(19) Europäisches Patentamt
European Patent Office
Office européen des brevets

(11) Veröffentlichungsnummer : **0 468 924 A2**

# (12) EUROPÄISCHE PATENTANMELDUNG

(21) Anmeldenummer : **91810577.6**

(22) Anmeldetag : **16.07.91**

(51) Int. Cl.[5] : **C07D 209/48,** A01N 43/00, C07D 471/04, C07D 487/04, C07D 207/408, C07D 211/82, C07D 513/04, C07D 265/26, // (C07D471/04, 235:00, 221:00), (C07D487/04, 249:00, 237:00), (C07D513/04, 285:00, 237:00)

(30) Priorität : **23.07.90 CH 2439/90**

(43) Veröffentlichungstag der Anmeldung :
**29.01.92 Patentblatt 92/05**

(84) Benannte Vertragsstaaten :
**AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Pissiotas, Georg, Dr.**
**Am Sonnenrain 71**
**W-7850 Lörrach (DE)**
Erfinder : **Moser, Hans, Dr.**
**Hauptstrasse 67B**
**CH-4312 Magden (CH)**
Erfinder : **Brunner, Hans-Georg, Dr.**
**Wannenstrasse 14**
**CH-4415 Lausen (CH)**
Erfinder : **Steiner, Eginhard, Dr.**
**Obere Hofackerstrasse 3**
**CH-4414 Füllinsdorf (CH)**

(54) **Neue Herbizide.**

(57) Cycloalkancarbonsäurederivate der Formel I

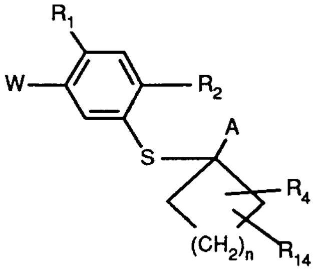

(I),

worin W für

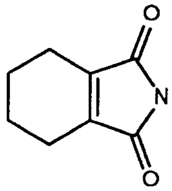 ($W_1$), 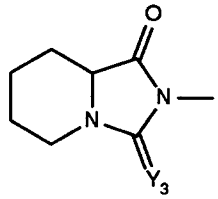 ($W_2$), 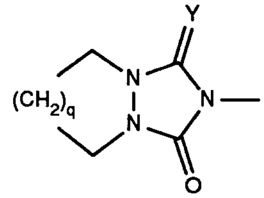 ($W_3$),

EP 0 468 924 A2

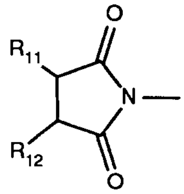 (W$_4$), 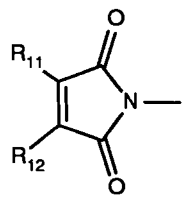 (W$_5$), 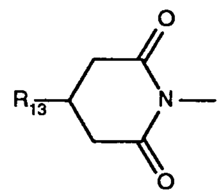 (W$_6$),

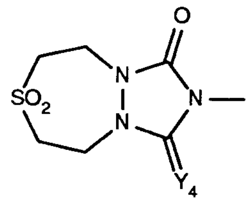 (W$_7$), 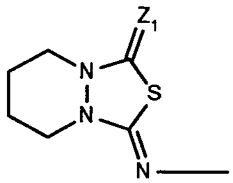 (W$_8$) 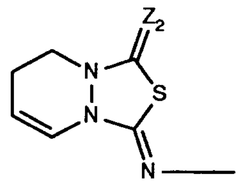 (W$_9$) oder

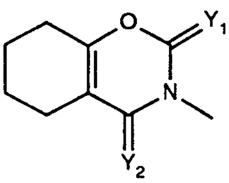 (W$_{10}$)

steht ; und
A CO-$R_3$ oder CN ;
$R_1$ Wasserstoff oder Fluor ;
$R_2$ Halogen oder Cyano ;
$R_3$ Chlor, X-$R_5$, Amino, $C_1$-$C_4$-Alkylamino, Di-$C_1$-$C_4$-alkylamino, $C_2$-$C_4$-Halogenalkylamino, Di-$C_2$-$C_4$-halogenalkylamino, $C_1$-$C_4$-Hydroxyalkylamino, Di-$C_2$-$C_4$-hydroxyalkylamino, $C_3$-$C_4$-Alkenylamino, Diallylamino, -N-Pyrrolidino, -N-Piperidino, -N-Morpholino, -N-Thiomorpholino, -N-Piperidazino, die Gruppe -O-N=C-($R_9$)$R_{10}$ oder die Gruppe -N-$R_5$($OR_5$) ;
$R_4$ und $R_{14}$ unabhängig voneinander Wasserstoff, Fluor, Chlor, Brom, $C_1$-$C_4$-Alkyl oder Trifluormethyl ;
$R_5$ Wasserstoff, $C_1$-$C_{10}$-Alkyl, $C_1$-$C_4$-Alkoxy-$C_1$-$C_4$-alkyl, Halogen-$C_1$-$C_8$-alkyl, $C_1$-$C_{10}$-Alkylthio-$C_1$-$C_4$-alkyl, Di-$C_1$-$C_4$-alkylamino-$C_1$-$C_4$-alkyl, Cyano-$C_1$-$C_8$-alkyl, $C_3$-$C_8$-Alkenyl, Halogen-$C_3$-$C_8$-alkenyl, $C_3$-$C_8$-Alkinyl, $C_3$-$C_7$-Cycloalkyl, $C_3$-$C_7$-Cycloalkyl-$C_1$-$C_4$-alkyl, Halogen-$C_3$-$C_7$-cycloalkyl oder Benzyl, das unsubstituiert oder am Phenylring gleich oder verschieden bis zu dreifach durch Halogen, $C_1$-$C_4$-Alkyl, Halogen-$C_1$-$C_4$-alkyl, Halogen-$C_1$-$C_4$-alkoxy oder $C_1$-$C_4$-alkoxy substituiert ist oder Alkali-, Erdalkali-Ionen und Ammoniumionen, die Gruppe -[$CHR_6$-$(CH_2)_m$]-$COOR_7$, oder die Gruppe [$CHR_6$-$(CH_2)_t$-$Si(R_6)_3$] ;
$R_6$ Wasserstoff oder $C_1$-$C_4$-Alkyl ;
$R_7$ Wasserstoff, $C_1$-$C_6$-Alkyl, $C_3$-$C_8$-Alkenyl, $C_3$-$C_8$-Alkinyl, $C_1$-$C_8$-Alkoxy-$C_2$-$C_8$-alkyl, $C_1$-$C_8$-Alkylthio-$C_2$-$C_8$-alkyl oder $C_3$-$C_7$-Cycloalkyl ;
$R_6$ $C_1$-$C_4$-Alkyl ;
$R_9$ $C_1$-$C_4$-Alkyl ;
$R_{10}$ $C_1$-$C_4$-Alkyl oder Phenyl ;
oder $R_9$ und $R_{10}$ zusammen mit dem Kohlenstoffatom, an welches sie gebunden sind, eien Cyclohexanring ;
$R_{11}$ $C_1$-$C_8$-Alkyl ;
$R_{12}$ Wasserstoff oder $C_1$-$C_8$-Alkyl ;
$R_{13}$ Wasserstoff, $C_1$-$C_6$-Alkyl, $C_3$-$C_7$-Cycloalkyl ; $C_1$-$C_4$-Alkoxy-$C_1$-$C_4$-Alkyl, $C_1$-$C_4$-Alkylthio-$C_1$-$C_4$-alkyl ; Halogen-$C_1$-$C_7$-alkyl, $C_3$-$C_7$-Alkenyl oder $C_3$-$C_7$-Alkinyl ;
X Sauerstoff oder Schwefel ;
Y Sauerstoff oder Schwefel ;
$Y_1$ Sauerstoff oder Schwefel ;
$Y_2$ Sauerstoff oder Schwefel ;
$Y_3$ Sauerstoff oder Schwefel ;
$Y_4$ Sauerstoff oder Schwefel ;
$Z_1$ Sauerstoff oder Schwefel ;
$Z_2$ Sauerstoff oder Schwefel ;
n 0, 1, 2, 3 oder 4 ;
m 0, 1, 2, 3 oder 4 ;
q 1 oder 2 ; und
t 0, 1, 2, 3 oder 4
bedeutet, unter Einschluss der Salze und Komplexe mit Säuren, Basen oder Komplexbildnern, sowie der möglichen Stereoisomeren, die in Form von Enantiomeren, Diastereomeren oder deren Gemische vorliegen, haben gute pre- und postemergent-selektive herbizide und wuchsregulierende Eigenschaften.

Die vorliegende Erfindung betrifft neue, herbizid wirksame und pflanzenwuchsregulierende Cycloalkancarbonsäurederivate, Verfahren zu ihrer Herstellung, die sie als Wirkstoffe enthaltende Mittel sowie deren Verwendung zum Bekämpfen von Unkräutern, vor allem selektiv in Nutzpflanzenkulturen oder zum Regulieren und Hemmen des Pflanzenwachstums.

S-Phenyl-thioglykolsäurederivate mit herbizider Wirkung sind aus den Europäischen Patentanmeldungen Nr. 0 238 711 und 0 304 920 sowie aus den US-Patenten USP 4,885,023, USP 4,684,397 und USP 4,801,408 bekannt. Die dort offenbarten Wirkstoffe können jedoch die Anforderungen bezüglich Wirkungsstärke und Selektivität nicht immer erfüllen. Es besteht somit ein Bedarf nach besser wirksamen und selektiveren Wirkstoffen.

Es wurden nun neue Cycloalkancarbonsäurederivate mit verbesserter herbizider und pflanzenwuchsregulierender Wirkung gefunden.

Die erfindungsgemässen Cycloalkancarbonsäurederivate entsprechen der Formel I

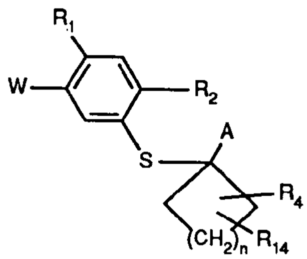

(I),

worin W für

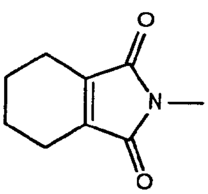 ($W_1$), 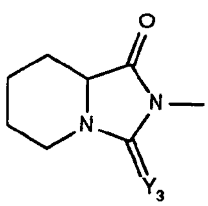 ($W_2$), 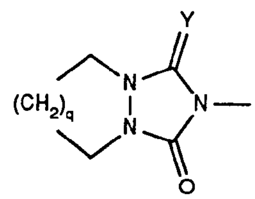 ($W_3$),

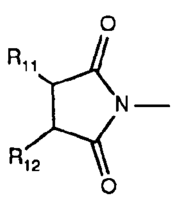 ($W_4$), 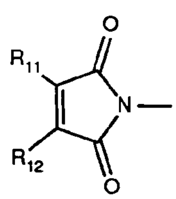 ($W_5$), 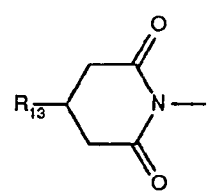 ($W_6$),

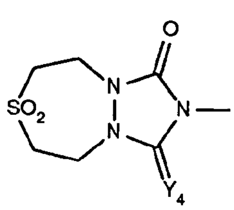 ($W_7$), 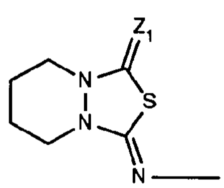 ($W_8$) 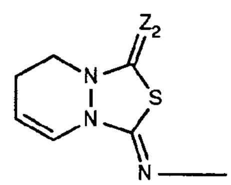 ($W_9$) oder

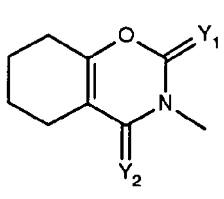 ($W_{10}$)

steht; und

A $CO-R_3$ oder CN;
$R_1$ Wasserstoff oder Fluor;
$R_2$ Halogen oder Cyano;
$R_3$ Chlor, $X-R_5$, Amino, $C_1$-$C_4$-Alkylamino, Di-$C_1$-$C_4$-alkylamino, $C_2$-$C_4$-Halogenalkyl-amino, Di-$C_2$-$C_4$-halogenalkylamino, $C_1$-$C_4$-Hydroxyalkylamino, Di-$C_1$-$C_4$-hydroxyalkylamino, $C_3$-$C_4$-Alkenylamino, Diallylamino, -N-Pyrrolidino, -N-Piperidino, -N-Morpholino, -N-Thiomorpholino, -N-Piperidazino, die Gruppe $-O-N=C-(R_9)R_{10}$ oder die Gruppe $-N-R_6(OR_6)$;
$R_4$ und $R_{14}$ unabhängig voneinander Wasserstoff, Fluor, Chlor, Brom, $C_1$-$C_4$-Alkyl oder Trifluormethyl;
$R_6$ Wasserstoff, $C_1$-$C_{10}$-Alkyl, $C_1$-$C_4$-Alkoxy-$C_1$-$C_4$-alkyl, Halogen-$C_1$-$C_8$-alkyl, $C_1$-$C_{10}$-Alkylthio-$C_1$-$C_4$-alkyl, Di-$C_1$-$C_4$-alkylamino-$C_1$-$C_4$-alkyl, Cyano-$C_1$-$C_8$-alkyl, $C_3$-$C_8$-Alkenyl, Halogen-$C_3$-$C_8$-alkenyl, $C_3$-$C_8$-Alkinyl, $C_3$-$C_7$-Cycloalkyl, $C_3$-$C_7$-Cycloalkyl-$C_1$-$C_4$-alkyl, Halogen-$C_3$-$C_7$-cycloalkyl oder Benzyl, das unsubstituiert oder am Phenylring gleich oder verschieden bis zu dreifach durch Halogen, $C_1$-$C_4$-Alkyl, Halogen-$C_1$-$C_4$-alkyl, Halogen-$C_1$-$C_4$-alkoxy oder $C_1$-$C_4$-alkoxy substituiert ist oder Alkali-, Erdalkali-Ionen und Ammoniumionen, die Gruppe $-[CHR_6-(CH_2)_m]-COOR_7$, oder die Gruppe $[CHR_6-(CH_2)_t-Si(R_8)_3]$;
$R_6$ Wasserstoff oder $C_1$-$C_4$-Alkyl;
$R_7$ Wasserstoff, $C_1$-$C_6$-Alkyl, $C_3$-$C_8$-Alkenyl, $C_3$-$C_8$-Alkinyl, $C_1$-$C_8$-Alkoxy-$C_2$-$C_8$-alkyl, $C_1$-$C_8$-Alkylthio-$C_2$-$C_8$-alkyl oder $C_3$-$C_7$-Cycloalkyl;
$R_6$ $C_1$-$C_4$-Alkyl;
$R_9$ $C_1$-$C_4$-Alkyl;
$R_{10}$ $C_1$-$C_4$-Alkyl oder Phenyl;
oder $R_9$ und $R_{10}$ zusammen mit dem Kohlenstoffatom, an welches sie gebunden sind, einen Cyclohexanring;
$R_{11}$ $C_1$-$C_8$-Alkyl;
$R_{12}$ Wasserstoff oder $C_1$-$C_8$-Alkyl;
$R_{13}$ Wasserstoff, $C_1$-$C_6$-Alkyl, $C_3$-$C_7$-Cycloalkyl: $C_1$-$C_4$-Alkoxy-$C_1$-$C_4$-Alkyl, $C_1$-$C_4$-Alkylthio-$C_1$-$C_4$-alkyl; Halogen-$C_1$-$C_7$-alkyl, $C_3$-$C_7$-Alkenyl oder $C_3$-$C_7$-Alkinyl;
X Sauerstoff oder Schwefel;
Y Sauerstoff oder Schwefel;
$Y_1$ Sauerstoff oder Schwefel;
$Y_2$ Sauerstoff oder Schwefel;
$Y_3$ Sauerstoff oder Schwefel;
$Y_4$ Sauerstoff oder Schwefel;
$Z_1$ Sauerstoff oder Schwefel;
$Z_2$ Sauerstoff oder Schwefel;
n 0, 1, 2, 3 oder 4;
m 0, 1, 2, 3 oder 4;
q 1 oder 2; und
t 0, 1, 2, 3 oder 4
bedeutet, unter Einschluss der Salze und Komplexe mit Säuren, Basen oder Komplexbildnern, sowie der möglichen Stereoisomeren, die in Form von Enantiomeren, Diastereomeren oder deren Gemische vorliegen.

In den in dieser Beschreibung verwendeten Definitionen umfassen die angegebenen generischen Begriffe, sowie die durch Kombination einzelner Unterbegriffe erhältlichen Einzelbedeutungen der Substituenten, beispielsweise die folgenden Einzelsubstituenten, wobei diese Aufzählung keine Einschränkung der Erfindung darstellt.

In den obigen Definitionen ist unter Halogen, Fluor, Chlor, Brom und Jod vorzugsweise Fluor, Chlor und Brom zu verstehen, insbesondere bei $R_2$ Chlor und Brom.

Alkyl ist beispielsweise Methyl, Ethyl, Isopropyl, n-Propyl, n-Butyl, iso-Butyl, sek-Butyl, tert-Butyl sowie die verschiedenen isomeren Pentyl-, Hexyl-, Heptyl-, Octyl-, Nonyl- und Decylradikale.

Halogenalkyl sind beispielsweise Fluormethyl, Difluormethyl, Trifluormethyl, Chlormethyl, Dichlormethyl, Trichlormethyl, 2,2,2-Trifluorethyl, 2-Fluorethyl, 2-Chlorethyl und 2,2,2-Trichlorethyl; vorzugsweise Trichlormethyl, Difluorchlormethyl, Trifluormethyl und Dichlorfluormethyl.

Alkoxy ist beispielsweise Methoxy, Ethoxy, Propyloxy, i-Propyloxy, n-Butyloxy, i-Butyloxy, s-Butyloxy und t-Butyloxy; vorzugsweise Methoxy und Ethoxy.

Halogenalkoxy ist z.B. Fluormethoxy, Difluormethoxy, Trifluormethoxy, 2,2,2-Trifluorethoxy, 1,1,2,2-Tetrafluorethoxy, 2-Fluorethoxy, 2-Chlorethoxy und 2,2,2-Trichlorethoxy; vorzugsweise Difluormethoxy, 2-Chlorethoxy und Trifluormethoxy.

Alkylthio ist beispielsweise Methylthio, Ethylthio, Propylthio, Isopropylthio, n-Butylthio, i-Butylthio, s-Butylthio, t-Butylthio oder die isomeren Pentylthio, vorzugsweise Methylthio und Ethylthio.

Unter Alkenyl ist geradkettiges oder verzweigtes Alkenyl zu verstehen, wie z.B. Vinyl, Allyl, Methallyl, 1-Methylvinyl, But-2-en-1-yl, 3-Pentenyl, 2-Hexenyl oder 3-Heptenyl. Bevorzugt sind Alkenylreste mit einer Kettenlänge von 2 bis 3 Kohlenstoffatomen.

Die in den Definitionen der Substituenten vorkommenden Alkinylreste können geradkettig oder verzweigt sein wie beispielsweise Ethinyl, Propargyl, 3-Butinyl, 1-Methylpropargyl, 2-Pentinyl oder 2-Hexinyl. Bevorzugt sind Ethinyl und Propargyl. Im Rahmen der vorliegenden Erfindung sind die an Sauerstoff oder Stickstoff gebundenen Alkenyl- und Alkinylgruppen in der Regel über ein gesättigtes Kohlenstoffatom gebunden.

Cycloalkyl steht beispielsweise für Cyclopropyl, Dimethylcyclopropyl, Cyclobutyl, Cyclopentyl, Methylcyclopentyl, Cyclohexyl oder Cycloheptyl, vorzugsweise aber für Cyclopropyl, Cyclopentyl oder Cyclohexyl.

Alkoxycarbonyl ist z.B.: Methoxycarbonyl, Ethoxycarbonyl, n-Propyloxycarbonyl, i-Propyloxycarbonyl und n-Butyloxycarbonyl, vorzugsweise Methoxycarbonyl und Ethoxycarbonyl.

Alkoxyalkyl ist z.B.: Methoxymethyl, Ethoxymethyl, Propyloxymethyl, Methoxyethyl, Ethoxyethyl, Propyloxyethyl, Methoxypropyl, Ethoxypropyl oder Propyloxypropyl.

Alkylthioalkyl ist z.B.: Methylthiomethyl, Ethylthiomethyl, Methylthioethyl, Ethylthioethyl, oder Isopropylthioethyl.

Alkylaminoalkyl ist z.B.: Methylaminoethyl, Dimethylaminoethyl, Ethylaminoethyl oder Diethylaminoethyl.

Cyanoalkyl ist z.B.: Cyanomethyl, Cyanoethyl oder Cyanopropyl.

Wenn $R_4$ und $R_{14}$ gleichzeitig verschieden von Wasserstoff sind und n eins bedeutet, sind $R_4$ und $R_{14}$ vorzugsweise am gleichen Kohlenstoffatom gebunden.

Halogencycloalkyl ist z.B.: 2,2-Dichlorcyclopropyl oder Pentachlorcyclohexyl.

Alkylsulfonyl ist z.B.: Methylsulfonyl, Ethylsulfonyl, Propylsulfonyl oder Butylsulfonyl. Bevorzugt sind Methyl- und Ethylsulfonyl.

Cycloalkoxycarbonyl ist z.B.: Cyclopentyloxycarbonyl oder Cyclohexyloxycarbonyl.

Phenyl, auch als Teil eines Substituenten wie Phenoxy, Phenylthio, Phenoxycarbonyl, Phenylaminocarbonyl, Benzyl oder Benzoyl, kann im allgemeinen unsubstituiert oder durch weitere Substituenten substituiert vorliegen. Die Substituenten können dann in ortho-, meta- und/oder para-Stellung stehen. Bevorzugte Substituentenstellungen sind die ortho- und para-Position zur Ringverknüpfungsstelle. Bevorzugte Substituenten sind Halogenatome.

In den weiteren Substituenten, welche aus mehreren Grundelementen zusammengesetzt sind, haben die Teilelemente die oben an Beispielen erläuterten Bedeutungen. Diese Aufzählungen bedeuten auch in diesen Fällen keine Einschränkung der Erfindung: sie haben illustrierenden Charakter.

Von den Verbindungen der Formel I sind diejenigen bevorzugt, in denen W für $W_1$, $W_2$, $W_3$, $W_6$, $W_8$ oder $W_9$ steht, wobei $R_{14}$ vorzugsweise Wasserstoff bedeutet.

Ferner sind aus dem Umfang der Formel I diejenigen Verbindungen bevorzugt, in denen $R_1$ für Fluor und $R_2$ für Chlor steht.

Bei einer besonders herausragenden Gruppe von Verbindungen der Formel I steht A für $CO-R_3$ und $R_3$ vorzugsweise für $X-R_5$. Aus dieser Gruppe sind diejenigen Verbindungen bevorzugt, in denen $R_5$ für $C_1$-$C_{10}$-Alkyl, insbesondere aber $C_1$-$C_4$-Alkyl steht. Ganz besonders gute biologische Wirkung zeigen diejenigen Verbindungen dieser Gruppe, bei denen W für $W_8$ oder $W_9$ steht. In den ganz besonders bevorzugten Verbindungen dieser Gruppe steht $R_4$ für Wasserstoff und X für Sauerstoff, wobei n vorzugsweise 1 bedeutet.

Als herausragende Einzelverbindungen aus dem Umfang der Formel I sind zu nennen:

1-[2-Chlor-4-fluor-5-(1,2,3,4,5,6-hexahydro-1,3-dioxo-isoindolyl)-phenylthio]-cyclopropancarbonsäuremethylester,

9-[4-Chlor-2-fluor-5-(1-methoxycarbonyl-cyclobutylthio)-phenylimino]-8-thia-1,6-diazabicyclo[4.3.0]-nonan-7-on;

9-[4-chlor-2-fluor-5-(1-methoxycarbonyl-cyclopropylthio)-phenylimino]-8-thia-1,6-diazabicyclo[4.3.0]-nonan-7-on; sowie

1-[4-Chlor-2-fluor-5-(1-methoxycarbonyl-cyclobutyl)-phenylthio]-4-trifluormethyl-piperidin-2,6-dion.

Die Verbindungen der Formel I, in welcher W für $W_1$, $W_4$, $W_5$ oder $W_6$ steht, können hergestellt werden, indem man die jeweiligen Anhydride der Reste $W_1$(Tetrahydrophtalsäureanhydrid), $W_4$(Maleinsäureanhydrid), $W_5$(Bernsteinsäureanhydrid) und $W_6$(Glutarsäureanhydrid) mit Aminen der Formel III

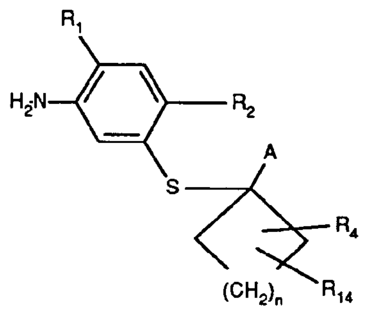

(III),

worin A, $R_1$, $R_2$, $R_4$, $R_{14}$ und n wie unter Formel I definiert sind, umsetzt. Beispielsweise lassen sich Verbindungen der Formel I, in welcher W für $W_6$ steht, herstellen, in dem man ein Glutarsäureanhydrid der Formel II, worin $R_{13}$ wie unter Formel I definiert ist, mit Aminen der Formel III, worin A, $R_1$, $R_2$, $R_4$ und n wie unter Formel I definiert sind, zu einem Glutarsäuremonoanilid der Formel IV umsetzt, und anschliessend das entstandene Monoamid der Formel IV mit einem Kondensationsmittel zu einem Cycloalkancarbonsäurederivat der Formel I cyclisiert.

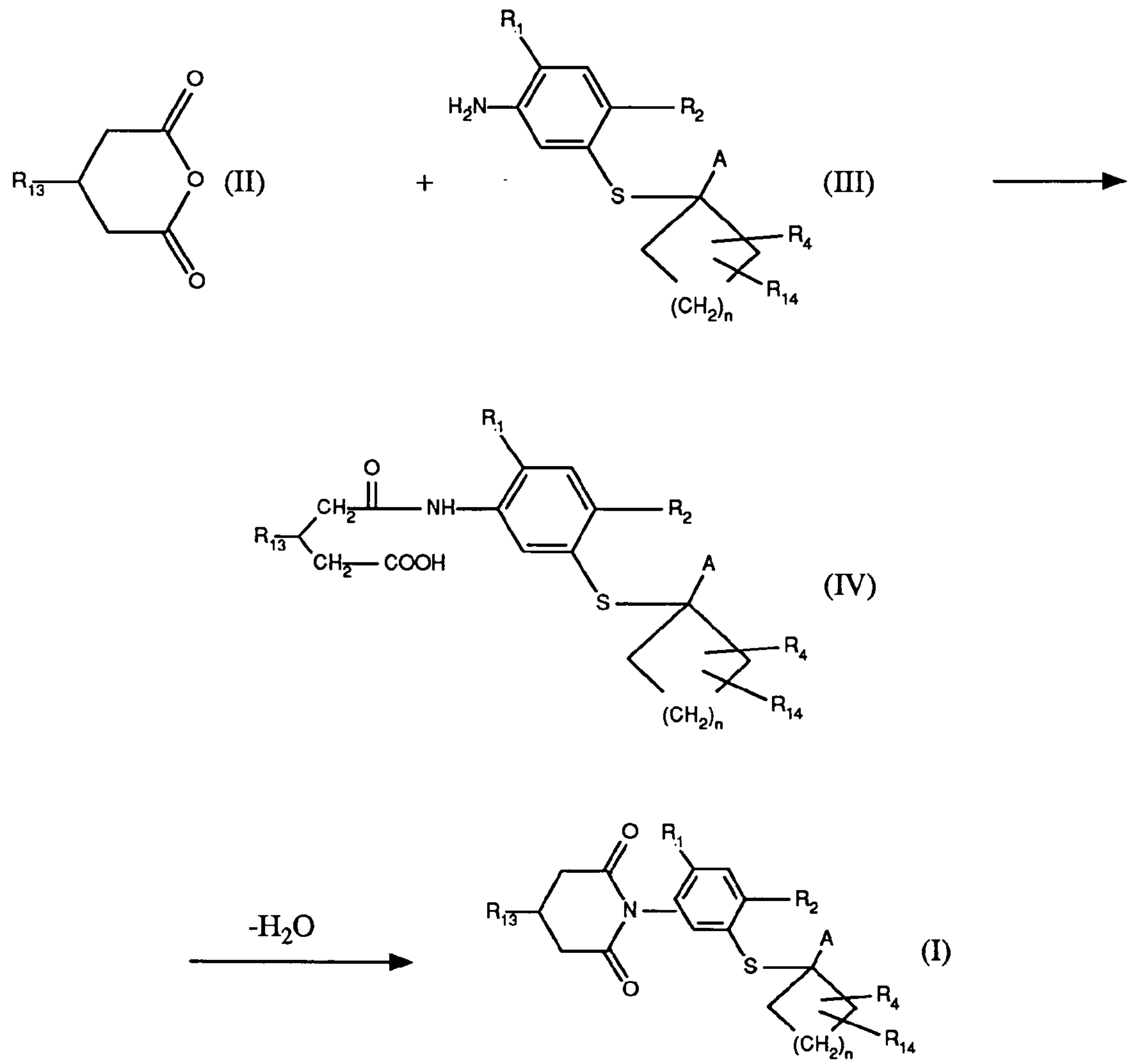


(II) + (III) (IV) (I)

Mit Vorteil führt man die obige Reaktion in einem inerten organischen Lösungsmittel aus. Die Reaktionstemperatur liegt im allgemeinen zwischen der Raumtemperatur und der Siedetemperatur des Reaktionsgemisches, vorzugsweise wird die Reaktionsmischung zum Rückfluß erhitzt. Die Kondensationsreaktion kann durch Zusatz von Kondensationskatalysatoren und Entfernung des entstehenden Wassers beschleunigt werden. Eine gleiche Wirkung erzielt man durch Zusatz von wasserentziehenden Mitteln, wie beispielsweise

Schwefelsäure. Als Lösungsmittel eignen sich insbesondere höhersiedende Kohlenwasserstoffe, niedere Alkancarbonsäuren sowie deren Ester und Amide, höhersiedende Ketone und Ether. Beispiele für geeignete Lösungsmittel sind: Benzol, Toluol, Xylol, Dimethylformamid, Dimethylacetamid, Essigsäure, Ethylacetat, Diisopropylether, Ethylenglykoldimethylether, Tetrahydrofuran oder 2-Butanon.

Geeignete Katalysatoren sind beispielsweise: p-Toluolsulfonsäure, Benzoesäure, 4-Dimethylaminopyridin, Schwefelsäure, Chlorwasserstoff oder Naphtalinsulfonsäure. In analoger Weise können die Verbindungen der Formel I, in denen W für $W_1$, $W_4$, oder $W_5$ steht, hergestellt werden.

Die Verbindungen der Formel II, bzw. die entsprechenden Anhydride der Reste $W_1$, $W_4$, und $W_5$ sowie die Verbindungen der Formel IV sind bekannt oder können analog zu bekannten Verfahren hergestellt werden.

Zur Synthese von Anhydriden der Formel II sowie den Anhydriden der Reste $W_1$, $W_4$, und $W_5$ sind in der Literatur verschiedene Wege beschrieben.

Als geeignet für die Herstellung der Verbindungen der Formel II erweist sich beispielsweise die in Schema 1 dargestellte Reaktionssequenz, die von einfachsten, allgemein verfügbaren Synthesebausteinen (Aldehyd und Cyanessigsäureester) ausgeht:

Schema 1

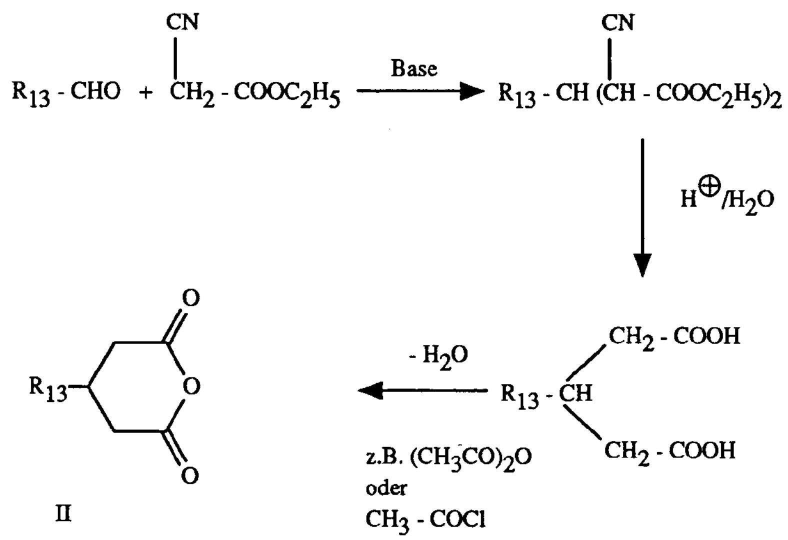


II

Das Verfahren gemäss Schema 1 ist unter anderem in folgenden Literaturstellen näher beschrieben: J. Chem. Soc. 117 (1920) 1465; J. Chem. Soc. 123 (1923), 3131; J. Chem. Soc. 1952, 4785; Rec. Trav. Chim. Pays-Bas 84 (1965), 1183 und J. Ind. Chem. Soc. 13 (1936), 322.

Eine andere Verfahrensvariante zur Herstellung der Glutarsäureanhydride der Formel II basiert auf einer Malonestersynthese und ist im Schema 2 zusammengefasst:

Schema 2

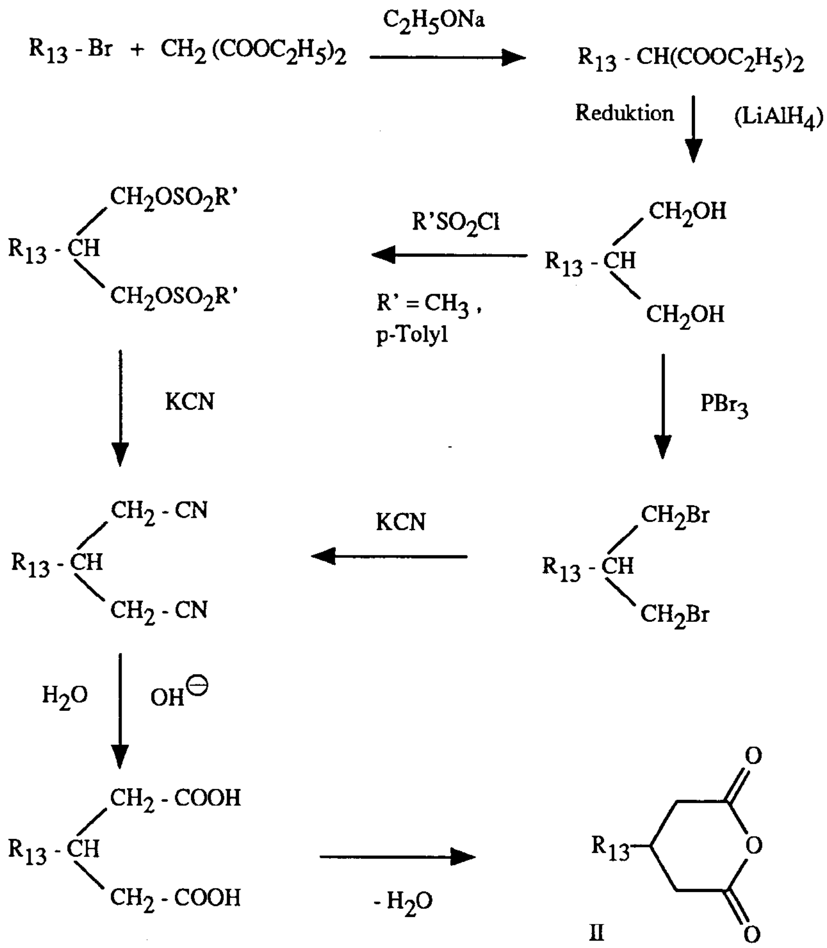


II

Auf analoge Weise können die Anhydride der Reste $W_1$, $W_4$, und $W_5$ hergestellt werden. Das in Schema 2 skizzierte Verfahren ist unter anderem aus folgenden Literaturstellen bekannt:
Chem. Soc. Perkin Trans. I, 1978, 1636; J. Am. Chem. Soc. 95 (1973), 7437; Org. Prep. Proc. Int. 7, (1975), 283.

Die Aniline der Formel III sind neu und wurden speziell für die Synthese der Verbindungen der Formel I entwickelt. Sie bilden daher ebenfalls einen Gegenstand der vorliegenden Erfindung. Sie können beispielsweise nach an sich bekannten Methoden durch

a) Reduktion der entsprechenden Nitroverbindungen durch katalytische Verfahren (z.B. $H_2/Pd/C$, oder $H_2/Pt$) oder

b) Umsetzung eines Thiophenols der Formel V mit einer Verbindung der Formel VI in Gegenwart einer Base nach folgendem Schema hergestellt werden:

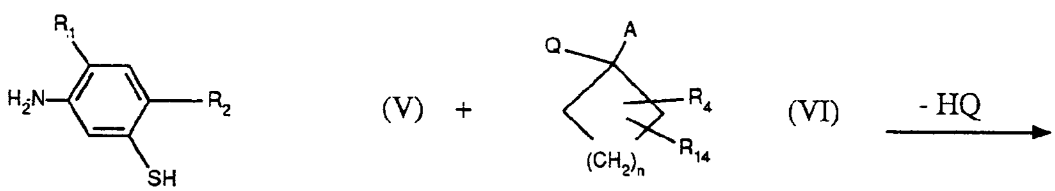


(V) + (VI)

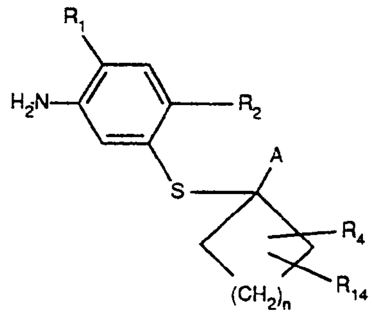

(III)

Im obigen Reaktionsschema steht Q für eine unter den Reaktionsbedingungen austauschbare Gruppe, wie z.B. Halogen, vorzugsweise Chlor, Brom oder Jod, oder für einen gewünschtenfalls im Phenylkern alkylierten oder halogenierten Phenylsulfonylrest oder für ein Radikal der Formel

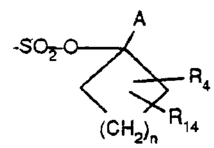

wobei die Substituenten A, $R_1$, $R_2$, $R_4$ und $R_{14}$ die unter Formel I angegebene Bedeutung haben und n für 1, 2, 3 oder 4 steht.

Die Thiophenole der Formel V sind aus EP-A-0 259 265 bekannt. Die Verbindungen der Formel VI sind bekannt oder lassen sich analog bekannter Methoden herstellen (siehe z.B. J. Org. Chem. **54**, 6096-6100 (1989)).

Die Verbindungen der Formel III können auch hergestellt werden, indem man ein Thiophenol der Formel V

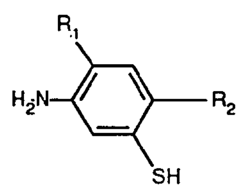

(V)

worin $R_1$ und $R_2$ die unter Formel I angegebene Bedeutung haben, in Gegenwart einer Base mit einer Verbindung der Formel XXI

$$X_1\text{-}C(R_{14}R_4)\text{-}(CH_2)_p\text{-}CH(A)\text{-}X_2 \qquad (XXI)$$

worin $X_1$ und $X_2$ unabhängig voneinander für Chlor oder Brom stehen, A und $R_4$ die unter Formel I angegebene Bedeutung haben und p 1, 2, 3, 4 oder 5 bedeutet, entweder

a) wenn A für CN steht:

zu einer Verbindung der Formel XXIII

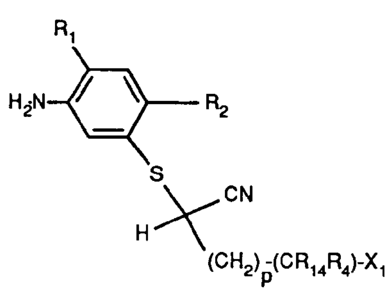

(XXIII)

umsetzt, welche anschließend in Gegenwart einer Base zur Verbindung der Formel III, worin A für CN steht, cyclisiert wird, oder

b) wenn A für COOH steht:

zu einer Verbindung der Formel XXIIIa

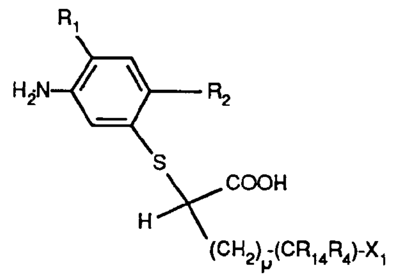

(XXIIIa)

umsetzt, welche nach an sich bekannten Methoden in Gegenwart einer Base in die Verbindungen der Formel XXIIIb

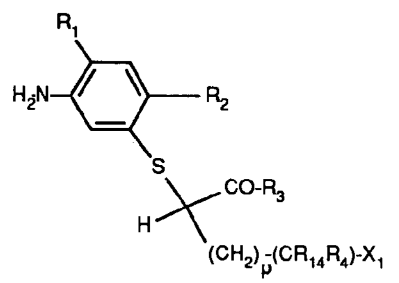

(XXIIIb)

überführt wird, welche anschließend in Gegenwart einer Base zur Verbindung der Formel III, worin A für $CO-R_3$ steht, cyclisiert wird. Die Position der Substituenten $R_4$ und $R_{14}$ in der Alkylenkette der Verbindung der Formel XXI kann variieren und ist abhängig von der gewünschten Substitutionsstellung von $R_4$ und $R_{14}$ am Cycloalkanring der Verbindungen der Formel III.

Die Verbindungen der Formel III, worin A für CN steht, können auch hergestellt werden, indem man eine Verbindung der Formel Va

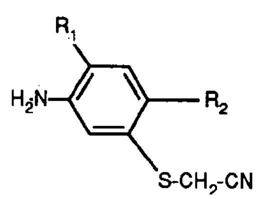

(Va)

worin $R_1$ und $R_2$ die unter Formel V angegebene Bedeutung haben, in Gegenwart einer Base mit einer Verbindung der Formel XXIa

$$X_1-C(R_{14}R_4)-(CH_2)_p-CH_2-X_2 \qquad (XXIa)$$

worin $X_1$, $X_2$, p, $R_{14}$ und $R_4$ die unter Formel XXI angegebene Bedeutung haben, cyclisiert. Bei der Verbindung der Formel XXIa ist die Position der Substituenten $R_4$ und $R_{14}$ in der Alkylenkette abhängig von der gewünschten Substitutionsstellung von $R_4$ und $R_{14}$ am Cycloalkanring der Verbindungen der Formel III. Derartige Reaktionen werden z.B. in Tetrahedron Letters **23**, 2391-2394 (1972) beschrieben.

Eine weitere Variante, die Verbindungen der Formel III, worin A für CN steht, herzustellen, besteht darin, daß man Verbindungen der Formel III, worin A für COCl steht, mit Ammoniak zum entsprechenden Acetamid umsetzt, welches durch Wasserabspaltung (z.B. Einwirkung von Phosphorpentoxid) in das gewünschte Nitril überführt werden kann. Derartige Reaktionen sind bekannt und dem Fachmann geläufig.

In der Formel XXI steht $X_1$ und $X_2$ vorzugsweise für Chlor. Geeignete Basen für diese Umsetzungen sind Oxide, Hydride, Hydroxide, Carbonate, Carbonsäuresalze oder Alkoholate eines Alkali- oder Erdalkalimetalls, Trialkylamine oder pyridinbasen. Als besonders gut geeignete Basen haben sich Natriummethylat, Natriumethylat, Natriumhydroxid, Kaliumhydroxid, Natriumcarbonat oder Kaliumcarbonat erwiesen. Für diese Reaktionen geeignete Lösungsmittel sind beispielsweise Verbindungen aus der Gruppe der offenkettigen oder cyclischen Ether, wie Dioxan, Tetrahydrofuran, Diethylether, Diisopropylether, Dimethoxymethan oder 1,2-Dimethoxyethan, aus der Gruppe der Alkohole wie Methanol, Ethanol oder Isopropanol oder aus der Gruppe der Ketone, wie Aceton, Methyl-ethylketon oder Methyl-isobutylketon. Die Umsetzungen erfolgen im allgemeinen bei Temperaturen von Raumtemperatur bis zur Siedetemperatur des Lösungsmittels.

Die Verbindungen der Formel I, worin W für $W_2$ steht und $Y_3$ Sauerstoff bedeutet, werden hergestellt, indem man ein Isocyanat der Formel VIIb

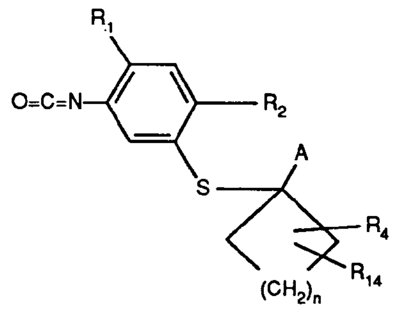

(VIIb)

worin $R_1$, $R_2$, $R_4$, $R_{14}$, A und n die unter Formel I angegebene Bedeutung haben, in einem organischem Lösungsmittel in Gegenwart einer Base bei einer Temperatur von 0 bis +150°C mit einem 2-Carboxylpiperidin der Formel XXIV

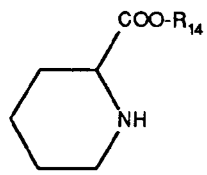

(XXIV)

worin $R_{14}$ für Wasserstoff, $C_1$-$C_6$-Alkyl oder Benzyl steht, zur Verbindung der Formel XXV

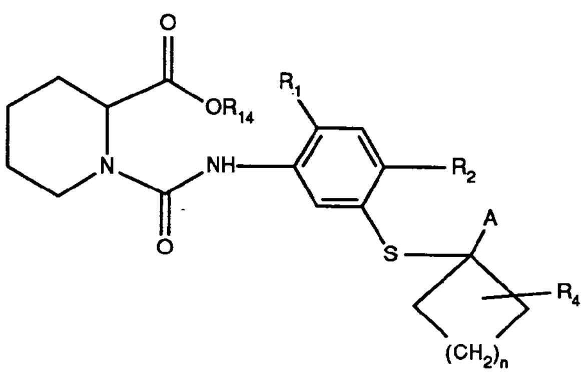

(XXV)

kondensiert, welche man anschließend unter sauren Bedingungen bei einer Temperatur von +50 bis +150°C zur Verbindung der Formel I cyclisiert. Derartige Reaktionen sind beispielsweise in EP-A-0 211 805 beschrieben.

Die Verbindungen der Formel I, worin W für $W_2$ steht und $Y_3$ Schwefel bedeutet, werden hergestellt, indem man ein Isothiocyanatcyanat der Formel VIIa

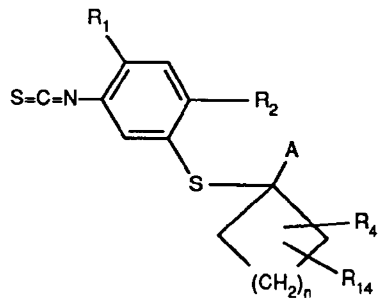

(VIIa)

worin $R_1$, $R_2$, $R_4$, $R_{14}$, A und n die unter Formel I angegebene Bedeutung haben, in einem organischem Lösungsmittel in Gegenwart einer Base bei einer Temperatur von 0 bis +150°C mit einem 2-Carboxylpiperidin der Formel XXIV

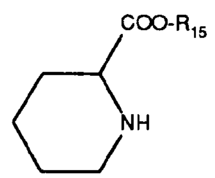

(XXIV)

worin $R_{15}$ für Wasserstoff, $C_1$-$C_6$-Alkyl oder Benzyl steht, zur Verbindung der Formel XXVa

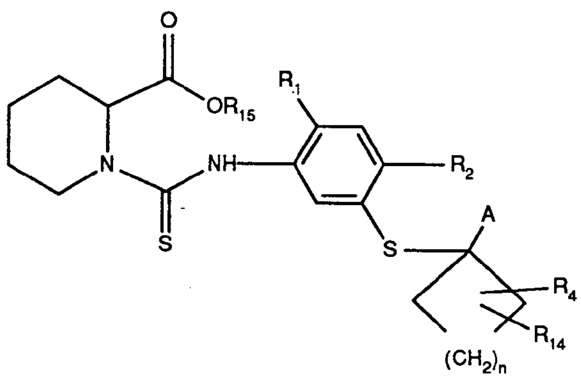

(XXVa)

kondensiert, welche man anschließend unter sauren Bedingungen bei einer Temperatur von +50 bis +150°C zur Verbindung der Formel I cyclisiert. Derartige Reaktionen sind ebenfalls in EP-A-0 211 805 beschrieben.

Die Verbindungen der Formel I, worin W für $W_3$ steht und Y Schwefel bedeutet, können hergestellt werden, indem man ein Isocyanat der Formel VIIb

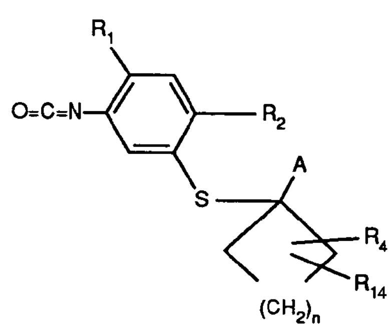

(VIIb)

worin $R_1$, $R_2$, $R_4$, $R_{14}$, A und n die unter Formel I angegebene Bedeutung haben, in einem organischem Lösungsmittel in Gegenwart einer Base bei einer Temperatur von 0 bis +150°C mit einer Verbindung der Formel XXVI

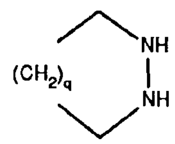

(XXVI)

worin q die unter Formel I angegebene Bedeutung hat, kondensiert, und die entstandene Verbindung der Formel XXVII

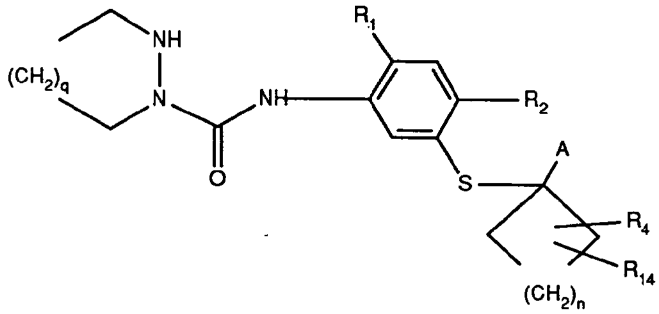

(XXVII)

anschließend in Gegenwart einer anorganischen Base bei einer Temperatur von 0 bis +150°C mit Thiophosgen umsetzt. Derartige Reaktionen sind beispielsweise in EP-A-0 211 805 beschrieben.

Die Verbindungen der Formel I, worin W für $W_3$ steht und Y Sauerstoff bedeutet, können hergestellt werden, indem man ein Isocyanat der Formel VIIb

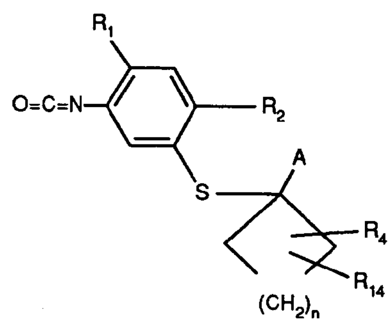

(VIIb)

worin $R_1$, $R_2$, $R_4$, $R_{14}$, A und n die unter Formel I angegebene Bedeutung haben, in einem organischem Lösungsmittel in Gegenwart einer Base bei einer Temperatur von 0 bis +150°C mit einer Verbindung der Formel XXVI

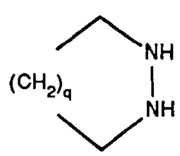

(XXVI)

worin q die unter Formel I angegebene Bedeutung hat, kondensiert, die entstandene Verbindung der Formel XXVII

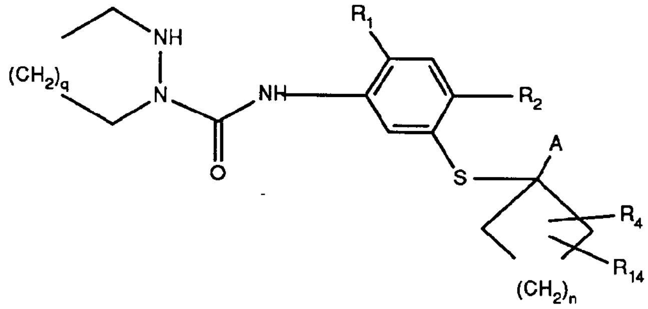

(XXVII)

anschließend in Gegenwart einer Base bei einer Temperatur von 0 bis +150°C mit einem Chlorkohlensäureester der Formel XXVIII

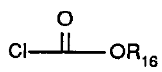

XXVIII

worin $R_{16}$ für $C_1$-$C_6$-Alkyl oder Benzyl steht, zu einer Verbindung der Formel XXIX

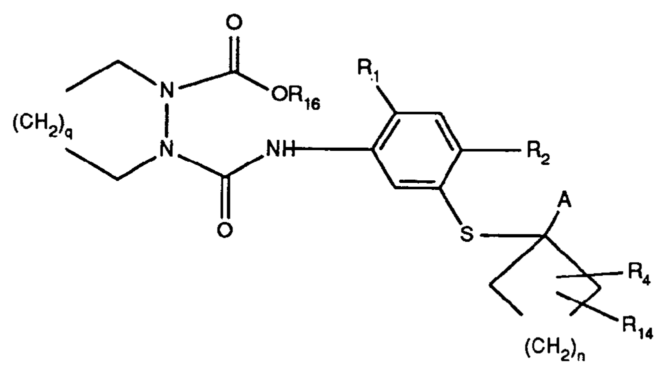

(XXIX)

umsetzt, welche man in einem inerten Lösungsmittel in Gegenwart einer Base bei einer Temperatur von +50 bis + 180°C zur Verbindung der Formel I cyclisiert. Derartige Reaktionen sind ebenfalls in EP-A-0 211 805 beschrieben.

Die Verbindungen der Formel I, worin W für $W_3$ steht und Y Schwefel bedeutet, können auch aus den Verbindungen der Formel I, worin W für $W_3$ steht und Y Sauerstoff bedeutet, hergestellt werden, indem man diese mit einem zur Einführung der Thioxogruppe befähigten Reagenz erhitzt. Solche Reagenzien sind beispielsweise Phosphorpentafluorid oder das Lawesson-Reagenz (2,4-Bis-(4-methoxyphenyl)-1,3-dithia-2,4-diphosphetan-2,4-disulfid). Derartige Umsetzungen sind bekannt und ihre Durchführung dem Fachmann geläufig.

Die Verbindungen der Formel I, in welcher W für $W_7$ steht und $Y_4$ Sauerstoff bedeutet, werden hergestellt, indem man ein Isocyanat der Formel VIIb

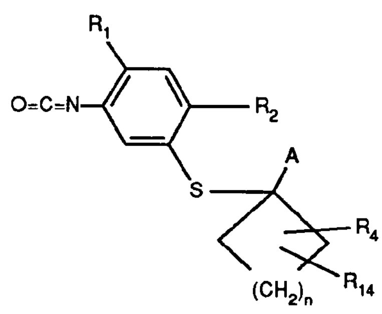

(VIIb)

worin die Substituenten A, $R_1$, $R_2$, $R_4$, $R_{14}$ sowie n die unter Formel I angegebene Bedeutung haben, mit einer Verbindung der Formel XXX

$H_2N\text{-}NH\text{-}COOR_{16}$ (XXX)

worin $R_{16}$ die oben angegebene Bedeutung hat, zur Verbindung der Formel XXXI

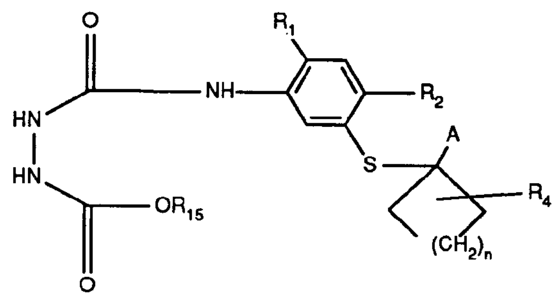

(XXXI)

umsetzt, welche man in Gegenwart einer Base bei einer Temperatur von +50 bis +180°C zur Verbindung der Formel XXXII

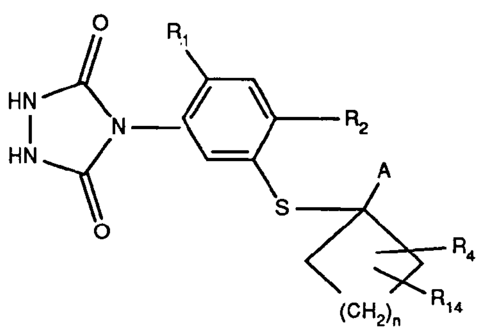

(XXXII)

cyclisiert, welche man bei einer Temperatur von 0 bis +150°C in Gegenwart einer katalytischen Menge an Base mit Divinylsulfon $(CH_2{=}CH)_2SO_2$ umsetzt. Derartige Reaktionen sind beispielsweise in EP-A 0 210 137 beschrieben.

Die Verbindungen der Formel I, in welcher W für $W_7$ steht und $Y_4$ Schwefel bedeutet, werden hergestellt, indem man ein Isothiocyanat der Formel VIIa

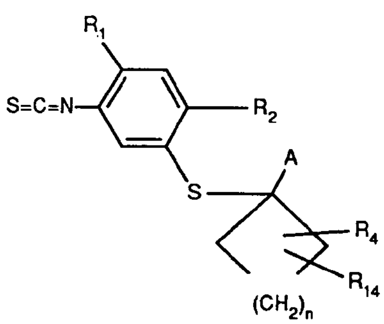

(VIIa)

worin die Substituenten A, $R_1$, $R_2$, $R_4$, $R_{14}$ sowie n die unter Formel I angegebene Bedeutung haben, mit einer Verbindung der Formel XXX

$H_2N\text{-}NH\text{-}COOR_{16}$ (XXX)

worin $R_{16}$ die oben angegebene Bedeutung hat, zur Verbindung der Formel XXXIa

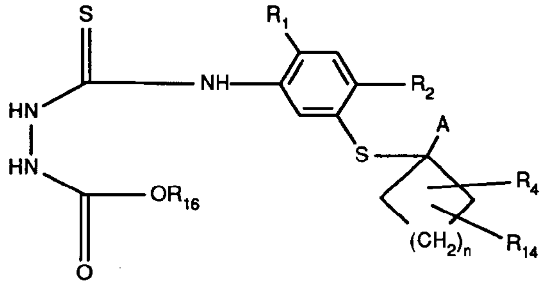

(XXXIa)

umsetzt, welche man in Gegenwart einer Base bei einer Temperatur von +50 bis + 180°C zur Verbindung der Formel XXXIIa

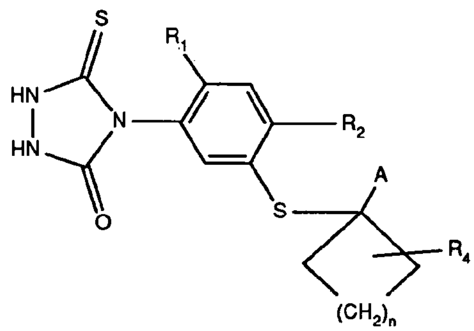

(XXXIIa)

cyclisiert, welche man bei einer Temperatur von 0 bis +150°C in Gegenwart einer katalytischen Menge an Base mit Divinylsulfon $(CH_2=CH)_2SO_2$ umsetzt. Derartige Reaktionen sind ebenfalls in EP-A 0 210 137 beschrieben.

Die Verbindungen der Formel I, in welcher W für $W_8$ steht, werden hergestellt, indem man ein Isothiocyanat der Formel VIIa

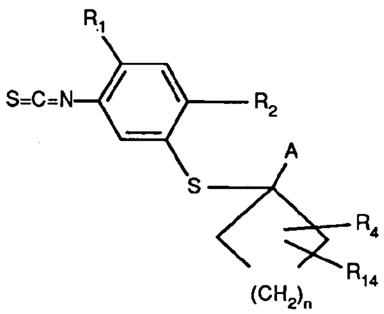

(VIIa),

worin die Substituenten A, $R_1$, $R_2$, $R_4$, $R_{14}$ sowie n die unter Formel I angegebene Bedeutung haben, mit einem Hexahydropyridazin der Formel VIII

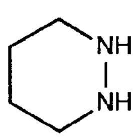

(VIII)

in die Verbindung der Formel IX

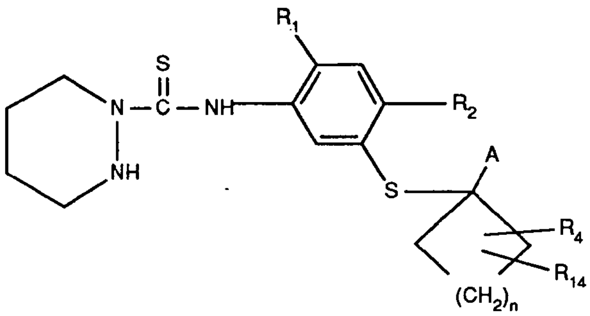

(IX)

überführt und diese anschliessend mit einer Verbindung der Formel X

$CZ_1Cl_2$ (X),

worin $Z_1$ für Sauerstoff oder Schwefel steht, in Gegenwart einer Base umsetzt.

Die Verbindungen der Formel I, in welcher W für $W_9$ steht, können hergestellt werden, indem man ein Isothiocyanat der Formel VIIa

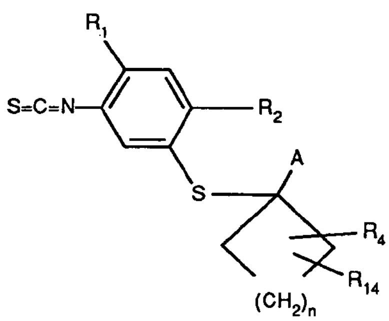

(VIIa),

worin die Substituenten A, $R_1$, $R_2$, $R_4$, $R_{14}$ sowie n die unter Formel I angegebene Bedeutung haben, mit einem 1,4,5,6-Tetrahydropyridazin der Formel XI

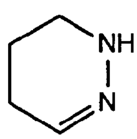

(XI)

in die Verbindung der Formel XII

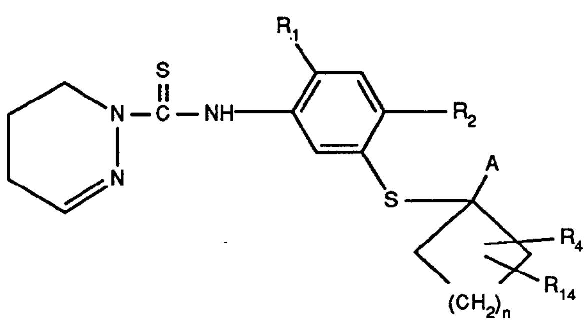

(XII)

überführt und diese anschliessend mit einer Verbindung der Formel Xa

$CZ_2Cl_2$ (Xa),

worin $Z_2$ für Sauerstoff oder Schwefel steht, in Gegenwart einer Base umsetzt.

Die Verbindungen der Formel, in welcher W für $W_9$ steht, können auch hergestellt werden, indem man ein Tetrahydropyridazin-3-on der Formel XIII

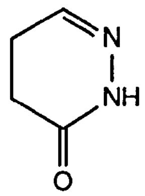

(XIII)

zum Hexahydropyridazin-3-on der Formel XIV

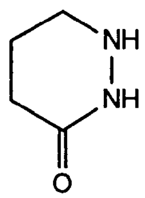

(XIV)

hydriert, dieses mit einem Isothiocyanat der Formel VIIa

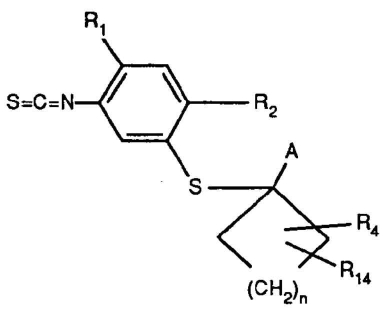

(VIIa)

worin die Substituenten A, $R_1$, $R_2$, $R_4$, $R_{14}$ sowie n die unter Formel I angegebene Bedeutung haben, zur Verbindung der Formel XVI

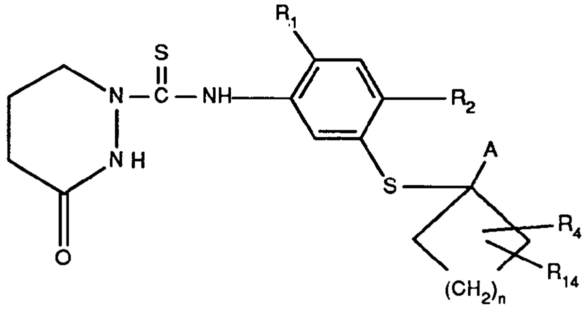

(XVI)

umsetzt, diese mit einer Verbindung der Formel Xa

$CZ_2Cl_2$ (Xa)

worin $Z_2$ für Sauerstoff oder Schwefel steht, oder mit $Cl_3(CO)_3Cl_3$ (Triphosgen) in die Verbindung der Formel XVII

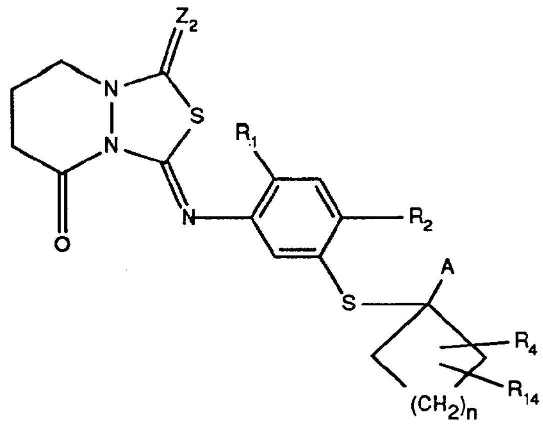

(XVII)

überführt, diese mit Natriumborhydrid zur Verbindung der Formel XVIII

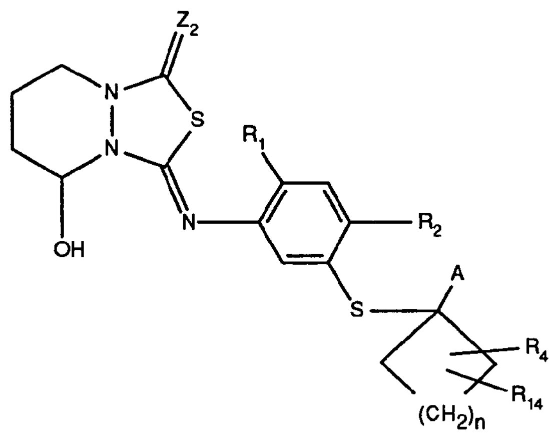

(XVIII)

umsetzt und diese anschliessend in Gegenwart von Säure dehydratisiert.

Verbindungen der Formel I, worin W für $W_8$ steht, können auch hergestellt werden, indem man Verbindungen der Formel I, worin W für $W_9$ steht, hydriert. Derartige Hydrierungsverfahren sowie die Hydrierung der Verbindung der Formel XIII zur Verbindung der Formel XIV sind dem Fachmann geläufig. Sie können beispielsweise mit Wasserstoff in Gegenwart von Edelmetallkatalysatoren wie Platin durchgeführt werden.

Die Umsetzung der Isothiocyanate der Formel VIIa mit den Pyridazinen der Formeln VIII und XI sowie den Pyridazin-3-onen der Formel XIV wird mit Vorteil in einem reaktionsinerten Lösungsmittel bei Temperaturen von -5 °C bis zur Siedetemperatur des Lösungsmittels, insbesondere von 0 bis +50°C, besonders bevorzugt bei Raumtemperatur, durchgeführt. Geeignete Lösungsmittel für diese Reaktion sind beispielsweise Toluol, Xylol, Essigsäureethylester oder Acetonitril.

Die Umsetzung der Verbindung der Formeln IX und XII mit der Verbindung der Formel X sowie die Umsetzung der Verbindung der Formel XVI mit der Verbindung der Formel X oder mit Triphosgen erfolgt vorteilhaft in einem reaktionsinerten Lösungsmittel bei niedrigen Temperaturen, vorzugsweise bei 0 bis +50°C, besonders bevorzugt bei 0 bis +15°C. Geeignete Basen für diese Reaktion sind beispielsweise Pyridin, Triethylamin oder N,N-Dimethylanilin. Als Lösungsmittel eignen sich beispielsweise 1,2-Dichlorethan, Dichlormethan oder Toluol.

Die Umsetzung der Verbindung der Formel XVII mit Natriumborhydrid in die Verbindung der Formel XVIII wird vorteilhaft bei Temperaturen von Raumtemperatur bis +50°C durchgeführt, wobei als Lösungsmittel Alkohole bevorzugt sind. Die anschliessende Dehydratisierung der Verbindung der Formel XVIII wird mit Vorteil in Toluol bei Rückflußtemperatur in Gegenwart von p-Toluolsulfonsäure, Schwefelsäure oder Trifluoressigsäure durchgeführt.

Die für die erfindungsgemässen Verbindungen der Formel I, worin W für $W_8$ oder $W_9$ steht, als Ausgangsprodukte verwendeten Pyridazine der Formeln VIII und XI sowie die Pyridazin-3-one der Formel XIV sind

bekannt oder lassen sich analog zu literaturbekannten Verfahren herstellen. Die Herstellung derartiger Verbindungen ist beispielsweise in Coll. Czechoslov. Chem. Commun. 33, 2087 (1968), Khimiya Geterotsiklicheskikh Soedinenii, 4, (3), 530-535 ( 1968), Beilstein, E III/IV 23, S. 465 sowie EP-A-0304920 beschrieben.

Die Verbindungen der Formel I, in welcher W für $W_{10}$ steht, können hergestellt werden durch Umsetzung von einem 2-Hydroxy-cyclohex-1-en-carbonsäure- oder -thiocarbonsäureanilid der Formel XIX

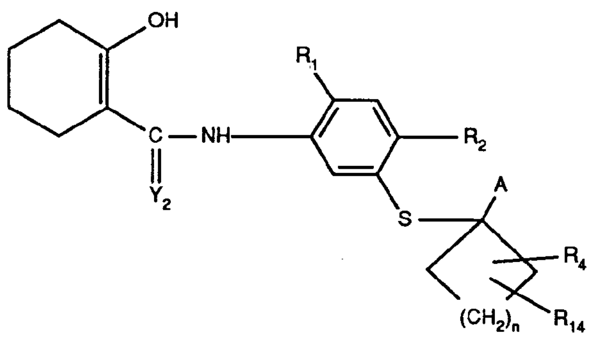

(XIX)

worin A, $R_1$, $R_2$, $R_4$, $R_{14}$, $Y_2$ und n unter der Formel I gegebene Bedeutung haben, mit einem reaktiven Kohlensäure- oder Thiokohlensäure-Derivat der Formel XX

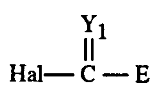

(XX)

worin $Y_1$ Sauerstoff oder Schwefel, Hal Chlor oder Brom und E Chlor, Brom oder $C_1$-$C_4$-Alkoxy bedeutet, wobei Ringschluß zur Verbindung der Formel I erfolgt.

Der Ringschluß erfolgt in Gegenwart der äquimolaren Menge einer organischen oder anorganischen Base in einem inerten Lösungs- oder Verdünnungsmittel bei einer Temperatur von Raumtemperatur bis zum Siedepunkt des Reaktionsgemisches.

Als reaktionsfähige Kohlensäure- beziehungsweise Thiokohlensäureester kommen z.B. Phosgen, Thiophosgen, Chlorameisensäure-ethylester, Chlorameisensäuremethylester, in Frage.

Als Lösungs- und Verdünnungsmittel eignen sich Aceton, Methylethylketon, Dioxan oder Tetrahydrofuran, Acetonitril, Toluol oder Chloroform.

Die Zwischenprodukte der Formel XIX sind neue Verbindungen. Sie wurden speziell zur Herstellung der Verbindungen der Formel I entwickelt. Die Zwischenprodukte der Formel XIX bilden daher ebenfalls einen Gegenstand der vorliegenden Erfindung. Um die Verbindungen der Formel XIX herzustellen, setzt man z.B. Morpholinocyclohexen mit einem Isocyanat oder Isothiocyanat der Formel VII um und hydrolisiert das entstandene 2-Morpholino-cyclohex-1-en-carbonsäureanilid bzw. 2-Morpholino-cyclohex-1-en-thiocarbonsäureanilid der Formel XXII anschliessend in wässrig saurer Lösung entsprechend dem Reaktionsschema:

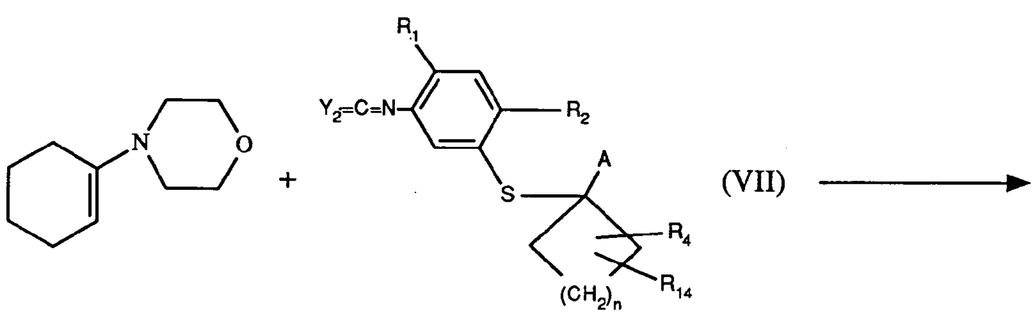

(VII)

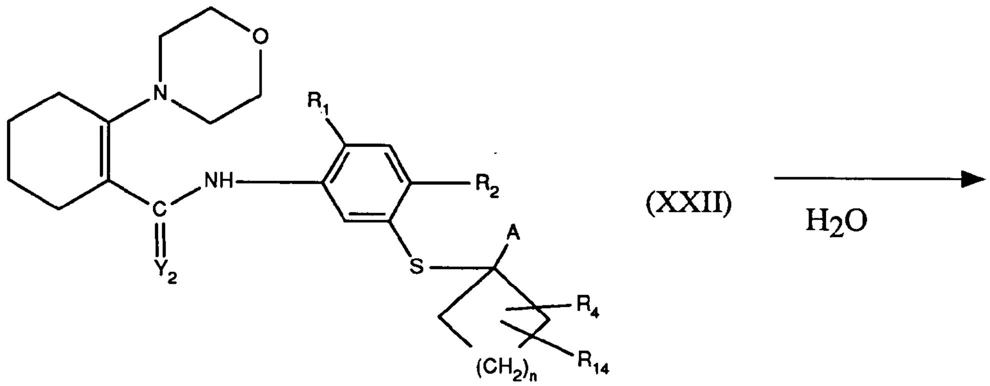

(XXII)

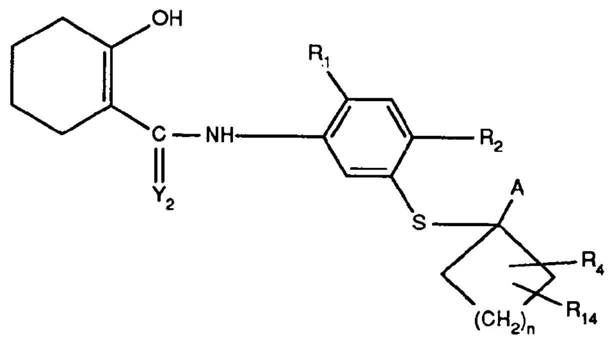
(XIX)

In den obigen Formeln haben die Substituenten die unter der Formel I gegebene Bedeutung.

1 -Morpholinocyclohex-1-en ist bekannt.

Die Isoyanate bzw. Isothiocyanate der Formel VII sind neu. Unter die Formel VII fallen die Isothiocyanate der Formel VIIa und die Isocyanate der Formel VIIb. Die Verbindungen der Formel VII wurden speziell zur Herstellung der Verbindungen der Formel I entwickelt und bilden ebenfalls einen Gegenstand der vorliegenden Erfindung. Die Isocyanate bzw. Isothiocyanate der Formel VII werden in an sich bekannter Weise durch Umsetzen der entsprechenden Amine der Formel III

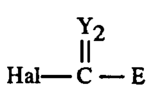 (XXa) + 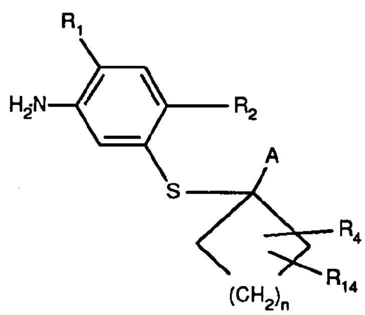 (III)

mit Phosgen oder Thiophosgen bzw. mit einem reaktiven Kohlensäure- oder Thiokohlensäureester der Formel XXa erhalten. Derartige Verfahren sind beispielsweise in EP-A-0304920 beschrieben.

In der Formel III haben die Substituenten die unter Formel I angegebene Bedeutung. In der Formel XXa bedeutet $Y_2$ Sauerstoff oder Schwefel, Hal Chlor oder Brom und E Chlor, Brom oder $C_1$-$C_4$-Alkoxy.

Die Kondensation des Isocyanates bzw. Isothiocyanates der Formel VII mit dem 1-Morpholinocyclohex-1-en geschieht im allgemeinen spontan unter Wärmeentwicklung. Es empfiehlt sich aber die Reaktion in einem absolutem Lösungsmittel, mit frisch destilliertem 1-Morpholinocyclohex-1-en unter Anschluss von Luftsauerstoff, z.B. durch Verdrängen der Luft im Reaktionsgefäss mit Stickstoff oder einem Edelgas vorzunehmen und die Reaktion nach Abklingen des exothermen Temperaturanstieges zu erhitzen.

Die vorstehend aufgeführten Verfahren können analog zu literaturbekannten Verfahren durchgeführt werden. Die in diesen Verfahren bevorzugten Reaktionsbedingungen, wie Temperatur, molare Verhältnisse der Edukte, Reaktionsführung. Lösungsmittel, gegebenenfalls erforderliche Reagentien, wie Säuren, Basen, wasserbindende Mittel usw., sind dem Fachmann geläufig.

Herstellungsbeispiele:

Beispiel H1: Herstellung von 1-(5-Amino-2-chlor-4-fluor-phenylthio)-cyclobutancarbonsäuremethylester:

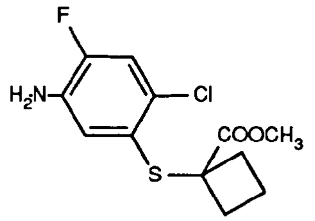

Zu einer bei Raumtemperatur gerührten Lösung aus 4,7 g Natriummethylat in 100 ml Methanol gibt man 16,6 g 5-Amino-2-chlor-4-fluor-phenylthiophenol hinzu. Nach tropenweiser Zugabe von 16,6 g 1-Brom-cyclobutan-carbonsäurem ethylester erwärmt man langsam zum Sieden. Nachdem die Reaktionsmischung 6 Stunden lang auf Rückflußtemperatur gehalten wurde, gibt man die Mischung in Eiswasser. Anschließend wird die organische Phase mit Ether extrahiert und über Natriumsulfat getrocknet. Nach Einengen der Lösung und chromatographischer Reinigung durch Kieselgelchromatographie erhält man 12 g 1-(5-Amino-2-chlor-4-fluor-phenylthio)-cyclobutancarbonsäuremethylester mit einem Schmelzpunkt von +94 bis +95 °C.

Beispiel H2: Herstellung von 1 -(2-chlor-4-fluor- 5-isothiocyanato-phenylthio)-cyclobutancarbonsäuremethylester:

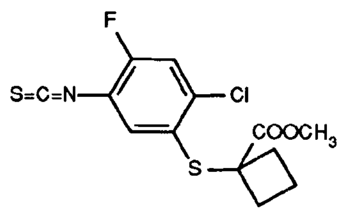

Zu einem Gemisch aus 12 g Calciumcarbonat und 8 ml Thiophosgen in 40 ml Ethylenchlorid und 40 ml Wasser gibt man unter Rühren bei einer Temperatur von +25 bis +30°C eine Lösung aus 12 g gemäß Beispiel H1 erhaltenen 1-(5-Amino-2-chlor-4-fluor-phenylthio)-cyclobutancarbonsäuremethylester in 30 ml Ethylenchlorid tropfenweise hinzu. Anschließend wird das Reaktionsgemisch 18 Stunden lang gerührt. Nach Abfiltrieren des entstandenen Niederschlages wird die organische Phase über Calciumchlorid getrocknet und anschließend im Vakuum eingeengt. Man erhält 13,1 g 2-(2-chlor-4-fluor-5-isothiocyanato-phenylthio)cyclobutancarbonsäuremethylester in Form eines Öles.

Beispiel H3: Herstellung von 1-(2-chlor-4-fluor-5-(1-hexahydropyridazinylthiocarbonylamino)-phenylthio] - cyclobutancarbonsäuremethylester:

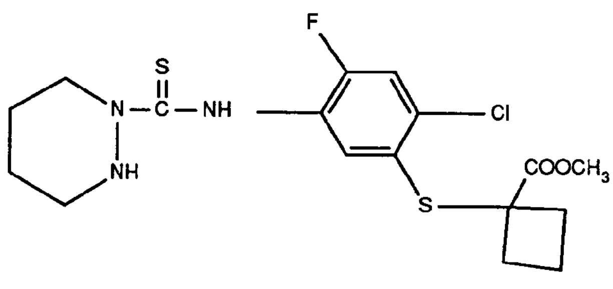

Zu einer Lösung aus 4 g Hexahydropyridazin in 30 ml Ethanol gibt man unter Rühren bei Raumtemperatur eine Lösung von 13,1 g gemäß Beispiel H2 erhaltenen 1-(2-chlor-4-fluor5-isothiocyanatophenylthio)-cyclobutancarbonsäuremethylesters in 200 ml Ethanol tropfenweise hinzu. Nach 3-stündigem Rühren bei Raumtemperatur wird die Reaktionsmischung im Vakuum eingedampft. Man erhält 10,8 g 1-[2-chlor-4-fluor-5-(1-hexahydropyridazinyl-thiocarbonylamino)-phenylthio]-cyclobutancarbonsäuremethylester mit einem Schmelzpunkt

von +112 bis +113°C.

Beispiel H4: Herstellung von 9-[4-chlor-2-fluor-5-(1-methoxycarbonylcyclobutylthio)-phenylimino]-8-thia-1,6-diazabicyclo[4.3.0]-nonan-7-on

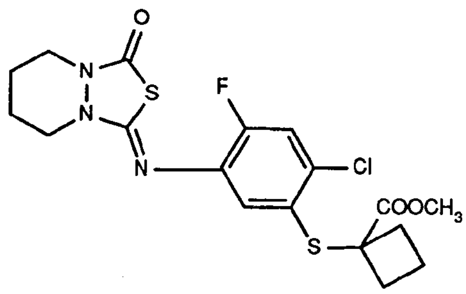

Zu einer Lösung von 10,4 g 1-[2-chlor-4-fluor-5-(1-hexahydropyridazinyl-thiocarbonylamino)-phenylthio] -cyclobutancarbonsäuremethylester und 10 ml Triethylamin in 100 ml Toluol gibt man unter Rühren bei einer Temperatur von + 10°C 12,5 ml 20%-ige toluolische Lösung von Phosgen tropfenweise hinzu. Anschließend läßt man die Reaktionsmischung 6 Stunden lang rühren und gibt sie dann in Eiswasser. Anschließend wird die organische Phase abgetrennt und über Natriumsulfat getrocknet. Nach dem Eindampfen und anschließender chromatographischer Reinigung mittels Kieselgelchromatographie erhält man 4,6 g 9-[4-chlor-2-fluor-5-(1-methoxycarbonylcyclobutylthio)-pheny limino]-8-thia-1,6-diazabicyclo[4.3.0]-nonan-7-on, $\eta_D^{22}$ 1,6010.

Beispiel H5: Herstellung von 1-[2-Chlor-4-fluor-5-(1,2,3,4,5,6-hexahydro-1,3-dioxo-isoindolyl)-phenylthio]-cyclobutancarbonsäuremethylester:

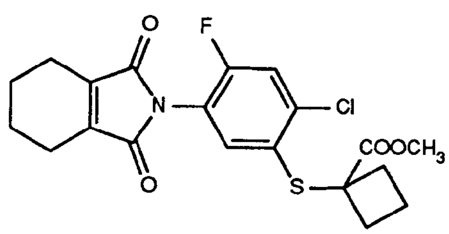

Ein Gemisch aus 4,4 g 1-(5-Amino-2-chlor-4-fluor-phenylthio)-cyclobutancarbonsäuremethylester und 2,4 g 3,4,5,6-Tetrahydrophtalsäureanhydrid in 60 ml Eisessig wird 5 Stunden lang unter Rühren am Rückfluß erhitzt. Anschließend wird das Reaktionsgemisch in Eiswasser gegeben und das ausgefallene klebrige Produkt mit Essigsäureethylester extrahiert. Nach Trocknen der organischen Phase über Natriumsulfat und Eindampfen im Vakuum wird der erhaltene Rückstand mit Petrolether versetzt, wobei die Substanz sich verfestigt. Nach dem Abfiltrieren erhält man 4 g 1-[2-Chlor-4-fluor-5-(1,2,3,4,5,6-hexahydro-1,3-dioxo-isoindolyl)-phenylthio]-cyclobutancarbonsäuremethylester mit einem Schmelzpunkt von +98°C.

Beispiel H6: Herstellung von 2-(5-Amino-2-chlor-4-fluor-phenylthio)-4-chlor-buttersäureethylester:

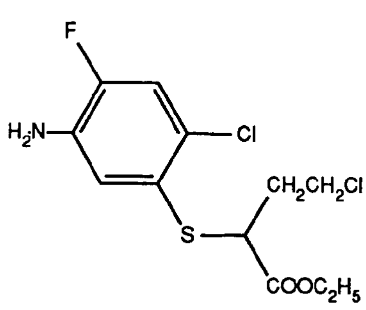

Zu einem Gemisch aus 14 g 5-Amino-2-chlor-4-fluor-thiophenol und 12,7 g Kaliumcarbonat in 280 ml

Methylethylketon gibt man bei Raumtemperatur 16,2 g 2,4-Dichlorbuttersäureethylester tropfenweise hinzu (exotherme Reaktion). Anschließend wird die Reaktionsmischung 18 Stunden am Rückfluß erhitzt. Nach dem Abkühlen der Reaktionsmischung wird der anorganische Teil der Mischung abfiltriert und mit Methylethylketon gewaschen. Nach Eindampfen der organischen Lösung und chromatographischer Reinigung des Rückstandes durch Kieselgelchromatographie erhält man 14,2 g 2-(5-Amino-2-chlor-4-fluor-phenylthio)-4-chlor-buttersäureethylester mit einem Schmelzpunkt von +97 bis +99°C.

Beispiel H7: Herstellung von 1-(5-Amino-2-chlor-4-fluor-phenylthio)-cyclopropancarbonsäure:

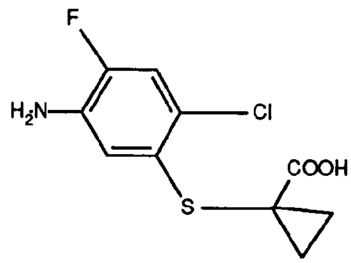

Zu einer Lösung aus 14,2 g 2-(5-Amino-2-chlor-4-fluor-phenylthio)-4-chlor-buttersäureeth ylester in 25 ml Ethanol gibt man unter Rühren bei einer Temperatur von +5°C eine Lösung von 2,4 g Kaliumhydroxid in 50 ml Ethanol tropfenweise hinzu. Nach 18-stündigem Rühren gibt man weitere 3-mal 0,9 g Kaliumhydroxid hinzu und rührt jeweils die Reaktionsmischung für 6 Stunden bis zum vollständigem Umsatz. Anschließend wird das Reaktionsgemisch eigeengt mit Wasser versetzt und mit Essigsäureethylester 3-mal extrahiert. Die vereinigten organischen Phasen werden über Natriumsulfat getrocknet und anschließend eingedampft. Nach Umkristallisation des Rückstands aus Essigsäureethylester/n-Hexan erhält man 8,4 g 1-(5-Amino-2-chlor-4-fluor-phenylthio)-cyclopropancarbonsäure mit einem Schmelzpunkt von +266 bis +267°C.

Beispiel H8: Herstellung von 1-[2-Chlor-4-fluor-5-(1,2,3,4,5,6-hexahydro-1,3-dioxoisoindolyl)-phenylthio]-cyclopropancarbonsäure:

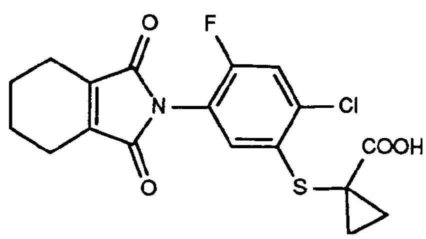

Ein Gemisch aus 2,4 g 1-(5-Amino-2-chlor-4-fluor-phenylthio)-cyclopropancarbonsäure, 1,5 g 3,4,5,6-Tetrahydrophtalsäureanhydrid und 100 ml Eisessig wird 18 Stunden lang unter Rühren am Rückfluß erhitzt. Anschließend wird das Reaktionsgemisch in Eiswasser gegeben und das ausgefallene Produkt mit Essigsäureethylester extrahiert. Nach Trocknen über Natriumsulfat und Eindampfen der Lösung erhält man 1,7 g 1-[2-Chlor-4-fluor-5-(1,2,3,4,5,6-hexahydro-1,3-dioxo-isoindolyl)-phenylthio]-cyclopropancarbonsäure mit einem Schmelzpunkt von +186 bis +188°C.

Beispiel H9: Herstellung von 1-[2-Chlor-4-fluor-5-(1,2,3,4,5,6-hexahydro1,3-dioxo-isoindolyl)-phenylthio]-cyclopropancarbonsäurechlor id:

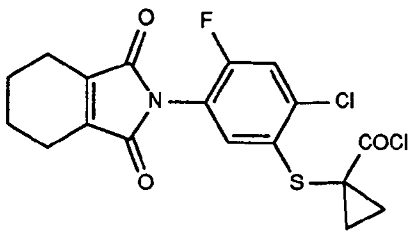

Zu einer Lösung aus 1 g 1-[2-Chlor-4-fluor-5-(1,2,3,4,5,6-hexahydro-1,3-dioxo-isoindolyl)-phenylthio]-cyclopropancarbonsäure und einem Tropfen Dimethylformamid in 40 ml Ethylenchlorid gibt man unter Rühren bei Raumtemperatur 3 ml Thionylchlorid hinzu und erhitzt anschließend für 3 Stunden am Rückfluß. Nach dem Eindampfen der Reaktionsmischung erhält man 1 g 1-[2-Chlor-4-fluor-5-(1,2,3,4,5,6-hexahydro-1,3-dioxo-isoindolyl)-phenylthio]-cyclopropancarbonsäurechlorid in Form eines Öles.

Beispiel H10: Herstellung von 1-[2-Chlor-4-fluor-5-(1,2,3,4,5,6-hexahydro-1,3-dioxo-isoindolyl)-phenylthio]-cyclopropancarbonsäuremethylester:

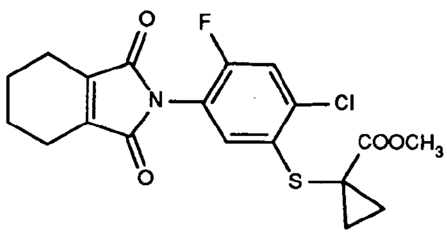

Zu einer Lösung aus 10 ml Methanol und 0,5 ml Triethylamin in 10 ml Essigsäuremethylester gibt man unter Rühren bei Raumtemperatur eine Lösung aus 1 g gemäß Beispiel H9 erhaltenen 1-[2-Chlor-4-fluor-5-(1,2,3,4,5,6-hexahydro-1,3-dioxo-isoindolyl)-phenylthio]-cyclopropancarbonsäurechlorids in 10 ml Essigsäuremethylester tropfenweise hinzu. Nach 3-stündigem Rühren bei Raumtemperatur filtriert man das ausgefallene Triethylaminhydrochlorid ab und dampft das Filtrat ein. Man erhält 0,9 g 1-[2-Chlor-4-fluor-5-(1,2,3,4,5,6-hexahydro-1,3-dioxo-isoindolyl)-phenylthio]-cyclopropancarbonsäuremethylester mit einem Schmelzpunkt von +142 bis +144°C.

Beispiel H11: Herstellung von N-[4-chlor-2-fluor-5-(1-methoxycarbonyl-cyclobutylthio)-phenyl] -3-trifluormethyl-glutarsäuremonoamid:

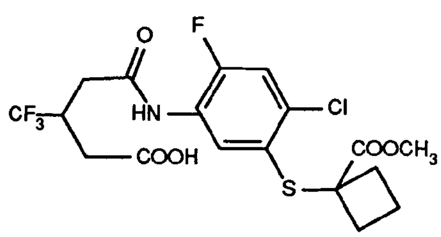

Ein Gemisch aus 3,6 g 3-Trifluormethyl-glutarsäureanhydrid und 5,8 g 1-(5-Amino-2-chlor-4-fluor-phenylthio)-cyclobutancarbonsäuremethylester in 65 ml Benzol wird unter Rühren 3 Stunden lang am Rückfluß erhitzt. Anschließend wird der entstandene Niederschlag abgetrennt, mit Diisopropylether gewaschen und getrocknet. Man erhält 7,9 g N-[4-chlor-2-fluor-5-(1-methoxycarbonyl-cyclobutylthio)-phenyl] -3-trifluormethyl-glutarsäuremonoamid mit einem Schmelzpunkt von +165 bis +167°C.

Beispiel H12: Herstellung von 1-[4-Chlor-2-fluor-5-(1-methoxycarbonyl-cyclobutyl)-phenylthio]-4-trifluormethyl-piperidin-2,6-dion:

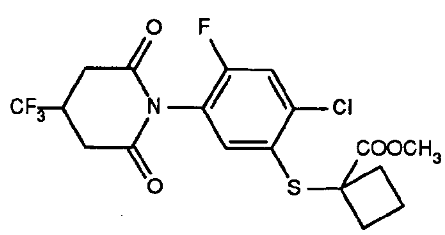

Ein Gemisch aus 7,9 g N-[4-chlor-2-fluor-5-(1-methoxycarbonyl-cyclobutylthio)-phenyl]-3-trifluormethyl-glutarsäuremonoamid und 1,4 g Natriumacetat in 34 ml Essigsäureanhydrid wird 30 Minuten lang bei einer

Temperatur von +100°C gerührt. Anschließend wird die Reaktionsmischung eingedampft und der Rückstand mit einer Mischung aus Diethylether und Eiswasser versetzt. Die Etherphase wird nacheinander mit Eiswasser, 10%-iger Natriumhydrogencarbonatlösung und gesättigter Kochsalzlösung gewaschen und danach über Natriumsulfat getrocknet. Das nach dem Abdampfen des Ethers zurückbleibende Öl wird aus n-Hexan unter Zugabe von wenig Diisopropylether und anschließend aus Methanol umkristallisiert. Man erhält 5 g 1-[4-Chlor-2-fluor-5-(1-methoxycarbonyl-cyclobutyl)-phenylthio]-4-trifluormethyl-piperidin-2,6-dion mit einem Schmelzpunkt von +115 bis +117°C.

Beispiel H13: Herstellung von 1-(5-Amino-2-chlor-4-fluor-phenylthio)-cyclopropancarbonsäureethyester:

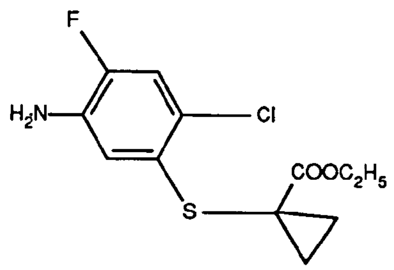

Ein Gemisch aus 12,5 g 2-(5-Amino-2-chlor-4-fluor-phenylthio)-4-chlor-buttersäureeth ylester, 10,6 g Kaliumcarbonat und 0,7 g Tetrabutylammoniumhydrogensulfat in 14 ml Toluol wird 40 Stunden bei +80°C gehalten. Anschließend werden die festen Teile der Reaktionsmischung abfiltriert und mit Aceton gewaschen. Nach dem Abdampfen des Lösungsmittels und Umkristallisieren aus n-Hexan und wenig Essigsäureethylester erhält man 7,9 g 1-(5-Amino-2-chlor-4-fluor-phenylthio)-cyclopropancarbonsäureethyester mit einem Schmelzpunkt von +96 bis +97°C.

Beispiel H14: Herstellung von 2-(5-Amino-2-chlor-4-fluor-phenylthio)-4-brom-buttersäuremethylester:

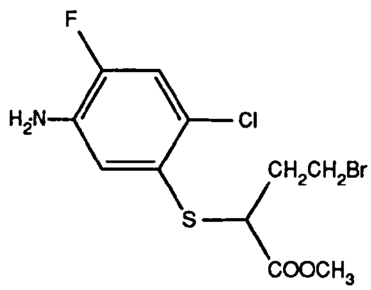

Zu einem Gemisch aus 17,8 g 5-Amino-2-chlor-4-fluor-thiophenol und 15,2 g Kaliumcarbonat in 160 ml Methylethylketon gibt man bei Raumtemperatur 28,6 g 2,4-Dibrombuttersäuremethylester tropfenweise hinzu (exotherme Reaktion). Anschließend wird die Reaktionsmischung 2 Stunden am Rückfluß erhitzt. Nach dem Abkühlen der Reaktionsmischung wird der anorganische Teil der Mischung abfiltriert und mit Methylethylketon gewaschen. Nach Eindampfen der organischen Lösung und chromatographischer Reinigung des Rückstandes durch Kieselgelchromatographie erhält man 25,5 g 2-(5-Amino-2-chlor-4-fluor-phenylthio)-4-brom-buttersäuremethylester mit einem Schmelzpunkt von +97 bis +99°C.

Beispiel H15: Herstellung von 1-(5-Amino-2-chlor-4-fluor-phenylthio)-cyclopropancarbonsäuremethyester:

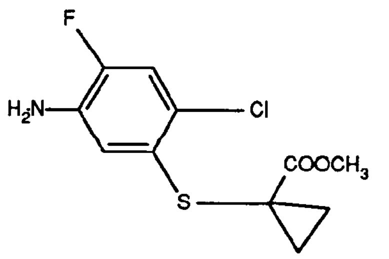

Ein Gemisch aus 12,5 g 2-(5-Amino-2-chlor-4-fluor-phenylthio)-4-brom-buttersäuremethylester, 30,4 g Kaliumcarbonat und 1,7 g Tetrabutylammoniumhydrogensulfat in 38 ml Toluol wird 40 Stunden bei +80°C gehalten. Anschließend werden die festen Teile der Reaktionsmischung abfiltriert und mit Aceton gewaschen. Nach dem Abdampfen des Lösungsmittels erhält man 17 g 1-(5-Amino-2-chlor-4-fluor-phenylthio)-cyclopropancarbonsäuremethyester mit einem Schmelzpunkt von +119 bis +124°C.

Beispiel H16: Herstellung von 1-(5-Amino-2-chlor-4-fluor-phenylthio)-cyclopropancarbonsäuremethyester:

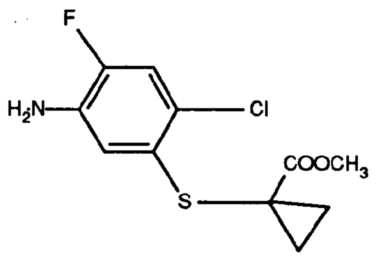

Eine Lösung aus 3,6 g 1-(5-Amino-2-chlor-4-fluor-phenylthio)-cyclopropancarbonsäure und einigen Tropfen Schwefelsäure in 50 ml Methanol wird solange am Rückfluß erhitzt, bis kein Ausgangsprodukt mehr nachweisbar ist. Anschließend wird das Methanol abgedampft, der Rückstand mit Wasser und Eessigsäureethylester versetzt und die organische Phase nacheinander mit Natriumhydrogencarbonatlösung, Wasser und gesättigter Natriumchloridlösung gewaschen. Nach dem Einengen und Reinigen des Rohprodukts mittels Kieselgelchromatographie erhält man 5,7 g 1-(5-Amino-2-chlor-4-fluor-phenylthio)-cyclopropancarbonsäuremethyester mit einem Schmelzpunkt von + 119 bis + 121 °C.

Beispiel H17: Herstellung von 1-(2-chlor-4-fluor-5-isothiocyanato-phenylthio)-cyclopropancarbonsäuremethyester:

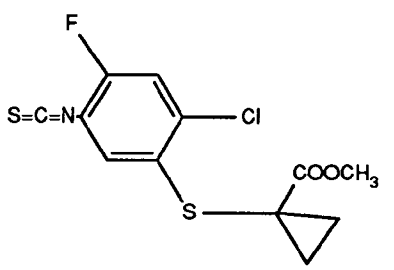

Zu einem Gemisch aus 3 g Calciumcarbonat, 2 ml Thiophosgen in 20 ml Ethylenchlorid und 20 ml Wasser gibt man unter Rühren bei Raumtemperatur eine Lösung aus 5,5 g 1-(5-Amino-2-chlor-4-fluor-phenylthio)-cyclopropancarbonsäuremethyester in 20 ml Ethylenchlorid tropfenweise hinzu. Nach 4-stündigem Rühren bei Raumtemperatur wird der anorganische Teil der Reaktionsmischung abfiltriert, die organische Phase über Calciumchlorid getrocknet und anschließend eingedampft. Man erhält 6,3 g 1-(2-chlor-4-fluor-5-isothiocyanato-phenylthio)-cyclopropancarbonsäuremethyester mit einem Schmelzpunkt von +100 bis +101°C.

Beispiel H18: Herstellung von 1-[2-chlor-4-fluor-5-(1-hexahydropyridazinyl-thiocarbonylamino)-phenylthio]-cyclopropancarbonsäuremethylester:

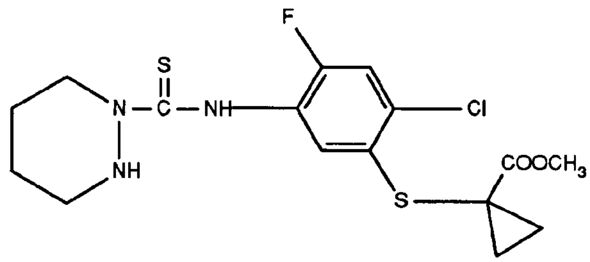

Zu einer Lösung aus 2,2 g Hexahydropyridazin in 30 ml Ethanol gibt man unter Rühren bei Raumtemperatur eine Lösung von 6,3 g 1-(2-chlor-4-fluor5-isothiocyanato-phenylthio)-cyclopropancarbonsäuremethylesters in 30 ml Ethanol tropfenweise hinzu. Nach 30-minütigem Rühren bei Raumtemperatur wird die Reaktionsmischung im Vakuum eingedampft. Man erhält 7,9 g 1-[2-chlor-4-fluor-5-(1-hexahydropyridazinyl-thiocarbonylamino)-phenylthio]-cyclopropancarbonsäuremethylester mit einem Schmelzpunkt von +156 bis +157°C.

Beispiel H19: Herstellung von 9-[4-chlor-2-fluor-5-(1-methoxycarbonyl-cyclopropylthio)-phenylimino] -8-thia-1,6-diazabicyclo[4.3.0]-nonan-7-on:

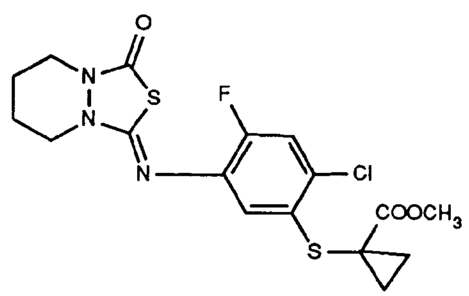

Zu einer Lösung von 3,7 g 1-[2-chlor-4-fluor-5-(1-hexahydropyridazinyl-thiocarbonylamino)-phenylthio]-cyclopropancarbonsäuremethylester und 4,6 ml Triethylamin in 100 ml Toluol gibt man unter Rühren bei einer Temperatur von o bis +10°C 5,7 ml 20%-ige toluolische Lösung von Phosgen tropfenweise hinzu. Anschließend läßt man die Reaktionsmischung 4 Stunden lang rühren und gibt sie dann in Eiswasser. Anschließend wird die organische Phase abgetrennt und über Natriumsulfat getrocknet. Nach dem Eindampfen und anschließender chromatographischer Reinigung mittels Kieselgelchromatographie erhält man 3,7 g 9-[4-chlor-2-fluor-5-(1-methoxycarbonyl-cyclopropylthio)-phenylimino]-8-thia-1,6-diazabicyclo[4.3.0]-nonan-7-on, mit einem Schmelzpunkt von +149°C.

Analog zu vorstehenden Beispielen und den in der Beschreibung genannten Herstellungsverfahren können die Verbindungen der Tabellen 1 bis 12 hergestellt werden:

Tabelle 1:

Verbindungen der Formel I mit W = $W_1$ und $R_{14}$ = H:

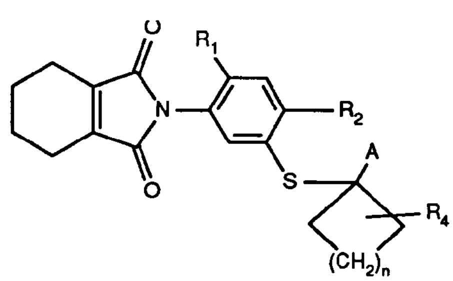

(I)

Tabelle 1

| Verb. Nr. | $R_1$ | $R_2$ | $R_3$ | $R_4$ | n | A | Smp. [°C] |
|---|---|---|---|---|---|---|---|
| 1.001 | F | Cl | -Cl | H | 0 | $-CO-R_3$ | |
| 1.002 | F | Cl | -OH | H | 0 | $-CO-R_3$ | 186-188 |
| 1.003 | F | Cl | $-OCH_3$ | H | 0 | $-CO-R_3$ | 142 |
| 1.004 | F | Cl | $-OC_2H_5$ | H | 0 | $-CO-R_3$ | 85-86 |
| 1.005 | F | Cl | $-OC_3H_7$ | H | 0 | $-CO-R_3$ | |
| 1.006 | F | Cl | 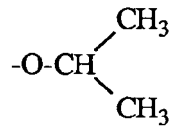 | H | 0 | $-CO-R_3$ | $n_D^{20} = 1,5368$ |
| 1.007 | F | Cl | $-OC_4H_9$ | H | 0 | $-CO-R_3$ | |
| 1.008 | F | Cl | $-O-CH(CH_3)-C_2H_5$ | H | 0 | $-CO-R_3$ | |
| 1.009 | F | Cl | 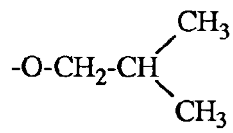 | H | 0 | $-CO-R_3$ | |
| 1.010 | F | Cl | $-OC_5H_{11}$ | H | 0 | $-CO-R_3$ | |
| 1.011 | F | Cl | $-O-CH_2-CH_2-O-CH_3$ | H | 0 | $-CO-R_3$ | |
| 1.012 | F | Cl | $-O-CH_2-CH_2-O-C_2H_5$ | H | 0 | $-CO-R_3$ | |
| 1.013 | F | Cl | $-O-CH-(CH_3)-CH_2-O-CH_3$ | H | 0 | $-CO-R_3$ | |
| 1.014 | F | Cl | $-O-CH_2-CH_2-S-CH_3$ | H | 0 | $-CO-R_3$ | |
| 1.015 | F | Cl | $-O-CH(CH_3)-CH_2-S-CH_3$ | H | 0 | $-CO-R_3$ | |
| 1.016 | F | Cl | $-O-CH(CH_3)-CH_2-S-C_2H_5$ | H | 0 | $-CO-R_3$ | |
| 1.017 | F | Cl | $-O-CH(CH_3)-CH_2-S-C_3H_7$ | H | 0 | $-CO-R_3$ | |
| 1.018 | F | Cl | 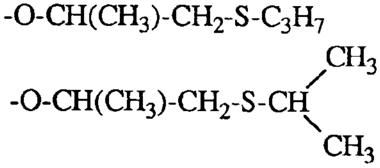 | H | 0 | $-CO-R_3$ | |
| 1.019 | F | Cl | $-O-CH(CH_3)-CH_2-S-C_4H_9$ | H | 0 | $-CO-R_3$ | |
| 1.020 | F | Cl | $-O-CH(CH_3)-CH_2-S-C_5H_{11}$ | H | 0 | $-CO-R_3$ | |
| 1.021 | F | Cl | 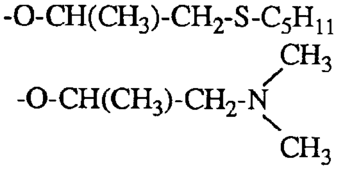 | H | 0 | $-CO-R_3$ | |

Tabelle 1 (Fortsetzung)

| Verb. Nr. | $R_1$ | $R_2$ | $R_3$ | $R_4$ | n | A | Smp. [°C] |
|---|---|---|---|---|---|---|---|
| 1.022 | F | Cl | 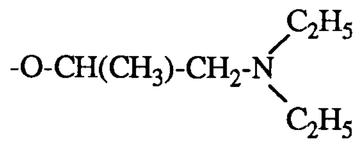 | H | 0 | $-CO-R_3$ | |
| 1.023 | F | Cl | $-NH_2$ | H | 0 | $-CO-R_3$ | |
| 1.024 | F | Cl | 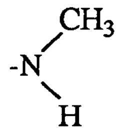 | H | 0 | $-CO-R_3$ | |
| 1.025 | F | Cl | 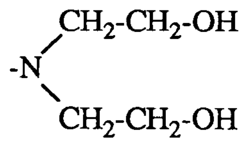 | H | 0 | $-CO-R_3$ | |
| 1.026 | F | Cl | $-NH-CH_2-CH=CH_2$ | H | 0 | $-CO-R_3$ | |
| 1.027 | F | Cl | $-N-(CH_2-CH=CH_2)_2$ | H | 0 | $-CO-R_3$ | |
| 1.028 | F | Cl | 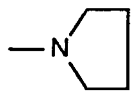 | H | 0 | $-CO-R_3$ | |
| 1.029 | F | Cl | 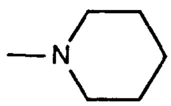 | H | 0 | $-CO-R_3$ | |
| 1.030 | F | Cl | 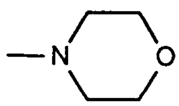 | H | 0 | $-CO-R_3$ | |
| 1.031 | F | Cl | 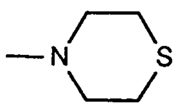 | H | 0 | $-CO-R_3$ | |
| 1.032 | F | Cl | 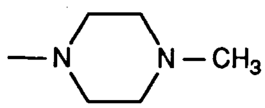 | H | 0 | $-CO-R_3$ | |
| 1.033 | F | Cl | 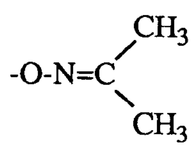 | H | 0 | $-CO-R_3$ | |
| 1.034 | F | Cl | $-O-CH_2-CH_2-Cl$ | H | 0 | $-CO-R_3$ | |
| 1.035 | F | Cl | $-O-CH_2-CN$ | H | 0 | $-CO-R_3$ | |
| 1.036 | F | Cl | 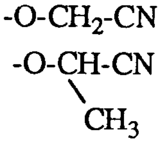 | H | 0 | $-CO-R_3$ | |
| 1.037 | F | Cl | $-O-CH_2-CH=CH_2$ | H | 0 | $-CO-R_3$ | |

Tabelle 1 (Fortsetzung)

| Verb. Nr. | $R_1$ | $R_2$ | $R_3$ | $R_4$ | n | A | Smp. [°C] |
|---|---|---|---|---|---|---|---|
| 1.038 | F | Cl | $-O-CH_2-CH=CHCl$ | H | 0 | $-CO-R_3$ | |
| 1.039 | F | Cl | $-O-CH_2-C=CH_2$<br>Cl | H | 0 | $-CO-R_3$ | |
| 1.040 | F | Cl | $-O-CH_2=C\equiv CH$ | H | 0 | $-CO-R_3$ | |
| 1.041 | F | Cl | $-O-CH-C\equiv CH$<br>$CH_3$ | H | 0 | $-CO-R_3$ | |
| 1.042 | F | Cl | 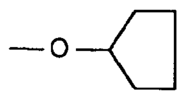 | H | 0 | $-CO-R_3$ | |
| 1.043 | F | Cl | 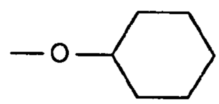 | H | 0 | $-CO-R_3$ | |
| 1.044 | F | Cl | 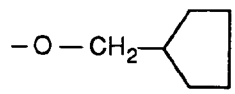 | H | 0 | $-CO-R_3$ | |
| 1.045 | F | Cl | 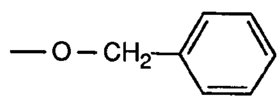 | H | 0 | $-CO-R_3$ | |
| 1.046 | F | Cl | 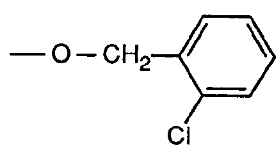 | H | 0 | $-CO-R_3$ | |
| 1.047 | F | Cl | 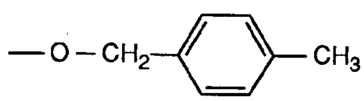 | H | 0 | $-CO-R_3$ | |
| 1.048 | F | Cl | $-S-CH_3$ | H | 0 | $-CO-R_3$ | |
| 1.049 | F | Cl | $-S-C_2H_5$ | H | 0 | $-CO-R_3$ | |
| 1.050 | F | Cl | $-S-C_3H_7$ | H | 0 | $-CO-R_3$ | |
| 1.051 | F | Cl | $-S-CH_2-CH=CH_2$ | H | 0 | $-CO-R_3$ | |
| 1.052 | F | Cl | $-S-CH_2-COOCH_3$ | H | 0 | $-CO-R_3$ | |
| 1.053 | F | Cl | $-S-CH_2-COOC_2H_5$ | H | 0 | $-CO-R_3$ | |
| 1.054 | F | Cl | $-S-CH_2-COOC_5H_{11}$ | H | 0 | $-CO-R_3$ | |
| 1.055 | F | Cl | $-S-CH(CH_3)-COOCH_3$ | H | 0 | $-CO-R_3$ | |
| 1.056 | F | Cl | $-S-(CH_3)-COOC_2H_5$ | H | 0 | $-CO-R_3$ | |

Tabelle 1 (Fortsetzung)

| Verb. Nr. | $R_1$ | $R_2$ | $R_3$ | $R_4$ | n | A | Smp. [°C] |
|---|---|---|---|---|---|---|---|
| 1.057 | F | Cl | $-S-CH(CH_3)-COOC_3H_7$ | H | 0 | $-CO-R_3$ | |
| 1.058 | F | Cl | $-S-CH_2-CH_2-COOCH_3$ | H | 0 | $-CO-R_3$ | |
| 1.059 | F | Cl | $-S-CH_2-COOCH_2-CH_2-O-CH_3$ | H | 0 | $-CO-R_3$ | |
| 1.060 | F | Cl | $-O-CH_2-COOCH_3$ | H | 0 | $-CO-R_3$ | |
| 1.061 | F | Cl | $-O-CH(CH_3)-COOCH_3$ | H | 0 | $-CO-R_3$ | |
| 1.062 | F | Cl | $-O-CH_2-COOC_5H_{11}$ | H | 0 | $-CO-R_3$ | |
| 1.063 | F | Cl | $-O-CH_2-CH_3-Si(CH_3)_3$ | H | 0 | $-CO-R_3$ | |
| 1.064 | F | Cl | -ONa | H | 0 | $-CO-R_3$ | |
| 1.065 | F | Br | -Cl | H | 0 | $-CO-R_3$ | |
| 1.066 | F | Br | -OH | H | 0 | $-CO-R_3$ | |
| 1.067 | F | Br | $-OCH_3$ | H | 0 | $-CO-R_3$ | |
| 1.068 | F | Br | $-OC_2H_5$ | H | 0 | $-CO-R_3$ | |
| 1.069 | F | Br | $-OC_3H_7$ | H | 0 | $-CO-R_3$ | |
| 1.070 | F | Br | 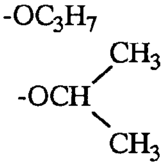 | H | 0 | $-CO-R_3$ | |
| 1.071 | F | Br | $-OC_4H_9$ | H | 0 | $-CO-R_3$ | |
| 1.072 | F | Br | $-OCH(CH_3)-CH_2-CH_3$ | H | 0 | $-CO-R_3$ | |
| 1.073 | F | Br | 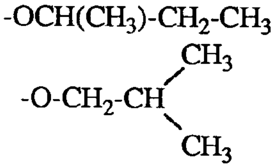 | H | 0 | $-CO-R_3$ | |
| 1.074 | F | Br | $-O-C_5H_{11}$ | H | 0 | $-CO-R_3$ | |
| 1.075 | F | Br | $-O-CH_2-CH_2-O-CH_3$ | H | 0 | $-CO-R_3$ | |
| 1.076 | F | Br | $-O-CH_2-CH_2-O-C_2H_5$ | H | 0 | $-CO-R_3$ | |
| 1.077 | F | Br | $-O-CH(CH_3)-CH_2-O-CH_3$ | H | 0 | $-CO-R_3$ | |
| 1.078 | F | Br | $-O-CH_2-CH_2-S-CH_3$ | H | 0 | $-CO-R_3$ | |
| 1.079 | F | Br | $-O-CH_2(CH_3)-S-CH_3$ | H | 0 | $-CO-R_3$ | |
| 1.080 | F | Br | $-O-CH-(CH_3)-S-C_2H_5$ | H | 0 | $-CO-R_3$ | |
| 1.081 | F | Br | $-O-CH(CH_3)-S-C_3H_7$ | H | 0 | $-CO-R_3$ | |
| 1.082 | F | Br | 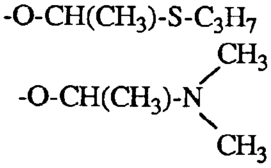 | H | 0 | $-CO-R_3$ | |
| 1.083 | F | Br | $-NH_2$ | H | 0 | $-CO-R_3$ | |

Tabelle 1 (Fortsetzung)

| Verb. Nr. | $R_1$ | $R_2$ | $R_3$ | $R_4$ | n | A | Smp. [°C] |
|---|---|---|---|---|---|---|---|
| 1.084 | F | Br | 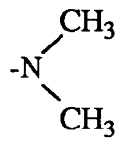 | H | 0 | $-CO-R_3$ | |
| 1.085 | F | Br | 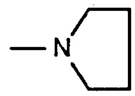 | H | 0 | $-CO-R_3$ | |
| 1.086 | F | Br | 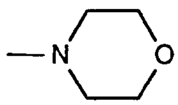 | H | 0 | $-CO-R_3$ | |
| 1.087 | F | Br | 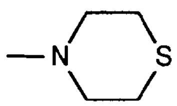 | H | 0 | $-CO-R_3$ | |
| 1.088 | F | Br | 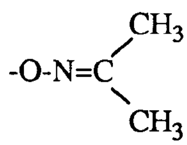 | H | 0 | $-CO-R_3$ | |
| 1.089 | F | Br | $-O-CH_2-CH_2-Cl$ | H | 0 | $-CO-R_3$ | |
| 1.090 | F | Br | $-O-CH_2-CN$ | H | 0 | $-CO-R_3$ | |
| 1.091 | F | Br | $-O-CH_2-CH=CH_2$ | H | 0 | $-CO-R_3$ | |
| 1.092 | F | Br | $-O-CH_2-C\equiv CH$ | H | 0 | $-CO-R_3$ | |
| 1.093 | F | Br | 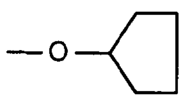 | H | 0 | $-CO-R_3$ | |
| 1.094 | F | Br | 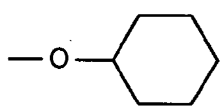 | H | 0 | $-CO-R_3$ | |
| 1.095 | F | Br | 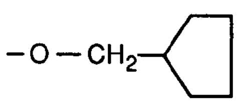 | H | 0 | $-CO-R_3$ | |
| 1.096 | F | Br | 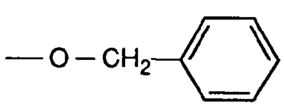 | H | 0 | $-CO-R_3$ | |
| 1.097 | F | Br | $-SCH_3$ | H | 0 | $-CO-R_3$ | |
| 1.098 | F | Br | $-S-CH_2-COOCH_3$ | H | 0 | $-CO-R_3$ | |
| 1.099 | F | Br | $-S-CH(CH_3)-COOCH_3$ | H | 0 | $-CO-R_3$ | |
| 1.100 | F | Br | $-O-CH_2-COOCH_3$ | H | 0 | $-CO-R_3$ | |

Tabelle 1 (Fortsetzung)

| Verb. Nr. | $R_1$ | $R_2$ | $R_3$ | $R_4$ | n | A | Smp. [°C] |
|---|---|---|---|---|---|---|---|
| 1.101 | F | Br | $-O-CH(CH_3)COOCH_3$ | H | 0 | $-CO-R_3$ | |
| 1.102 | F | CN | -Cl | H | 0 | $-CO-R_3$ | |
| 1.103 | F | CN | -OH | H | 0 | $-CO-R_3$ | |
| 1.104 | F | CN | $-OCH_3$ | H | 0 | $-CO-R_3$ | |
| 1.105 | H | Cl | -Cl | H | 0 | $-CO-R_3$ | |
| 1.106 | H | Cl | -OH | H | 0 | $-CO-R_3$ | |
| 1.107 | H | Cl | $-OCH_3$ | H | 0 | $-CO-R_3$ | |
| 1.108 | H | Cl | $-OC_2H_5$ | H | 0 | $-CO-R_3$ | |
| 1.109 | H | Cl | 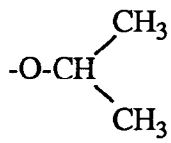 | H | 0 | $-CO-R_3$ | |
| 1.110 | H | Cl | $-O-CH_2-COOCH_3$ | H | 0 | $-CO-R_3$ | |
| 1.111 | H | Cl | $-O-CH(CH_3)COOCH_3$ | H | 0 | $-CO-R_3$ | |
| 1.112 | H | Cl | $-S-CH_2-COOCH_3$ | H | 0 | $-CO-R_3$ | |
| 1.113 | H | Cl | $-S-CH(CH_3)COOCH_3$ | H | 0 | $-CO-R_3$ | |
| 1.114 | H | Cl | 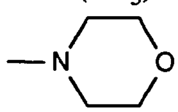 | H | 0 | $-CO-R_3$ | |
| 1.115 | F | Cl | -Cl | H | 1 | $-CO-R_3$ | |
| 1.116 | F | Cl | -OH | H | 1 | $-CO-R_3$ | |
| 1.117 | F | Cl | $-OCH_3$ | H | 1 | $-CO-R_3$ | 98 |
| 1.118 | F | Cl | $-OC_2H_5$ | H | 1 | $-CO-R_3$ | 122-123 |
| 1.119 | F | Cl | $-OC_3H_7$ | H | 1 | $-CO-R_3$ | |
| 1.120 | F | Cl | 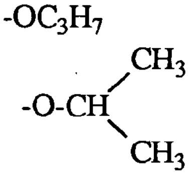 | H | 1 | $-CO-R_3$ | |
| 1.121 | F | Cl | $-OC_4H_9$ | H | 1 | $-CO-R_3$ | |
| 1.122 | F | Cl | $-O-CH(CH_3)-C_2H_5$ | H | 1 | $-CO-R_3$ | |
| 1.123 | F | Cl | 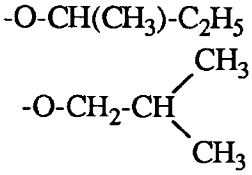 | H | 1 | $-CO-R_3$ | |
| 1.124 | F | Cl | $-OC_5H_{11}$ | H | 1 | $-CO-R_3$ | |
| 1.125 | F | Cl | $-O-CH_2-CH_2-O-CH_3$ | H | 1 | $-CO-R_3$ | |
| 1.126 | F | Cl | $-O-CH_2-CH_2-O-C_2H_5$ | H | 1 | $-CO-R_3$ | |

Tabelle 1 (Fortsetzung)

| Verb. Nr. | $R_1$ | $R_2$ | $R_3$ | $R_4$ | n | A | Smp. [°C] |
|---|---|---|---|---|---|---|---|
| 1.127 | F | Cl | $-O-CH-(CH_3)-CH_2-O-CH_3$ | H | 1 | $-CO-R_3$ | |
| 1.128 | F | Cl | $-O-CH_2-CH_2-S-CH_3$ | H | 1 | $-CO-R_3$ | |
| 1.129 | F | Cl | $-O-CH(CH_3)-CH_2-S-CH_3$ | H | 1 | $-CO-R_3$ | |
| 1.130 | F | Cl | $-O-CH(CH_3)-CH_2-S-C_2H_5$ | H | 1 | $-CO-R_3$ | |
| 1.131 | F | Cl | $-O-CH(CH_3)-CH_2-S-C_3H_7$ | H | 1 | $-CO-R_3$ | |
| 1.132 | F | Cl | 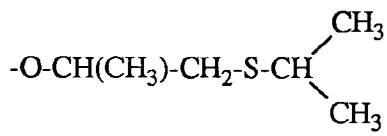 | H | 1 | $-CO-R_3$ | |
| 1.133 | F | Cl | $-O-CH(CH_3)-CH_2-S-C_4H_9$ | H | 1 | $-CO-R_3$ | |
| 1.134 | F | Cl | $-O-CH(CH_3)-CH_2-S-C_5H_{11}$ | H | 1 | $-CO-R_3$ | |
| 1.135 | F | Cl | 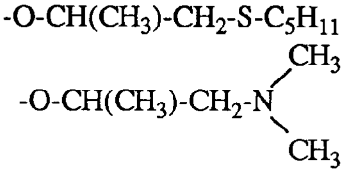 | H | 1 | $-CO-R_3$ | |
| 1.136 | F | Cl | 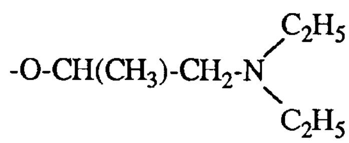 | H | 1 | $-CO-R_3$ | |
| 1.137 | F | Cl | $-NH_2$ | H | 1 | $-CO-R_3$ | |
| 1.138 | F | Cl | 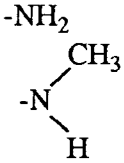 | H | 1 | $-CO-R_3$ | |
| 1.139 | F | Cl | 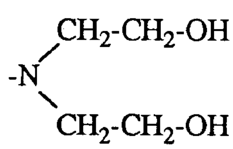 | H | 1 | $-CO-R_3$ | |
| 1.140 | F | Cl | $-NH-CH_2-CH=CH_2$ | H | 1 | $-CO-R_3$ | |
| 1.141 | F | Cl | $-N-(CH_2-CH=CH_2)_2$ | H | 1 | $-CO-R_3$ | |
| 1.142 | F | Cl | 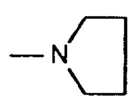 | H | 1 | $-CO-R_3$ | |
| 1.143 | F | Cl | 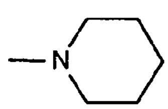 | H | 1 | $-CO-R_3$ | |

Tabelle 1 (Fortsetzung)

| Verb. Nr. | $R_1$ | $R_2$ | $R_3$ | $R_4$ | n | A | Smp. [°C] |
|---|---|---|---|---|---|---|---|
| 1.144 | F | Cl | 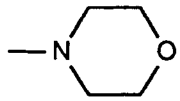 | H | 1 | $-CO-R_3$ | |
| 1.145 | F | Cl | 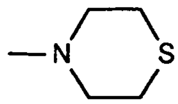 | H | 1 | $-CO-R_3$ | |
| 1.146 | F | Cl | 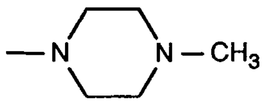 | H | 1 | $-CO-R_3$ | |
| 1.147 | F | Cl | 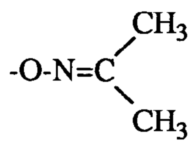 | H | 1 | $-CO-R_3$ | |
| 1.148 | F | Cl | $-O-CH_2-CH_2-Cl$ | H | 1 | $-CO-R_3$ | |
| 1.149 | F | Cl | $-O-CH_2-CN$ | H | 1 | $-CO-R_3$ | |
| 1.150 | F | Cl | 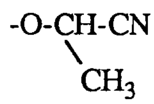 | H | 1 | $-CO-R_3$ | |
| 1.151 | F | Cl | $-O-CH_2-CH=CH_2$ | H | 1 | $-CO-R_3$ | |
| 1.152 | F | Cl | $-O-CH_2-CH=CHCl$ | H | 1 | $-CO-R_3$ | |
| 1.153 | F | Cl | 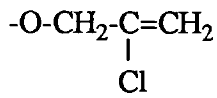 | H | 1 | $-CO-R_3$ | |
| 1.154 | F | Cl | $-O-CH_2=C\equiv CH$ | H | 1 | $-CO-R_3$ | |
| 1.155 | F | Cl | 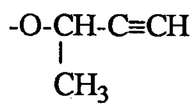 | H | 1 | $-CO-R_3$ | |
| 1.156 | F | Cl | 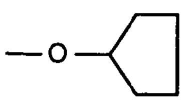 | H | 1 | $-CO-R_3$ | |
| 1.157 | F | Cl | 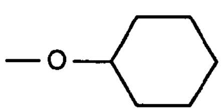 | H | 1 | $-CO-R_3$ | |
| 1.158 | F | Cl | 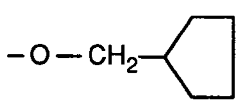 | H | 1 | $-CO-R_3$ | |
| 1.159 | F | Cl | 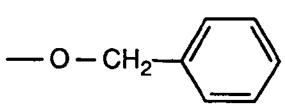 | H | 1 | $-CO-R_3$ | |

Tabelle 1 (Fortsetzung)

| Verb. Nr. | $R_1$ | $R_2$ | $R_3$ | $R_4$ | n | A | Smp. [°C] |
|---|---|---|---|---|---|---|---|
| 1.160 | F | Cl | 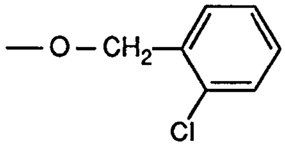 | H | 1 | $-CO-R_3$ | |
| 1.161 | F | Cl | 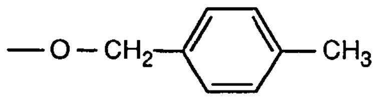 | H | 1 | $-CO-R_3$ | |
| 1.162 | F | Cl | $-S-CH_3$ | H | 1 | $-CO-R_3$ | |
| 1.163 | F | Cl | $-S-C_2H_5$ | H | 1 | $-CO-R_3$ | |
| 1.164 | F | Cl | $-S-C_3H_7$ | H | 1 | $-CO-R_3$ | |
| 1.165 | F | Cl | $-S-CH_2-CH=CH_2$ | H | 1 | $-CO-R_3$ | |
| 1.166 | F | Cl | $-S-CH_2-COOCH_3$ | H | 1 | $-CO-R_3$ | |
| 1.167 | F | Cl | $-S-CH_2-COOC_2H_5$ | H | 1 | $-CO-R_3$ | |
| 1.168 | F | Cl | $-S-CH_2-COOC_5H_{11}$ | H | 1 | $-CO-R_3$ | |
| 1.169 | F | Cl | $-S-CH(CH_3)-COOCH_3$ | H | 1 | $-CO-R_3$ | |
| 1.170 | F | Cl | $-S-(CH_3)-COOC_2H_5$ | H | 1 | $-CO-R_3$ | |
| 1.171 | F | Cl | $-S-CH(CH_3)-COOC_3H_7$ | H | 1 | $-CO-R_3$ | |
| 1.172 | F | Cl | $-S-CH_2-CH_2-COOCH_3$ | H | 1 | $-CO-R_3$ | |
| 1.173 | F | Cl | $-S-CH_2-COOCH_2-CH_2-O-CH_3$ | H | 1 | $-CO-R_3$ | |
| 1.174 | F | Cl | $-O-CH_2-COOCH_3$ | H | 1 | $-CO-R_3$ | |
| 1.175 | F | Cl | $-O-CH(CH_3)-COOCH_3$ | H | 1 | $-CO-R_3$ | |
| 1.176 | F | Cl | $-O-CH_2-COOC_5H_{11}$ | H | 1 | $-CO-R_3$ | |
| 1.177 | F | Cl | $-O-CH_2-CH_3-Si(CH_3)_3$ | H | 1 | $-CO-R_3$ | |
| 1.178 | F | Cl | -ONa | H | 1 | $-CO-R_3$ | |
| 1.179 | F | Br | -Cl | H | 1 | $-CO-R_3$ | |
| 1.180 | F | Br | -OH | H | 1 | $-CO-R_3$ | |
| 1.181 | F | Br | $-OCH_3$ | H | 1 | $-CO-R_3$ | |
| 1.182 | F | Br | $-OC_2H_5$ | H | 1 | $-CO-R_3$ | |
| 1.183 | F | Br | $-OC_3H_7$ | H | 1 | $-CO-R_3$ | |
| 1.184 | F | Br | 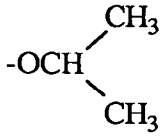 | H | 1 | $-CO-R_3$ | |
| 1.185 | F | Br | $-OC_4H_9$ | H | 1 | $-CO-R_3$ | |

Tabelle 1 (Fortsetzung)

| Verb. Nr. | $R_1$ | $R_2$ | $R_3$ | $R_4$ | n | A | Smp. [°C] |
|---|---|---|---|---|---|---|---|
| 1.186 | F | Br | $-OCH(CH_3)-CH_2-CH_3$ | H | 1 | $-CO-R_3$ | |
| 1.187 | F | Br | 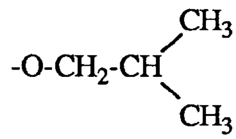 | H | 1 | $-CO-R_3$ | |
| 1.188 | F | Br | $-O-C_5H_{11}$ | H | 1 | $-CO-R_3$ | |
| 1.189 | F | Br | $-O-CH_2-CH_2-O-CH_3$ | H | 1 | $-CO-R_3$ | |
| 1.190 | F | Br | $-O-CH_2-CH_2-O-C_2H_5$ | H | 1 | $-CO-R_3$ | |
| 1.191 | F | Br | $-O-CH(CH_3)-CH_2-O-CH_3$ | H | 1 | $-CO-R_3$ | |
| 1.192 | F | Br | $-O-CH_2-CH_2-S-CH_3$ | H | 1 | $-CO-R_3$ | |
| 1.193 | F | Br | $-O-CH_2(CH_3)-S-CH_3$ | H | 1 | $-CO-R_3$ | |
| 1.194 | F | Br | $-O-CH-(CH_3)-S-C_2H_5$ | H | 1 | $-CO-R_3$ | |
| 1.195 | F | Br | $-O-CH(CH_3)-S-C_3H_7$ | H | 1 | $-CO-R_3$ | |
| 1.196 | F | Br | 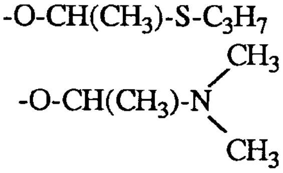 | H | 1 | $-CO-R_3$ | |
| 1.197 | F | Br | $-NH_2$ | H | 1 | $-CO-R_3$ | |
| 1.198 | F | Br | 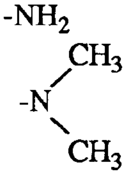 | H | 1 | $-CO-R_3$ | |
| 1.199 | F | Br | 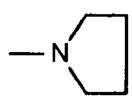 | H | 1 | $-CO-R_3$ | |
| 1.200 | F | Br | 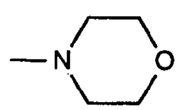 | H | 1 | $-CO-R_3$ | |
| 1.201 | F | Br | 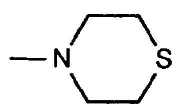 | H | 1 | $-CO-R_3$ | |
| 1.202 | F | Br | 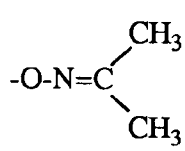 | H | 1 | $-CO-R_3$ | |
| 1.203 | F | Br | $-O-CH_2-CH_2-Cl$ | H | 1 | $-CO-R_3$ | |
| 1.204 | F | Br | $-O-CH_2-CN$ | H | 1 | $-CO-R_3$ | |
| 1.205 | F | Br | $-O-CH_2-CH=CH_2$ | H | 1 | $-CO-R_3$ | |
| 1.206 | F | Br | $-O-CH_2-C\equiv CH$ | H | 1 | $-CO-R_3$ | |

Tabelle 1 (Fortsetzung)

| Verb. Nr. | $R_1$ | $R_2$ | $R_3$ | $R_4$ | n | A | Smp. [°C] |
|---|---|---|---|---|---|---|---|
| 1.207 | F | Br | 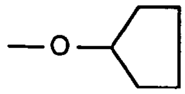 | H | 1 | $-CO-R_3$ | |
| 1.208 | F | Br | 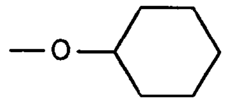 | H | 1 | $-CO-R_3$ | |
| 1.209 | F | Br | 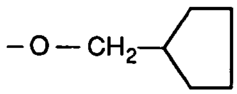 | H | 1 | $-CO-R_3$ | |
| 1.210 | F | Br | 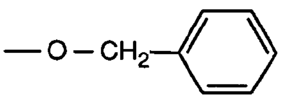 | H | 1 | $-CO-R_3$ | |
| 1.211 | F | Br | $-SCH_3$ | H | 1 | $-CO-R_3$ | |
| 1.212 | F | Br | $-S-CH_2-COOCH_3$ | H | 1 | $-CO-R_3$ | |
| 1.213 | F | Br | $-S-CH(CH_3)-COOCH_3$ | H | 1 | $-CO-R_3$ | |
| 1.214 | F | Br | $-O-CH_2-COOCH_3$ | H | 1 | $-CO-R_3$ | |
| 1.215 | F | Br | $-O-CH(CH_3)COOCH_3$ | H | 1 | $-CO-R_3$ | |
| 1.216 | F | CN | -Cl | H | 1 | $-CO-R_3$ | |
| 1.217 | F | CN | -OH | H | 1 | $-CO-R_3$ | |
| 1.218 | F | CN | $-OCH_3$ | H | 1 | $-CO-R_3$ | |
| 1.219 | H | Cl | -Cl | H | 1 | $-CO-R_3$ | |
| 1.220 | H | Cl | -OH | H | 1 | $-CO-R_3$ | |
| 1.221 | H | Cl | $-OCH_3$ | H | 1 | $-CO-R_3$ | |
| 1.222 | H | Cl | $-OC_2H_5$ | H | 1 | $-CO-R_3$ | |
| 1.223 | H | Cl | 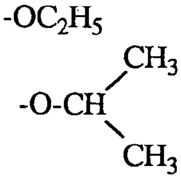 | H | 1 | $-CO-R_3$ | |
| 1.224 | H | Cl | $-O-CH_2-COOCH_3$ | H | 1 | $-CO-R_3$ | |
| 1.225 | H | Cl | $-O-CH(CH_3)COOCH_3$ | H | 1 | $-CO-R_3$ | |
| 1.226 | H | Cl | $-S-CH_2-COOCH_3$ | H | 1 | $-CO-R_3$ | |
| 1.227 | H | Cl | $-S-CH(CH_3)COOCH_3$ | H | 1 | $-CO-R_3$ | |
| 1.228 | H | Cl | 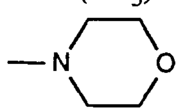 | H | 1 | $-CO-R_3$ | |
| 1.229 | F | Cl | Cl | H | 2 | $-CO-R_3$ | |

Tabelle 1 (Fortsetzung)

| Verb. Nr. | $R_1$ | $R_2$ | $R_3$ | $R_4$ | n | A | Smp. [°C] |
|---|---|---|---|---|---|---|---|
| 1.230 | F | Cl | OH | H | 2 | $-CO-R_3$ | |
| 1.231 | F | Cl | $OCH_3$ | H | 2 | $-CO-R_3$ | |
| 1.232 | F | Cl | $-OC_2H_5$ | H | 2 | $-CO-R_3$ | |
| 1.233 | F | Cl | 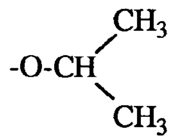 | H | 2 | $-CO-R_3$ | |
| 1.234 | F | Cl | $-O-CH_2-CH_2-O-CH_3$ | H | 2 | $-CO-R_3$ | |
| 1.235 | F | Cl | $-O-CH(CH_3)-CH_2-S-CH_3$ | H | 2 | $-CO-R_3$ | |
| 1.236 | F | Cl | $-O-CH_2-COOCH_3$ | H | 2 | $-CO-R_3$ | |
| 1.237 | F | Cl | $-S-CH_2-COOCH_3$ | H | 2 | $-CO-R_3$ | |
| 1.238 | F | Cl | $-CH_2-CH=CH_2$ | H | 2 | $-CO-R_3$ | |
| 1.239 | F | Cl | $-CH_2-C\equiv CH$ | H | 2 | $-CO-R_3$ | |
| 1.240 | F | Cl | Cl | H | 3 | $-CO-R_3$ | |
| 1.241 | F | Cl | OH | H | 3 | $-CO-R_3$ | |
| 1.242 | F | Cl | $OCH_3$ | H | 3 | $-CO-R_3$ | 102-103 |
| 1.243 | F | Cl | $OC_2H_5$ | H | 3 | $-CO-R_3$ | |
| 1.244 | F | Cl | 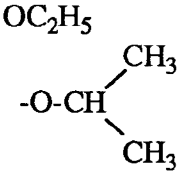 | H | 3 | $-CO-R_3$ | |
| 1.245 | F | Cl | $-O-CH_2-CH_2-O-CH$ | H | 3 | $-CO-R_3$ | |
| 1.246 | F | Cl | $-O-CH(CH_3)-CH_2-S-CH_3$ | H | 3 | $-CO-R_3$ | |
| 1.247 | F | Cl | $-O-CH_2-COOCH_3$ | H | 3 | $-CO-R_3$ | |
| 1.248 | F | Cl | $-S-CH_2-COOCH_3$ | H | 3 | $-CO-R_3$ | |
| 1.249 | F | Cl | $-O-CH_2-C\equiv CH$ | H | 3 | $-CO-R_3$ | |
| 1.250 | F | Cl | -Cl | Cl | 0 | $-CO-R_3$ | |
| 1.251 | F | Cl | -OH | Cl | 0 | $-CO-R_3$ | |
| 1.252 | F | Cl | $-OCH_3$ | Cl | 0 | $-CO-R_3$ | |
| 1.253 | F | Cl | $-OC_2H_5$ | Cl | 0 | $-CO-R_3$ | |
| 1.254 | F | Cl | 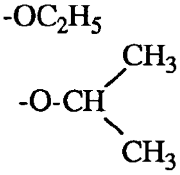 | Cl | 0 | $-CO-R_3$ | |
| 1.255 | F | Cl | $-O-CH_2-COOCH_3$ | Cl | 0 | $-CO-R_3$ | |
| 1.256 | F | Cl | $-S-CH_2-COOCH_3$ | Cl | 0 | $-CO-R_3$ | |
| 1.257 | F | Cl | $-OCH_3$ | Br | 0 | $-CO-R_3$ | |

Tabelle 1 (Fortsetzung)

| Verb. Nr. | $R_1$ | $R_2$ | $R_3$ | $R_4$ | n | A | Smp. [°C] |
|---|---|---|---|---|---|---|---|
| 1.258 | F | Cl | 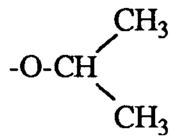 | Br | 0 | $-CO-R_3$ | |
| 1.259 | F | Cl | $-OCH_3$ | F | 0 | $-CO-R_3$ | |
| 1.260 | F | Cl | $-OCH_3$ | $CH_3$ | 0 | $-CO-R_3$ | |
| 1.261 | F | Cl | $-OC_2H_5$ | $CH_3$ | 0 | $-CO-R_3$ | |
| 1.262 | F | Cl | 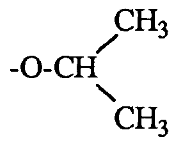 | $CH_3$ | 0 | $-CO-R_3$ | |
| 1.263 | F | Cl | $-O-CH_2-CH_2-O-CH_3$ | $CH_3$ | 0 | $-CO-R_3$ | |
| 1.264 | F | Cl | $-O-CH_2-COOCH_3$ | $CH_3$ | 0 | $-CO-R_3$ | |
| 1.265 | F | Cl | $-O-CH(CH_3)COOCH_3$ | $CH_3$ | 0 | $-CO-R_3$ | |
| 1.266 | F | Cl | $-S-CH_2-COOCH_3$ | $CH_3$ | 0 | $-CO-R_3$ | |
| 1.267 | F | Cl | $-OCH_3$ | $CF_3$ | 0 | $-CO-R_3$ | |
| 1.268 | F | Cl | $-OC_2H_5$ | $CF_3$ | 0 | $-CO-R_3$ | |
| 1.269 | F | Cl | 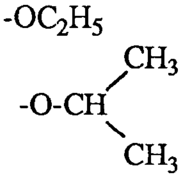 | $CF_3$ | 0 | $-CO-R_3$ | |
| 1.270 | F | Cl | $-O-CH_2-COOCH_3$ | $CF_3$ | 0 | $-CO-R_3$ | |
| 1.271 | F | Cl | $-S-CH_2-COOCH_3$ | $CF_3$ | 0 | $-CO-R_3$ | |
| 1.272 | F | Cl | - | H | 0 | -CN | |
| 1.273 | F | Cl | - | H | 1 | -CN | |
| 1.274 | F | Cl | - | H | 2 | -CN | |
| 1.275 | F | Cl | - | H | 3 | -CN | |
| 1.276 | F | Cl | - | H | 4 | -CN | |

Tabelle 2:

Verbindungen der Formel I, worin W für $W_2$, $Y_3$ für Sauerstoff und $R_{14}$ für Wasserstoff steht:

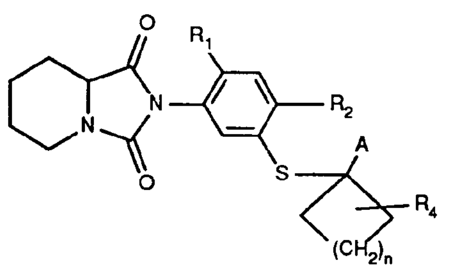

(I)

Tabelle 2

| Verb. Nr. | $R_1$ | $R_2$ | $R_3$ | $R_4$ | n | A | Smp. [°C] |
|---|---|---|---|---|---|---|---|
| 2.001 | F | Cl | -Cl | H | 0 | $-CO-R_3$ | |
| 2.002 | F | Cl | -OH | H | 0 | $-CO-R_3$ | |
| 2.003 | F | Cl | $-OCH_3$ | H | 0 | $-CO-R_3$ | |
| 2.004 | F | Cl | $-OC_2H_5$ | H | 0 | $-CO-R_3$ | |
| 2.005 | F | Cl | $-OC_3H_7$ | H | 0 | $-CO-R_3$ | |
| 2.006 | F | Cl | 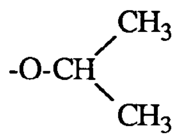 | H | 0 | $-CO-R_3$ | |
| 2.007 | F | Cl | $-OC_4H_9$ | H | 0 | $-CO-R_3$ | |
| 2.008 | F | Cl | $-O-CH(CH_3)-C_2H_5$ | H | 0 | $-CO-R_3$ | |
| 2.009 | F | Cl | 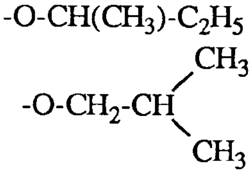 | H | 0 | $-CO-R_3$ | |
| 2.010 | F | Cl | $-OC_5H_{11}$ | H | 0 | $-CO-R_3$ | |
| 2.011 | F | Cl | $-O-CH_2-CH_2-O-CH_3$ | H | 0 | $-CO-R_3$ | |
| 2.012 | F | Cl | $-O-CH_2-CH_2-O-C_2H_5$ | H | 0 | $-CO-R_3$ | |
| 2.013 | F | Cl | $-O-CH-(CH_3)-CH_2-O-CH_3$ | H | 0 | $-CO-R_3$ | |
| 2.014 | F | Cl | $-O-CH_2-CH_2-S-CH_3$ | H | 0 | $-CO-R_3$ | |
| 2.015 | F | Cl | $-O-CH(CH_3)-CH_2-S-CH_3$ | H | 0 | $-CO-R_3$ | |
| 2.016 | F | Cl | $-O-CH(CH_3)-CH_2-S-C_2H_5$ | H | 0 | $-CO-R_3$ | |
| 2.017 | F | Cl | $-O-CH(CH_3)-CH_2-S-C_3H_7$ | H | 0 | $-CO-R_3$ | |
| 2.018 | F | Cl | 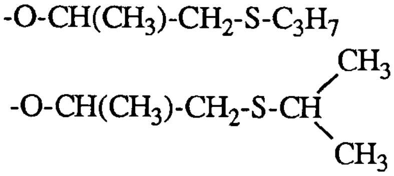 | H | 0 | $-CO-R_3$ | |
| 2.019 | F | Cl | $-O-CH(CH_3)-CH_2-S-C_4H_9$ H 0 | | | $-CO-R_3$ | |
| 2.020 | F | Cl | $-O-CH(CH_3)-CH_2-S-C_5H_{11}$ | H | 0 | $-CO-R_3$ | |
| 2.021 | F | Cl | 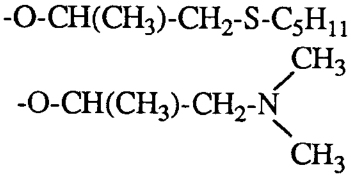 | H | 0 | $-CO-R_3$ | |

Tabelle 2 (Fortsetzung)

| Verb. Nr. | $R_1$ | $R_2$ | $R_3$ | $R_4$ | n | A | Smp. [°C] |
|---|---|---|---|---|---|---|---|
| 2.022 | F | Cl | 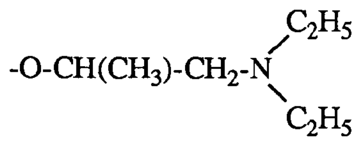 | H | 0 | $-CO-R_3$ | |
| 2.023 | F | Cl | $-NH_2$ | H | 0 | $-CO-R_3$ | |
| 2.024 | F | Cl | 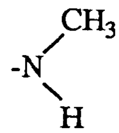 | H | 0 | $-CO-R_3$ | |
| 2.025 | F | Cl | 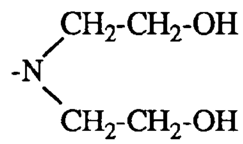 | H | 0 | $-CO-R_3$ | |
| 2.026 | F | Cl | $-NH-CH_2-CH=CH_2$ | H | 0 | $-CO-R_3$ | |
| 2.027 | F | Cl | $-N-(CH_2-CH=CH_2)_2$ | H | 0 | $-CO-R_3$ | |
| 2.028 | F | Cl | 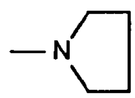 | H | 0 | $-CO-R_3$ | |
| 2.029 | F | Cl | 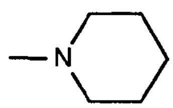 | H | 0 | $-CO-R_3$ | |
| 2.030 | F | Cl | 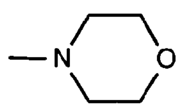 | H | 0 | $-CO-R_3$ | |
| 2.031 | F | Cl | 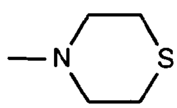 | H | 0 | $-CO-R_3$ | |
| 2.032 | F | Cl | 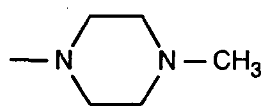 | H | 0 | $-CO-R_3$ | |
| 2.033 | F | Cl | 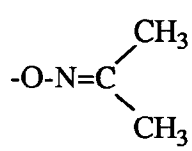 | H | 0 | $-CO-R_3$ | |
| 2.034 | F | Cl | $-O-CH_2-CH_2-Cl$ | H | 0 | $-CO-R_3$ | |
| 2.035 | F | Cl | $-O-CH_2-CN$ | H | 0 | $-CO-R_3$ | |
| 2.036 | F | Cl | 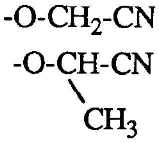 | H | 0 | $-CO-R_3$ | |
| 2.037 | F | Cl | $-O-CH_2-CH=CH_2$ | H | 0 | $-CO-R_3$ | |

Tabelle 2 (Fortsetzung)

| Verb. Nr. | $R_1$ | $R_2$ | $R_3$ | $R_4$ | n | A | Smp. [°C] |
|---|---|---|---|---|---|---|---|
| 2.038 | F | Cl | $-O-CH_2-CH=CHCl$ | H | 0 | $-CO-R_3$ | |
| 2.039 | F | Cl | 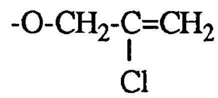 | H | 0 | $-CO-R_3$ | |
| 2.040 | F | Cl | $-O-CH_2=C\equiv CH$ | H | 0 | $-CO-R_3$ | |
| 2.041 | F | Cl | 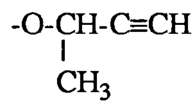 | H | 0 | $-CO-R_3$ | |
| 2.042 | F | Cl | 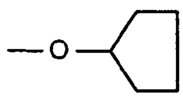 | H | 0 | $-CO-R_3$ | |
| 2.043 | F | Cl | 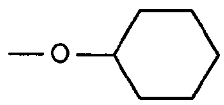 | H | 0 | $-CO-R_3$ | |
| 2.044 | F | Cl | 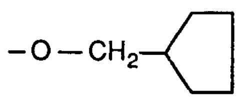 | H | 0 | $-CO-R_3$ | |
| 2.045 | F | Cl | 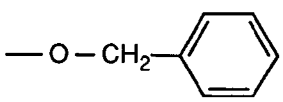 | H | 0 | $-CO-R_3$ | |
| 2.046 | F | Cl | 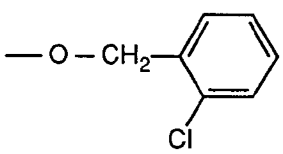 | H | 0 | $-CO-R_3$ | |
| 2.047 | F | Cl | 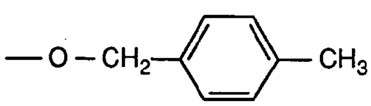 | H | 0 | $-CO-R_3$ | |
| 2.048 | F | Cl | $-S-CH_3$ | H | 0 | $-CO-R_3$ | |
| 2.049 | F | Cl | $-S-C_2H_5$ | H | 0 | $-CO-R_3$ | |
| 2.050 | F | Cl | $-S-C_3H_7$ | H | 0 | $-CO-R_3$ | |
| 2.051 | F | Cl | $-S-CH_2-CH=CH_2$ | H | 0 | $-CO-R_3$ | |
| 2.052 | F | Cl | $-S-CH_2-COOCH_3$ | H | 0 | $-CO-R_3$ | |
| 2.053 | F | Cl | $-S-CH_2-COOC_2H_5$ | H | 0 | $-CO-R_3$ | |
| 2.054 | F | Cl | $-S-CH_2-COOC_5H_{11}$ | H | 0 | $-CO-R_3$ | |
| 2.055 | F | Cl | $-S-CH(CH_3)-COOCH_3$ | H | 0 | $-CO-R_3$ | |
| 2.056 | F | Cl | $-S-(CH_3)-COOC_2H_5$ | H | 0 | $-CO-R_3$ | |

Tabelle 2 (Fortsetzung)

| Verb. Nr. | $R_1$ | $R_2$ | $R_3$ | $R_4$ | n | A | Smp. [°C] |
|---|---|---|---|---|---|---|---|
| 2.057 | F | Cl | $-S-CH(CH_3)-COOC_3H_7$ | H | 0 | $-CO-R_3$ | |
| 2.058 | F | Cl | $-S-CH_2-CH_2-COOCH_3$ | H | 0 | $-CO-R_3$ | |
| 2.059 | F | Cl | $-S-CH_2-COOCH_2-CH_2-O-CH_3$ | H | 0 | $-CO-R_3$ | |
| 2.060 | F | Cl | $-O-CH_2-COOCH_3$ | H | 0 | $-CO-R_3$ | |
| 2.061 | F | Cl | $-O-CH(CH_3)-COOCH_3$ | H | 0 | $-CO-R_3$ | |
| 2.062 | F | Cl | $-O-CH_2-COOC_5H_{11}$ | H | 0 | $-CO-R_3$ | |
| 2.063 | F | Cl | $-O-CH_2-CH_3-Si(CH_3)_3$ | H | 0 | $-CO-R_3$ | |
| 2.064 | F | Cl | -ONa | H | 0 | $-CO-R_3$ | |
| 2.065 | F | Br | -Cl | H | 0 | $-CO-R_3$ | |
| 2.066 | F | Br | -OH | H | 0 | $-CO-R_3$ | |
| 2.067 | F | Br | $-OCH_3$ | H | 0 | $-CO-R_3$ | |
| 2.068 | F | Br | $-OC_2H_5$ | H | 0 | $-CO-R_3$ | |
| 2.069 | F | Br | $-OC_3H_7$ | H | 0 | $-CO-R_3$ | |
| 2.070 | F | Br | 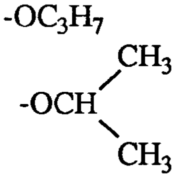 | H | 0 | $-CO-R_3$ | |
| 2.071 | F | Br | $-OC_4H_9$ | H | 0 | $-CO-R_3$ | |
| 2.072 | F | Br | $-OCH(CH_3)-CH_2-CH_3$ | H | 0 | $-CO-R_3$ | |
| 2.073 | F | Br | 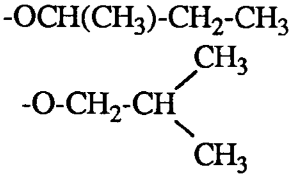 | H | 0 | $-CO-R_3$ | |
| 2.074 | F | Br | $-O-C_5H_{11}$ | H | 0 | $-CO-R_3$ | |
| 2.075 | F | Br | $-O-CH_2-CH_2-O-CH_3$ | H | 0 | $-CO-R_3$ | |
| 2.076 | F | Br | $-O-CH_2-CH_2-O-C_2H_5$ | H | 0 | $-CO-R_3$ | |
| 2.077 | F | Br | $-O-CH(CH_3)-CH_2-O-CH_3$ | H | 0 | $-CO-R_3$ | |
| 2.078 | F | Br | $-O-CH_2-CH_2-S-CH_3$ | H | 0 | $-CO-R_3$ | |
| 2.079 | F | Br | $-O-CH_2(CH_3)-S-CH_3$ | H | 0 | $-CO-R_3$ | |
| 2.080 | F | Br | $-O-CH-(CH_3)-S-C_2H_5$ | H | 0 | $-CO-R_3$ | |
| 2.081 | F | Br | $-O-CH(CH_3)-S-C_3H_7$ | H | 0 | $-CO-R_3$ | |
| 2.082 | F | Br | 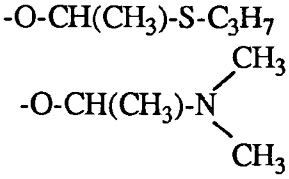 | H | 0 | $-CO-R_3$ | |
| 2.083 | F | Br | $-NH_2$ | H | 0 | $-CO-R_3$ | |

Tabelle 2 (Fortsetzung)

| Verb. Nr. | $R_1$ | $R_2$ | $R_3$ | $R_4$ | n | A | Smp. [°C] |
|---|---|---|---|---|---|---|---|
| 2.084 | F | Br | 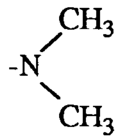 | H | 0 | $-CO-R_3$ | |
| 2.085 | F | Br | 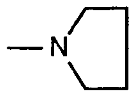 | H | 0 | $-CO-R_3$ | |
| 2.086 | F | Br | 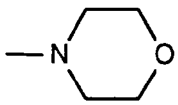 | H | 0 | $-CO-R_3$ | |
| 2.087 | F | Br | 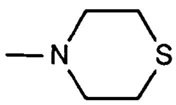 | H | 0 | $-CO-R_3$ | |
| 2.088 | F | Br | 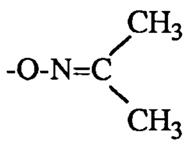 | H | 0 | $-CO-R_3$ | |
| 2.089 | F | Br | $-O-CH_2-CH_2-Cl$ | H | 0 | $-CO-R_3$ | |
| 2.090 | F | Br | $-O-CH_2-CN$ | H | 0 | $-CO-R_3$ | |
| 2.091 | F | Br | $-O-CH_2-CH=CH_2$ | H | 0 | $-CO-R_3$ | |
| 2.092 | F | Br | $-O-CH_2-C\equiv CH$ | H | 0 | $-CO-R_3$ | |
| 2.093 | F | Br | 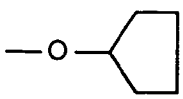 | H | 0 | $-CO-R_3$ | |
| 2.094 | F | Br | 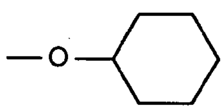 | H | 0 | $-CO-R_3$ | |
| 2.095 | F | Br | 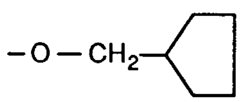 | H | 0 | $-CO-R_3$ | |
| 2.096 | F | Br | 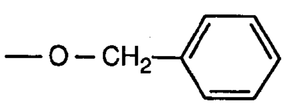 | H | 0 | $-CO-R_3$ | |
| 2.097 | F | Br | $-SCH_3$ | H | 0 | $-CO-R_3$ | |
| 2.098 | F | Br | $-S-CH_2-COOCH_3$ | H | 0 | $-CO-R_3$ | |
| 2.099 | F | Br | $-S-CH(CH_3)-COOCH_3$ | H | 0 | $-CO-R_3$ | |
| 2.100 | F | Br | $-O-CH_2-COOCH_3$ | H | 0 | $-CO-R_3$ | |

Tabelle 2 (Fortsetzung)

| Verb. Nr. | $R_1$ | $R_2$ | $R_3$ | $R_4$ | n | A | Smp. [°C] |
|---|---|---|---|---|---|---|---|
| 2.101 | F | Br | $-O-CH(CH_3)COOCH_3$ | H | 0 | $-CO-R_3$ | |
| 2.102 | F | CN | -Cl | H | 0 | $-CO-R_3$ | |
| 2.103 | F | CN | -OH | H | 0 | $-CO-R_3$ | |
| 2.104 | F | CN | $-OCH_3$ | H | 0 | $-CO-R_3$ | |
| 2.105 | H | Cl | -Cl | H | 0 | $-CO-R_3$ | |
| 2.106 | H | Cl | -OH | H | 0 | $-CO-R_3$ | |
| 2.107 | H | Cl | $-OCH_3$ | H | 0 | $-CO-R_3$ | |
| 2.108 | H | Cl | $-OC_2H_5$ | H | 0 | $-CO-R_3$ | |
| 2.109 | H | Cl | 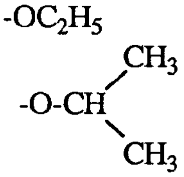 | H | 0 | $-CO-R_3$ | |
| 2.110 | H | Cl | $-O-CH_2-COOCH_3$ | H | 0 | $-CO-R_3$ | |
| 2.111 | H | Cl | $-O-CH(CH_3)COOCH_3$ | H | 0 | $-CO-R_3$ | |
| 2.112 | H | Cl | $-S-CH_2-COOCH_3$ | H | 0 | $-CO-R_3$ | |
| 2.113 | H | Cl | $-S-CH(CH_3)COOCH_3$ | H | 0 | $-CO-R_3$ | |
| 2.114 | H | Cl | 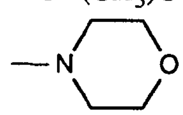 | H | 0 | $-CO-R_3$ | |
| 2.115 | F | Cl | -Cl | H | 1 | $-CO-R_3$ | |
| 2.116 | F | Cl | -OH | H | 1 | $-CO-R_3$ | |
| 2.117 | F | Cl | $-OCH_3$ | H | 1 | $-CO-R_3$ | 107-108 |
| 2.118 | F | Cl | $-OC_2H_5$ | H | 1 | $-CO-R_3$ | |
| 2.119 | F | Cl | $-OC_3H_7$ | H | 1 | $-CO-R_3$ | |
| 2.120 | F | Cl | 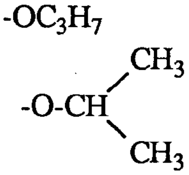 | H | 1 | $-CO-R_3$ | |
| 2.121 | F | Cl | $-OC_4H_9$ | H | 1 | $-CO-R_3$ | |
| 2.122 | F | Cl | $-O-CH(CH_3)-C_2H_5$ | H | 1 | $-CO-R_3$ | |
| 2.123 | F | Cl | 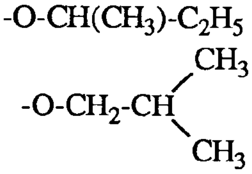 | H | 1 | $-CO-R_3$ | |
| 2.124 | F | Cl | $-OC_5H_{11}$ | H | 1 | $-CO-R_3$ | |
| 2.125 | F | Cl | $-O-CH_2-CH_2-O-CH_3$ | H | 1 | $-CO-R_3$ | |
| 2.126 | F | Cl | $-O-CH_2-CH_2-O-C_2H_5$ | H | 1 | $-CO-R_3$ | |

Tabelle 2 (Fortsetzung)

| Verb. Nr. | $R_1$ | $R_2$ | $R_3$ | $R_4$ | n | A | Smp. [°C] |
|---|---|---|---|---|---|---|---|
| 2.127 | F | Cl | $-O-CH-(CH_3)-CH_2-O-CH_3$ | H | 1 | $-CO-R_3$ | |
| 2.128 | F | Cl | $-O-CH_2-CH_2-S-CH_3$ | H | 1 | $-CO-R_3$ | |
| 2.129 | F | Cl | $-O-CH(CH_3)-CH_2-S-CH_3$ | H | 1 | $-CO-R_3$ | |
| 2.130 | F | Cl | $-O-CH(CH_3)-CH_2-S-C_2H_5$ | H | 1 | $-CO-R_3$ | |
| 2.131 | F | Cl | $-O-CH(CH_3)-CH_2-S-C_3H_7$ | H | 1 | $-CO-R_3$ | |
| 2.132 | F | Cl | 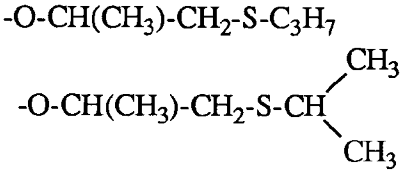 | H | 1 | $-CO-R_3$ | |
| 2.133 | F | Cl | $-O-CH(CH_3)-CH_2-S-C_4H_9$ | H | 1 | $-CO-R_3$ | |
| 2.134 | F | Cl | $-O-CH(CH_3)-CH_2-S-C_5H_{11}$ | H | 1 | $-CO-R_3$ | |
| 2.135 | F | Cl | 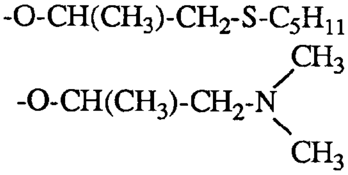 | H | 1 | $-CO-R_3$ | |
| 2.136 | F | Cl | 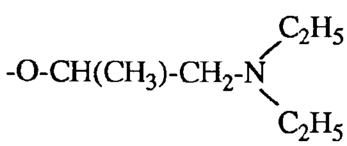 | H | 1 | $-CO-R_3$ | |
| 2.137 | F | Cl | $-NH_2$ | H | 1 | $-CO-R_3$ | |
| 2.138 | F | Cl | 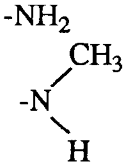 | H | 1 | $-CO-R_3$ | |
| 2.139 | F | Cl | 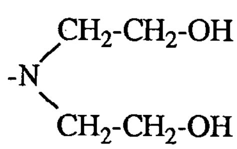 | H | 1 | $-CO-R_3$ | |
| 2.140 | F | Cl | $-NH-CH_2-CH=CH_2$ | H | 1 | $-CO-R_3$ | |
| 2.141 | F | Cl | $-N-(CH_2-CH=CH_2)_2$ | H | 1 | $-CO-R_3$ | |
| 2.142 | F | Cl | 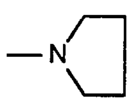 | H | 1 | $-CO-R_3$ | |
| 2.143 | F | Cl | 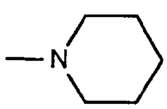 | H | 1 | $-CO-R_3$ | |

Tabelle 2 (Fortsetzung)

| Verb. Nr. | $R_1$ | $R_2$ | $R_3$ | $R_4$ | n | A | Smp. [°C] |
|---|---|---|---|---|---|---|---|
| 2.144 | F | Cl | 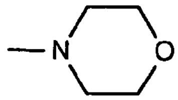 | H | 1 | $-CO-R_3$ | |
| 2.145 | F | Cl | 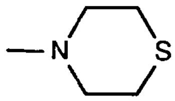 | H | 1 | $-CO-R_3$ | |
| 2.146 | F | Cl | 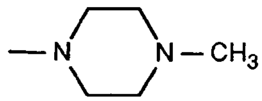 | H | 1 | $-CO-R_3$ | |
| 2.147 | F | Cl | 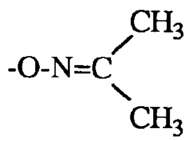 | H | 1 | $-CO-R_3$ | |
| 2.148 | F | Cl | $-O-CH_2-CH_2-Cl$ | H | 1 | $-CO-R_3$ | |
| 2.149 | F | Cl | $-O-CH_2-CN$ | H | 1 | $-CO-R_3$ | |
| 2.150 | F | Cl | 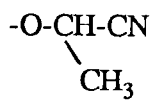 | H | 1 | $-CO-R_3$ | |
| 2.151 | F | Cl | $-O-CH_2-CH=CH_2$ | H | 1 | $-CO-R_3$ | |
| 2.152 | F | Cl | $-O-CH_2-CH=CHCl$ | H | 1 | $-CO-R_3$ | |
| 2.153 | F | Cl | 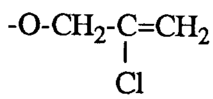 | H | 1 | $-CO-R_3$ | |
| 2.154 | F | Cl | $-O-CH_2=C \equiv CH$ | H | 1 | $-CO-R_3$ | |
| 2.155 | F | Cl | 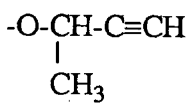 | H | 1 | $-CO-R_3$ | |
| 2.156 | F | Cl | 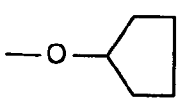 | H | 1 | $-CO-R_3$ | |
| 2.157 | F | Cl | 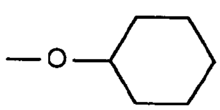 | H | 1 | $-CO-R_3$ | |
| 2.158 | F | Cl | 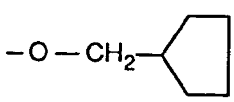 | H | 1 | $-CO-R_3$ | |
| 2.159 | F | Cl | 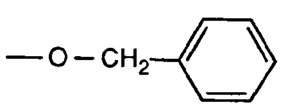 | H | 1 | $-CO-R_3$ | |

Tabelle 2 (Fortsetzung)

| Verb. Nr. | $R_1$ | $R_2$ | $R_3$ | $R_4$ | n | A | Smp. [°C] |
|---|---|---|---|---|---|---|---|
| 2.160 | F | Cl | 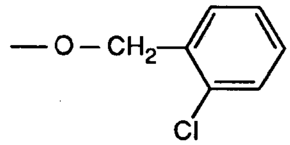 | H | 1 | $-CO-R_3$ | |
| 2.161 | F | Cl | 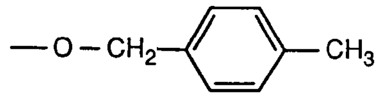 | H | 1 | $-CO-R_3$ | |
| 2.162 | F | Cl | $-S-CH_3$ | H | 1 | $-CO-R_3$ | |
| 2.163 | F | Cl | $-S-C_2H_5$ | H | 1 | $-CO-R_3$ | |
| 2.164 | F | Cl | $-S-C_3H_7$ | H | 1 | $-CO-R_3$ | |
| 2.165 | F | Cl | $-S-CH_2-CH=CH_2$ | H | 1 | $-CO-R_3$ | |
| 2.166 | F | Cl | $-S-CH_2-COOCH_3$ | H | 1 | $-CO-R_3$ | |
| 2.167 | F | Cl | $-S-CH_2-COOC_2H_5$ | H | 1 | $-CO-R_3$ | |
| 2.168 | F | Cl | $-S-CH_2-COOC_5H_{11}$ | H | 1 | $-CO-R_3$ | |
| 2.169 | F | Cl | $-S-CH(CH_3)-COOCH_3$ | H | 1 | $-CO-R_3$ | |
| 2.170 | F | Cl | $-S-(CH_3)-COOC_2H_5$ | H | 1 | $-CO-R_3$ | |
| 2.171 | F | Cl | $-S-CH(CH_3)-COOC_3H_7$ | H | 1 | $-CO-R_3$ | |
| 2.172 | F | Cl | $-S-CH_2-CH_2-COOCH_3$ | H | 1 | $-CO-R_3$ | |
| 2.173 | F | Cl | $-S-CH_2-COOCH_2-CH_2-O-CH_3$ | H | 1 | $-CO-R_3$ | |
| 2.174 | F | Cl | $-O-CH_2-COOCH_3$ | H | 1 | $-CO-R_3$ | |
| 2.175 | F | Cl | $-O-CH(CH_3)-COOCH_3$ | H | 1 | $-CO-R_3$ | |
| 2.176 | F | Cl | $-O-CH_2-COOC_5H_{11}$ | H | 1 | $-CO-R_3$ | |
| 2.177 | F | Cl | $-O-CH_2-CH_3-Si(CH_3)_3$ | H | 1 | $-CO-R_3$ | |
| 2.178 | F | Cl | -ONa | H | 1 | $-CO-R_3$ | |
| 2.179 | F | Br | -Cl | H | 1 | $-CO-R_3$ | |
| 2.180 | F | Br | -OH | H | 1 | $-CO-R_3$ | |
| 2.181 | F | Br | $-OCH_3$ | H | 1 | $-CO-R_3$ | |
| 2.182 | F | Br | $-OC_2H_5$ | H | 1 | $-CO-R_3$ | |
| 2.183 | F | Br | $-OC_3H_7$ | H | 1 | $-CO-R_3$ | |
| 2.184 | F | Br | 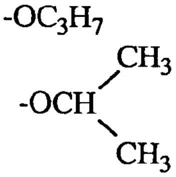 | H | 1 | $-CO-R_3$ | |
| 2.185 | F | Br | $-OC_4H_9$ | H | 1 | $-CO-R_3$ | |

Tabelle 2 (Fortsetzung)

| Verb. Nr. | $R_1$ | $R_2$ | $R_3$ | $R_4$ | n | A | Smp. [°C] |
|---|---|---|---|---|---|---|---|
| 2.186 | F | Br | $-OCH(CH_3)-CH_2-CH_3$ | H | 1 | $-CO-R_3$ | |
| 2.187 | F | Br | 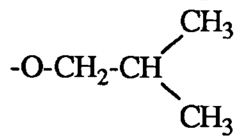 | H | 1 | $-CO-R_3$ | |
| 2.188 | F | Br | $-O-C_5H_{11}$ | H | 1 | $-CO-R_3$ | |
| 2.189 | F | Br | $-O-CH_2-CH_2-O-CH_3$ | H | 1 | $-CO-R_3$ | |
| 2.190 | F | Br | $-O-CH_2-CH_2-O-C_2H_5$ | H | 1 | $-CO-R_3$ | |
| 2.191 | F | Br | $-O-CH(CH_3)-CH_2-O-CH_3$ | H | 1 | $-CO-R_3$ | |
| 2.192 | F | Br | $-O-CH_2-CH_2-S-CH_3$ | H | 1 | $-CO-R_3$ | |
| 2.193 | F | Br | $-O-CH_2(CH_3)-S-CH_3$ | H | 1 | $-CO-R_3$ | |
| 2.194 | F | Br | $-O-CH-(CH_3)-S-C_2H_5$ | H | 1 | $-CO-R_3$ | |
| 2.195 | F | Br | $-O-CH(CH_3)-S-C_3H_7$ | H | 1 | $-CO-R_3$ | |
| 2.196 | F | Br | 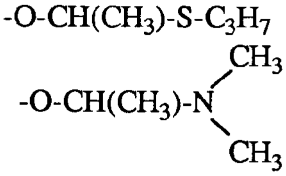 | H | 1 | $-CO-R_3$ | |
| 2.197 | F | Br | $-NH_2$ | H | 1 | $-CO-R_3$ | |
| 2.198 | F | Br | 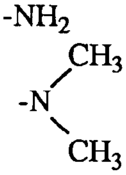 | H | 1 | $-CO-R_3$ | |
| 2.199 | F | Br | 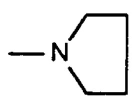 | H | 1 | $-CO-R_3$ | |
| 2.200 | F | Br | 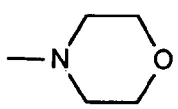 | H | 1 | $-CO-R_3$ | |
| 2.201 | F | Br | 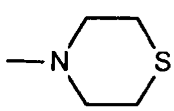 | H | 1 | $-CO-R_3$ | |
| 2.202 | F | Br | 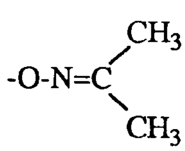 | H | 1 | $-CO-R_3$ | |
| 2.203 | F | Br | $-O-CH_2-CH_2-Cl$ | H | 1 | $-CO-R_3$ | |
| 2.204 | F | Br | $-O-CH_2-CN$ | H | 1 | $-CO-R_3$ | |
| 2.205 | F | Br | $-O-CH_2-CH=CH_2$ | H | 1 | $-CO-R_3$ | |
| 2.206 | F | Br | $-O-CH_2-C\equiv CH$ | H | 1 | $-CO-R_3$ | |

Tabelle 2 (Fortsetzung)

| Verb. Nr. | $R_1$ | $R_2$ | $R_3$ | $R_4$ | n | A | Smp. [°C] |
|---|---|---|---|---|---|---|---|
| 2.207 | F | Br | 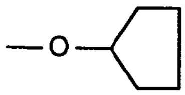 | H | 1 | $-CO-R_3$ | |
| 2.208 | F | Br | 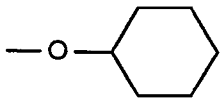 | H | 1 | $-CO-R_3$ | |
| 2.209 | F | Br | 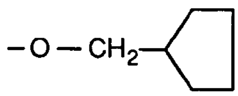 | H | 1 | $-CO-R_3$ | |
| 2.210 | F | Br | 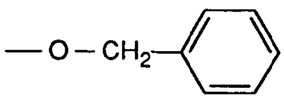 | H | 1 | $-CO-R_3$ | |
| 2.211 | F | Br | $-SCH_3$ | H | 1 | $-CO-R_3$ | |
| 2.212 | F | Br | $-S-CH_2-COOCH_3$ | H | 1 | $-CO-R_3$ | |
| 2.213 | F | Br | $-S-CH(CH_3)-COOCH_3$ | H | 1 | $-CO-R_3$ | |
| 2.214 | F | Br | $-O-CH_2-COOCH_3$ | H | 1 | $-CO-R_3$ | |
| 2.215 | F | Br | $-O-CH(CH_3)COOCH_3$ | H | 1 | $-CO-R_3$ | |
| 2.216 | F | CN | -Cl | H | 1 | $-CO-R_3$ | |
| 2.217 | F | CN | -OH | H | 1 | $-CO-R_3$ | |
| 2.218 | F | CN | $-OCH_3$ | H | 1 | $-CO-R_3$ | |
| 2.219 | H | Cl | -Cl | H | 1 | $-CO-R_3$ | |
| 2.220 | H | Cl | -OH | H | 1 | $-CO-R_3$ | |
| 2.221 | H | Cl | $-OCH_3$ | H | 1 | $-CO-R_3$ | |
| 2.222 | H | Cl | $-OC_2H_5$ | H | 1 | $-CO-R_3$ | |
| 2.223 | H | Cl | 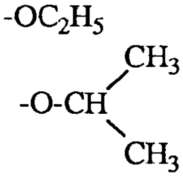 | H | 1 | $-CO-R_3$ | |
| 2.224 | H | Cl | $-O-CH_2-COOCH_3$ | H | 1 | $-CO-R_3$ | |
| 2.225 | H | Cl | $-O-CH(CH_3)COOCH_3$ | H | 1 | $-CO-R_3$ | |
| 2.226 | H | Cl | $-S-CH_2-COOCH_3$ | H | 1 | $-CO-R_3$ | |
| 2.227 | H | Cl | $-S-CH(CH_3)COOCH_3$ | H | 1 | $-CO-R_3$ | |
| 2.228 | H | Cl | 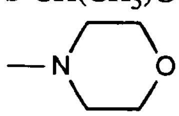 | H | 1 | $-CO-R_3$ | |
| 2.229 | F | Cl | Cl | H | 2 | $-CO-R_3$ | |

Tabelle 2 (Fortsetzung)

| Verb. Nr. | $R_1$ | $R_2$ | $R_3$ | $R_4$ | n | A | Smp. [°C] |
|---|---|---|---|---|---|---|---|
| 2.230 | F | Cl | OH | H | 2 | $-CO-R_3$ | |
| 2.231 | F | Cl | $OCH_3$ | H | 2 | $-CO-R_3$ | |
| 2.232 | F | Cl | $-OC_2H_5$ | H | 2 | $-CO-R_3$ | |
| 2.233 | F | Cl | 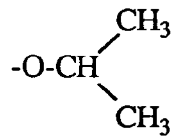 | H | 2 | $-CO-R_3$ | |
| 2.234 | F | Cl | $-O-CH_2-CH_2-O-CH_3$ | H | 2 | $-CO-R_3$ | |
| 2.235 | F | Cl | $-O-CH(CH_3)-CH_2-S-CH_3$ | H | 2 | $-CO-R_3$ | |
| 2.236 | F | Cl | $-O-CH_2-COOCH_3$ | H | 2 | $-CO-R_3$ | |
| 2.237 | F | Cl | $-S-CH_2-COOCH_3$ | H | 2 | $-CO-R_3$ | |
| 2.238 | F | Cl | $-CH_2-CH=CH_2$ | H | 2 | $-CO-R_3$ | |
| 2.239 | F | Cl | $-CH_2-C\equiv CH$ | H | 2 | $-CO-R_3$ | |
| 2.240 | F | Cl | Cl | H | 3 | $-CO-R_3$ | |
| 2.241 | F | Cl | OH | H | 3 | $-CO-R_3$ | |
| 2.242 | F | Cl | $OCH_3$ | H | 3 | $-CO-R_3$ | |
| 2.243 | F | Cl | $OC_2H_5$ | H | 3 | $-CO-R_3$ | |
| 2.244 | F | Cl | 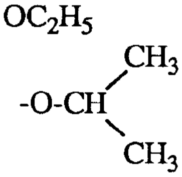 | H | 3 | $-CO-R_3$ | |
| 2.245 | F | Cl | $-O-CH_2-CH_2-O-CH$ | H | 3 | $-CO-R_3$ | |
| 2.246 | F | Cl | $-O-CH(CH_3)-CH_2-S-CH_3$ | H | 3 | $-CO-R_3$ | |
| 2.247 | F | Cl | $-O-CH_2-COOCH_3$ | H | 3 | $-CO-R_3$ | |
| 2.248 | F | Cl | $-S-CH_2-COOCH_3$ | H | 3 | $-CO-R_3$ | |
| 2.249 | F | Cl | $-O-CH_2-C\equiv CH$ | H | 3 | $-CO-R_3$ | |
| 2.250 | F | Cl | -Cl | Cl | 0 | $-CO-R_3$ | |
| 2.251 | F | Cl | -OH | Cl | 0 | $-CO-R_3$ | |
| 2.252 | F | Cl | $-OCH_3$ | Cl | 0 | $-CO-R_3$ | |
| 2.253 | F | Cl | $-OC_2H_5$ | Cl | 0 | $-CO-R_3$ | |
| 2.254 | F | Cl | 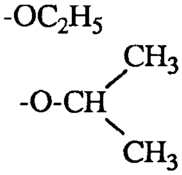 | Cl | 0 | $-CO-R_3$ | |
| 2.255 | F | Cl | $-O-CH_2-COOCH_3$ | Cl | 0 | $-CO-R_3$ | |
| 2.256 | F | Cl | $-S-CH_2-COOCH_3$ | Cl | 0 | $-CO-R_3$ | |
| 2.257 | F | Cl | $-OCH_3$ | Br | 0 | $-CO-R_3$ | |

Tabelle 2 (Fortsetzung)

| Verb. Nr. | $R_1$ | $R_2$ | $R_3$ | $R_4$ | n | A | Smp. [°C] |
|---|---|---|---|---|---|---|---|
| 2.258 | F | Cl | 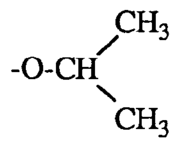 | Br | 0 | $-CO-R_3$ | |
| 2.259 | F | Cl | $-OCH_3$ | F | 0 | $-CO-R_3$ | |
| 2.260 | F | Cl | $-OCH_3$ | $CH_3$ | 0 | $-CO-R_3$ | |
| 2.261 | F | Cl | $-OC_2H_5$ | $CH_3$ | 0 | $-CO-R_3$ | |
| 2.262 | F | Cl | 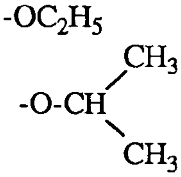 | $CH_3$ | 0 | $-CO-R_3$ | |
| 2.263 | F | Cl | $-O-CH_2-CH_2-O-CH_3$ | $CH_3$ | 0 | $-CO-R_3$ | |
| 2.264 | F | Cl | $-O-CH_2-COOCH_3$ | $CH_3$ | 0 | $-CO-R_3$ | |
| 2.265 | F | Cl | $-O-CH(CH_3)COOCH_3$ | $CH_3$ | 0 | $-CO-R_3$ | |
| 2.266 | F | Cl | $-S-CH_2-COOCH_3$ | $CH_3$ | 0 | $-CO-R_3$ | |
| 2.267 | F | Cl | $-OCH_3$ | $CF_3$ | 0 | $-CO-R_3$ | |
| 2.268 | F | Cl | $-OC_2H_5$ | $CF_3$ | 0 | $-CO-R_3$ | |
| 2.269 | F | Cl | 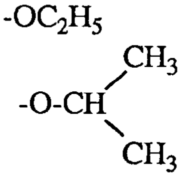 | $CF_3$ | 0 | $-CO-R_3$ | |
| 2.270 | F | Cl | $-O-CH_2-COOCH_3$ | $CF_3$ | 0 | $-CO-R_3$ | |
| 2.271 | F | Cl | $-S-CH_2-COOCH_3$ | $CF_3$ | 0 | $-CO-R_3$ | |
| 2.272 | F | Cl | - | H | 0 | -CN | |
| 2.273 | F | Cl | - | H | 1 | -CN | |
| 2.274 | F | Cl | - | H | 2 | -CN | |
| 2.275 | F | Cl | - | H | 3 | -CN | |
| 2.276 | F | Cl | - | H | 4 | -CN | |

Tabelle 3:

Verbindungen der Formel I mit W = $W_3$ und $R_{14}$ = H:

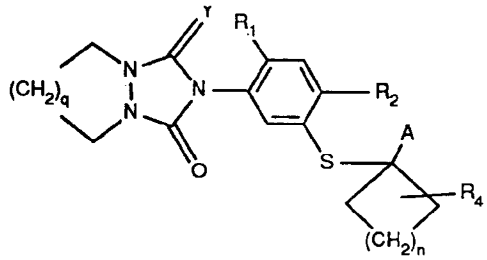

(I)

Tabelle 3

| Verb. Nr. | $R_1$ | $R_2$ | $R_3$ | $R_4$ | n | A | Y | q | Smp. [°C] |
|---|---|---|---|---|---|---|---|---|---|
| 3.001 | F | Cl | -Cl | H | 0 | $-CO-R_3$ | O | 1 | |
| 3.002 | F | Cl | -OH | H | 0 | $-CO-R_3$ | O | 2 | |
| 3.003 | F | Cl | $-OCH_3$ | H | 0 | $-CO-R_3$ | O | 2 | |
| 3.004 | F | Cl | $-OC_2H_5$ | H | 0 | $-CO-R_3$ | O | 2 | |
| 3.005 | F | Cl | $-OC_3H_7$ | H | 0 | $-CO-R_3$ | O | 2 | |
| 3.006 | F | Cl | 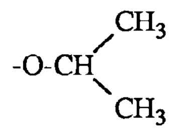 | H | 0 | $-CO-R_3$ | O | 2 | |
| 3.007 | F | Cl | $-OC_4H_9$ | H | 0 | $-CO-R_3$ | O | 2 | |
| 3.008 | F | Cl | $-O-CH(CH_3)-C_2H_5$ | H | 0 | $-CO-R_3$ | O | 2 | |
| 3.009 | F | Cl | 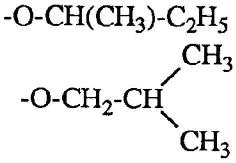 | H | 0 | $-CO-R_3$ | O | 2 | |
| 3.010 | F | Cl | $-OC_5H_{11}$ | H | 0 | $-CO-R_3$ | O | 2 | |
| 3.011 | F | Cl | $-O-CH_2-CH_2-O-CH_3$ | H | 0 | $-CO-R_3$ | O | 2 | |
| 3.012 | F | Cl | $-O-CH_2-CH_2-O-C_2H_5$ | H | 0 | $-CO-R_3$ | O | 2 | |
| 3.013 | F | Cl | $-O-CH-(CH_3)-CH_2-O-CH_3$ | H | 0 | $-CO-R_3$ | O | 2 | |
| 3.014 | F | Cl | $-O-CH_2-CH_2-S-CH_3$ | H | 0 | $-CO-R_3$ | O | 2 | |
| 3.015 | F | Cl | $-O-CH(CH_3)-CH_2-S-CH_3$ | H | 0 | $-CO-R_3$ | O | 2 | |
| 3.016 | F | Cl | $-O-CH(CH_3)-CH_2-S-C_2H_5$ | H | 0 | $-CO-R_3$ | O | 2 | |
| 3.017 | F | Cl | $-O-CH(CH_3)-CH_2-S-C_3H_7$ | H | 0 | $-CO-R_3$ | O | 2 | |
| 3.018 | F | Cl | 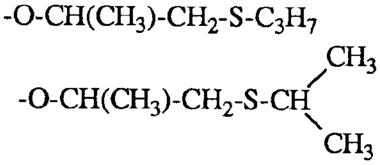 | H | 0 | $-CO-R_3$ | O | 2 | |
| 3.019 | F | Cl | $-O-CH(CH_3)-CH_2-S-C_4H_9$ | H | 0 | $-CO-R_3$ | O | 2 | |
| 3.020 | F | Cl | $-O-CH(CH_3)-CH_2-S-C_5H_{11}$ | H | 0 | $-CO-R_3$ | O | 2 | |
| 3.021 | F | Cl | 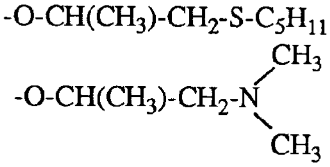 | H | 0 | $-CO-R_3$ | O | 2 | |

Tabelle 3 (Fortsetzung)

| Verb. Nr. | $R_1$ | $R_2$ | $R_3$ | $R_4$ | n | A | Y | q | Smp. [°C] |
|---|---|---|---|---|---|---|---|---|---|
| 3.022 | F | Cl | 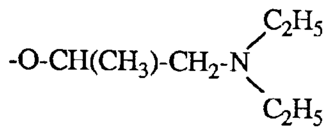 | H | 0 | $-CO-R_3$ | O | 1 | |
| 3.023 | F | Cl | $-NH_2$ | H | 0 | $-CO-R_3$ | O | 2 | |
| 3.024 | F | Cl | 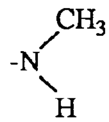 | H | 0 | $-CO-R_3$ | O | 2 | |
| 3.025 | F | Cl | 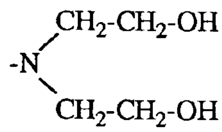 | H | 0 | $-CO-R_3$ | O | 2 | |
| 3.026 | F | Cl | $-NH-CH_2-CH=CH_2$ | H | 0 | $-CO-R_3$ | O | 2 | |
| 3.027 | F | Cl | $-N-(CH_2-CH=CH_2)_2$ | H | 0 | $-CO-R_3$ | O | 2 | |
| 3.028 | F | Cl | 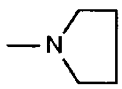 | H | 0 | $-CO-R_3$ | O | 2 | |
| 3.029 | F | Cl | 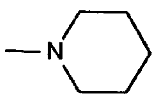 | H | 0 | $-CO-R_3$ | O | 2 | |
| 3.030 | F | Cl | 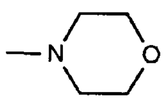 | H | 0 | $-CO-R_3$ | O | 2 | |
| 3.031 | F | Cl | 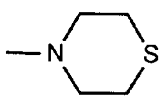 | H | 0 | $-CO-R_3$ | O | 2 | |
| 3.032 | F | Cl | 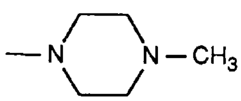 | H | 0 | $-CO-R_3$ | O | 2 | |
| 3.033 | F | Cl | 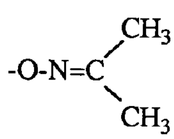 | H | 0 | $-CO-R_3$ | O | 2 | |
| 3.034 | F | Cl | $-O-CH_2-CH_2-Cl$ | H | 0 | $-CO-R_3$ | O | 2 | |
| 3.035 | F | Cl | $-O-CH_2-CN$ | H | 0 | $-CO-R_3$ | O | 2 | |
| 3.036 | F | Cl | 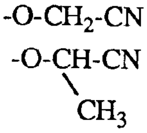 | H | 0 | $-CO-R_3$ | O | 2 | |
| 3.037 | F | Cl | $-O-CH_2-CH=CH_2$ | H | 0 | $-CO-R_3$ | O | 2 | |

Tabelle 3 (Fortsetzung)

| Verb. Nr. | $R_1$ | $R_2$ | $R_3$ | $R_4$ | n | A | Y | q | Smp. [°C] |
|---|---|---|---|---|---|---|---|---|---|
| 3.038 | F | Cl | $-O-CH_2-CH=CHCl$ | H | 0 | $-CO-R_3$ | O | 2 | |
| 3.039 | F | Cl | $-O-CH_2-C=CH_2$<br>I<br>Cl | H | 0 | $-CO-R_3$ | O | 2 | |
| 3.040 | F | Cl | $-O-CH_2=C\equiv CH$ | H | 0 | $-CO-R_3$ | O | 2 | |
| 3.041 | F | Cl | $-O-CH-C\equiv CH$<br>I<br>$CH_3$ | H | 0 | $-CO-R_3$ | O | 2 | |
| 3.042 | F | Cl | 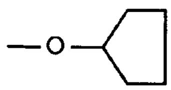 | H | 0 | $-CO-R_3$ | O | 2 | |
| 3.043 | F | Cl | 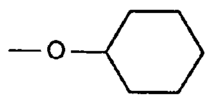 | H | 0 | $-CO-R_3$ | O | 2 | |
| 3.044 | F | Cl | 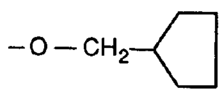 | H | 0 | $-CO-R_3$ | O | | |
| 3.045 | F | Cl | 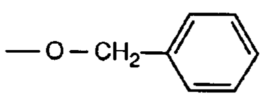 | H | 0 | $-CO-R_3$ | O | 2 | |
| 3.046 | F | Cl | 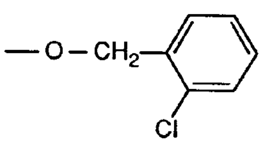 | H | 0 | $-CO-R_3$ | O | 2 | |
| 3.047 | F | Cl | 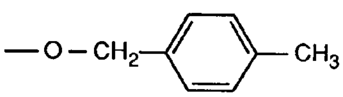 | H | 0 | $-CO-R_3$ | O | 2 | |
| 3.048 | F | Cl | $-S-CH_3$ | H | 0 | $-CO-R_3$ | O | 2 | |
| 3.049 | F | Cl | $-S-C_2H_5$ | H | 0 | $-CO-R_3$ | O | 2 | |
| 3.050 | F | Cl | $-S-C_3H_7$ | H | 0 | $-CO-R_3$ | O | 2 | |
| 3.051 | F | Cl | $-S-CH_2-CH=CH_2$ | H | 0 | $-CO-R_3$ | O | 2 | |
| 3.052 | F | Cl | $-S-CH_2-COOCH_3$ | H | 0 | $-CO-R_3$ | O | 2 | |
| 3.053 | F | Cl | $-S-CH_2-COOC_2H_5$ | H | 0 | $-CO-R_3$ | O | 2 | |
| 3.054 | F | Cl | $-S-CH_2-COOC_5H_{11}$ | H | 0 | $-CO-R_3$ | O | 2 | |
| 3.055 | F | Cl | $-S-CH(CH_3)-COOCH_3$ | H | 0 | $-CO-R_3$ | O | 2 | |
| 3.056 | F | Cl | $-S-(CH_3)-COOC_2H_5$ | H | 0 | $-CO-R_3$ | O | 2 | |

Tabelle 3 (Fortsetzung)

| Verb. Nr. | $R_1$ | $R_2$ | $R_3$ | $R_4$ | n | A | Y | q | Smp. [°C] |
|---|---|---|---|---|---|---|---|---|---|
| 3.057 | F | Cl | $-S-CH(CH_3)-COOC_3H_7$ | H | 0 | $-CO-R_3$ | O | 2 | |
| 3.058 | F | Cl | $-S-CH_2-CH_2-COOCH_3$ | H | 0 | $-CO-R_3$ | O | 2 | |
| 3.059 | F | Cl | $-S-CH_2-COOCH_2-CH_2-O-CH_3$ | H | 0 | $-CO-R_3$ | O | 2 | |
| 3.060 | F | Cl | $-O-CH_2-COOCH_3$ | H | 0 | $-CO-R_3$ | O | 2 | |
| 3.061 | F | Cl | $-O-CH(CH_3)-COOCH_3$ | H | 0 | $-CO-R_3$ | O | 2 | |
| 3.062 | F | Cl | $-O-CH_2-COOC_5H_{11}$ | H | 0 | $-CO-R_3$ | O | 2 | |
| 3.063 | F | Cl | $-O-CH_2-CH_3-Si(CH_3)_3$ | H | 0 | $-CO-R_3$ | O | 2 | |
| 3.064 | F | Cl | -ONa | H | 0 | $-CO-R_3$ | O | 2 | |
| 3.065 | F | Br | -Cl | H | 0 | $-CO-R_3$ | O | 2 | |
| 3.066 | F | Br | -OH | H | 0 | $-CO-R_3$ | O | 2 | |
| 3.067 | F | Br | $-OCH_3$ | H | 0 | $-CO-R_3$ | O | 2 | |
| 3.068 | F | Br | $-OC_2H_5$ | H | 0 | $-CO-R_3$ | O | 2 | |
| 3.069 | F | Br | $-OC_3H_7$ | H | 0 | $-CO-R_3$ | O | 2 | |
| 3.070 | F | Br | 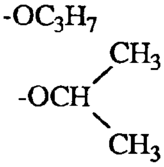 | H | 0 | $-CO-R_3$ | O | 2 | |
| 3.071 | F | Br | $-OC_4H_9$ | H | 0 | $-CO-R_3$ | O | 2 | |
| 3.072 | F | Br | $-OCH(CH_3)-CH_2-CH_3$ | H | 0 | $-CO-R_3$ | O | 2 | |
| 3.073 | F | Br | 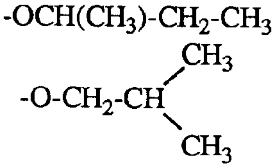 | H | 0 | $-CO-R_3$ | O | 2 | |
| 3.074 | F | Br | $-O-C_5H_{11}$ | H | 0 | $-CO-R_3$ | O | 2 | |
| 3.075 | F | Br | $-O-CH_2-CH_2-O-CH_3$ | H | 0 | $-CO-R_3$ | O | 2 | |
| 3.076 | F | Br | $-O-CH_2-CH_2-O-C_2H_5$ | H | 0 | $-CO-R_3$ | O | 2 | |
| 3.077 | F | Br | $-O-CH(CH_3)-CH_2-O-CH_3$ | H | 0 | $-CO-R_3$ | O | 2 | |
| 3.078 | F | Br | $-O-CH_2-CH_2-S-CH_3$ | H | 0 | $-CO-R_3$ | O | 2 | |
| 3.079 | F | Br | $-O-CH_2(CH_3)-S-CH_3$ | H | 0 | $-CO-R_3$ | O | 2 | |
| 3.080 | F | Br | $-O-CH-(CH_3)-S-C_2H_5$ | H | 0 | $-CO-R_3$ | O | 2 | |
| 3.081 | F | Br | $-O-CH(CH_3)-S-C_3H_7$ | H | 0 | $-CO-R_3$ | O | 2 | |
| 3.082 | F | Br | 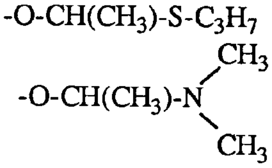 | H | 0 | $-CO-R_3$ | O | 2 | |
| 3.083 | F | Br | $-NH_2$ | H | 0 | $-CO-R_3$ | O | 2 | |

Tabelle 3 (Fortsetzung)

| Verb. Nr. | $R_1$ | $R_2$ | $R_3$ | $R_4$ | n | A | Y | q | Smp. [°C] |
|---|---|---|---|---|---|---|---|---|---|
| 3.084 | F | Br | 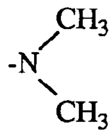 | H | 0 | $-CO-R_3$ | O | 2 | |
| 3.085 | F | Br | 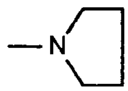 | H | 0 | $-CO-R_3$ | O | 2 | |
| 3.086 | F | Br | 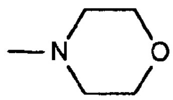 | H | 0 | $-CO-R_3$ | O | 2 | |
| 3.087 | F | Br | 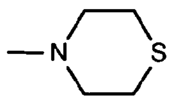 | H | 0 | $-CO-R_3$ | O | 2 | |
| 3.088 | F | Br | 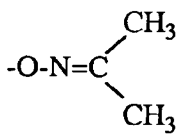 | H | 0 | $-CO-R_3$ | O | 2 | |
| 3.089 | F | Br | $-O-CH_2-CH_2-Cl$ | H | 0 | $-CO-R_3$ | O | 2 | |
| 3.090 | F | Br | $-O-CH_2-CN$ | H | 0 | $-CO-R_3$ | S | 2 | |
| 3.091 | F | Br | $-O-CH_2-CH=CH_2$ | H | 0 | $-CO-R_3$ | S | 2 | |
| 3.092 | F | Br | $-O-CH_2-C\equiv CH$ | H | 0 | $-CO-R_3$ | S | 2 | |
| 3.093 | F | Br | 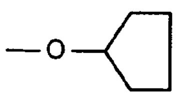 | H | 0 | $-CO-R_3$ | S | 2 | |
| 3.094 | F | Br | 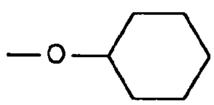 | H | 0 | $-CO-R_3$ | S | 2 | |
| 3.095 | F | Br | 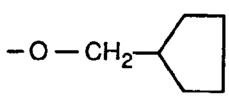 | H | 0 | $-CO-R_3$ | S | 2 | |
| 3.096 | F | Br | 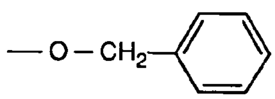 | H | 0 | $-CO-R_3$ | S | 2 | |
| 3.097 | F | Br | $-SCH_3$ | H | 0 | $-CO-R_3$ | S | 2 | |
| 3.098 | F | Br | $-S-CH_2-COOCH_3$ | H | 0 | $-CO-R_3$ | S | 2 | |
| 3.099 | F | Br | $-S-CH(CH_3)-COOCH_3$ | H | 0 | $-CO-R_3$ | S | 2 | |
| 3.100 | F | Br | $-O-CH_2-COOCH_3$ | H | 0 | $-CO-R_3$ | S | 2 | |

Tabelle 3 (Fortsetzung)

| Verb. Nr. | $R_1$ | $R_2$ | $R_3$ | $R_4$ | n | A | Y | q | Smp. [°C] |
|---|---|---|---|---|---|---|---|---|---|
| 3.101 | F | Br | $-O-CH(CH_3)COOCH_3$ | H | 0 | $-CO-R_3$ | S | 2 | |
| 3.102 | F | CN | -Cl | H | 0 | $-CO-R_3$ | S | 2 | |
| 3.103 | F | CN | -OH | H | 0 | $-CO-R_3$ | S | 2 | |
| 3.104 | F | CN | $-OCH_3$ | H | 0 | $-CO-R_3$ | S | 2 | |
| 3.105 | H | Cl | -Cl | H | 0 | $-CO-R_3$ | S | 2 | |
| 3.106 | H | Cl | -OH | H | 0 | $-CO-R_3$ | S | 2 | |
| 3.107 | H | Cl | $-OCH_3$ | H | 0 | $-CO-R_3$ | S | 2 | |
| 3.108 | H | Cl | $-OC_2H_5$ | H | 0 | $-CO-R_3$ | S | 2 | |
| 3.109 | H | Cl | 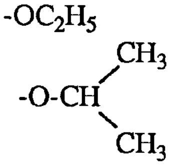 | H | 0 | $-CO-R_3$ | S | 2 | |
| 3.110 | H | Cl | $-O-CH_2-COOCH_3$ | H | 0 | $-CO-R_3$ | S | 2 | |
| 3.111 | H | Cl | $-O-CH(CH_3)COOCH_3$ | H | 0 | $-CO-R_3$ | S | 2 | |
| 3.112 | H | Cl | $-S-CH_2-COOCH_3$ | H | 0 | $-CO-R_3$ | S | 2 | |
| 3.113 | H | Cl | $-S-CH(CH_3)COOCH_3$ | H | 0 | $-CO-R_3$ | S | 2 | |
| 3.114 | H | Cl | 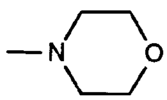 | H | 0 | $-CO-R_3$ | S | 2 | |
| 3.115 | F | Cl | -Cl | H | 1 | $-CO-R_3$ | O | 2 | |
| 3.116 | F | Cl | -OH | H | 1 | $-CO-R_3$ | O | 2 | |
| 3.117 | F | Cl | $-OCH_3$ | H | 1 | $-CO-R_3$ | O | 2 | |
| 3.118 | F | Cl | $-OC_2H_5$ | H | 1 | $-CO-R_3$ | O | 2 | |
| 3.119 | F | Cl | $-OC_3H_7$ | H | 1 | $-CO-R_3$ | O | 2 | |
| 3.120 | F | Cl | 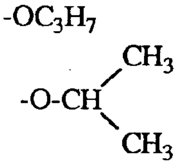 | H | 1 | $-CO-R_3$ | O | 2 | |
| 3.121 | F | Cl | $-OC_4H_9$ | H | 1 | $-CO-R_3$ | O | 2 | |
| 3.122 | F | Cl | $-O-CH(CH_3)-C_2H_5$ | H | 1 | $-CO-R_3$ | O | 2 | |
| 3.123 | F | Cl | 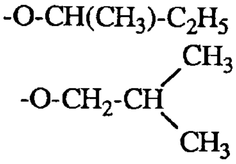 | H | 1 | $-CO-R_3$ | O | 2 | |
| 3.124 | F | Cl | $-OC_5H_{11}$ | H | 1 | $-CO-R_3$ | O | 2 | |
| 3.125 | F | Cl | $-O-CH_2-CH_2-O-CH_3$ | H | 1 | $-CO-R_3$ | O | 2 | |
| 3.126 | F | Cl | $-O-CH_2-CH_2-O-C_2H_5$ | H | 1 | $-CO-R_3$ | O | 2 | |

Tabelle 3 (Fortsetzung)

| Verb. Nr. | $R_1$ | $R_2$ | $R_3$ | $R_4$ | n | A | Y | q | Smp. [°C] |
|---|---|---|---|---|---|---|---|---|---|
| 3.127 | F | Cl | $-O-CH-(CH_3)-CH_2-O-CH_3$ | H | 1 | $-CO-R_3$ | O | 2 | |
| 3.128 | F | Cl | $-O-CH_2-CH_2-S-CH_3$ | H | 1 | $-CO-R_3$ | O | 2 | |
| 3.129 | F | Cl | $-O-CH(CH_3)-CH_2-S-CH_3$ | H | 1 | $-CO-R_3$ | O | 2 | |
| 3.130 | F | Cl | $-O-CH(CH_3)-CH_2-S-C_2H_5$ | H | 1 | $-CO-R_3$ | O | 2 | |
| 3.131 | F | Cl | $-O-CH(CH_3)-CH_2-S-C_3H_7$ | H | 1 | $-CO-R_3$ | O | 2 | |
| 3.132 | F | Cl | 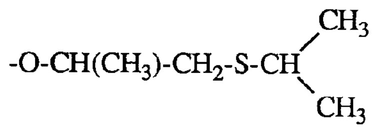 | H | 1 | $-CO-R_3$ | O | 2 | |
| 3.133 | F | Cl | $-O-CH(CH_3)-CH_2-S-C_4H_9$ | H | 1 | $-CO-R_3$ | O | 2 | |
| 3.134 | F | Cl | $-O-CH(CH_3)-CH_2-S-C_5H_{11}$ | H | 1 | $-CO-R_3$ | O | 2 | |
| 3.135 | F | Cl | 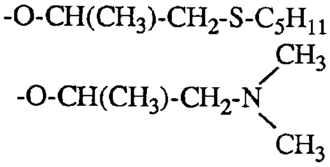 | H | 1 | $-CO-R_3$ | O | 2 | |
| 3.136 | F | Cl | 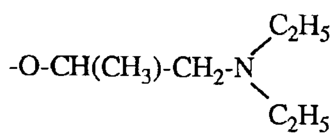 | H | 1 | $-CO-R_3$ | O | 2 | |
| 3.137 | F | Cl | $-NH_2$ | H | 1 | $-CO-R_3$ | O | 2 | |
| 3.138 | F | Cl | 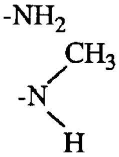 | H | 1 | $-CO-R_3$ | O | 2 | |
| 3.139 | F | Cl | 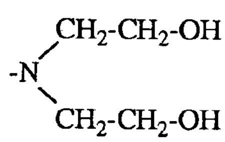 | H | 1 | $-CO-R_3$ | O | 2 | |
| 3.140 | F | Cl | $-NH-CH_2-CH=CH_2$ | H | 1 | $-CO-R_3$ | O | 2 | |
| 3.141 | F | Cl | $-N-(CH_2-CH=CH_2)_2$ | H | 1 | $-CO-R_3$ | O | 2 | |
| 3.142 | F | Cl | 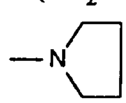 | H | 1 | $-CO-R_3$ | O | 2 | |
| 3.143 | F | Cl | 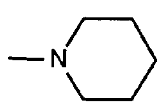 | H | 1 | $-CO-R_3$ | O | 2 | |

Tabelle 3 (Fortsetzung)

| Verb. Nr. | $R_1$ | $R_2$ | $R_3$ | $R_4$ | n | A | Y | q | Smp. [°C] |
|---|---|---|---|---|---|---|---|---|---|
| 3.144 | F | Cl | 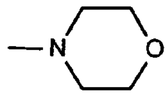 | H | 1 | $-CO-R_3$ | O | | |
| 3.145 | F | Cl | 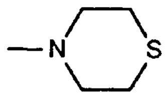 | H | 1 | $-CO-R_3$ | O | 2 | |
| 3.146 | F | Cl | 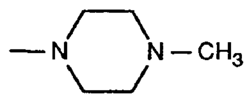 | H | 1 | $-CO-R_3$ | O | 2 | |
| 3.147 | F | Cl | $-O-N=C(CH_3)_2$ | H | 1 | $-CO-R_3$ | O | 2 | |
| 3.148 | F | Cl | $-O-CH_2-CH_2-Cl$ | H | 1 | $-CO-R_3$ | O | 2 | |
| 3.149 | F | Cl | $-O-CH_2-CN$ | H | 1 | $-CO-R_3$ | O | 2 | |
| 3.150 | F | Cl | $-O-CH(CH_3)-CN$ | H | 1 | $-CO-R_3$ | O | 2 | |
| 3.151 | F | Cl | $-O-CH_2-CH=CH_2$ | H | 1 | $-CO-R_3$ | O | 2 | |
| 3.152 | F | Cl | $-O-CH_2-CH=CHCl$ | H | 1 | $-CO-R_3$ | O | 2 | |
| 3.153 | F | Cl | $-O-CH_2-C(Cl)=CH_2$ | H | 1 | $-CO-R_3$ | O | 2 | |
| 3.154 | F | Cl | $-O-CH_2=C\equiv CH$ | H | 1 | $-CO-R_3$ | O | 2 | |
| 3.155 | F | Cl | $-O-CH(CH_3)-C\equiv CH$ | H | 1 | $-CO-R_3$ | O | 2 | |
| 3.156 | F | Cl | 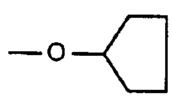 | H | 1 | $-CO-R_3$ | O | 2 | |
| 3.157 | F | Cl | 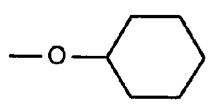 | H | 1 | $-CO-R_3$ | O | 2 | |
| 3.158 | F | Cl | 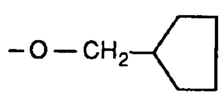 | H | 1 | $-CO-R_3$ | O | 2 | |
| 3.159 | F | Cl | 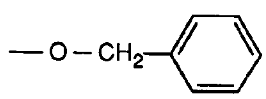 | H | 1 | $-CO-R_3$ | O | 2 | |

Tabelle 3 (Fortsetzung)

| Verb. Nr. | $R_1$ | $R_2$ | $R_3$ | $R_4$ | n | A | Y | q | Smp. [°C] |
|---|---|---|---|---|---|---|---|---|---|
| 3.160 | F | Cl | 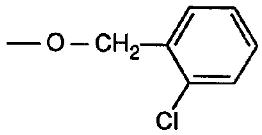 | H | 1 | $-CO-R_3$ | O | | |
| 3.161 | F | Cl | 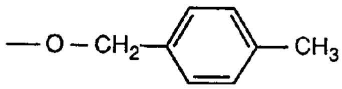 | H | 1 | $-CO-R_3$ | O | 2 | |
| 3.162 | F | Cl | $-S-CH_3$ | H | 1 | $-CO-R_3$ | O | 2 | |
| 3.163 | F | Cl | $-S-C_2H_5$ | H | 1 | $-CO-R_3$ | O | 2 | |
| 3.164 | F | Cl | $-S-C_3H_7$ | H | 1 | $-CO-R_3$ | O | 2 | |
| 3.165 | F | Cl | $-S-CH_2-CH=CH_2$ | H | 1 | $-CO-R_3$ | O | 2 | |
| 3.166 | F | Cl | $-S-CH_2-COOCH_3$ | H | 1 | $-CO-R_3$ | O | 2 | |
| 3.167 | F | Cl | $-S-CH_2-COOC_2H_5$ | H | 1 | $-CO-R_3$ | O | 2 | |
| 3.168 | F | Cl | $-S-CH_2-COOC_5H_{11}$ | H | 1 | $-CO-R_3$ | O | 2 | |
| 3.169 | F | Cl | $-S-CH(CH_3)-COOCH_3$ | H | 1 | $-CO-R_3$ | O | 2 | |
| 3.170 | F | Cl | $-S-(CH_3)-COOC_2H_5$ | H | 1 | $-CO-R_3$ | O | 2 | |
| 3.171 | F | Cl | $-S-CH(CH_3)-COOC_3H_7$ | H | 1 | $-CO-R_3$ | O | 2 | |
| 3.172 | F | Cl | $-S-CH_2-CH_2-COOCH_3$ | H | 1 | $-CO-R_3$ | O | 2 | |
| 3.173 | F | Cl | $-S-CH_2-COOCH_2-CH_2-O-CH_3$ | H | 1 | $-CO-R_3$ | O | 2 | |
| 3.174 | F | Cl | $-O-CH_2-COOCH_3$ | H | 1 | $-CO-R_3$ | O | 2 | |
| 3.175 | F | Cl | $-O-CH(CH_3)-COOCH_3$ | H | 1 | $-CO-R_3$ | O | 2 | |
| 3.176 | F | Cl | $-O-CH_2-COOC_5H_{11}$ | H | 1 | $-CO-R_3$ | O | 2 | |
| 3.177 | F | Cl | $-O-CH_2-CH_3-Si(CH_3)_3$ | H | 1 | $-CO-R_3$ | O | 2 | |
| 3.178 | F | Cl | -ONa | H | 1 | $-CO-R_3$ | O | 2 | |
| 3.179 | F | Br | -Cl | H | 1 | $-CO-R_3$ | O | 2 | |
| 3.180 | F | Br | -OH | H | 1 | $-CO-R_3$ | O | 2 | |
| 3.181 | F | Br | $-OCH_3$ | H | 1 | $-CO-R_3$ | O | 2 | |
| 3.182 | F | Br | $-OC_2H_5$ | H | 1 | $-CO-R_3$ | O | 2 | |
| 3.183 | F | Br | $-OC_3H_7$ | H | 1 | $-CO-R_3$ | O | 2 | |
| 3.184 | F | Br | 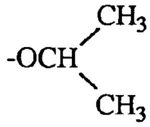 | H | 1 | $-CO-R_3$ | O | 2 | |
| 3.185 | F | Br | $-OC_4H_9$ | H | 1 | $-CO-R_3$ | O | 2 | |

Tabelle 3 (Fortsetzung)

| Verb. Nr. | $R_1$ | $R_2$ | $R_3$ | $R_4$ | n | A | Y | q | Smp. [°C] |
|---|---|---|---|---|---|---|---|---|---|
| 3.186 | F | Br | $-OCH(CH_3)-CH_2-CH_3$ | H | 1 | $-CO-R_3$ | O | 2 | |
| 3.187 | F | Br | 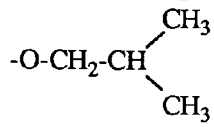 | H | 1 | $-CO-R_3$ | O | 2 | |
| 3.188 | F | Br | $-O-C_5H_{11}$ | H | 1 | $-CO-R_3$ | O | 2 | |
| 3.189 | F | Br | $-O-CH_2-CH_2-O-CH_3$ | H | 1 | $-CO-R_3$ | O | 2 | |
| 3.190 | F | Br | $-O-CH_2-CH_2-O-C_2H_5$ | H | 1 | $-CO-R_3$ | O | 2 | |
| 3.191 | F | Br | $-O-CH(CH_3)-CH_2-O-CH_3$ | H | 1 | $-CO-R_3$ | O | 2 | |
| 3.192 | F | Br | $-O-CH_2-CH_2-S-CH_3$ | H | 1 | $-CO-R_3$ | O | 2 | |
| 3.193 | F | Br | $-O-CH_2(CH_3)-S-CH_3$ | H | 1 | $-CO-R_3$ | O | 2 | |
| 3.194 | F | Br | $-O-CH-(CH_3)-S-C_2H_5$ | H | 1 | $-CO-R_3$ | O | 2 | |
| 3.195 | F | Br | $-O-CH(CH_3)-S-C_3H_7$ | H | 1 | $-CO-R_3$ | O | 2 | |
| 3.196 | F | Br | 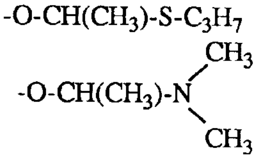 | H | 1 | $-CO-R_3$ | O | 2 | |
| 3.197 | F | Br | $-NH_2$ | H | 1 | $-CO-R_3$ | O | 2 | |
| 3.198 | F | Br | 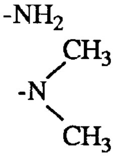 | H | 1 | $-CO-R_3$ | O | 2 | |
| 3.199 | F | Br | 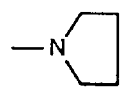 | H | 1 | $-CO-R_3$ | O | 2 | |
| 3.200 | F | Br | 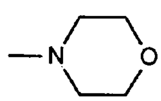 | H | 1 | $-CO-R_3$ | O | 2 | |
| 3.201 | F | Br | 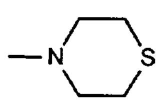 | H | 1 | $-CO-R_3$ | O | 2 | |
| 3.202 | F | Br | 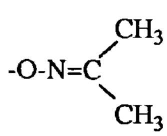 | H | 1 | $-CO-R_3$ | O | 2 | |
| 3.203 | F | Br | $-O-CH_2-CH_2-Cl$ | H | 1 | $-CO-R_3$ | O | 2 | |
| 3.204 | F | Br | $-O-CH_2-CN$ | H | 1 | $-CO-R_3$ | O | 2 | |
| 3.205 | F | Br | $-O-CH_2-CH=CH_2$ | H | 1 | $-CO-R_3$ | O | 2 | |
| 3.206 | F | Br | $-O-CH_2-C\equiv CH$ | H | 1 | $-CO-R_3$ | O | 2 | |

Tabelle 3 (Fortsetzung)

| Verb. Nr. | $R_1$ | $R_2$ | $R_3$ | $R_4$ | n | A | Y | q | Smp. [°C] |
|---|---|---|---|---|---|---|---|---|---|
| 3.207 | F | Br | 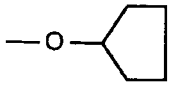 | H | 1 | $-CO-R_3$ | O | 2 | |
| 3.208 | F | Br | 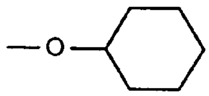 | H | 1 | $-CO-R_3$ | O | 2 | |
| 3.209 | F | Br | 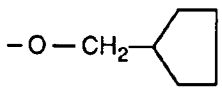 | H | 1 | $-CO-R_3$ | O | 2 | |
| 3.210 | F | Br | 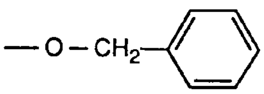 | H | 1 | $-CO-R_3$ | O | 2 | |
| 3.211 | F | Br | $-SCH_3$ | H | 1 | $-CO-R_3$ | O | 2 | |
| 3.212 | F | Br | $-S-CH_2-COOCH_3$ | H | 1 | $-CO-R_3$ | O | 2 | |
| 3.213 | F | Br | $-S-CH(CH_3)-COOCH_3$ | H | 1 | $-CO-R_3$ | O | 2 | |
| 3.214 | F | Br | $-O-CH_2-COOCH_3$ | H | 1 | $-CO-R_3$ | O | 2 | |
| 3.215 | F | Br | $-O-CH(CH_3)COOCH_3$ | H | 1 | $-CO-R_3$ | O | 2 | |
| 3.216 | F | CN | -Cl | H | 1 | $-CO-R_3$ | O | 2 | |
| 3.217 | F | CN | -OH | H | 1 | $-CO-R_3$ | O | 2 | |
| 3.218 | F | CN | $-OCH_3$ | H | 1 | $-CO-R_3$ | O | 2 | |
| 3.219 | H | Cl | -Cl | H | 1 | $-CO-R_3$ | O | 2 | |
| 3.220 | H | Cl | -OH | H | 1 | $-CO-R_3$ | O | 2 | |
| 3.221 | H | Cl | $-OCH_3$ | H | 1 | $-CO-R_3$ | O | 2 | |
| 3.222 | H | Cl | $-OC_2H_5$ | H | 1 | $-CO-R_3$ | O | 2 | |
| 3.223 | H | Cl | 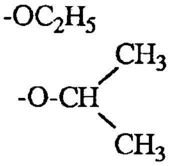 | H | 1 | $-CO-R_3$ | O | 2 | |
| 3.224 | H | Cl | $-O-CH_2-COOCH_3$ | H | 1 | $-CO-R_3$ | O | 2 | |
| 3.225 | H | Cl | $-O-CH(CH_3)COOCH_3$ | H | 1 | $-CO-R_3$ | O | 2 | |
| 3.226 | H | Cl | $-S-CH_2-COOCH_3$ | H | 1 | $-CO-R_3$ | O | 2 | |
| 3.227 | H | Cl | $-S-CH(CH_3)COOCH_3$ | H | 1 | $-CO-R_3$ | O | 2 | |
| 3.228 | H | Cl | 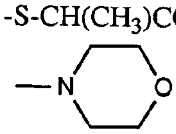 | H | 1 | $-CO-R_3$ | O | 2 | |
| 3.229 | F | Cl | Cl | H | 2 | $-CO-R_3$ | O | 2 | |

Tabelle 3 (Fortsetzung)

| Verb. Nr. | $R_1$ | $R_2$ | $R_3$ | $R_4$ | n | A | Y | q | Smp. [°C] |
|---|---|---|---|---|---|---|---|---|---|
| 3.230 | F | Cl | OH | H | 2 | $-CO-R_3$ | O | 2 | |
| 3.231 | F | Cl | $OCH_3$ | H | 2 | $-CO-R_3$ | O | 2 | |
| 3.232 | F | Cl | $-OC_2H_5$ | H | 2 | $-CO-R_3$ | O | 2 | |
| 3.233 | F | Cl | 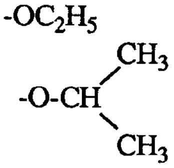 | H | 2 | $-CO-R_3$ | O | 2 | |
| 3.234 | F | Cl | $-O-CH_2-CH_2-O-CH_3$ | H | 2 | $-CO-R_3$ | O | 2 | |
| 3.235 | F | Cl | $-O-CH(CH_3)-CH_2-S-CH_3$ | H | 2 | $-CO-R_3$ | O | 2 | |
| 3.236 | F | Cl | $-O-CH_2-COOCH_3$ | H | 2 | $-CO-R_3$ | O | 2 | |
| 3.237 | F | Cl | $-S-CH_2-COOCH_3$ | H | 2 | $-CO-R_3$ | O | 2 | |
| 3.238 | F | Cl | $-CH_2-CH=CH_2$ | H | 2 | $-CO-R_3$ | O | 2 | |
| 3.239 | F | Cl | $-CH_2-C\equiv CH$ | H | 2 | $-CO-R_3$ | O | 2 | |
| 3.240 | F | Cl | Cl | H | 3 | $-CO-R_3$ | O | 2 | |
| 3.241 | F | Cl | OH | H | 3 | $-CO-R_3$ | O | 2 | |
| 3.242 | F | Cl | $OCH_3$ | H | 3 | $-CO-R_3$ | O | 2 | |
| 3.243 | F | Cl | $OC_2H_5$ | H | 3 | $-CO-R_3$ | O | 2 | |
| 3.244 | F | Cl | 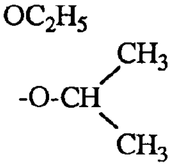 | H | 3 | $-CO-R_3$ | O | 2 | |
| 3.245 | F | Cl | $-O-CH_2-CH_2-O-CH$ | H | 3 | $-CO-R_3$ | O | 2 | |
| 3.246 | F | Cl | $-O-CH(CH_3)-CH_2-S-CH_3$ | H | 3 | $-CO-R_3$ | O | 2 | |
| 3.247 | F | Cl | $-O-CH_2-COOCH_3$ | H | 3 | $-CO-R_3$ | O | 2 | |
| 3.248 | F | Cl | $-S-CH_2-COOCH_3$ | H | 3 | $-CO-R_3$ | O | 2 | |
| 3.249 | F | Cl | $-O-CH_2-C\equiv CH$ | H | 3 | $-CO-R_3$ | O | 2 | |
| 3.250 | F | Cl | -Cl | Cl | 0 | $-CO-R_3$ | O | 2 | |
| 3.251 | F | Cl | -OH | Cl | 0 | $-CO-R_3$ | O | 2 | |
| 3.252 | F | Cl | $-OCH_3$ | Cl | 0 | $-CO-R_3$ | O | 2 | |
| 3.253 | F | Cl | $-OC_2H_5$ | Cl | 0 | $-CO-R_3$ | O | 2 | |
| 3.254 | F | Cl | 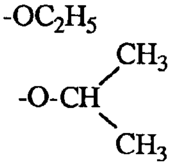 | Cl | 0 | $-CO-R_3$ | O | 2 | |
| 3.255 | F | Cl | $-O-CH_2-COOCH_3$ | Cl | 0 | $-CO-R_3$ | O | 2 | |
| 3.256 | F | Cl | $-S-CH_2-COOCH_3$ | Cl | 0 | $-CO-R_3$ | O | 2 | |
| 3.257 | F | Cl | $-OCH_3$ | Br | 0 | $-CO-R_3$ | O | 2 | |

Tabelle 3 (Fortsetzung)

| Verb. Nr. | $R_1$ | $R_2$ | $R_3$ | $R_4$ | n | A | Y | q | Smp. [°C] |
|---|---|---|---|---|---|---|---|---|---|
| 3.258 | F | Cl | 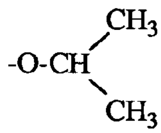 | Br | 0 | $-CO-R_3$ | O | 2 | |
| 3.259 | F | Cl | $-OCH_3$ | F | 0 | $-CO-R_3$ | O | 2 | |
| 3.260 | F | Cl | $-OCH_3$ | $CH_3$ | 0 | $-CO-R_3$ | O | 2 | |
| 3.261 | F | Cl | $-OC_2H_5$ | $CH_3$ | 0 | $-CO-R_3$ | O | 2 | |
| 3.262 | F | Cl | 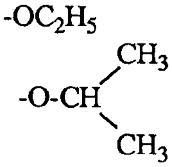 | $CH_3$ | 0 | $-CO-R_3$ | O | 2 | |
| 3.263 | F | Cl | $-O-CH_2-CH_2-O-CH_3$ | $CH_3$ | 0 | $-CO-R_3$ | O | 2 | |
| 3.264 | F | Cl | $-O-CH_2-COOCH_3$ | $CH_3$ | 0 | $-CO-R_3$ | O | 2 | |
| 3.265 | F | Cl | $-O-CH(CH_3)COOCH_3$ | $CH_3$ | 0 | $-CO-R_3$ | O | 2 | |
| 3.266 | F | Cl | $-S-CH_2-COOCH_3$ | $CH_3$ | 0 | $-CO-R_3$ | O | 2 | |
| 3.267 | F | Cl | $-OCH_3$ | $CF_3$ | 0 | $-CO-R_3$ | O | 2 | |
| 3.268 | F | Cl | $-OC_2H_5$ | $CF_3$ | 0 | $-CO-R_3$ | O | 2 | |
| 3.269 | F | Cl | 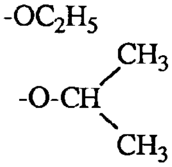 | $CF_3$ | 0 | $-CO-R_3$ | O | 2 | |
| 3.270 | F | Cl | $-O-CH_2-COOCH_3$ | $CF_3$ | 0 | $-CO-R_3$ | O | 2 | |
| 3.271 | F | Cl | $-S-CH_2-COOCH_3$ | $CF_3$ | 0 | $-CO-R_3$ | O | 2 | |
| 3.272 | F | Cl | - | H | 0 | -CN | O | 2 | |
| 3.273 | F | Cl | - | H | 1 | -CN | O | 2 | |
| 3.274 | F | Cl | - | H | 2 | -CN | O | 2 | |
| 3.275 | F | Cl | - | H | 3 | -CN | O | 2 | |
| 3.276 | F | Cl | - | H | 4 | -CN | O | 2 | |
| 3.277 | F | CL | $-OCH_3$ | H | 0 | $-CO-R_3$ | S | 2 | (harzig) |

Tabelle 4a:

Verbindungen der Formel I mit $W = W_4$, $A = -CO-R_3$ und $R_{14} = H$:

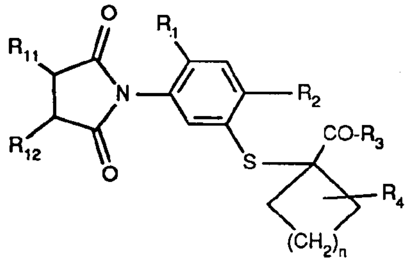

(I)

Tabelle 4a

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.001 | F | Cl | -Cl | H | 0 | $-CH_3$ | $-C_2H_5$ | |
| 4.002 | F | Cl | -OH | H | 0 | $-CH_3$ | $-C_2H_5$ | |
| 4.003 | F | Cl | $-OCH_3$ | H | 0 | $-CH_3$ | $-C_2H_5$ | |
| 4.004 | F | Cl | $-OC_2H_5$ | H | 0 | $-CH_3$ | $-C_2H_5$ | |
| 4.005 | F | Cl | $-OC_3H_7$ | H | 0 | $-CH_3$ | $-C_2H_5$ | |
| 4.006 | F | Cl | 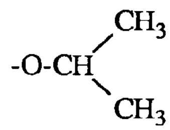 | H | 0 | $-CH_3$ | $-C_2H_5$ | |
| 4.007 | F | Cl | $-OC_4H_9$ | H | 0 | $-CH_3$ | $-C_2H_5$ | |
| 4.008 | F | Cl | $-O-CH(CH_3)-C_2H_5$ | H | 0 | $-CH_3$ | $-C_2H_5$ | |
| 4.009 | F | Cl | 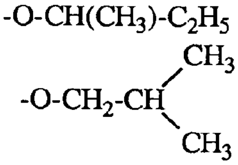 | H | 0 | $-CH_3$ | $-C_2H_5$ | |
| 4.010 | F | Cl | $-OC_5H_{11}$ | H | 0 | $-CH_3$ | $-C_2H_5$ | |
| 4.011 | F | Cl | $-O-CH_2-CH_2-O-CH_3$ | H | 0 | $-CH_3$ | $-C_2H_5$ | |
| 4.012 | F | Cl | $-O-CH_2-CH_2-O-C_2H_5$ | H | 0 | $-CH_3$ | $-C_2H_5$ | |
| 4.013 | F | Cl | $-O-CH-(CH_3)-CH_2-O-CH_3$ | H | 0 | $-CH_3$ | $-C_2H_5$ | |
| 4.014 | F | Cl | $-O-CH_2-CH_2-S-CH_3$ | H | 0 | $-CH_3$ | $-C_2H_5$ | |
| 4.015 | F | Cl | $-O-CH(CH_3)-CH_2-S-CH_3$ | H | 0 | $-CH_3$ | $-C_2H_5$ | |
| 4.016 | F | Cl | $-O-CH(CH_3)-CH_2-S-C_2H_5$ | H | 0 | $-CH_3$ | $-C_2H_5$ | |
| 4.017 | F | Cl | $-O-CH(CH_3)-CH_2-S-C_3H_7$ | H | 0 | $-CH_3$ | $-C_2H_5$ | |
| 4.018 | F | Cl | 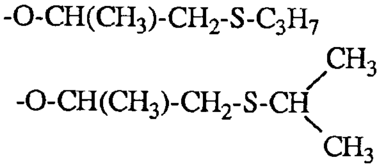 | H | 0 | $-CH_3$ | $-C_2H_5$ | |
| 4.019 | F | Cl | $-O-CH(CH_3)-CH_2-S-C_4H_9$ | H | 0 | $-CH_3$ | $-C_2H_5$ | |
| 4.020 | F | Cl | $-O-CH(CH_3)-CH_2-S-C_5H_{11}$ | H | 0 | $-CH_3$ | $-C_2H_5$ | |
| 4.021 | F | Cl | 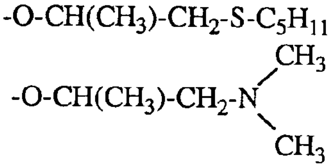 | H | 0 | $-CH_3$ | $-C_2H_5$ | |

Tabelle 4a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.022 | F | Cl | 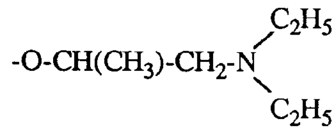 | H | 0 | $-CH_3$ | $-C_2H_5$ | |
| 4.023 | F | Cl | $-NH_2$ | H | 0 | $-CH_3$ | $-C_2H_5$ | |
| 4.024 | F | Cl | 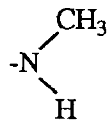 | H | 0 | $-CH_3$ | $-C_2H_5$ | |
| 4.025 | F | Cl | 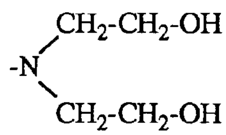 | H | 0 | $-CH_3$ | $-C_2H_5$ | |
| 4.026 | F | Cl | $-NH-CH_2-CH=CH_2$ | H | 0 | $-CH_3$ | $-C_2H_5$ | |
| 4.027 | F | Cl | $-N-(CH_2-CH=CH_2)_2$ | H | 0 | $-CH_3$ | $-C_2H_5$ | |
| 4.028 | F | Cl | 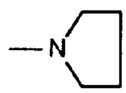 | H | 0 | $-CH_3$ | $-C_2H_5$ | |
| 4.029 | F | Cl | 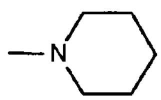 | H | 0 | $-CH_3$ | $-C_2H_5$ | |
| 4.030 | F | Cl | 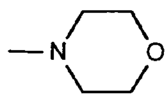 | H | 0 | $-CH_3$ | $-C_2H_5$ | |
| 4.031 | F | Cl | 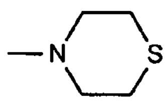 | H | 0 | $-CH_3$ | $-C_2H_5$ | |
| 4.032 | F | Cl | 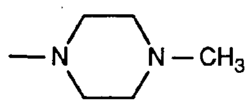 | H | 0 | $-CH_3$ | $-C_2H_5$ | |
| 4.033 | F | Cl | 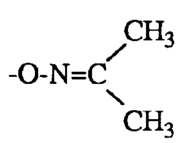 | H | 0 | $-CH_3$ | $-C_2H_5$ | |
| 4.034 | F | Cl | $-O-CH_2-CH_2-Cl$ | H | 0 | $-CH_3$ | $-C_2H_5$ | |
| 4.035 | F | Cl | $-O-CH_2-CN$ | H | 0 | $-CH_3$ | $-C_2H_5$ | |
| 4.036 | F | Cl | 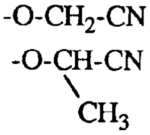 | H | 0 | $-CH_3$ | $-C_2H_5$ | |
| 4.037 | F | Cl | $-O-CH_2-CH=CH_2$ | H | 0 | $-CH_3$ | $-C_2H_5$ | |

Tabelle 4a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.038 | F | Cl | $-O-CH_2-CH=CHCl$ | H | 0 | $-CH_3$ | $-C_2H_5$ | |
| 4.039 | F | Cl | $-O-CH_2-C=CH_2$<br>I<br>Cl | H | 0 | $-CH_3$ | $-C_2H_5$ | |
| 4.040 | F | Cl | $-O-CH_2=C≡CH$ | H | 0 | $-CH_3$ | $-C_2H_5$ | |
| 4.041 | F | Cl | $-O-CH-C≡CH$<br>I<br>$CH_3$ | H | 0 | $-CH_3$ | $-C_2H_5$ | |
| 4.042 | F | Cl | 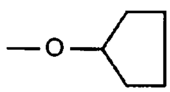 | H | 0 | $-CH_3$ | $-C_2H_5$ | |
| 4.043 | F | Cl | 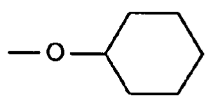 | H | 0 | $-CH_3$ | $-C_2H_5$ | |
| 4.044 | F | Cl | 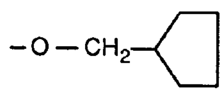 | H | 0 | $-CH_3$ | $-C_2H_5$ | |
| 4.045 | F | Cl | 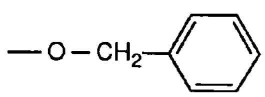 | H | 0 | $-CH_3$ | $-C_2H_5$ | |
| 4.046 | F | Cl | 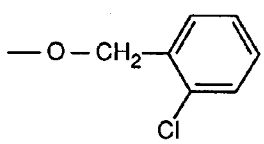 | H | 0 | $-CH_3$ | $-C_2H_5$ | |
| 4.047 | F | Cl | 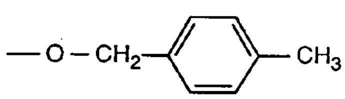 | H | 0 | $-CH_3$ | $-C_2H_5$ | |
| 4.048 | F | Cl | $-S-CH_3$ | H | 0 | $-CH_3$ | $-C_2H_5$ | |
| 4.049 | F | Cl | $-S-C_2H_5$ | H | 0 | $-CH_3$ | $-C_2H_5$ | |
| 4.050 | F | Cl | $-S-C_3H_7$ | H | 0 | $-CH_3$ | $-C_2H_5$ | |
| 4.051 | F | Cl | $-S-CH_2-CH=CH_2$ | H | 0 | $-CH_3$ | $-C_2H_5$ | |
| 4.052 | F | Cl | $-S-CH_2-COOCH_3$ | H | 0 | $-CH_3$ | $-C_2H_5$ | |
| 4.053 | F | Cl | $-S-CH_2-COOC_2H_5$ | H | 0 | $-CH_3$ | $-C_2H_5$ | |
| 4.054 | F | Cl | $-S-CH_2-COOC_5H_{11}$ | H | 0 | $-CH_3$ | $-C_2H_5$ | |
| 4.055 | F | Cl | $-S-CH(CH_3)-COOCH_3$ | H | 0 | $-CH_3$ | $-C_2H_5$ | |
| 4.056 | F | Cl | $-S-(CH_3)-COOC_2H_5$ | H | 0 | $-CH_3$ | $-C_2H_5$ | |

Tabelle 4a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.057 | F | Cl | $-S-CH(CH_3)-COOC_3H_7$ | H | 0 | $-CH_3$ | $-C_2H_5$ | |
| 4.058 | F | Cl | $-S-CH_2-CH_2-COOCH_3$ | H | 0 | $-CH_3$ | $-C_2H_5$ | |
| 4.059 | F | Cl | $-S-CH_2-COOCH_2-CH_2-O-CH_3$ | H | 0 | $-CH_3$ | $-C_2H_5$ | |
| 4.060 | F | Cl | $-O-CH_2-COOCH_3$ | H | 0 | $-CH_3$ | $-C_2H_5$ | |
| 4.061 | F | Cl | $-O-CH(CH_3)-COOCH_3$ | H | 0 | $-CH_3$ | $-C_2H_5$ | |
| 4.062 | F | Cl | $-O-CH_2-COOC_5H_{11}$ | H | 0 | $-CH_3$ | $-C_2H_5$ | |
| 4.063 | F | Cl | $-O-CH_2-CH_3-Si(CH_3)_3$ | H | 0 | $-CH_3$ | $-C_2H_5$ | |
| 4.064 | F | Cl | -ONa | H | 0 | $-CH_3$ | $-C_2H_5$ | |
| 4.065 | F | Br | -Cl | H | 0 | $-CH_3$ | $-C_2H_5$ | |
| 4.066 | F | Br | -OH | H | 0 | $-CH_3$ | $-C_2H_5$ | |
| 4.067 | F | Br | $-OCH_3$ | H | 0 | $-CH_3$ | $-C_2H_5$ | |
| 4.068 | F | Br | $-OC_2H_5$ | H | 0 | $-CH_3$ | $-C_2H_5$ | |
| 4.069 | F | Br | $-OC_3H_7$ | H | 0 | $-CH_3$ | $-C_2H_5$ | |
| 4.070 | F | Br | 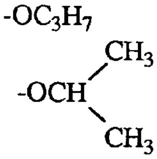 | H | 0 | $-CH_3$ | $-C_2H_5$ | |
| 4.071 | F | Br | $-OC_4H_9$ | H | 0 | $-CH_3$ | $-C_2H_5$ | |
| 4.072 | F | Br | $-OCH(CH_3)-CH_2-CH_3$ | H | 0 | $-CH_3$ | $-C_2H_5$ | |
| 4.073 | F | Br | 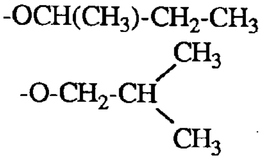 | H | 0 | $-CH_3$ | $-C_2H_5$ | |
| 4.074 | F | Br | $-O-C_5H_{11}$ | H | 0 | $-CH_3$ | $-C_2H_5$ | |
| 4.075 | F | Br | $-O-CH_2-CH_2-O-CH_3$ | H | 0 | $-CH_3$ | $-C_2H_5$ | |
| 4.076 | F | Br | $-O-CH_2-CH_2-O-C_2H_5$ | H | 0 | $-CH_3$ | $-C_2H_5$ | |
| 4.077 | F | Br | $-O-CH(CH_3)-CH_2-O-CH_3$ | H | 0 | $-CH_3$ | $-C_2H_5$ | |
| 4.078 | F | Br | $-O-CH_2-CH_2-S-CH_3$ | H | 0 | $-CH_3$ | $-C_2H_5$ | |
| 4.079 | F | Br | $-O-CH_2(CH_3)-S-CH_3$ | H | 0 | $-CH_3$ | $-C_2H_5$ | |
| 4.080 | F | Br | $-O-CH-(CH_3)-S-C_2H_5$ | H | 0 | $-CH_3$ | $-C_2H_5$ | |
| 4.081 | F | Br | $-O-CH(CH_3)-S-C_3H_7$ | H | 0 | $-CH_3$ | $-C_2H_5$ | |
| 4.082 | F | Br | 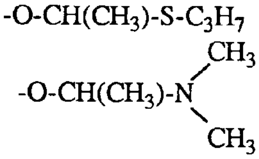 | H | 0 | $-CH_3$ | $-C_2H_5$ | |
| 4.083 | F | Br | $-NH_2$ | H | 0 | $-CH_3$ | $-C_2H_5$ | |

Tabelle 4a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.084 | F | Br | 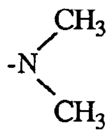 | H | 0 | $-CH_3$ | $-C_2H_5$ | |
| 4.085 | F | Br | 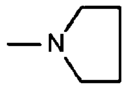 | H | 0 | $-CH_3$ | $-C_2H_5$ | |
| 4.086 | F | Br | 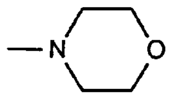 | H | 0 | $-CH_3$ | $-C_2H_5$ | |
| 4.087 | F | Br | 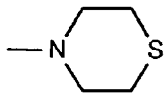 | H | 0 | $-CH_3$ | $-C_2H_5$ | |
| 4.088 | F | Br | 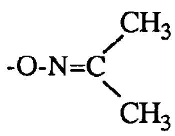 | H | 0 | $-CH_3$ | $-C_2H_5$ | |
| 4.089 | F | Br | $-O-CH_2-CH_2-Cl$ | H | 0 | $-CH_3$ | $-C_2H_5$ | |
| 4.090 | F | Br | $-O-CH_2-CN$ | H | 0 | $-CH_3$ | $-C_2H_5$ | |
| 4.091 | F | Br | $-O-CH_2-CH=CH_2$ | H | 0 | $-CH_3$ | $-C_2H_5$ | |
| 4.092 | F | Br | $-O-CH_2-C\equiv CH$ | H | 0 | $-CH_3$ | $-C_2H_5$ | |
| 4.093 | F | Br | 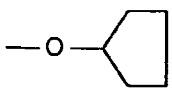 | H | 0 | $-CH_3$ | $-C_2H_5$ | |
| 4.094 | F | Br | 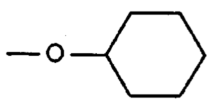 | H | 0 | $-CH_3$ | $-C_2H_5$ | |
| 4.095 | F | Br | 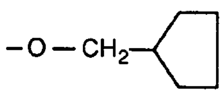 | H | 0 | $-CH_3$ | $-C_2H_5$ | |
| 4.096 | F | Br | 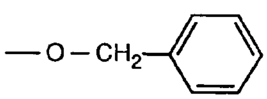 | H | 0 | $-CH_3$ | $-C_2H_5$ | |
| 4.097 | F | Br | $-SCH_3$ | H | 0 | $-CH_3$ | $-C_2H_5$ | |
| 4.098 | F | Br | $-S-CH_2-COOCH_3$ | H | 0 | $-CH_3$ | $-C_2H_5$ | |
| 4.099 | F | Br | $-S-CH(CH_3)-COOCH_3$ | H | 0 | $-CH_3$ | $-C_2H_5$ | |
| 4.100 | F | Br | $-O-CH_2-COOCH_3$ | H | 0 | $-CH_3$ | $-C_2H_5$ | |

Tabelle 4a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.101 | F | Br | $-O-CH(CH_3)COOCH_3$ | H | 0 | $-CH_3$ | $-C_2H_5$ | |
| 4.102 | F | CN | -Cl | H | 0 | $-CH_3$ | $-C_2H_5$ | |
| 4.103 | F | CN | -OH | H | 0 | $-CH_3$ | $-C_2H_5$ | |
| 4.104 | F | CN | $-OCH_3$ | H | 0 | $-CH_3$ | $-C_2H_5$ | |
| 4.105 | H | Cl | -Cl | H | 0 | $-CH_3$ | $-C_2H_5$ | |
| 4.106 | H | Cl | -OH | H | 0 | $-CH_3$ | $-C_2H_5$ | |
| 4.107 | H | Cl | $-OCH_3$ | H | 0 | $-CH_3$ | $-C_2H_5$ | |
| 4.108 | H | Cl | $-OC_2H_5$ | H | 0 | $-CH_3$ | $-C_2H_5$ | |
| 4.109 | H | Cl | 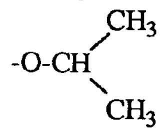 | H | 0 | $-CH_3$ | $-C_2H_5$ | |
| 4.110 | H | Cl | $-O-CH_2-COOCH_3$ | H | 0 | $-CH_3$ | $-C_2H_5$ | |
| 4.111 | H | Cl | $-O-CH(CH_3)COOCH_3$ | H | 0 | $-CH_3$ | $-C_2H_5$ | |
| 4.112 | H | Cl | $-S-CH_2-COOCH_3$ | H | 0 | $-CH_3$ | $-C_2H_5$ | |
| 4.113 | H | Cl | $-S-CH(CH_3)COOCH_3$ | H | 0 | $-CH_3$ | $-C_2H_5$ | |
| 4.114 | H | Cl | 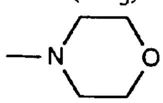 | H | 0 | $-CH_3$ | $-C_2H_5$ | |
| 4.115 | F | Cl | -Cl | H | 1 | $-CH_3$ | $-C_2H_5$ | |
| 4.116 | F | Cl | -OH | H | 1 | $-CH_3$ | $-C_2H_5$ | |
| 4.117 | F | Cl | $-OCH_3$ | H | 1 | $-CH_3$ | $-C_2H_5$ | 53-58 |
| 4.118 | F | Cl | $-OC_2H_5$ | H | 1 | $-CH_3$ | $-C_2H_5$ | |
| 4.119 | F | Cl | $-OC_3H_7$ | H | 1 | $-CH_3$ | $-C_2H_5$ | |
| 4.120 | F | Cl | 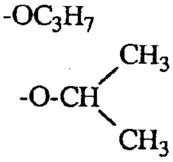 | H | 1 | $-CH_3$ | $-C_2H_5$ | |
| 4.121 | F | Cl | $-OC_4H_9$ | H | 1 | $-CH_3$ | $-C_2H_5$ | |
| 4.122 | F | Cl | $-O-CH(CH_3)-C_2H_5$ | H | 1 | $-CH_3$ | $-C_2H_5$ | |
| 4.123 | F | Cl | 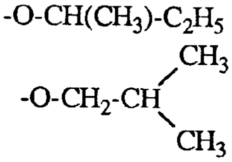 | H | 1 | $-CH_3$ | $-C_2H_5$ | |
| 4.124 | F | Cl | $-OC_5H_{11}$ | H | 1 | $-CH_3$ | $-C_2H_5$ | |
| 4.125 | F | Cl | $-O-CH_2-CH_2-O-CH_3$ | H | 1 | $-CH_3$ | $-C_2H_5$ | |
| 4.126 | F | Cl | $-O-CH_2-CH_2-O-C_2H_5$ | H | 1 | $-CH_3$ | $-C_2H_5$ | |

Tabelle 4a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.127 | F | Cl | $-O-CH-(CH_3)-CH_2-O-CH_3$ | H | 1 | $-CH_3$ | $-C_2H_5$ | |
| 4.128 | F | Cl | $-O-CH_2-CH_2-S-CH_3$ | H | 1 | $-CH_3$ | $-C_2H_5$ | |
| 4.129 | F | Cl | $-O-CH(CH_3)-CH_2-S-CH_3$ | H | 1 | $-CH_3$ | $-C_2H_5$ | |
| 4.130 | F | Cl | $-O-CH(CH_3)-CH_2-S-C_2H_5$ | H | 1 | $-CH_3$ | $-C_2H_5$ | |
| 4.131 | F | Cl | $-O-CH(CH_3)-CH_2-S-C_3H_7$ | H | 1 | $-CH_3$ | $-C_2H_5$ | |
| 4.132 | F | Cl | 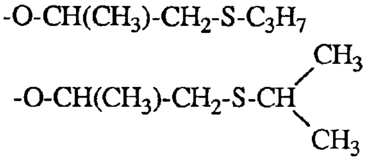 | H | 1 | $-CH_3$ | $-C_2H_5$ | |
| 4.133 | F | Cl | $-O-CH(CH_3)-CH_2-S-C_4H_9$ | H | 1 | $-CH_3$ | $-C_2H_5$ | |
| 4.134 | F | Cl | $-O-CH(CH_3)-CH_2-S-C_5H_{11}$ | H | 1 | $-CH_3$ | $-C_2H_5$ | |
| 4.135 | F | Cl | 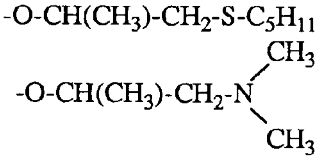 | H | 1 | $-CH_3$ | $-C_2H_5$ | |
| 4.136 | F | Cl | 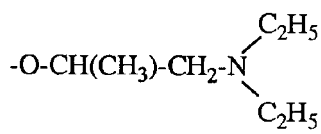 | H | 1 | $-CH_3$ | $-C_2H_5$ | |
| 4.137 | F | Cl | $-NH_2$ | H | 1 | $-CH_3$ | $-C_2H_5$ | |
| 4.138 | F | Cl | 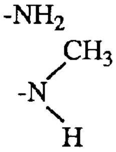 | H | 1 | $-CH_3$ | $-C_2H_5$ | |
| 4.139 | F | Cl | 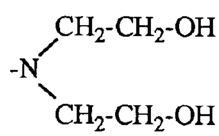 | H | 1 | $-CH_3$ | $-C_2H_5$ | |
| 4.140 | F | Cl | $-NH-CH_2-CH=CH_2$ | H | 1 | $-CH_3$ | $-C_2H_5$ | |
| 4.141 | F | Cl | $-N-(CH_2-CH=CH_2)_2$ | H | 1 | $-CH_3$ | $-C_2H_5$ | |
| 4.142 | F | Cl | 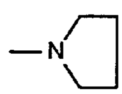 | H | 1 | $-CH_3$ | $-C_2H_5$ | |
| 4.143 | F | Cl | 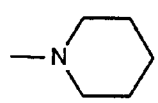 | H | 1 | $-CH_3$ | $-C_2H_5$ | |

Tabelle 4a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.144 | F | Cl | 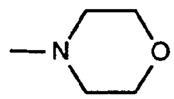 | H | 1 | $-CH_3$ | $-C_2H_5$ | |
| 4.145 | F | Cl | 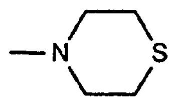 | H | 1 | $-CH_3$ | $-C_2H_5$ | |
| 4.146 | F | Cl | 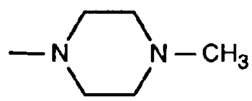 | H | 1 | $-CH_3$ | $-C_2H_5$ | |
| 4.147 | F | Cl | 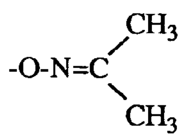 | H | 1 | $-CH_3$ | $-C_2H_5$ | |
| 4.148 | F | Cl | $-O-CH_2-CH_2-Cl$ | H | 1 | $-CH_3$ | $-C_2H_5$ | |
| 4.149 | F | Cl | $-O-CH_2-CN$ | H | 1 | $-CH_3$ | $-C_2H_5$ | |
| 4.150 | F | Cl | 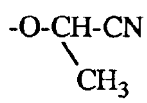 | H | 1 | $-CH_3$ | $-C_2H_5$ | |
| 4.151 | F | Cl | $-O-CH_2-CH=CH_2$ | H | 1 | $-CH_3$ | $-C_2H_5$ | |
| 4.152 | F | Cl | $-O-CH_2-CH=CHCl$ | H | 1 | $-CH_3$ | $-C_2H_5$ | |
| 4.153 | F | Cl | 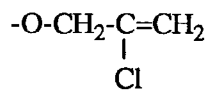 | H | 1 | $-CH_3$ | $-C_2H_5$ | |
| 4.154 | F | Cl | $-O-CH_2=C\equiv CH$ | H | 1 | $-CH_3$ | $-C_2H_5$ | |
| 4.155 | F | Cl | 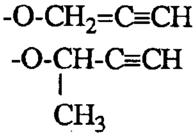 | H | 1 | $-CH_3$ | $-C_2H_5$ | |
| 4.156 | F | Cl | 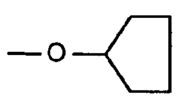 | H | 1 | $-CH_3$ | $-C_2H_5$ | |
| 4.157 | F | Cl | 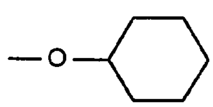 | H | 1 | $-CH_3$ | $-C_2H_5$ | |
| 4.158 | F | Cl | 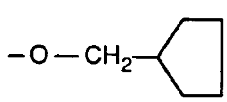 | H | 1 | $-CH_3$ | $-C_2H_5$ | |
| 4.159 | F | Cl | 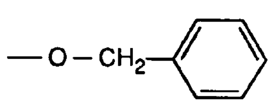 | H | 1 | $-CH_3$ | $-C_2H_5$ | |

Tabelle 4a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.160 | F | Cl | 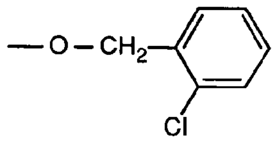 | H | 1 | $-CH_3$ | $-C_2H_5$ | |
| 4.161 | F | Cl | 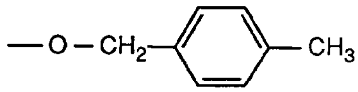 | H | 1 | $-CH_3$ | $-C_2H_5$ | |
| 4.162 | F | Cl | $-S-CH_3$ | H | 1 | $-CH_3$ | $-C_2H_5$ | |
| 4.163 | F | Cl | $-S-C_2H_5$ | H | 1 | $-CH_3$ | $-C_2H_5$ | |
| 4.164 | F | Cl | $-S-C_3H_7$ | H | 1 | $-CH_3$ | $-C_2H_5$ | |
| 4.165 | F | Cl | $-S-CH_2-CH=CH_2$ | H | 1 | $-CH_3$ | $-C_2H_5$ | |
| 4.166 | F | Cl | $-S-CH_2-COOCH_3$ | H | 1 | $-CH_3$ | $-C_2H_5$ | |
| 4.167 | F | Cl | $-S-CH_2-COOC_2H_5$ | H | 1 | $-CH_3$ | $-C_2H_5$ | |
| 4.168 | F | Cl | $-S-CH_2-COOC_5H_{11}$ | H | 1 | $-CH_3$ | $-C_2H_5$ | |
| 4.169 | F | Cl | $-S-CH(CH_3)-COOCH_3$ | H | 1 | $-CH_3$ | $-C_2H_5$ | |
| 4.170 | F | Cl | $-S-(CH_3)-COOC_2H_5$ | H | 1 | $-CH_3$ | $-C_2H_5$ | |
| 4.171 | F | Cl | $-S-CH(CH_3)-COOC_3H_7$ | H | 1 | $-CH_3$ | $-C_2H_5$ | |
| 4.172 | F | Cl | $-S-CH_2-CH_2-COOCH_3$ | H | 1 | $-CH_3$ | $-C_2H_5$ | |
| 4.173 | F | Cl | $-S-CH_2-COOCH_2-CH_2-O-CH_3$ | H | 1 | $-CH_3$ | $-C_2H_5$ | |
| 4.174 | F | Cl | $-O-CH_2-COOCH_3$ | H | 1 | $-CH_3$ | $-C_2H_5$ | |
| 4.175 | F | Cl | $-O-CH(CH_3)-COOCH_3$ | H | 1 | $-CH_3$ | $-C_2H_5$ | |
| 4.176 | F | Cl | $-O-CH_2-COOC_5H_{11}$ | H | 1 | $-CH_3$ | $-C_2H_5$ | |
| 4.177 | F | Cl | $-O-CH_2-CH_3-Si(CH_3)_3$ | H | 1 | $-CH_3$ | $-C_2H_5$ | |
| 4.178 | F | Cl | -ONa | H | 1 | $-CH_3$ | $-C_2H_5$ | |
| 4.179 | F | Br | -Cl | H | 1 | $-CH_3$ | $-C_2H_5$ | |
| 4.180 | F | Br | -OH | H | 1 | $-CH_3$ | $-C_2H_5$ | |
| 4.181 | F | Br | $-OCH_3$ | H | 1 | $-CH_3$ | $-C_2H_5$ | |
| 4.182 | F | Br | $-OC_2H_5$ | H | 1 | $-CH_3$ | $-C_2H_5$ | |
| 4.183 | F | Br | $-OC_3H_7$ | H | 1 | $-CH_3$ | $-C_2H_5$ | |
| 4.184 | F | Br | 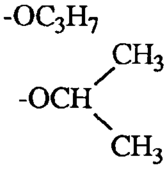 | H | 1 | $-CH_3$ | $-C_2H_5$ | |
| 4.185 | F | Br | $-OC_4H_9$ | H | 1 | $-CH_3$ | $-C_2H_5$ | |

Tabelle 4a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.186 | F | Br | $-OCH(CH_3)-CH_2-CH_3$ | H | 1 | $-CH_3$ | $-C_2H_5$ | |
| 4.187 | F | Br | 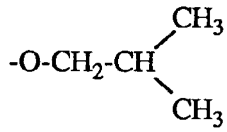 | H | 1 | $-CH_3$ | $-C_2H_5$ | |
| 4.188 | F | Br | $-O-C_5H_{11}$ | H | 1 | $-CH_3$ | $-C_2H_5$ | |
| 4.189 | F | Br | $-O-CH_2-CH_2-O-CH_3$ | H | 1 | $-CH_3$ | $-C_2H_5$ | |
| 4.190 | F | Br | $-O-CH_2-CH_2-O-C_2H_5$ | H | 1 | $-CH_3$ | $-C_2H_5$ | |
| 4.191 | F | Br | $-O-CH(CH_3)-CH_2-O-CH_3$ | H | 1 | $-CH_3$ | $-C_2H_5$ | |
| 4.192 | F | Br | $-O-CH_2-CH_2-S-CH_3$ | H | 1 | $-CH_3$ | $-C_2H_5$ | |
| 4.193 | F | Br | $-O-CH_2(CH_3)-S-CH_3$ | H | 1 | $-CH_3$ | $-C_2H_5$ | |
| 4.194 | F | Br | $-O-CH-(CH_3)-S-C_2H_5$ | H | 1 | $-CH_3$ | $-C_2H_5$ | |
| 4.195 | F | Br | $-O-CH(CH_3)-S-C_3H_7$ | H | 1 | $-CH_3$ | $-C_2H_5$ | |
| 4.196 | F | Br | 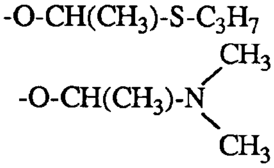 | H | 1 | $-CH_3$ | $-C_2H_5$ | |
| 4.197 | F | Br | $-NH_2$ | H | 1 | $-CH_3$ | $-C_2H_5$ | |
| 4.198 | F | Br | 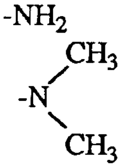 | H | 1 | $-CH_3$ | $-C_2H_5$ | |
| 4.199 | F | Br | 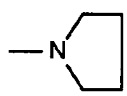 | H | 1 | $-CH_3$ | $-C_2H_5$ | |
| 4.200 | F | Br | 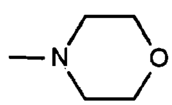 | H | 1 | $-CH_3$ | $-C_2H_5$ | |
| 4.201 | F | Br | 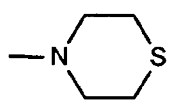 | H | 1 | $-CH_3$ | $-C_2H_5$ | |
| 4.202 | F | Br | 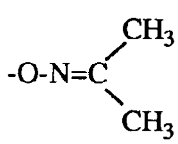 | H | 1 | $-CH_3$ | $-C_2H_5$ | |
| 4.203 | F | Br | $-O-CH_2-CH_2-Cl$ | H | 1 | $-CH_3$ | $-C_2H_5$ | |
| 4.204 | F | Br | $-O-CH_2-CN$ | H | 1 | $-CH_3$ | $-C_2H_5$ | |
| 4.205 | F | Br | $-O-CH_2-CH=CH_2$ | H | 1 | $-CH_3$ | $-C_2H_5$ | |
| 4.206 | F | Br | $-O-CH_2-C\equiv CH$ | H | 1 | $-CH_3$ | $-C_2H_5$ | |

Tabelle 4a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.207 | F | Br | 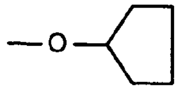 | H | 1 | $-CH_3$ | $-C_2H_5$ | |
| 4.208 | F | Br | 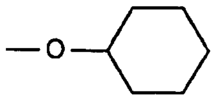 | H | 1 | $-CH_3$ | $-C_2H_5$ | |
| 4.209 | F | Br | 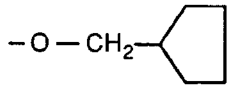 | H | 1 | $-CH_3$ | $-C_2H_5$ | |
| 4.210 | F | Br | 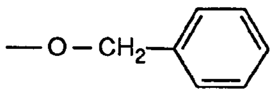 | H | 1 | $-CH_3$ | $-C_2H_5$ | |
| 4.211 | F | Br | $-SCH_3$ | H | 1 | $-CH_3$ | $-C_2H_5$ | |
| 4.212 | F | Br | $-S-CH_2-COOCH_3$ | H | 1 | $-CH_3$ | $-C_2H_5$ | |
| 4.213 | F | Br | $-S-CH(CH_3)-COOCH_3$ | H | 1 | $-CH_3$ | $-C_2H_5$ | |
| 4.214 | F | Br | $-O-CH_2-COOCH_3$ | H | 1 | $-CH_3$ | $-C_2H_5$ | |
| 4.215 | F | Br | $-O-CH(CH_3)COOCH_3$ | H | 1 | $-CH_3$ | $-C_2H_5$ | |
| 4.216 | F | CN | -Cl | H | 1 | $-CH_3$ | $-C_2H_5$ | |
| 4.217 | F | CN | -OH | H | 1 | $-CH_3$ | $-C_2H_5$ | |
| 4.218 | F | CN | $-OCH_3$ | H | 1 | $-CH_3$ | $-C_2H_5$ | |
| 4.219 | H | Cl | -Cl | H | 1 | $-CH_3$ | $-C_2H_5$ | |
| 4.220 | H | Cl | -OH | H | 1 | $-CH_3$ | $-C_2H_5$ | |
| 4.221 | H | Cl | $-OCH_3$ | H | 1 | $-CH_3$ | $-C_2H_5$ | |
| 4.222 | H | Cl | $-OC_2H_5$ | H | 1 | $-CH_3$ | $-C_2H_5$ | |
| 4.223 | H | Cl | 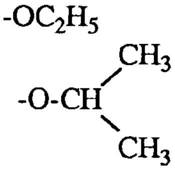 | H | 1 | $-CH_3$ | $-C_2H_5$ | |
| 4.224 | H | Cl | $-O-CH_2-COOCH_3$ | H | 1 | $-CH_3$ | $-C_2H_5$ | |
| 4.225 | H | Cl | $-O-CH(CH_3)COOCH_3$ | H | 1 | $-CH_3$ | $-C_2H_5$ | |
| 4.226 | H | Cl | $-S-CH_2-COOCH_3$ | H | 1 | $-CH_3$ | $-C_2H_5$ | |
| 4.227 | H | Cl | $-S-CH(CH_3)COOCH_3$ | H | 1 | $-CH_3$ | $-C_2H_5$ | |
| 4.228 | H | Cl | 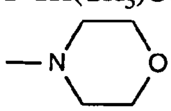 | H | 1 | $-CH_3$ | $-C_2H_5$ | |
| 4.229 | F | Cl | Cl | H | 2 | $-CH_3$ | $-C_2H_5$ | |

Tabelle 4a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.230 | F | Cl | OH | H | 2 | $-CH_3$ | $-C_2H_5$ | |
| 4.231 | F | Cl | $OCH_3$ | H | 2 | $-CH_3$ | $-C_2H_5$ | |
| 4.232 | F | Cl | $-OC_2H_5$ | H | 2 | $-CH_3$ | $-C_2H_5$ | |
| 4.233 | F | Cl | 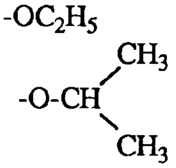 | H | 2 | $-CH_3$ | $-C_2H_5$ | |
| 4.234 | F | Cl | $-O-CH_2-CH_2-O-CH_3$ | H | 2 | $-CH_3$ | $-C_2H_5$ | |
| 4.235 | F | Cl | $-O-CH(CH_3)-CH_2-S-CH_3$ | H | 2 | $-CH_3$ | $-C_2H_5$ | |
| 4.236 | F | Cl | $-O-CH_2-COOCH_3$ | H | 2 | $-CH_3$ | $-C_2H_5$ | |
| 4.237 | F | Cl | $-S-CH_2-COOCH_3$ | H | 2 | $-CH_3$ | $-C_2H_5$ | |
| 4.238 | F | Cl | $-CH_2-CH=CH_2$ | H | 2 | $-CH_3$ | $-C_2H_5$ | |
| 4.239 | F | Cl | $-CH_2-C\equiv CH$ | H | 2 | $-CH_3$ | $-C_2H_5$ | |
| 4.240 | F | Cl | Cl | H | 3 | $-CH_3$ | $-C_2H_5$ | |
| 4.241 | F | Cl | OH | H | 3 | $-CH_3$ | $-C_2H_5$ | |
| 4.242 | F | Cl | $OCH_3$ | H | 3 | $-CH_3$ | $-C_2H_5$ | |
| 4.243 | F | Cl | $OC_2H_5$ | H | 3 | $-CH_3$ | $-C_2H_5$ | |
| 4.244 | F | Cl | 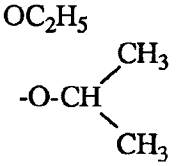 | H | 3 | $-CH_3$ | $-C_2H_5$ | |
| 4.245 | F | Cl | $-O-CH_2-CH_2-O-CH$ | H | 3 | $-CH_3$ | $-C_2H_5$ | |
| 4.246 | F | Cl | $-O-CH(CH_3)-CH_2-S-CH_3$ | H | 3 | $-CH_3$ | $-C_2H_5$ | |
| 4.247 | F | Cl | $-O-CH_2-COOCH_3$ | H | 3 | $-CH_3$ | $-C_2H_5$ | |
| 4.248 | F | Cl | $-S-CH_2-COOCH_3$ | H | 3 | $-CH_3$ | $-C_2H_5$ | |
| 4.249 | F | Cl | $-O-CH_2-C\equiv CH$ | H | 3 | $-CH_3$ | $-C_2H_5$ | |
| 4.250 | F | Cl | -Cl | Cl | 0 | $-CH_3$ | $-C_2H_5$ | |
| 4.251 | F | Cl | -OH | Cl | 0 | $-CH_3$ | $-C_2H_5$ | |
| 4.252 | F | Cl | $-OCH_3$ | Cl | 0 | $-CH_3$ | $-C_2H_5$ | |
| 4.253 | F | Cl | $-OC_2H_5$ | Cl | 0 | $-CH_3$ | $-C_2H_5$ | |
| 4.254 | F | Cl | 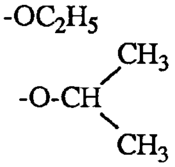 | Cl | 0 | $-CH_3$ | $-C_2H_5$ | |
| 4.255 | F | Cl | $-O-CH_2-COOCH_3$ | Cl | 0 | $-CH_3$ | $-C_2H_5$ | |
| 4.256 | F | Cl | $-S-CH_2-COOCH_3$ | Cl | 0 | $-CH_3$ | $-C_2H_5$ | |
| 4.257 | F | Cl | $-OCH_3$ | Br | 0 | $-CH_3$ | $-C_2H_5$ | |

Tabelle 4a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.258 | F | Cl | 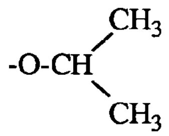 | Br | 0 | $-CH_3$ | $-C_2H_5$ | |
| 4.259 | F | Cl | $-OCH_3$ | F | 0 | $-CH_3$ | $-C_2H_5$ | |
| 4.260 | F | Cl | $-OCH_3$ | $CH_3$ | 0 | $-CH_3$ | $-C_2H_5$ | |
| 4.261 | F | Cl | $-OC_2H_5$ | $CH_3$ | 0 | $-CH_3$ | $-C_2H_5$ | |
| 4.262 | F | Cl | 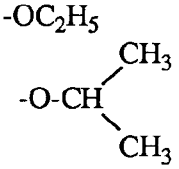 | $CH_3$ | 0 | $-CH_3$ | $-C_2H_5$ | |
| 4.263 | F | Cl | $-O-CH_2-CH_2-O-CH_3$ | $CH_3$ | 0 | $-CH_3$ | $-C_2H_5$ | |
| 4.264 | F | Cl | $-O-CH_2-COOCH_3$ | $CH_3$ | 0 | $-CH_3$ | $-C_2H_5$ | |
| 4.265 | F | Cl | $-O-CH(CH_3)COOCH_3$ | $CH_3$ | 0 | $-CH_3$ | $-C_2H_5$ | |
| 4.266 | F | Cl | $-S-CH_2-COOCH_3$ | $CH_3$ | 0 | $-CH_3$ | $-C_2H_5$ | |
| 4.267 | F | Cl | $-OCH_3$ | $CF_3$ | 0 | $-CH_3$ | $-C_2H_5$ | |
| 4.268 | F | Cl | $-OC_2H_5$ | $CF_3$ | 0 | $-CH_3$ | $-C_2H_5$ | |
| 4.269 | F | Cl | 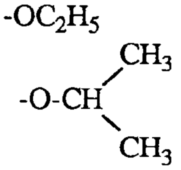 | $CF_3$ | 0 | $-CH_3$ | $-C_2H_5$ | |
| 4.270 | F | Cl | $-O-CH_2-COOCH_3$ | $CF_3$ | 0 | $-CH_3$ | $-C_2H_5$ | |
| 4.271 | F | Cl | $-S-CH_2-COOCH_3$ | $CF_3$ | 0 | $-CH_3$ | $-C_2H_5$ | |

Tabelle 4b:

Verbindungen der Formel I mit $W = W_4$, $A = -CN$ und $R_{14} = H$:

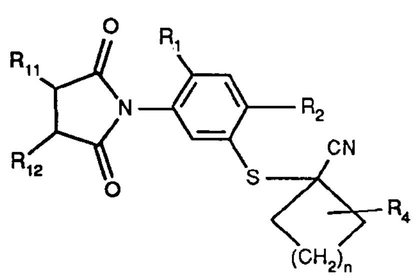

(I)

Tabelle 4b

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 4.272 | F | Cl | - | H | 0 | $-CH_3$ | $-C_2H_5$ | |
| 4.273 | F | Cl | - | H | 1 | $-CH_3$ | $-C_2H_5$ | |
| 4.274 | F | Cl | - | H | 2 | $-CH_3$ | $-C_2H_5$ | |
| 4.275 | F | Cl | - | H | 3 | $-CH_3$ | $-C_2H_5$ | |
| 4.276 | F | Cl | - | H | 4 | $-CH_3$ | $-C_2H_5$ | |

Tabelle 5a:

Verbindungen der Formel I mit W = $W_5$, R = $-CO-R_3$ und $R_{14}$ = H:

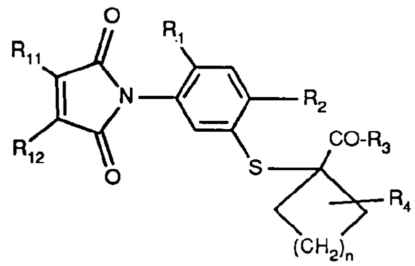

(I)

<u>Tabelle 5a</u>

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.001 | F | Cl | -Cl | H | 0 | $-CH_3$ | $-C_2H_5$ | |
| 5.002 | F | Cl | -OH | H | 0 | $-CH_3$ | $-C_2H_5$ | |
| 5.003 | F | Cl | $-OCH_3$ | H | 0 | $-CH_3$ | $-C_2H_5$ | |
| 5.004 | F | Cl | $-OC_2H_5$ | H | 0 | $-CH_3$ | $-C_2H_5$ | |
| 5.005 | F | Cl | $-OC_3H_7$ | H | 0 | $-CH_3$ | $-C_2H_5$ | |
| 5.006 | F | Cl | 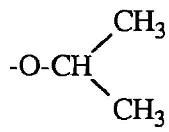 | H | 0 | $-CH_3$ | $-C_2H_5$ | |
| 5.007 | F | Cl | $-OC_4H_9$ | H | 0 | $-CH_3$ | $-C_2H_5$ | |
| 5.008 | F | Cl | $-O-CH(CH_3)-C_2H_5$ | H | 0 | $-CH_3$ | $-C_2H_5$ | |
| 5.009 | F | Cl | 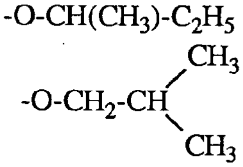 | H | 0 | $-CH_3$ | $-C_2H_5$ | |
| 5.010 | F | Cl | $-OC_5H_{11}$ | H | 0 | $-CH_3$ | $-C_2H_5$ | |
| 5.011 | F | Cl | $-O-CH_2-CH_2-O-CH_3$ | H | 0 | $-CH_3$ | $-C_2H_5$ | |
| 5.012 | F | Cl | $-O-CH_2-CH_2-O-C_2H_5$ | H | 0 | $-CH_3$ | $-C_2H_5$ | |
| 5.013 | F | Cl | $-O-CH-(CH_3)-CH_2-O-CH_3$ | H | 0 | $-CH_3$ | $-C_2H_5$ | |
| 5.014 | F | Cl | $-O-CH_2-CH_2-S-CH_3$ | H | 0 | $-CH_3$ | $-C_2H_5$ | |
| 5.015 | F | Cl | $-O-CH(CH_3)-CH_2-S-CH_3$ | H | 0 | $-CH_3$ | $-C_2H_5$ | |
| 5.016 | F | Cl | $-O-CH(CH_3)-CH_2-S-C_2H_5$ | H | 0 | $-CH_3$ | $-C_2H_5$ | |
| 5.017 | F | Cl | $-O-CH(CH_3)-CH_2-S-C_3H_7$ | H | 0 | $-CH_3$ | $-C_2H_5$ | |
| 5.018 | F | Cl | 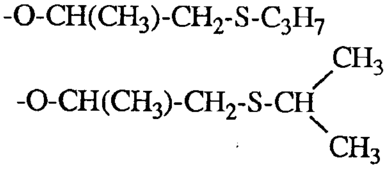 | H | 0 | $-CH_3$ | $-C_2H_5$ | |
| 5.019 | F | Cl | $-O-CH(CH_3)-CH_2-S-C_4H_9$ | H | 0 | $-CH_3$ | $-C_2H_5$ | |
| 5.020 | F | Cl | $-O-CH(CH_3)-CH_2-S-C_5H_{11}$ | H | 0 | $-CH_3$ | $-C_2H_5$ | |
| 5.021 | F | Cl | 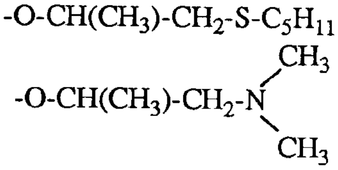 | H | 0 | $-CH_3$ | $-C_2H_5$ | |

Tabelle 5a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.022 | F | Cl | 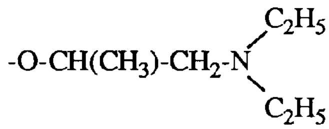 | H | 0 | $-CH_3$ | $-C_2H_5$ | |
| 5.023 | F | Cl | $-NH_2$ | H | 0 | $-CH_3$ | $-C_2H_5$ | |
| 5.024 | F | Cl | 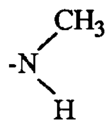 | H | 0 | $-CH_3$ | $-C_2H_5$ | |
| 5.025 | F | Cl | 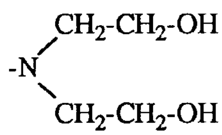 | H | 0 | $-CH_3$ | $-C_2H_5$ | |
| 5.026 | F | Cl | $-NH-CH_2-CH=CH_2$ | H | 0 | $-CH_3$ | $-C_2H_5$ | |
| 5.027 | F | Cl | $-N-(CH_2-CH=CH_2)_2$ | H | 0 | $-CH_3$ | $-C_2H_5$ | |
| 5.028 | F | Cl | 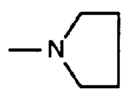 | H | 0 | $-CH_3$ | $-C_2H_5$ | |
| 5.029 | F | Cl | 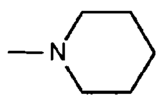 | H | 0 | $-CH_3$ | $-C_2H_5$ | |
| 5.030 | F | Cl | 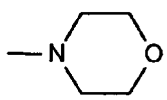 | H | 0 | $-CH_3$ | $-C_2H_5$ | |
| 5.031 | F | Cl | 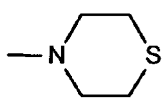 | H | 0 | $-CH_3$ | $-C_2H_5$ | |
| 5.032 | F | Cl | 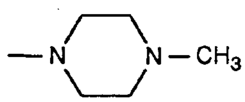 | H | 0 | $-CH_3$ | $-C_2H_5$ | |
| 5.033 | F | Cl | 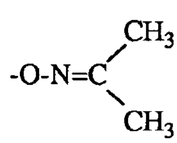 | H | 0 | $-CH_3$ | $-C_2H_5$ | |
| 5.034 | F | Cl | $-O-CH_2-CH_2-Cl$ | H | 0 | $-CH_3$ | $-C_2H_5$ | |
| 5.035 | F | Cl | $-O-CH_2-CN$ | H | 0 | $-CH_3$ | $-C_2H_5$ | |
| 5.036 | F | Cl | 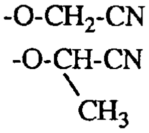 | H | 0 | $-CH_3$ | $-C_2H_5$ | |
| 5.037 | F | Cl | $-O-CH_2-CH=CH_2$ | H | 0 | $-CH_3$ | $-C_2H_5$ | |

Tabelle 5a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.038 | F | Cl | $-O-CH_2-CH=CHCl$ | H | 0 | $-CH_3$ | $-C_2H_5$ | |
| 5.039 | F | Cl | $-O-CH_2-C=CH_2$<br>\|<br>Cl | H | 0 | $-CH_3$ | $-C_2H_5$ | |
| 5.040 | F | Cl | $-O-CH_2=C≡CH$ | H | 0 | $-CH_3$ | $-C_2H_5$ | |
| 5.041 | F | Cl | $-O-CH-C≡CH$<br>\|<br>$CH_3$ | H | 0 | $-CH_3$ | $-C_2H_5$ | |
| 5.042 | F | Cl | 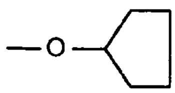 | H | 0 | $-CH_3$ | $-C_2H_5$ | |
| 5.043 | F | Cl | 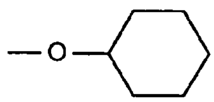 | H | 0 | $-CH_3$ | $-C_2H_5$ | |
| 5.044 | F | Cl | 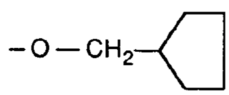 | H | 0 | $-CH_3$ | $-C_2H_5$ | |
| 5.045 | F | Cl | 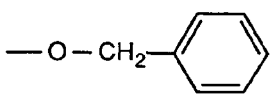 | H | 0 | $-CH_3$ | $-C_2H_5$ | |
| 5.046 | F | Cl | 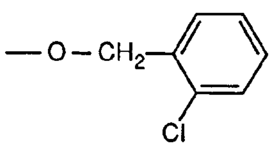 | H | 0 | $-CH_3$ | $-C_2H_5$ | |
| 5.047 | F | Cl | 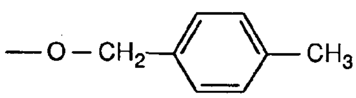 | H | 0 | $-CH_3$ | $-C_2H_5$ | |
| 5.048 | F | Cl | $-S-CH_3$ | H | 0 | $-CH_3$ | $-C_2H_5$ | |
| 5.049 | F | Cl | $-S-C_2H_5$ | H | 0 | $-CH_3$ | $-C_2H_5$ | |
| 5.050 | F | Cl | $-S-C_3H_7$ | H | 0 | $-CH_3$ | $-C_2H_5$ | |
| 5.051 | F | Cl | $-S-CH_2-CH=CH_2$ | H | 0 | $-CH_3$ | $-C_2H_5$ | |
| 5.052 | F | Cl | $-S-CH_2-COOCH_3$ | H | 0 | $-CH_3$ | $-C_2H_5$ | |
| 5.053 | F | Cl | $-S-CH_2-COOC_2H_5$ | H | 0 | $-CH_3$ | $-C_2H_5$ | |
| 5.054 | F | Cl | $-S-CH_2-COOC_5H_{11}$ | H | 0 | $-CH_3$ | $-C_2H_5$ | |
| 5.055 | F | Cl | $-S-CH(CH_3)-COOCH_3$ | H | 0 | $-CH_3$ | $-C_2H_5$ | |
| 5.056 | F | Cl | $-S-(CH_3)-COOC_2H_5$ | H | 0 | $-CH_3$ | $-C_2H_5$ | |

Tabelle 5a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.057 | F | Cl | $-S-CH(CH_3)-COOC_3H_7$ | H | 0 | $-CH_3$ | $-C_2H_5$ | |
| 5.058 | F | Cl | $-S-CH_2-CH_2-COOCH_3$ | H | 0 | $-CH_3$ | $-C_2H_5$ | |
| 5.059 | F | Cl | $-S-CH_2-COOCH_2-CH_2-O-CH_3$ | H | 0 | $-CH_3$ | $-C_2H_5$ | |
| 5.060 | F | Cl | $-O-CH_2-COOCH_3$ | H | 0 | $-CH_3$ | $-C_2H_5$ | |
| 5.061 | F | Cl | $-O-CH(CH_3)-COOCH_3$ | H | 0 | $-CH_3$ | $-C_2H_5$ | |
| 5.062 | F | Cl | $-O-CH_2-COOC_5H_{11}$ | H | 0 | $-CH_3$ | $-C_2H_5$ | |
| 5.063 | F | Cl | $-O-CH_2-CH_3-Si(CH_3)_3$ | H | 0 | $-CH_3$ | $-C_2H_5$ | |
| 5.064 | F | Cl | -ONa | H | 0 | $-CH_3$ | $-C_2H_5$ | |
| 5.065 | F | Br | -Cl | H | 0 | $-CH_3$ | $-C_2H_5$ | |
| 5.066 | F | Br | -OH | H | 0 | $-CH_3$ | $-C_2H_5$ | |
| 5.067 | F | Br | $-OCH_3$ | H | 0 | $-CH_3$ | $-C_2H_5$ | |
| 5.068 | F | Br | $-OC_2H_5$ | H | 0 | $-CH_3$ | $-C_2H_5$ | |
| 5.069 | F | Br | $-OC_3H_7$ | H | 0 | $-CH_3$ | $-C_2H_5$ | |
| 5.070 | F | Br | 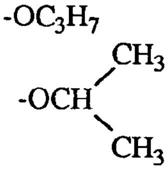 | H | 0 | $-CH_3$ | $-C_2H_5$ | |
| 5.071 | F | Br | $-OC_4H_9$ | H | 0 | $-CH_3$ | $-C_2H_5$ | |
| 5.072 | F | Br | $-OCH(CH_3)-CH_2-CH_3$ | H | 0 | $-CH_3$ | $-C_2H_5$ | |
| 5.073 | F | Br | 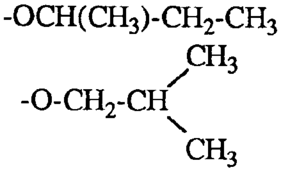 | H | 0 | $-CH_3$ | $-C_2H_5$ | |
| 5.074 | F | Br | $-O-C_5H_{11}$ | H | 0 | $-CH_3$ | $-C_2H_5$ | |
| 5.075 | F | Br | $-O-CH_2-CH_2-O-CH_3$ | H | 0 | $-CH_3$ | $-C_2H_5$ | |
| 5.076 | F | Br | $-O-CH_2-CH_2-O-C_2H_5$ | H | 0 | $-CH_3$ | $-C_2H_5$ | |
| 5.077 | F | Br | $-O-CH(CH_3)-CH_2-O-CH_3$ | H | 0 | $-CH_3$ | $-C_2H_5$ | |
| 5.078 | F | Br | $-O-CH_2-CH_2-S-CH_3$ | H | 0 | $-CH_3$ | $-C_2H_5$ | |
| 5.079 | F | Br | $-O-CH_2(CH_3)-S-CH_3$ | H | 0 | $-CH_3$ | $-C_2H_5$ | |
| 5.080 | F | Br | $-O-CH-(CH_3)-S-C_2H_5$ | H | 0 | $-CH_3$ | $-C_2H_5$ | |
| 5.081 | F | Br | $-O-CH(CH_3)-S-C_3H_7$ | H | 0 | $-CH_3$ | $-C_2H_5$ | |
| 5.082 | F | Br | 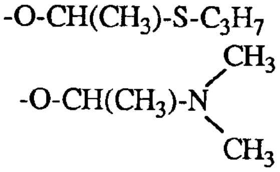 | H | 0 | $-CH_3$ | $-C_2H_5$ | |
| 5.083 | F | Br | $-NH_2$ | H | 0 | $-CH_3$ | $-C_2H_5$ | |

Tabelle 5a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.084 | F | Br | 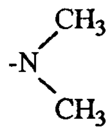 | H | 0 | $-CH_3$ | $-C_2H_5$ | |
| 5.085 | F | Br | 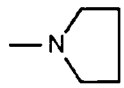 | H | 0 | $-CH_3$ | $-C_2H_5$ | |
| 5.086 | F | Br | 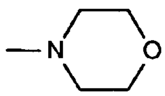 | H | 0 | $-CH_3$ | $-C_2H_5$ | |
| 5.087 | F | Br | 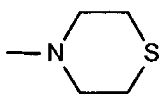 | H | 0 | $-CH_3$ | $-C_2H_5$ | |
| 5.088 | F | Br | 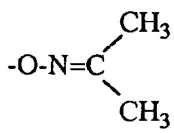 | H | 0 | $-CH_3$ | $-C_2H_5$ | |
| 5.089 | F | Br | $-O-CH_2-CH_2-Cl$ | H | 0 | $-CH_3$ | $-C_2H_5$ | |
| 5.090 | F | Br | $-O-CH_2-CN$ | H | 0 | $-CH_3$ | $-C_2H_5$ | |
| 5.091 | F | Br | $-O-CH_2-CH=CH_2$ | H | 0 | $-CH_3$ | $-C_2H_5$ | |
| 5.092 | F | Br | $-O-CH_2-C\equiv CH$ | H | 0 | $-CH_3$ | $-C_2H_5$ | |
| 5.093 | F | Br | 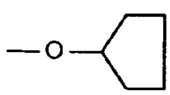 | H | 0 | $-CH_3$ | $-C_2H_5$ | |
| 5.094 | F | Br | 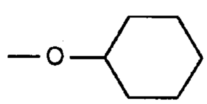 | H | 0 | $-CH_3$ | $-C_2H_5$ | |
| 5.095 | F | Br | 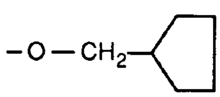 | H | 0 | $-CH_3$ | $-C_2H_5$ | |
| 5.096 | F | Br | 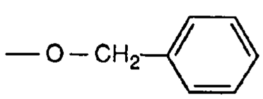 | H | 0 | $-CH_3$ | $-C_2H_5$ | |
| 5.097 | F | Br | $-SCH_3$ | H | 0 | $-CH_3$ | $-C_2H_5$ | |
| 5.098 | F | Br | $-S-CH_2-COOCH_3$ | H | 0 | $-CH_3$ | $-C_2H_5$ | |
| 5.099 | F | Br | $-S-CH(CH_3)-COOCH_3$ | H | 0 | $-CH_3$ | $-C_2H_5$ | |
| 5.100 | F | Br | $-O-CH_2-COOCH_3$ | H | 0 | $-CH_3$ | $-C_2H_5$ | |

Tabelle 5a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.101 | F | Br | $-O-CH(CH_3)COOCH_3$ | H | 0 | $-CH_3$ | $-C_2H_5$ | |
| 5.102 | F | CN | -Cl | H | 0 | $-CH_3$ | $-C_2H_5$ | |
| 5.103 | F | CN | -OH | H | 0 | $-CH_3$ | $-C_2H_5$ | |
| 5.104 | F | CN | $-OCH_3$ | H | 0 | $-CH_3$ | $-C_2H_5$ | |
| 5.105 | H | Cl | -Cl | H | 0 | $-CH_3$ | $-C_2H_5$ | |
| 5.106 | H | Cl | -OH | H | 0 | $-CH_3$ | $-C_2H_5$ | |
| 5.107 | H | Cl | $-OCH_3$ | H | 0 | $-CH_3$ | $-C_2H_5$ | |
| 5.108 | H | Cl | $-OC_2H_5$ | H | 0 | $-CH_3$ | $-C_2H_5$ | |
| 5.109 | H | Cl | 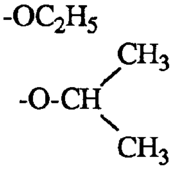 | H | 0 | $-CH_3$ | $-C_2H_5$ | |
| 5.110 | H | Cl | $-O-CH_2-COOCH_3$ | H | 0 | $-CH_3$ | $-C_2H_5$ | |
| 5.111 | H | Cl | $-O-CH(CH_3)COOCH_3$ | H | 0 | $-CH_3$ | $-C_2H_5$ | |
| 5.112 | H | Cl | $-S-CH_2-COOCH_3$ | H | 0 | $-CH_3$ | $-C_2H_5$ | |
| 5.113 | H | Cl | $-S-CH(CH_3)COOCH_3$ | H | 0 | $-CH_3$ | $-C_2H_5$ | |
| 5.114 | H | Cl | 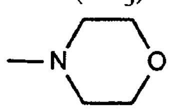 | H | 0 | $-CH_3$ | $-C_2H_5$ | |
| 5.115 | F | Cl | -Cl | H | 1 | $-CH_3$ | $-C_2H_5$ | |
| 5.116 | F | Cl | -OH | H | 1 | $-CH_3$ | $-C_2H_5$ | |
| 5.117 | F | Cl | $-OCH_3$ | H | 1 | $-CH_3$ | $-C_2H_5$ | |
| 5.118 | F | Cl | $-OC_2H_5$ | H | 1 | $-CH_3$ | $-C_2H_5$ | |
| 5.119 | F | Cl | $-OC_3H_7$ | H | 1 | $-CH_3$ | $-C_2H_5$ | |
| 5.120 | F | Cl | 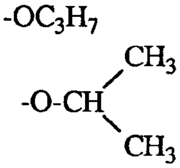 | H | 1 | $-CH_3$ | $-C_2H_5$ | |
| 5.121 | F | Cl | $-OC_4H_9$ | H | 1 | $-CH_3$ | $-C_2H_5$ | |
| 5.122 | F | Cl | $-O-CH(CH_3)-C_2H_5$ | H | 1 | $-CH_3$ | $-C_2H_5$ | |
| 5.123 | F | Cl | 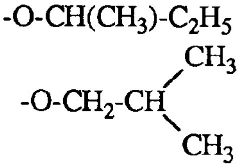 | H | 1 | $-CH_3$ | $-C_2H_5$ | |
| 5.124 | F | Cl | $-OC_5H_{11}$ | H | 1 | $-CH_3$ | $-C_2H_5$ | |
| 5.125 | F | Cl | $-O-CH_2-CH_2-O-CH_3$ | H | 1 | $-CH_3$ | $-C_2H_5$ | |
| 5.126 | F | Cl | $-O-CH_2-CH_2-O-C_2H_5$ | H | 1 | $-CH_3$ | $-C_2H_5$ | |

Tabelle 5a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.127 | F | Cl | $-O-CH-(CH_3)-CH_2-O-CH_3$ | H | 1 | $-CH_3$ | $-C_2H_5$ | |
| 5.128 | F | Cl | $-O-CH_2-CH_2-S-CH_3$ | H | 1 | $-CH_3$ | $-C_2H_5$ | |
| 5.129 | F | Cl | $-O-CH(CH_3)-CH_2-S-CH_3$ | H | 1 | $-CH_3$ | $-C_2H_5$ | |
| 5.130 | F | Cl | $-O-CH(CH_3)-CH_2-S-C_2H_5$ | H | 1 | $-CH_3$ | $-C_2H_5$ | |
| 5.131 | F | Cl | $-O-CH(CH_3)-CH_2-S-C_3H_7$ | H | 1 | $-CH_3$ | $-C_2H_5$ | |
| 5.132 | F | Cl | 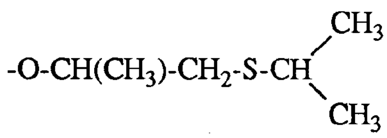 | H | 1 | $-CH_3$ | $-C_2H_5$ | |
| 5.133 | F | Cl | $-O-CH(CH_3)-CH_2-S-C_4H_9$ | H | 1 | $-CH_3$ | $-C_2H_5$ | |
| 5.134 | F | Cl | $-O-CH(CH_3)-CH_2-S-C_5H_{11}$ | H | 1 | $-CH_3$ | $-C_2H_5$ | |
| 5.135 | F | Cl | 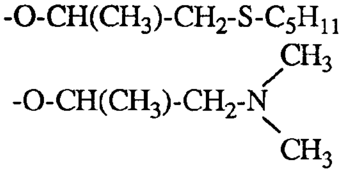 | H | 1 | $-CH_3$ | $-C_2H_5$ | |
| 5.136 | F | Cl | 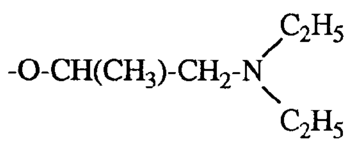 | H | 1 | $-CH_3$ | $-C_2H_5$ | |
| 5.137 | F | Cl | $-NH_2$ | H | 1 | $-CH_3$ | $-C_2H_5$ | |
| 5.138 | F | Cl | 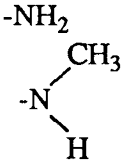 | H | 1 | $-CH_3$ | $-C_2H_5$ | |
| 5.139 | F | Cl | 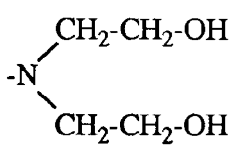 | H | 1 | $-CH_3$ | $-C_2H_5$ | |
| 5.140 | F | Cl | $-NH-CH_2-CH=CH_2$ | H | 1 | $-CH_3$ | $-C_2H_5$ | |
| 5.141 | F | Cl | $-N-(CH_2-CH=CH_2)_2$ | H | 1 | $-CH_3$ | $-C_2H_5$ | |
| 5.142 | F | Cl | 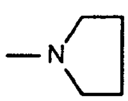 | H | 1 | $-CH_3$ | $-C_2H_5$ | |
| 5.143 | F | Cl | 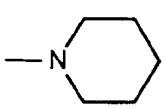 | H | 1 | $-CH_3$ | $-C_2H_5$ | |

Tabelle 5a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.144 | F | Cl | 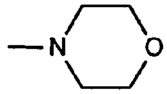 | H | 1 | $-CH_3$ | $-C_2H_5$ | |
| 5.145 | F | Cl | 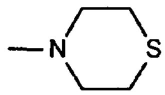 | H | 1 | $-CH_3$ | $-C_2H_5$ | |
| 5.146 | F | Cl | 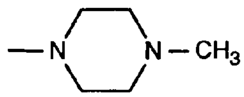 | H | 1 | $-CH_3$ | $-C_2H_5$ | |
| 5.147 | F | Cl | 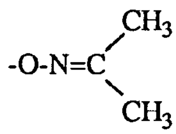 | H | 1 | $-CH_3$ | $-C_2H_5$ | |
| 5.148 | F | Cl | $-O-CH_2-CH_2-Cl$ | H | 1 | $-CH_3$ | $-C_2H_5$ | |
| 5.149 | F | Cl | $-O-CH_2-CN$ | H | 1 | $-CH_3$ | $-C_2H_5$ | |
| 5.150 | F | Cl | 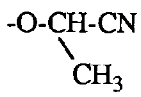 | H | 1 | $-CH_3$ | $-C_2H_5$ | |
| 5.151 | F | Cl | $-O-CH_2-CH=CH_2$ | H | 1 | $-CH_3$ | $-C_2H_5$ | |
| 5.152 | F | Cl | $-O-CH_2-CH=CHCl$ | H | 1 | $-CH_3$ | $-C_2H_5$ | |
| 5.153 | F | Cl | 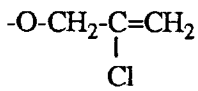 | H | 1 | $-CH_3$ | $-C_2H_5$ | |
| 5.154 | F | Cl | $-O-CH_2=C\equiv CH$ | H | 1 | $-CH_3$ | $-C_2H_5$ | |
| 5.155 | F | Cl | 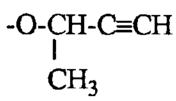 | H | 1 | $-CH_3$ | $-C_2H_5$ | |
| 5.156 | F | Cl | 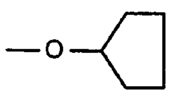 | H | 1 | $-CH_3$ | $-C_2H_5$ | |
| 5.157 | F | Cl | 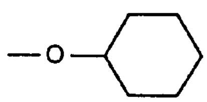 | H | 1 | $-CH_3$ | $-C_2H_5$ | |
| 5.158 | F | Cl | 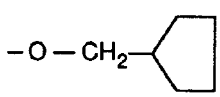 | H | 1 | $-CH_3$ | $-C_2H_5$ | |
| 5.159 | F | Cl | 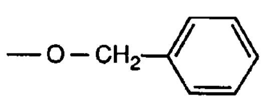 | H | 1 | $-CH_3$ | $-C_2H_5$ | |

Tabelle 5a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.160 | F | Cl | 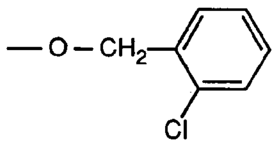 | H | 1 | $-CH_3$ | $-C_2H_5$ | |
| 5.161 | F | Cl | 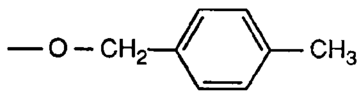 | H | 1 | $-CH_3$ | $-C_2H_5$ | |
| 5.162 | F | Cl | $-S-CH_3$ | H | 1 | $-CH_3$ | $-C_2H_5$ | |
| 5.163 | F | Cl | $-S-C_2H_5$ | H | 1 | $-CH_3$ | $-C_2H_5$ | |
| 5.164 | F | Cl | $-S-C_3H_7$ | H | 1 | $-CH_3$ | $-C_2H_5$ | |
| 5.165 | F | Cl | $-S-CH_2-CH=CH_2$ | H | 1 | $-CH_3$ | $-C_2H_5$ | |
| 5.166 | F | Cl | $-S-CH_2-COOCH_3$ | H | 1 | $-CH_3$ | $-C_2H_5$ | |
| 5.167 | F | Cl | $-S-CH_2-COOC_2H_5$ | H | 1 | $-CH_3$ | $-C_2H_5$ | |
| 5.168 | F | Cl | $-S-CH_2-COOC_5H_{11}$ | H | 1 | $-CH_3$ | $-C_2H_5$ | |
| 5.169 | F | Cl | $-S-CH(CH_3)-COOCH_3$ | H | 1 | $-CH_3$ | $-C_2H_5$ | |
| 5.170 | F | Cl | $-S-(CH_3)-COOC_2H_5$ | H | 1 | $-CH_3$ | $-C_2H_5$ | |
| 5.171 | F | Cl | $-S-CH(CH_3)-COOC_3H_7$ | H | 1 | $-CH_3$ | $-C_2H_5$ | |
| 5.172 | F | Cl | $-S-CH_2-CH_2-COOCH_3$ | H | 1 | $-CH_3$ | $-C_2H_5$ | |
| 5.173 | F | Cl | $-S-CH_2-COOCH_2-CH_2-O-CH_3$ | H | 1 | $-CH_3$ | $-C_2H_5$ | |
| 5.174 | F | Cl | $-O-CH_2-COOCH_3$ | H | 1 | $-CH_3$ | $-C_2H_5$ | |
| 5.175 | F | Cl | $-O-CH(CH_3)-COOCH_3$ | H | 1 | $-CH_3$ | $-C_2H_5$ | |
| 5.176 | F | Cl | $-O-CH_2-COOC_5H_{11}$ | H | 1 | $-CH_3$ | $-C_2H_5$ | |
| 5.177 | F | Cl | $-O-CH_2-CH_3-Si(CH_3)_3$ | H | 1 | $-CH_3$ | $-C_2H_5$ | |
| 5.178 | F | Cl | -ONa | H | 1 | $-CH_3$ | $-C_2H_5$ | |
| 5.179 | F | Br | -Cl | H | 1 | $-CH_3$ | $-C_2H_5$ | |
| 5.180 | F | Br | -OH | H | 1 | $-CH_3$ | $-C_2H_5$ | |
| 5.181 | F | Br | $-OCH_3$ | H | 1 | $-CH_3$ | $-C_2H_5$ | |
| 5.182 | F | Br | $-OC_2H_5$ | H | 1 | $-CH_3$ | $-C_2H_5$ | |
| 5.183 | F | Br | $-OC_3H_7$ | H | 1 | $-CH_3$ | $-C_2H_5$ | |
| 5.184 | F | Br | 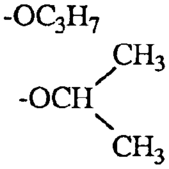 | H | 1 | $-CH_3$ | $-C_2H_5$ | |
| 5.185 | F | Br | $-OC_4H_9$ | H | 1 | $-CH_3$ | $-C_2H_5$ | |

Tabelle 5a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.186 | F | Br | $-OCH(CH_3)-CH_2-CH_3$ | H | 1 | $-CH_3$ | $-C_2H_5$ | |
| 5.187 | F | Br | 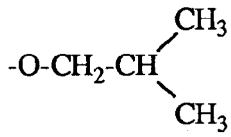 | H | 1 | $-CH_3$ | $-C_2H_5$ | |
| 5.188 | F | Br | $-O-C_5H_{11}$ | H | 1 | $-CH_3$ | $-C_2H_5$ | |
| 5.189 | F | Br | $-O-CH_2-CH_2-O-CH_3$ | H | 1 | $-CH_3$ | $-C_2H_5$ | |
| 5.190 | F | Br | $-O-CH_2-CH_2-O-C_2H_5$ | H | 1 | $-CH_3$ | $-C_2H_5$ | |
| 5.191 | F | Br | $-O-CH(CH_3)-CH_2-O-CH_3$ | H | 1 | $-CH_3$ | $-C_2H_5$ | |
| 5.192 | F | Br | $-O-CH_2-CH_2-S-CH_3$ | H | 1 | $-CH_3$ | $-C_2H_5$ | |
| 5.193 | F | Br | $-O-CH_2(CH_3)-S-CH_3$ | H | 1 | $-CH_3$ | $-C_2H_5$ | |
| 5.194 | F | Br | $-O-CH-(CH_3)-S-C_2H_5$ | H | 1 | $-CH_3$ | $-C_2H_5$ | |
| 5.195 | F | Br | $-O-CH(CH_3)-S-C_3H_7$ | H | 1 | $-CH_3$ | $-C_2H_5$ | |
| 5.196 | F | Br | 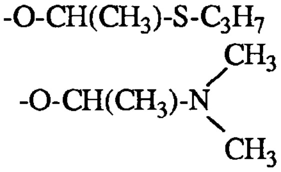 | H | 1 | $-CH_3$ | $-C_2H_5$ | |
| 5.197 | F | Br | $-NH_2$ | H | 1 | $-CH_3$ | $-C_2H_5$ | |
| 5.198 | F | Br | 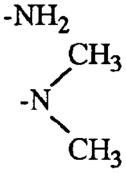 | H | 1 | $-CH_3$ | $-C_2H_5$ | |
| 5.199 | F | Br | 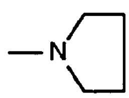 | H | 1 | $-CH_3$ | $-C_2H_5$ | |
| 5.200 | F | Br | 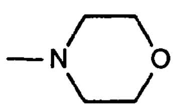 | H | 1 | $-CH_3$ | $-C_2H_5$ | |
| 5.201 | F | Br | 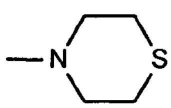 | H | 1 | $-CH_3$ | $-C_2H_5$ | |
| 5.202 | F | Br | 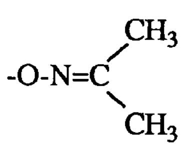 | H | 1 | $-CH_3$ | $-C_2H_5$ | |
| 5.203 | F | Br | $-O-CH_2-CH_2-Cl$ | H | 1 | $-CH_3$ | $-C_2H_5$ | |
| 5.204 | F | Br | $-O-CH_2-CN$ | H | 1 | $-CH_3$ | $-C_2H_5$ | |
| 5.205 | F | Br | $-O-CH_2-CH=CH_2$ | H | 1 | $-CH_3$ | $-C_2H_5$ | |
| 5.206 | F | Br | $-O-CH_2-C\equiv CH$ | H | 1 | $-CH_3$ | $-C_2H_5$ | |

<u>Tabelle 5a</u> (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.207 | F | Br | 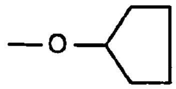 | H | 1 | $-CH_3$ | $-C_2H_5$ | |
| 5.208 | F | Br | 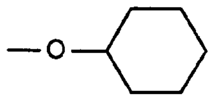 | H | 1 | $-CH_3$ | $-C_2H_5$ | |
| 5.209 | F | Br | 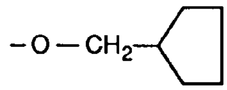 | H | 1 | $-CH_3$ | $-C_2H_5$ | |
| 5.210 | F | Br | 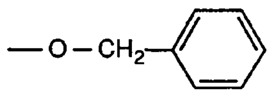 | H | 1 | $-CH_3$ | $-C_2H_5$ | |
| 5.211 | F | Br | $-SCH_3$ | H | 1 | $-CH_3$ | $-C_2H_5$ | |
| 5.212 | F | Br | $-S-CH_2-COOCH_3$ | H | 1 | $-CH_3$ | $-C_2H_5$ | |
| 5.213 | F | Br | $-S-CH(CH_3)-COOCH_3$ | H | 1 | $-CH_3$ | $-C_2H_5$ | |
| 5.214 | F | Br | $-O-CH_2-COOCH_3$ | H | 1 | $-CH_3$ | $-C_2H_5$ | |
| 5.215 | F | Br | $-O-CH(CH_3)COOCH_3$ | H | 1 | $-CH_3$ | $-C_2H_5$ | |
| 5.216 | F | CN | -Cl | H | 1 | $-CH_3$ | $-C_2H_5$ | |
| 5.217 | F | CN | -OH | H | 1 | $-CH_3$ | $-C_2H_5$ | |
| 5.218 | F | CN | $-OCH_3$ | H | 1 | $-CH_3$ | $-C_2H_5$ | |
| 5.219 | H | Cl | -Cl | H | 1 | $-CH_3$ | $-C_2H_5$ | |
| 5.220 | H | Cl | -OH | H | 1 | $-CH_3$ | $-C_2H_5$ | |
| 5.221 | H | Cl | $-OCH_3$ | H | 1 | $-CH_3$ | $-C_2H_5$ | |
| 5.222 | H | Cl | $-OC_2H_5$ | H | 1 | $-CH_3$ | $-C_2H_5$ | |
| 5.223 | H | Cl | 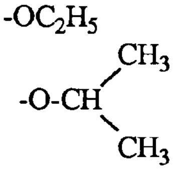 | H | 1 | $-CH_3$ | $-C_2H_5$ | |
| 5.224 | H | Cl | $-O-CH_2-COOCH_3$ | H | 1 | $-CH_3$ | $-C_2H_5$ | |
| 5.225 | H | Cl | $-O-CH(CH_3)COOCH_3$ | H | 1 | $-CH_3$ | $-C_2H_5$ | |
| 5.226 | H | Cl | $-S-CH_2-COOCH_3$ | H | 1 | $-CH_3$ | $-C_2H_5$ | |
| 5.227 | H | Cl | $-S-CH(CH_3)COOCH_3$ | H | 1 | $-CH_3$ | $-C_2H_5$ | |
| 5.228 | H | Cl | 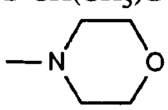 | H | 1 | $-CH_3$ | $-C_2H_5$ | |
| 5.229 | F | Cl | Cl | H | 2 | $-CH_3$ | $-C_2H_5$ | |

Tabelle 5a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.230 | F | Cl | OH | H | 2 | $-CH_3$ | $-C_2H_5$ | |
| 5.231 | F | Cl | $OCH_3$ | H | 2 | $-CH_3$ | $-C_2H_5$ | |
| 5.232 | F | Cl | $-OC_2H_5$ | H | 2 | $-CH_3$ | $-C_2H_5$ | |
| 5.233 | F | Cl | 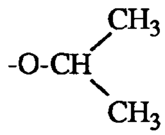 | H | 2 | $-CH_3$ | $-C_2H_5$ | |
| 5.234 | F | Cl | $-O-CH_2-CH_2-O-CH_3$ | H | 2 | $-CH_3$ | $-C_2H_5$ | |
| 5.235 | F | Cl | $-O-CH(CH_3)-CH_2-S-CH_3$ | H | 2 | $-CH_3$ | $-C_2H_5$ | |
| 5.236 | F | Cl | $-O-CH_2-COOCH_3$ | H | 2 | $-CH_3$ | $-C_2H_5$ | |
| 5.237 | F | Cl | $-S-CH_2-COOCH_3$ | H | 2 | $-CH_3$ | $-C_2H_5$ | |
| 5.238 | F | Cl | $-CH_2-CH=CH_2$ | H | 2 | $-CH_3$ | $-C_2H_5$ | |
| 5.239 | F | Cl | $-CH_2-C\equiv CH$ | H | 2 | $-CH_3$ | $-C_2H_5$ | |
| 5.240 | F | Cl | Cl | H | 3 | $-CH_3$ | $-C_2H_5$ | |
| 5.241 | F | Cl | OH | H | 3 | $-CH_3$ | $-C_2H_5$ | |
| 5.242 | F | Cl | $OCH_3$ | H | 3 | $-CH_3$ | $-C_2H_5$ | |
| 5.243 | F | Cl | $OC_2H_5$ | H | 3 | $-CH_3$ | $-C_2H_5$ | |
| 5.244 | F | Cl | 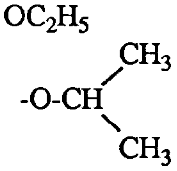 | H | 3 | $-CH_3$ | $-C_2H_5$ | |
| 5.245 | F | Cl | $-O-CH_2-CH_2-O-CH$ | H | 3 | $-CH_3$ | $-C_2H_5$ | |
| 5.246 | F | Cl | $-O-CH(CH_3)-CH_2-S-CH_3$ | H | 3 | $-CH_3$ | $-C_2H_5$ | |
| 5.247 | F | Cl | $-O-CH_2-COOCH_3$ | H | 3 | $-CH_3$ | $-C_2H_5$ | |
| 5.248 | F | Cl | $-S-CH_2-COOCH_3$ | H | 3 | $-CH_3$ | $-C_2H_5$ | |
| 5.249 | F | Cl | $-O-CH_2-C\equiv CH$ | H | 3 | $-CH_3$ | $-C_2H_5$ | |
| 5.250 | F | Cl | -Cl | Cl | 0 | $-CH_3$ | $-C_2H_5$ | |
| 5.251 | F | Cl | -OH | Cl | 0 | $-CH_3$ | $-C_2H_5$ | |
| 5.252 | F | Cl | $-OCH_3$ | Cl | 0 | $-CH_3$ | $-C_2H_5$ | |
| 5.253 | F | Cl | $-OC_2H_5$ | Cl | 0 | $-CH_3$ | $-C_2H_5$ | |
| 5.254 | F | Cl | 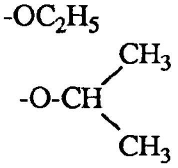 | Cl | 0 | $-CH_3$ | $-C_2H_5$ | |
| 5.255 | F | Cl | $-O-CH_2-COOCH_3$ | Cl | 0 | $-CH_3$ | $-C_2H_5$ | |
| 5.256 | F | Cl | $-S-CH_2-COOCH_3$ | Cl | 0 | $-CH_3$ | $-C_2H_5$ | |
| 5.257 | F | Cl | $-OCH_3$ | Br | 0 | $-CH_3$ | $-C_2H_5$ | |

Tabelle 5a (Fortsetzung)

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.258 | F | Cl | 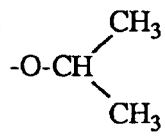 | Br | 0 | $-CH_3$ | $-C_2H_5$ | |
| 5.259 | F | Cl | $-OCH_3$ | F | 0 | $-CH_3$ | $-C_2H_5$ | |
| 5.260 | F | Cl | $-OCH_3$ | $CH_3$ | 0 | $-CH_3$ | $-C_2H_5$ | |
| 5.261 | F | Cl | $-OC_2H_5$ | $CH_3$ | 0 | $-CH_3$ | $-C_2H_5$ | |
| 5.262 | F | Cl | 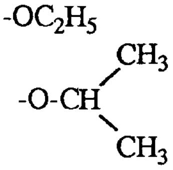 | $CH_3$ | 0 | $-CH_3$ | $-C_2H_5$ | |
| 5.263 | F | Cl | $-O-CH_2-CH_2-O-CH_3$ | $CH_3$ | 0 | $-CH_3$ | $-C_2H_5$ | |
| 5.264 | F | Cl | $-O-CH_2-COOCH_3$ | $CH_3$ | 0 | $-CH_3$ | $-C_2H_5$ | |
| 5.265 | F | Cl | $-O-CH(CH_3)COOCH_3$ | $CH_3$ | 0 | $-CH_3$ | $-C_2H_5$ | |
| 5.266 | F | Cl | $-S-CH_2-COOCH_3$ | $CH_3$ | 0 | $-CH_3$ | $-C_2H_5$ | |
| 5.267 | F | Cl | $-OCH_3$ | $CF_3$ | 0 | $-CH_3$ | $-C_2H_5$ | |
| 5.268 | F | Cl | $-OC_2H_5$ | $CF_3$ | 0 | $-CH_3$ | $-C_2H_5$ | |
| 5.269 | F | Cl | 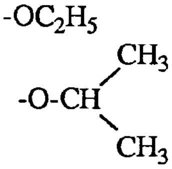 | $CF_3$ | 0 | $-CH_3$ | $-C_2H_5$ | |
| 5.270 | F | Cl | $-O-CH_2-COOCH_3$ | $CF_3$ | 0 | $-CH_3$ | $-C_2H_5$ | |
| 5.271 | F | Cl | $-S-CH_2-COOCH_3$ | $CF_3$ | 0 | $-CH_3$ | $-C_2H_5$ | |

Tabelle 5b:

Verbindungen der Formel I mit W = $W_5$, R = -CN und $R_{14}$ = H:

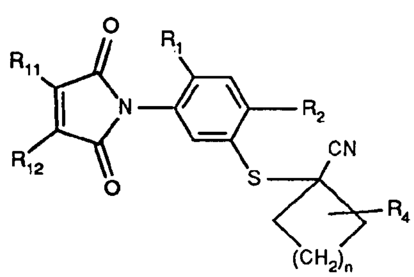 (I)

Tabelle 5b

| Verb. Nr. | $R_1$ | $R_2$ | $R_3$ | $R_4$ | n | $R_{11}$ | $R_{12}$ | Smp. [°C] |
|---|---|---|---|---|---|---|---|---|
| 5.272 | F | Cl | - | H | 0 | $-CH_3$ | $-C_2H_5$ | |
| 5.273 | F | Cl | - | H | 1 | $-CH_3$ | $-C_2H_5$ | |
| 5.274 | F | Cl | - | H | 2 | $-CH_3$ | $-C_2H_5$ | |
| 5.275 | F | Cl | - | H | 3 | $-CH_3$ | $-C_2H_5$ | |
| 5.276 | F | Cl | - | H | 4 | $-CH_3$ | $-C_2H_5$ | |

Tabelle 6a:

Verbindungen der Formel I mit W = $W_6$, A = $-CO-R_3$ und $R_{14}$ = H:

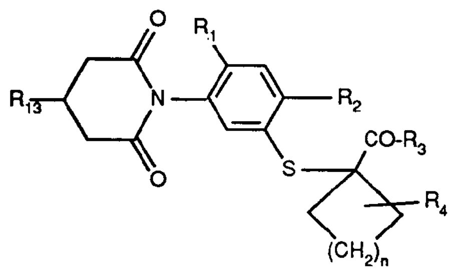

(I)

Tabelle 6a

| Verb. Nr. | $R_1$ | $R_2$ | $R_3$ | $R_4$ | n | $R_{13}$ | Smp. [°C] |
|---|---|---|---|---|---|---|---|
| 6.001 | F | Cl | -Cl | H | 0 | $CF_3$ | |
| 6.002 | F | Cl | -OH | H | 0 | $CF_3$ | |
| 6.003 | F | Cl | $-OCH_3$ | H | 0 | $CF_3$ | |
| 6.004 | F | Cl | $-OC_2H_5$ | H | 0 | $CF_3$ | |
| 6.005 | F | Cl | $-OC_3H_7$ | H | 0 | $CF_3$ | |
| 6.006 | F | Cl | 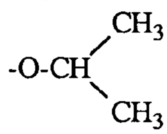 | H | 0 | $CF_3$ | |
| 6.007 | F | Cl | $-OC_4H_9$ | H | 0 | $CF_3$ | |
| 6.008 | F | Cl | $-O-CH(CH_3)-C_2H_5$ | H | 0 | $CF_3$ | |
| 6.009 | F | Cl | 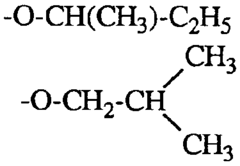 | H | 0 | $CF_3$ | |
| 6.010 | F | Cl | $-OC_5H_{11}$ | H | 0 | $CF_3$ | |
| 6.011 | F | Cl | $-O-CH_2-CH_2-O-CH_3$ | H | 0 | $CF_3$ | |
| 6.012 | F | Cl | $-O-CH_2-CH_2-O-C_2H_5$ | H | 0 | $CF_3$ | |
| 6.013 | F | Cl | $-O-CH-(CH_3)-CH_2-O-CH_3$ | H | 0 | $CF_3$ | |
| 6.014 | F | Cl | $-O-CH_2-CH_2-S-CH_3$ | H | 0 | $CF_3$ | |
| 6.015 | F | Cl | $-O-CH(CH_3)-CH_2-S-CH_3$ | H | 0 | $CF_3$ | |
| 6.016 | F | Cl | $-O-CH(CH_3)-CH_2-S-C_2H_5$ | H | 0 | $CF_3$ | |
| 6.017 | F | Cl | $-O-CH(CH_3)-CH_2-S-C_3H_7$ | H | 0 | $CF_3$ | |
| 6.018 | F | Cl | 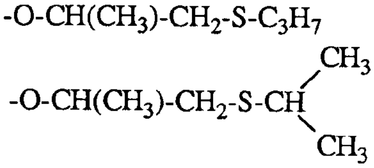 | H | 0 | $CF_3$ | |
| 6.019 | F | Cl | $-O-CH(CH_3)-CH_2-S-C_4H_9$ | H | 0 | $CF_3$ | |
| 6.020 | F | Cl | $-O-CH(CH_3)-CH_2-S-C_5H_{11}$ | H | 0 | $CF_3$ | |
| 6.021 | F | Cl | 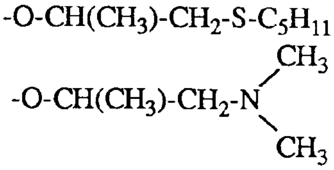 | H | 0 | $CF_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.022 | F | Cl | 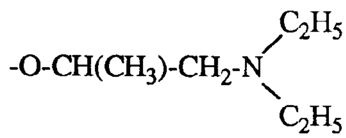 | H | 0 | $CF_3$ | |
| 6.023 | F | Cl | $-NH_2$ | H | 0 | $CF_3$ | |
| 6.024 | F | Cl | 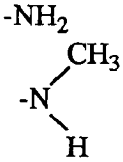 | H | 0 | $CF_3$ | |
| 6.025 | F | Cl | 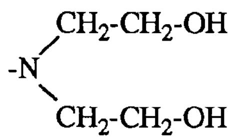 | H | 0 | $CF_3$ | |
| 6.026 | F | Cl | $-NH-CH_2-CH=CH_2$ | H | 0 | $CF_3$ | |
| 6.027 | F | Cl | $-N-(CH_2-CH=CH_2)_2$ | H | 0 | $CF_3$ | |
| 6.028 | F | Cl | 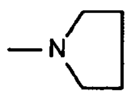 | H | 0 | $CF_3$ | |
| 6.029 | F | Cl | 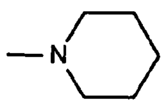 | H | 0 | $CF_3$ | |
| 6.030 | F | Cl | 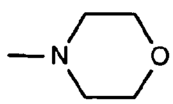 | H | 0 | $CF_3$ | |
| 6.031 | F | Cl | 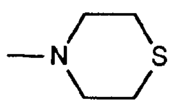 | H | 0 | $CF_3$ | |
| 6.032 | F | Cl | 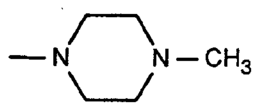 | H | 0 | $CF_3$ | |
| 6.033 | F | Cl | 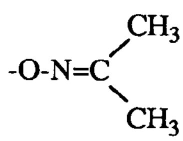 | H | 0 | $CF_3$ | |
| 6.034 | F | Cl | $-O-CH_2-CH_2-Cl$ | H | 0 | $CF_3$ | |
| 6.035 | F | Cl | $-O-CH_2-CN$ | H | 0 | $CF_3$ | |
| 6.036 | F | Cl | 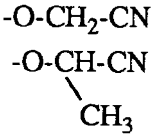 | H | 0 | $CF_3$ | |
| 6.037 | F | Cl | $-O-CH_2-CH=CH_2$ | H | 0 | $CF_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.038 | F | Cl | $-O-CH_2-CH=CHCl$ | H | 0 | $CF_3$ | |
| 6.039 | F | Cl | $-O-CH_2-C=CH_2$<br>\|<br>Cl | H | 0 | $CF_3$ | |
| 6.040 | F | Cl | $-O-CH_2=C\equiv CH$ | H | 0 | $CF_3$ | |
| 6.041 | F | Cl | $-O-CH-C\equiv CH$<br>\|<br>$CH_3$ | H | 0 | $CF_3$ | |
| 6.042 | F | Cl | 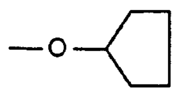 | H | 0 | $CF_3$ | |
| 6.043 | F | Cl | 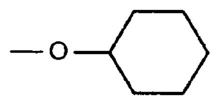 | H | 0 | $CF_3$ | |
| 6.044 | F | Cl | 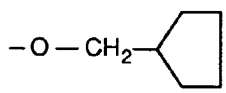 | H | 0 | $CF_3$ | |
| 6.045 | F | Cl | 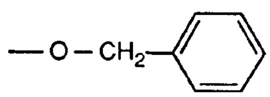 | H | 0 | $CF_3$ | |
| 6.046 | F | Cl | 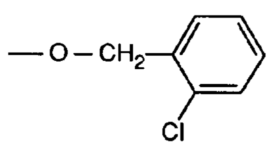 | H | 0 | $CF_3$ | |
| 6.047 | F | Cl | 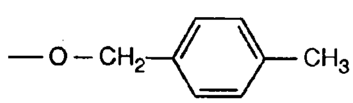 | H | 0 | $CF_3$ | |
| 6.048 | F | Cl | $-S-CH_3$ | H | 0 | $CF_3$ | |
| 6.049 | F | Cl | $-S-C_2H_5$ | H | 0 | $CF_3$ | |
| 6.050 | F | Cl | $-S-C_3H_7$ | H | 0 | $CF_3$ | |
| 6.051 | F | Cl | $-S-CH_2-CH=CH_2$ | H | 0 | $CF_3$ | |
| 6.052 | F | Cl | $-S-CH_2-COOCH_3$ | H | 0 | $CF_3$ | |
| 6.053 | F | Cl | $-S-CH_2-COOC_2H_5$ | H | 0 | $CF_3$ | |
| 6.054 | F | Cl | $-S-CH_2-COOC_5H_{11}$ | H | 0 | $CF_3$ | |
| 6.055 | F | Cl | $-S-CH(CH_3)-COOCH_3$ | H | 0 | $CF_3$ | |
| 6.056 | F | Cl | $-S-(CH_3)-COOC_2H_5$ | H | 0 | $CF_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.057 | F | Cl | $-S-CH(CH_3)-COOC_3H_7$ | H | 0 | $CF_3$ | |
| 6.058 | F | Cl | $-S-CH_2-CH_2-COOCH_3$ | H | 0 | $CF_3$ | |
| 6.059 | F | Cl | $-S-CH_2-COOCH_2-CH_2-O-CH_3$ | H | 0 | $CF_3$ | |
| 6.060 | F | Cl | $-O-CH_2-COOCH_3$ | H | 0 | $CF_3$ | |
| 6.061 | F | Cl | $-O-CH(CH_3)-COOCH_3$ | H | 0 | $CF_3$ | |
| 6.062 | F | Cl | $-O-CH_2-COOC_5H_{11}$ | H | 0 | $CF_3$ | |
| 6.063 | F | Cl | $-O-CH_2-CH_3-Si(CH_3)_3$ | H | 0 | $CF_3$ | |
| 6.064 | F | Cl | -ONa | H | 0 | $CF_3$ | |
| 6.065 | F | Br | -Cl | H | 0 | $CF_3$ | |
| 6.066 | F | Br | -OH | H | 0 | $CF_3$ | |
| 6.067 | F | Br | $-OCH_3$ | H | 0 | $CF_3$ | |
| 6.068 | F | Br | $-OC_2H_5$ | H | 0 | $CF_3$ | |
| 6.069 | F | Br | $-OC_3H_7$ | H | 0 | $CF_3$ | |
| 6.070 | F | Br | 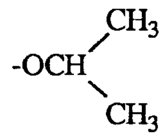 | H | 0 | $CF_3$ | |
| 6.071 | F | Br | $-OC_4H_9$ | H | 0 | $CF_3$ | |
| 6.072 | F | Br | $-OCH(CH_3)-CH_2-CH_3$ | H | 0 | $CF_3$ | |
| 6.073 | F | Br | 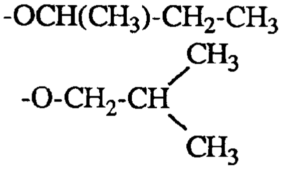 | H | 0 | $CF_3$ | |
| 6.074 | F | Br | $-O-C_5H_{11}$ | H | 0 | $CF_3$ | |
| 6.075 | F | Br | $-O-CH_2-CH_2-O-CH_3$ | H | 0 | $CF_3$ | |
| 6.076 | F | Br | $-O-CH_2-CH_2-O-C_2H_5$ | H | 0 | $CF_3$ | |
| 6.077 | F | Br | $-O-CH(CH_3)-CH_2-O-CH_3$ | H | 0 | $CF_3$ | |
| 6.078 | F | Br | $-O-CH_2-CH_2-S-CH_3$ | H | 0 | $CF_3$ | |
| 6.079 | F | Br | $-O-CH_2(CH_3)-S-CH_3$ | H | 0 | $CF_3$ | |
| 6.080 | F | Br | $-O-CH-(CH_3)-S-C_2H_5$ | H | 0 | $CF_3$ | |
| 6.081 | F | Br | $-O-CH(CH_3)-S-C_3H_7$ | H | 0 | $CF_3$ | |
| 6.082 | F | Br | 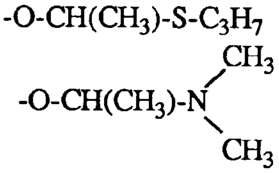 | H | 0 | $CF_3$ | |
| 6.083 | F | Br | $-NH_2$ | H | 0 | $CF_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.084 | F | Br | 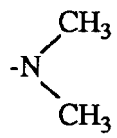 | H | 0 | $CF_3$ | |
| 6.085 | F | Br | 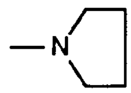 | H | 0 | $CF_3$ | |
| 6.086 | F | Br | 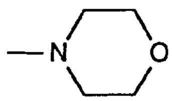 | H | 0 | $CF_3$ | |
| 6.087 | F | Br | 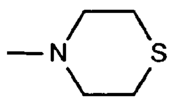 | H | 0 | $CF_3$ | |
| 6.088 | F | Br | 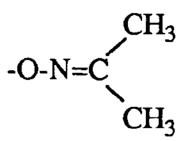 | H | 0 | $CF_3$ | |
| 6.089 | F | Br | $-O-CH_2-CH_2-Cl$ | H | 0 | $CF_3$ | |
| 6.090 | F | Br | $-O-CH_2-CN$ | H | 0 | $CF_3$ | |
| 6.091 | F | Br | $-O-CH_2-CH=CH_2$ | H | 0 | $CF_3$ | |
| 6.092 | F | Br | $-O-CH_2-C≡CH$ | H | 0 | $CF_3$ | |
| 6.093 | F | Br | 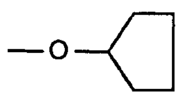 | H | 0 | $CF_3$ | |
| 6.094 | F | Br | 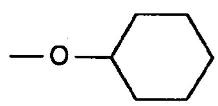 | H | 0 | $CF_3$ | |
| 6.095 | F | Br | 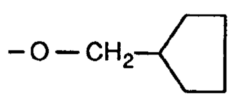 | H | 0 | $CF_3$ | |
| 6.096 | F | Br | 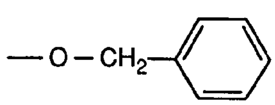 | H | 0 | $CF_3$ | |
| 6.097 | F | Br | $-SCH_3$ | H | 0 | $CF_3$ | |
| 6.098 | F | Br | $-S-CH_2-COOCH_3$ | H | 0 | $CF_3$ | |
| 6.099 | F | Br | $-S-CH(CH_3)-COOCH_3$ | H | 0 | $CF_3$ | |
| 6.100 | F | Br | $-O-CH_2-COOCH_3$ | H | 0 | $CF_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.101 | F | Br | $-O-CH(CH_3)COOCH_3$ | H | 0 | $CF_3$ | |
| 6.102 | F | CN | -Cl | H | 0 | $CF_3$ | |
| 6.103 | F | CN | -OH | H | 0 | $CF_3$ | |
| 6.104 | F | CN | $-OCH_3$ | H | 0 | $CF_3$ | |
| 6.105 | H | Cl | -Cl | H | 0 | $CF_3$ | |
| 6.106 | H | Cl | -OH | H | 0 | $CF_3$ | |
| 6.107 | H | Cl | $-OCH_3$ | H | 0 | $CF_3$ | |
| 6.108 | H | Cl | $-OC_2H_5$ | H | 0 | $CF_3$ | |
| 6.109 | H | Cl | 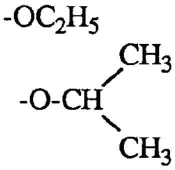 | H | 0 | $CF_3$ | |
| 6.110 | H | Cl | $-O-CH_2-COOCH_3$ | H | 0 | $CF_3$ | |
| 6.111 | H | Cl | $-O-CH(CH_3)COOCH_3$ | H | 0 | $CF_3$ | |
| 6.112 | H | Cl | $-S-CH_2-COOCH_3$ | H | 0 | $CF_3$ | |
| 6.113 | H | Cl | $-S-CH(CH_3)COOCH_3$ | H | 0 | $CF_3$ | |
| 6.114 | H | Cl | 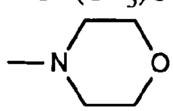 | H | 0 | $CF_3$ | |
| 6.115 | F | Cl | -Cl | H | 1 | $CF_3$ | |
| 6.116 | F | Cl | -OH | H | 1 | $CF_3$ | |
| 6.117 | F | Cl | $-OCH_3$ | H | 1 | $CF_3$ | 115-117 |
| 6.118 | F | Cl | $-OC_2H_5$ | H | 1 | $CF_3$ | |
| 6.119 | F | Cl | $-OC_3H_7$ | H | 1 | $CF_3$ | |
| 6.120 | F | Cl | 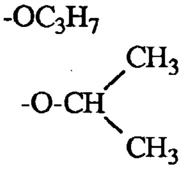 | H | 1 | $CF_3$ | |
| 6.121 | F | Cl | $-OC_4H_9$ | H | 1 | $CF_3$ | |
| 6.122 | F | Cl | $-O-CH(CH_3)-C_2H_5$ | H | 1 | $CF_3$ | |
| 6.123 | F | Cl | 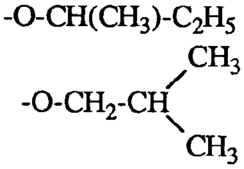 | H | 1 | $CF_3$ | |
| 6.124 | F | Cl | $-OC_5H_{11}$ | H | 1 | $CF_3$ | |
| 6.125 | F | Cl | $-O-CH_2-CH_2-O-CH_3$ | H | 1 | $CF_3$ | |
| 6.126 | F | Cl | $-O-CH_2-CH_2-O-C_2H_5$ | H | 1 | $CF_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.127 | F | Cl | $-O-CH-(CH_3)-CH_2-O-CH_3$ | H | 1 | $CF_3$ | |
| 6.128 | F | Cl | $-O-CH_2-CH_2-S-CH_3$ | H | 1 | $CF_3$ | |
| 6.129 | F | Cl | $-O-CH(CH_3)-CH_2-S-CH_3$ | H | 1 | $CF_3$ | |
| 6.130 | F | Cl | $-O-CH(CH_3)-CH_2-S-C_2H_5$ | H | 1 | $CF_3$ | |
| 6.131 | F | Cl | $-O-CH(CH_3)-CH_2-S-C_3H_7$ | H | 1 | $CF_3$ | |
| 6.132 | F | Cl | 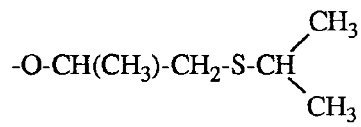 | H | 1 | $CF_3$ | |
| 6.133 | F | Cl | $-O-CH(CH_3)-CH_2-S-C_4H_9$ | H | 1 | $CF_3$ | |
| 6.134 | F | Cl | $-O-CH(CH_3)-CH_2-S-C_5H_{11}$ | H | 1 | $CF_3$ | |
| 6.135 | F | Cl | 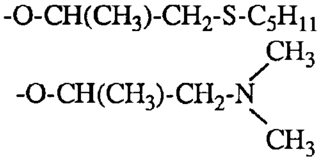 | H | 1 | $CF_3$ | |
| 6.136 | F | Cl | 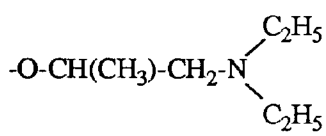 | H | 1 | $CF_3$ | |
| 6.137 | F | Cl | $-NH_2$ | H | 1 | $CF_3$ | |
| 6.138 | F | Cl | 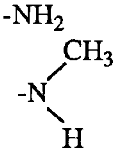 | H | 1 | $CF_3$ | |
| 6.139 | F | Cl | 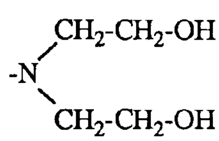 | H | 1 | $CF_3$ | |
| 6.140 | F | Cl | $-NH-CH_2-CH=CH_2$ | H | 1 | $CF_3$ | |
| 6.141 | F | Cl | $-N-(CH_2-CH=CH_2)_2$ | H | 1 | $CF_3$ | |
| 6.142 | F | Cl | 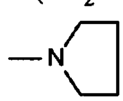 | H | 1 | $CF_3$ | |
| 6.143 | F | Cl | 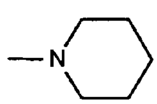 | H | 1 | $CF_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.144 | F | Cl | 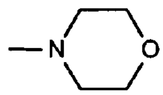 | H | 1 | $CF_3$ | |
| 6.145 | F | Cl | 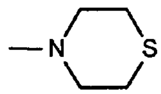 | H | 1 | $CF_3$ | |
| 6.146 | F | Cl | 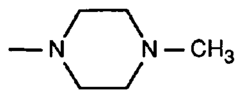 | H | 1 | $CF_3$ | |
| 6.147 | F | Cl | 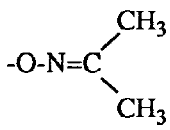 | H | 1 | $CF_3$ | |
| 6.148 | F | Cl | $-O-CH_2-CH_2-Cl$ | H | 1 | $CF_3$ | |
| 6.149 | F | Cl | $-O-CH_2-CN$ | H | 1 | $CF_3$ | |
| 6.150 | F | Cl | 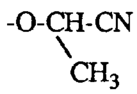 | H | 1 | $CF_3$ | |
| 6.151 | F | Cl | $-O-CH_2-CH=CH_2$ | H | 1 | $CF_3$ | |
| 6.152 | F | Cl | $-O-CH_2-CH=CHCl$ | H | 1 | $CF_3$ | |
| 6.153 | F | Cl | 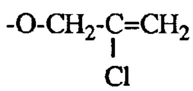 | H | 1 | $CF_3$ | |
| 6.154 | F | Cl | $-O-CH_2=C\equiv CH$ | H | 1 | $CF_3$ | |
| 6.155 | F | Cl | 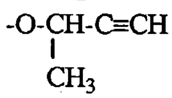 | H | 1 | $CF_3$ | |
| 6.156 | F | Cl | 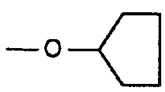 | H | 1 | $CF_3$ | |
| 6.157 | F | Cl | 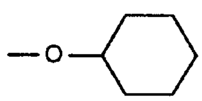 | H | 1 | $CF_3$ | |
| 6.158 | F | Cl | 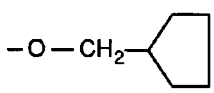 | H | 1 | $CF_3$ | |
| 6.159 | F | Cl | 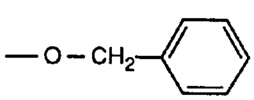 | H | 1 | $CF_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.160 | F | Cl | 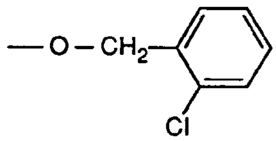 | H | 1 | $CF_3$ | |
| 6.161 | F | Cl | 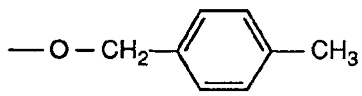 | H | 1 | $CF_3$ | |
| 6.162 | F | Cl | $-S-CH_3$ | H | 1 | $CF_3$ | |
| 6.163 | F | Cl | $-S-C_2H_5$ | H | 1 | $CF_3$ | |
| 6.164 | F | Cl | $-S-C_3H_7$ | H | 1 | $CF_3$ | |
| 6.165 | F | Cl | $-S-CH_2-CH=CH_2$ | H | 1 | $CF_3$ | |
| 6.166 | F | Cl | $-S-CH_2-COOCH_3$ | H | 1 | $CF_3$ | |
| 6.167 | F | Cl | $-S-CH_2-COOC_2H_5$ | H | 1 | $CF_3$ | |
| 6.168 | F | Cl | $-S-CH_2-COOC_5H_{11}$ | H | 1 | $CF_3$ | |
| 6.169 | F | Cl | $-S-CH(CH_3)-COOCH_3$ | H | 1 | $CF_3$ | |
| 6.170 | F | Cl | $-S-(CH_3)-COOC_2H_5$ | H | 1 | $CF_3$ | |
| 6.171 | F | Cl | $-S-CH(CH_3)-COOC_3H_7$ | H | 1 | $CF_3$ | |
| 6.172 | F | Cl | $-S-CH_2-CH_2-COOCH_3$ | H | 1 | $CF_3$ | |
| 6.173 | F | Cl | $-S-CH_2-COOCH_2-CH_2-O-CH_3$ | H | 1 | $CF_3$ | |
| 6.174 | F | Cl | $-O-CH_2-COOCH_3$ | H | 1 | $CF_3$ | |
| 6.175 | F | Cl | $-O-CH(CH_3)-COOCH_3$ | H | 1 | $CF_3$ | |
| 6.176 | F | Cl | $-O-CH_2-COOC_5H_{11}$ | H | 1 | $CF_3$ | |
| 6.177 | F | Cl | $-O-CH_2-CH_3-Si(CH_3)_3$ | H | 1 | $CF_3$ | |
| 6.178 | F | Cl | -ONa | H | 1 | $CF_3$ | |
| 6.179 | F | Br | -Cl | H | 1 | $CF_3$ | |
| 6.180 | F | Br | -OH | H | 1 | $CF_3$ | |
| 6.181 | F | Br | $-OCH_3$ | H | 1 | $CF_3$ | |
| 6.182 | F | Br | $-OC_2H_5$ | H | 1 | $CF_3$ | |
| 6.183 | F | Br | $-OC_3H_7$ | H | 1 | $CF_3$ | |
| 6.184 | F | Br | 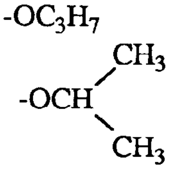 | H | 1 | $CF_3$ | |
| 6.185 | F | Br | $-OC_4H_9$ | H | 1 | $CF_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.186 | F | Br | $-OCH(CH_3)-CH_2-CH_3$ | H | 1 | $CF_3$ | |
| 6.187 | F | Br | 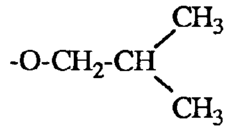 | H | 1 | $CF_3$ | |
| 6.188 | F | Br | $-O-C_5H_{11}$ | H | 1 | $CF_3$ | |
| 6.189 | F | Br | $-O-CH_2-CH_2-O-CH_3$ | H | 1 | $CF_3$ | |
| 6.190 | F | Br | $-O-CH_2-CH_2-O-C_2H_5$ | H | 1 | $CF_3$ | |
| 6.191 | F | Br | $-O-CH(CH_3)-CH_2-O-CH_3$ | H | 1 | $CF_3$ | |
| 6.192 | F | Br | $-O-CH_2-CH_2-S-CH_3$ | H | 1 | $CF_3$ | |
| 6.193 | F | Br | $-O-CH_2(CH_3)-S-CH_3$ | H | 1 | $CF_3$ | |
| 6.194 | F | Br | $-O-CH-(CH_3)-S-C_2H_5$ | H | 1 | $CF_3$ | |
| 6.195 | F | Br | $-O-CH(CH_3)-S-C_3H_7$ | H | 1 | $CF_3$ | |
| 6.196 | F | Br | 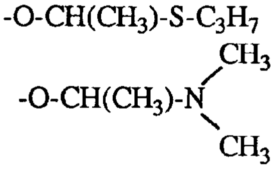 | H | 1 | $CF_3$ | |
| 6.197 | F | Br | $-NH_2$ | H | 1 | $CF_3$ | |
| 6.198 | F | Br | 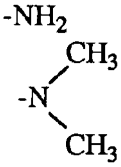 | H | 1 | $CF_3$ | |
| 6.199 | F | Br | 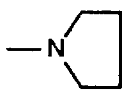 | H | 1 | $CF_3$ | |
| 6.200 | F | Br | 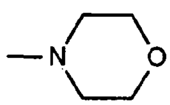 | H | 1 | $CF_3$ | |
| 6.201 | F | Br | 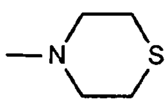 | H | 1 | $CF_3$ | |
| 6.202 | F | Br | 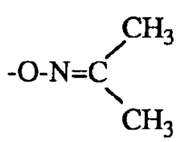 | H | 1 | $CF_3$ | |
| 6.203 | F | Br | $-O-CH_2-CH_2-Cl$ | H | 1 | $CF_3$ | |
| 6.204 | F | Br | $-O-CH_2-CN$ | H | 1 | $CF_3$ | |
| 6.205 | F | Br | $-O-CH_2-CH=CH_2$ | H | 1 | $CF_3$ | |
| 6.206 | F | Br | $-O-CH_2-C\equiv CH$ | H | 1 | $CF_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.207 | F | Br | 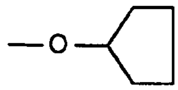 | H | 1 | $CF_3$ | |
| 6.208 | F | Br | 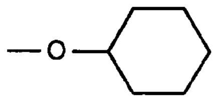 | H | 1 | $CF_3$ | |
| 6.209 | F | Br | 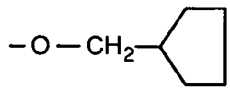 | H | 1 | $CF_3$ | |
| 6.210 | F | Br | 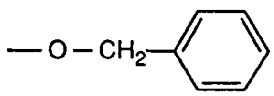 | H | 1 | $CF_3$ | |
| 6.211 | F | Br | $-SCH_3$ | H | 1 | $CF_3$ | |
| 6.212 | F | Br | $-S-CH_2-COOCH_3$ | H | 1 | $CF_3$ | |
| 6.213 | F | Br | $-S-CH(CH_3)-COOCH_3$ | H | 1 | $CF_3$ | |
| 6.214 | F | Br | $-O-CH_2-COOCH_3$ | H | 1 | $CF_3$ | |
| 6.215 | F | Br | $-O-CH(CH_3)COOCH_3$ | H | 1 | $CF_3$ | |
| 6.216 | F | CN | -Cl | H | 1 | $CF_3$ | |
| 6.217 | F | CN | -OH | H | 1 | $CF_3$ | |
| 6.218 | F | CN | $-OCH_3$ | H | 1 | $CF_3$ | |
| 6.219 | H | Cl | -Cl | H | 1 | $CF_3$ | |
| 6.220 | H | Cl | -OH | H | 1 | $CF_3$ | |
| 6.221 | H | Cl | $-OCH_3$ | H | 1 | $CF_3$ | |
| 6.222 | H | Cl | $-OC_2H_5$ | H | 1 | $CF_3$ | |
| 6.223 | H | Cl | 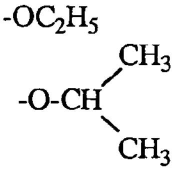 | H | 1 | $CF_3$ | |
| 6.224 | H | Cl | $-O-CH_2-COOCH_3$ | H | 1 | $CF_3$ | |
| 6.225 | H | Cl | $-O-CH(CH_3)COOCH_3$ | H | 1 | $CF_3$ | |
| 6.226 | H | Cl | $-S-CH_2-COOCH_3$ | H | 1 | $CF_3$ | |
| 6.227 | H | Cl | $-S-CH(CH_3)COOCH_3$ | H | 1 | $CF_3$ | |
| 6.228 | H | Cl | 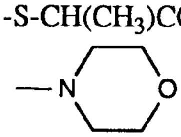 | H | 1 | $CF_3$ | |
| 6.229 | F | Cl | Cl | H | 2 | $CF_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.230 | F | Cl | OH | H | 2 | $CF_3$ | |
| 6.231 | F | Cl | $OCH_3$ | H | 2 | $CF_3$ | |
| 6.232 | F | Cl | $-OC_2H_5$ | H | 2 | $CF_3$ | |
| 6.233 | F | Cl | 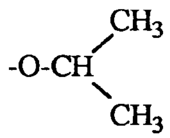 | H | 2 | $CF_3$ | |
| 6.234 | F | Cl | $-O-CH_2-CH_2-O-CH_3$ | H | 2 | $CF_3$ | |
| 6.235 | F | Cl | $-O-CH(CH_3)-CH_2-S-CH_3$ | H | 2 | $CF_3$ | |
| 6.236 | F | Cl | $-O-CH_2-COOCH_3$ | H | 2 | $CF_3$ | |
| 6.237 | F | Cl | $-S-CH_2-COOCH_3$ | H | 2 | $CF_3$ | |
| 6.238 | F | Cl | $-CH_2-CH=CH_2$ | H | 2 | $CF_3$ | |
| 6.239 | F | Cl | $-CH_2-C\equiv CH$ | H | 2 | $CF_3$ | |
| 6.240 | F | Cl | Cl | H | 3 | $CF_3$ | |
| 6.241 | F | Cl | OH | H | 3 | $CF_3$ | |
| 6.242 | F | Cl | $OCH_3$ | H | 3 | $CF_3$ | |
| 6.243 | F | Cl | $OC_2H_5$ | H | 3 | $CF_3$ | |
| 6.244 | F | Cl | 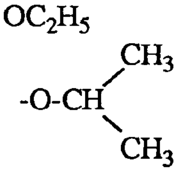 | H | 3 | $CF_3$ | |
| 6.245 | F | Cl | $-O-CH_2-CH_2-O-CH$ | H | 3 | $CF_3$ | |
| 6.246 | F | Cl | $-O-CH(CH_3)-CH_2-S-CH_3$ | H | 3 | $CF_3$ | |
| 6.247 | F | Cl | $-O-CH_2-COOCH_3$ | H | 3 | $CF_3$ | |
| 6.248 | F | Cl | $-S-CH_2-COOCH_3$ | H | 3 | $CF_3$ | |
| 6.249 | F | Cl | $-O-CH_2-C\equiv CH$ | H | 3 | $CF_3$ | |
| 6.250 | F | Cl | -Cl | Cl | 0 | $CF_3$ | |
| 6.251 | F | Cl | -OH | Cl | 0 | $CF_3$ | |
| 6.252 | F | Cl | $-OCH_3$ | Cl | 0 | $CF_3$ | |
| 6.253 | F | Cl | $-OC_2H_5$ | Cl | 0 | $CF_3$ | |
| 6.254 | F | Cl | 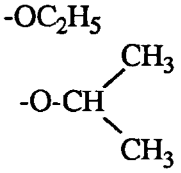 | Cl | 0 | $CF_3$ | |
| 6.255 | F | Cl | $-O-CH_2-COOCH_3$ | Cl | 0 | $CF_3$ | |
| 6.256 | F | Cl | $-S-CH_2-COOCH_3$ | Cl | 0 | $CF_3$ | |
| 6.257 | F | Cl | $-OCH_3$ | Br | 0 | $CF_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.258 | F | Cl | 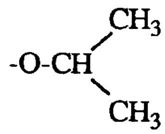 | Br | 0 | $CF_3$ | |
| 6.259 | F | Cl | $-OCH_3$ | F | 0 | $CF_3$ | |
| 6.260 | F | Cl | $-OCH_3$ | $CH_3$ | 0 | $CF_3$ | |
| 6.261 | F | Cl | $-OC_2H_5$ | $CH_3$ | 0 | $CF_3$ | |
| 6.262 | F | Cl | 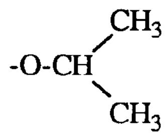 | $CH_3$ | 0 | $CF_3$ | |
| 6.263 | F | Cl | $-O-CH_2-CH_2-O-CH_3$ | $CH_3$ | 0 | $CF_3$ | |
| 6.264 | F | Cl | $-O-CH_2-COOCH_3$ | $CH_3$ | 0 | $CF_3$ | |
| 6.265 | F | Cl | $-O-CH(CH_3)COOCH_3$ | $CH_3$ | 0 | $CF_3$ | |
| 6.266 | F | Cl | $-S-CH_2-COOCH_3$ | $CH_3$ | 0 | $CF_3$ | |
| 6.267 | F | Cl | $-OCH_3$ | $CF_3$ | 0 | $CF_3$ | |
| 6.268 | F | Cl | $-OC_2H_5$ | $CF_3$ | 0 | $CF_3$ | |
| 6.269 | F | Cl | 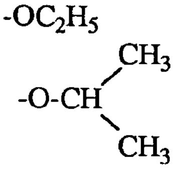 | $CF_3$ | 0 | $CF_3$ | |
| 6.270 | F | Cl | $-O-CH_2-COOCH_3$ | $CF_3$ | 0 | $CF_3$ | |
| 6.271 | F | Cl | $-S-CH_2-COOCH_3$ | $CF_3$ | 0 | $CF_3$ | |
| 6.272 | F | Cl | -Cl | H | 0 | $CH_3$ | |
| 6.273 | F | Cl | -OH | H | 0 | $CH_3$ | |
| 6.274 | F | Cl | $-OCH_3$ | H | 0 | $CH_3$ | |
| 6.275 | F | Cl | $-OC_2H_5$ | H | 0 | $CH_3$ | |
| 6.276 | F | Cl | $-OC_3H_7$ | H | 0 | $CH_3$ | |
| 6.277 | F | Cl | 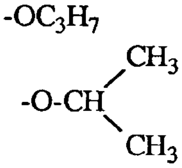 | H | 0 | $CH_3$ | |
| 6.278 | F | Cl | $-OC_4H_9$ | H | 0 | $CH_3$ | |
| 6.279 | F | Cl | $-O-CH(CH_3)-C_2H_5$ | H | 0 | $CH_3$ | |

<u>Tabelle 6a</u> (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.280 | F | Cl | 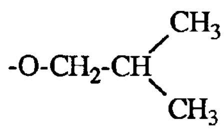 | H | 0 | $CH_3$ | |
| 6.281 | F | Cl | $-OC_5H_{11}$ | H | 0 | $CH_3$ | |
| 6.282 | F | Cl | $-O-CH_2-CH_2-O-CH_3$ | H | 0 | $CH_3$ | |
| 6.283 | F | Cl | $-O-CH_2-CH_2-O-C_2H_5$ | H | 0 | $CH_3$ | |
| 6.284 | F | Cl | $-O-CH-(CH_3)-CH_2-O-CH_3$ | H | 0 | $CH_3$ | |
| 6.285 | F | Cl | $-O-CH_2-CH_2-S-CH_3$ | H | 0 | $CH_3$ | |
| 6.286 | F | Cl | $-O-CH(CH_3)-CH_2-S-CH_3$ | H | 0 | $CH_3$ | |
| 6.287 | F | Cl | $-O-CH(CH_3)-CH_2-S-C_2H_5$ | H | 0 | $CH_3$ | |
| 6.288 | F | Cl | $-O-CH(CH_3)-CH_2-S-C_3H_7$ | H | 0 | $CH_3$ | |
| 6.289 | F | Cl | 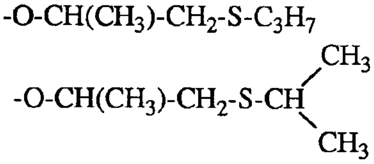 | H | 0 | $CH_3$ | |
| 6.290 | F | Cl | $-O-CH(CH_3)-CH_2-S-C_4H_9$ | H | 0 | $CH_3$ | |
| 6.291 | F | Cl | $-O-CH(CH_3)-CH_2-S-C_5H_{11}$ | H | 0 | $CH_3$ | |
| 6.292 | F | Cl | 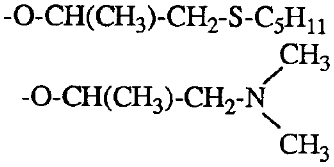 | H | 0 | $CH_3$ | |
| 6.293 | F | Cl | 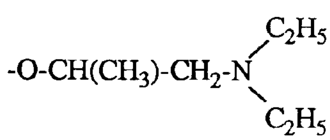 | H | 0 | $CH_3$ | |
| 6.294 | F | Cl | $-NH_2$ | H | 0 | $CH_3$ | |
| 6.295 | F | Cl | 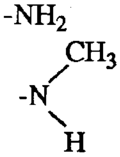 | H | 0 | $CH_3$ | |
| 6.296 | F | Cl | 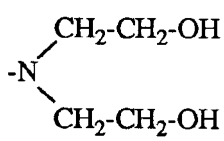 | H | 0 | $CH_3$ | |
| 6.297 | F | Cl | $-NH-CH_2-CH=CH_2$ | H | 0 | $CH_3$ | |
| 6.298 | F | Cl | $-N-(CH_2-CH=CH_2)_2$ | H | 0 | $CH_3$ | |
| 6.299 | F | Cl | 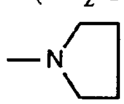 | H | 0 | $CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.300 | F | Cl | 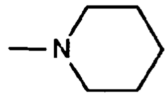 | H | 0 | $CH_3$ | |
| 6.301 | F | Cl | 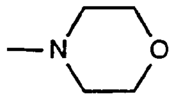 | H | 0 | $CH_3$ | |
| 6.302 | F | Cl | 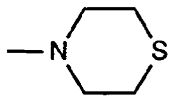 | H | 0 | $CH_3$ | |
| 6.303 | F | Cl | 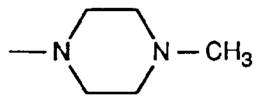 | H | 0 | $CH_3$ | |
| 6.304 | F | Cl | 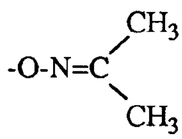 | H | 0 | $CH_3$ | |
| 6.305 | F | Cl | $-O-CH_2-CH_2-Cl$ | H | 0 | $CH_3$ | |
| 6.306 | F | Cl | $-O-CH_2-CN$ | H | 0 | $CH_3$ | |
| 6.307 | F | Cl | 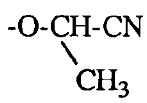 | H | 0 | $CH_3$ | |
| 6.308 | F | Cl | $-O-CH_2-CH=CH_2$ | H | 0 | $CH_3$ | |
| 6.309 | F | Cl | $-O-CH_2-CH=CHCl$ | H | 0 | $CH_3$ | |
| 6.310 | F | Cl | 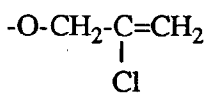 | H | 0 | $CH_3$ | |
| 6.311 | F | Cl | $-O-CH_2=C\equiv CH$ | H | 0 | $CH_3$ | |
| 6.312 | F | Cl | 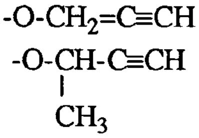 | H | 0 | $CH_3$ | |
| 6.313 | F | Cl | 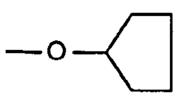 | H | 0 | $CH_3$ | |
| 6.314 | F | Cl | 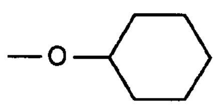 | H | 0 | $CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.315 | F | Cl | 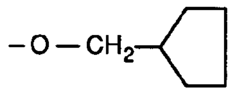 | H | 0 | $CH_3$ | |
| 6.316 | F | Cl | 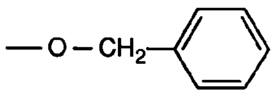 | H | 0 | $CH_3$ | |
| 6.317 | F | Cl | 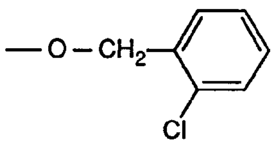 | H | 0 | $CH_3$ | |
| 6.318 | F | Cl | 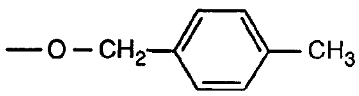 | H | 0 | $CH_3$ | |
| 6.319 | F | Cl | $-S-CH_3$ | H | 0 | $CH_3$ | |
| 6.320 | F | Cl | $-S-C_2H_5$ | H | 0 | $CH_3$ | |
| 6.321 | F | Cl | $-S-C_3H_7$ | H | 0 | $CH_3$ | |
| 6.322 | F | Cl | $-S-CH_2-CH=CH_2$ | H | 0 | $CH_3$ | |
| 6.323 | F | Cl | $-S-CH_2-COOCH_3$ | H | 0 | $CH_3$ | |
| 6.324 | F | Cl | $-S-CH_2-COOC_2H_5$ | H | 0 | $CH_3$ | |
| 6.325 | F | Cl | $-S-CH_2-COOC_5H_{11}$ | H | 0 | $CH_3$ | |
| 6.326 | F | Cl | $-S-CH(CH_3)-COOCH_3$ | H | 0 | $CH_3$ | |
| 6.327 | F | Cl | $-S-(CH_3)-COOC_2H_5$ | H | 0 | $CH_3$ | |
| 6.328 | F | Cl | $-S-CH(CH_3)-COOC_3H_7$ | H | 0 | $CH_3$ | |
| 6.329 | F | Cl | $-S-CH_2-CH_2-COOCH_3$ | H | 0 | $CH_3$ | |
| 6.330 | F | Cl | $-S-CH_2-COOCH_2-CH_2-O-CH_3$ | H | 0 | $CH_3$ | |
| 6.331 | F | Cl | $-O-CH_2-COOCH_3$ | H | 0 | $CH_3$ | |
| 6.332 | F | Cl | $-O-CH(CH_3)-COOCH_3$ | H | 0 | $CH_3$ | |
| 6.333 | F | Cl | $-O-CH_2-COOC_5H_{11}$ | H | 0 | $CH_3$ | |
| 6.334 | F | Cl | $-O-CH_2-CH_3-Si(CH_3)_3$ | H | 0 | $CH_3$ | |
| 6.335 | F | Cl | -ONa | H | 0 | $CH_3$ | |
| 6.336 | F | Br | -Cl | H | 0 | $CH_3$ | |
| 6.337 | F | Br | -OH | H | 0 | $CH_3$ | |
| 6.338 | F | Br | $-OCH_3$ | H | 0 | $CH_3$ | |
| 6.339 | F | Br | $-OC_2H_5$ | H | 0 | $CH_3$ | |
| 6.340 | F | Br | $-OC_3H_7$ | H | 0 | $CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.341 | F | Br | 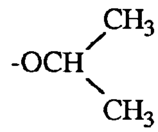 | H | 0 | $CH_3$ | |
| 6.342 | F | Br | $-OC_4H_9$ | H | 0 | $CH_3$ | |
| 6.343 | F | Br | $-OCH(CH_3)-CH_2-CH_3$ | H | 0 | $CH_3$ | |
| 6.344 | F | Br | 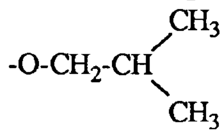 | H | 0 | $CH_3$ | |
| 6.345 | F | Br | $-O-C_5H_{11}$ | H | 0 | $CH_3$ | |
| 6.346 | F | Br | $-O-CH_2-CH_2-O-CH_3$ | H | 0 | $CH_3$ | |
| 6.347 | F | Br | $-O-CH_2-CH_2-O-C_2H_5$ | H | 0 | $CH_3$ | |
| 6.348 | F | Br | $-O-CH(CH_3)-CH_2-O-CH_3$ | H | 0 | $CH_3$ | |
| 6.349 | F | Br | $-O-CH_2-CH_2-S-CH_3$ | H | 0 | $CH_3$ | |
| 6.350 | F | Br | $-O-CH_2(CH_3)-S-CH_3$ | H | 0 | $CH_3$ | |
| 6.351 | F | Br | $-O-CH-(CH_3)-S-C_2H_5$ | H | 0 | $CH_3$ | |
| 6.352 | F | Br | $-O-CH(CH_3)-S-C_3H_7$ | H | 0 | $CH_3$ | |
| 6.353 | F | Br | 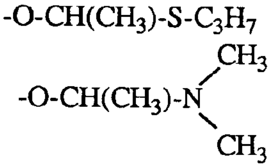 | H | 0 | $CH_3$ | |
| 6.354 | F | Br | $-NH_2$ | H | 0 | $CH_3$ | |
| 6.355 | F | Br | 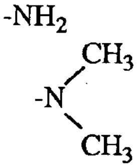 | H | 0 | $CH_3$ | |
| 6.356 | F | Br | 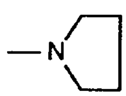 | H | 0 | $CH_3$ | |
| 6.357 | F | Br | 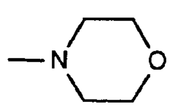 | H | 0 | $CH_3$ | |
| 6.358 | F | Br | 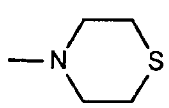 | H | 0 | $CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.359 | F | Br | 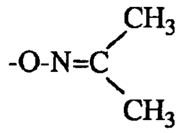 | H | 0 | $CH_3$ | |
| 6.360 | F | Br | $-O-CH_2-CH_2-Cl$ | H | 0 | $CH_3$ | |
| 6.361 | F | Br | $-O-CH_2-CN$ | H | 0 | $CH_3$ | |
| 6.362 | F | Br | $-O-CH_2-CH=CH_2$ | H | 0 | $CH_3$ | |
| 6.363 | F | Br | $-O-CH_2-C \equiv CH$ | H | 0 | $CH_3$ | |
| 6.364 | F | Br | 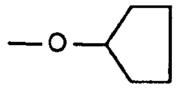 | H | 0 | $CH_3$ | |
| 6.365 | F | Br | 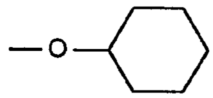 | H | 0 | $CH_3$ | |
| 6.366 | F | Br | 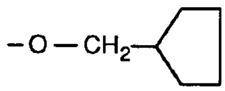 | H | 0 | $CH_3$ | |
| 6.367 | F | Br | 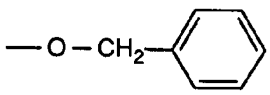 | H | 0 | $CH_3$ | |
| 6.368 | F | Br | $-SCH_3$ | H | 0 | $CH_3$ | |
| 6.369 | F | Br | $-S-CH_2-COOCH_3$ | H | 0 | $CH_3$ | |
| 6.370 | F | Br | $-S-CH(CH_3)-COOCH_3$ | H | 0 | $CH_3$ | |
| 6.371 | F | Br | $-O-CH_2-COOCH_3$ | H | 0 | $CH_3$ | |
| 6.372 | F | Br | $-O-CH(CH_3)COOCH_3$ | H | 0 | $CH_3$ | |
| 6.373 | F | CN | -Cl | H | 0 | $CH_3$ | |
| 6.374 | F | CN | -OH | H | 0 | $CH_3$ | |
| 6.375 | F | CN | $-OCH_3$ | H | 0 | $CH_3$ | |
| 6.376 | H | Cl | -Cl | H | 0 | $CH_3$ | |
| 6.377 | H | Cl | -OH | H | 0 | $CH_3$ | |
| 6.378 | H | Cl | $-OCH_3$ | H | 0 | $CH_3$ | |
| 6.379 | H | Cl | $-OC_2H_5$ | H | 0 | $CH_3$ | |
| 6.380 | H | Cl | 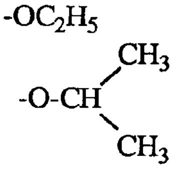 | H | 0 | $CH_3$ | |
| 6.381 | H | Cl | $-O-CH_2-COOCH_3$ | H | 0 | $CH_3$ | |
| 6.382 | H | Cl | $-O-CH(CH_3)COOCH_3$ | H | 0 | $CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.383 | H | Cl | $-S-CH_2-COOCH_3$ | H | 0 | $CH_3$ | |
| 6.384 | H | Cl | $-S-CH(CH_3)COOCH_3$ | H | 0 | $CH_3$ | |
| 6.385 | H | Cl | 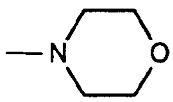 | H | 0 | $CH_3$ | |
| 6.386 | F | Cl | -Cl | H | 1 | $CH_3$ | |
| 6.387 | F | Cl | -OH | H | 1 | $CH_3$ | |
| 6.388 | F | Cl | $-OCH_3$ | H | 1 | $CH_3$ | |
| 6.389 | F | Cl | $-OC_2H_5$ | H | 1 | $CH_3$ | |
| 6.390 | F | Cl | $-OC_3H_7$ | H | 1 | $CH_3$ | |
| 6.391 | F | Cl | 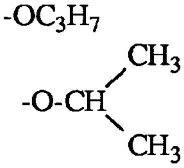 | H | 1 | $CH_3$ | |
| 6.392 | F | Cl | $-OC_4H_9$ | H | 1 | $CH_3$ | |
| 6.393 | F | Cl | $-O-CH(CH_3)-C_2H_5$ | H | 1 | $CH_3$ | |
| 6.394 | F | Cl | 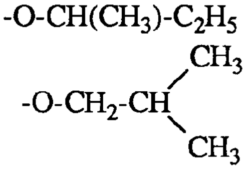 | H | 1 | $CH_3$ | |
| 6.395 | F | Cl | $-OC_5H_{11}$ | H | 1 | $CH_3$ | |
| 6.396 | F | Cl | $-O-CH_2-CH_2-O-CH_3$ | H | 1 | $CH_3$ | |
| 6.397 | F | Cl | $-O-CH_2-CH_2-O-C_2H_5$ | H | 1 | $CH_3$ | |
| 6.398 | F | Cl | $-O-CH-(CH_3)-CH_2-O-CH_3$ | H | 1 | $CH_3$ | |
| 6.399 | F | Cl | $-O-CH_2-CH_2-S-CH_3$ | H | 1 | $CH_3$ | |
| 6.400 | F | Cl | $-O-CH(CH_3)-CH_2-S-CH_3$ | H | 1 | $CH_3$ | |
| 6.401 | F | Cl | $-O-CH(CH_3)-CH_2-S-C_2H_5$ | H | 1 | $CH_3$ | |
| 6.402 | F | Cl | $-O-CH(CH_3)-CH_2-S-C_3H_7$ | H | 1 | $CH_3$ | |
| 6.403 | F | Cl | 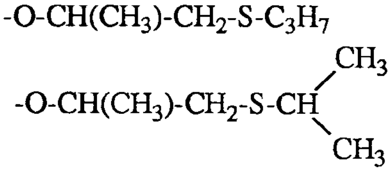 | H | 1 | $CH_3$ | |
| 6.404 | F | Cl | $-O-CH(CH_3)-CH_2-S-C_4H_9$ | H | 1 | $CH_3$ | |
| 6.405 | F | Cl | $-O-CH(CH_3)-CH_2-S-C_5H_{11}$ | H | 1 | $CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.406 | F | Cl | 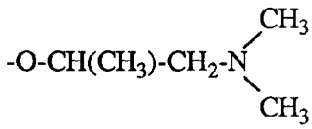 | H | 1 | $CH_3$ | |
| 6.407 | F | Cl | 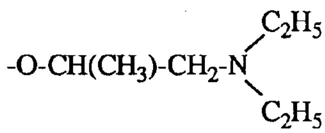 | H | 1 | $CH_3$ | |
| 6.408 | F | Cl | $-NH_2$ | H | 1 | $CH_3$ | |
| 6.409 | F | Cl | 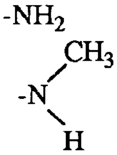 | H | 1 | $CH_3$ | |
| 6.410 | F | Cl | 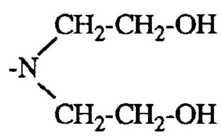 | H | 1 | $CH_3$ | |
| 6.411 | F | Cl | $-NH-CH_2-CH=CH_2$ | H | 1 | $CH_3$ | |
| 6.412 | F | Cl | $-N-(CH_2-CH=CH_2)_2$ | H | 1 | $CH_3$ | |
| 6.413 | F | Cl | 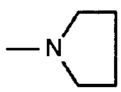 | H | 1 | $CH_3$ | |
| 6.414 | F | Cl | 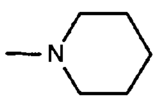 | H | 1 | $CH_3$ | |
| 6.415 | F | Cl | 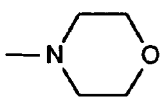 | H | 1 | $CH_3$ | |
| 6.416 | F | Cl | 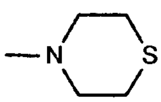 | H | 1 | $CH_3$ | |
| 6.417 | F | Cl | 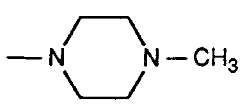 | H | 1 | $CH_3$ | |
| 6.418 | F | Cl | 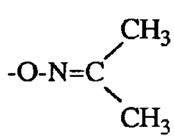 | H | 1 | $CH_3$ | |
| 6.419 | F | Cl | $-O-CH_2-CH_2-Cl$ | H | 1 | $CH_3$ | |
| 6.420 | F | Cl | $-O-CH_2-CN$ | H | 1 | $CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.421 | F | Cl | -O-CH-CN<br>\\<br>$CH_3$ | H | 1 | $CH_3$ | |
| 6.422 | F | Cl | $-O-CH_2-CH=CH_2$ | H | 1 | $CH_3$ | |
| 6.423 | F | Cl | $-O-CH_2-CH=CHCl$ | H | 1 | $CH_3$ | |
| 6.424 | F | Cl | $-O-CH_2-C=CH_2$<br>\|<br>Cl | H | 1 | $CH_3$ | |
| 6.425 | F | Cl | $-O-CH_2=C\equiv CH$ | H | 1 | $CH_3$ | |
| 6.426 | F | Cl | $-O-CH-C\equiv CH$<br>\|<br>$CH_3$ | H | 1 | $CH_3$ | |
| 6.427 | F | Cl | 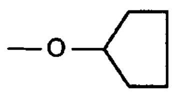 | H | 1 | $CH_3$ | |
| 6.428 | F | Cl | 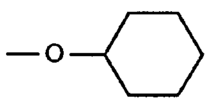 | H | 1 | $CH_3$ | |
| 6.429 | F | Cl | 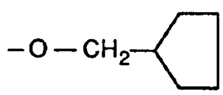 | H | 1 | $CH_3$ | |
| 6.430 | F | Cl | 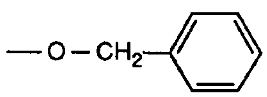 | H | 1 | $CH_3$ | |
| 6.431 | F | Cl | 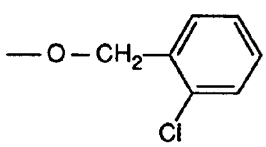 | H | 1 | $CH_3$ | |
| 6.432 | F | Cl | 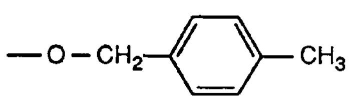 | H | 1 | $CH_3$ | |
| 6.433 | F | Cl | $-S-CH_3$ | H | 1 | $CH_3$ | |
| 6.434 | F | Cl | $-S-C_2H_5$ | H | 1 | $CH_3$ | |
| 6.435 | F | Cl | $-S-C_3H_7$ | H | 1 | $CH_3$ | |
| 6.436 | F | Cl | $-S-CH_2-CH=CH_2$ | H | 1 | $CH_3$ | |
| 6.437 | F | Cl | $-S-CH_2-COOCH_3$ | H | 1 | $CH_3$ | |
| 6.438 | F | Cl | $-S-CH_2-COOC_2H_5$ | H | 1 | $CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.439 | F | Cl | $-S-CH_2-COOC_5H_{11}$ | H | 1 | $CH_3$ | |
| 6.440 | F | Cl | $-S-CH(CH_3)-COOCH_3$ | H | 1 | $CH_3$ | |
| 6.441 | F | Cl | $-S-(CH_3)-COOC_2H_5$ | H | 1 | $CH_3$ | |
| 6.442 | F | Cl | $-S-CH(CH_3)-COOC_3H_7$ | H | 1 | $CH_3$ | |
| 6.443 | F | Cl | $-S-CH_2-CH_2-COOCH_3$ | H | 1 | $CH_3$ | |
| 6.444 | F | Cl | $-S-CH_2-COOCH_2-CH_2-O-CH_3$ | H | 1 | $CH_3$ | |
| 6.445 | F | Cl | $-O-CH_2-COOCH_3$ | H | 1 | $CH_3$ | |
| 6.446 | F | Cl | $-O-CH(CH_3)-COOCH_3$ | H | 1 | $CH_3$ | |
| 6.447 | F | Cl | $-O-CH_2-COOC_5H_{11}$ | H | 1 | $CH_3$ | |
| 6.448 | F | Cl | $-O-CH_2-CH_3-Si(CH_3)_3$ | H | 1 | $CH_3$ | |
| 6.449 | F | Cl | -ONa | H | 1 | $CH_3$ | |
| 6.450 | F | Br | -Cl | H | 1 | $CH_3$ | |
| 6.451 | F | Br | -OH | H | 1 | $CH_3$ | |
| 6.452 | F | Br | $-OCH_3$ | H | 1 | $CH_3$ | |
| 6.453 | F | Br | $-OC_2H_5$ | H | 1 | $CH_3$ | |
| 6.454 | F | Br | $-OC_3H_7$ | H | 1 | $CH_3$ | |
| 6.455 | F | Br | 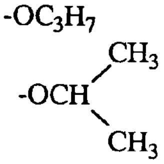 | H | 1 | $CH_3$ | |
| 6.456 | F | Br | $-OC_4H_9$ | H | 1 | $CH_3$ | |
| 6.457 | F | Br | $-OCH(CH_3)-CH_2-CH_3$ | H | 1 | $CH_3$ | |
| 6.458 | F | Br | 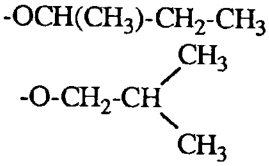 | H | 1 | $CH_3$ | |
| 6.459 | F | Br | $-O-C_5H_{11}$ | H | 1 | $CH_3$ | |
| 6.460 | F | Br | $-O-CH_2-CH_2-O-CH_3$ | H | 1 | $CH_3$ | |
| 6.461 | F | Br | $-O-CH_2-CH_2-O-C_2H_5$ | H | 1 | $CH_3$ | |
| 6.462 | F | Br | $-O-CH(CH_3)-CH_2-O-CH_3$ | H | 1 | $CH_3$ | |
| 6.463 | F | Br | $-O-CH_2-CH_2-S-CH_3$ | H | 1 | $CH_3$ | |
| 6.464 | F | Br | $-O-CH_2(CH_3)-S-CH_3$ | H | 1 | $CH_3$ | |
| 6.465 | F | Br | $-O-CH-(CH_3)-S-C_2H_5$ | H | 1 | $CH_3$ | |
| 6.466 | F | Br | $-O-CH(CH_3)-S-C_3H_7$ | H | 1 | $CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.467 | F | Br | 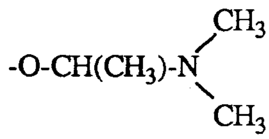 | H | 1 | $CH_3$ | |
| 6.468 | F | Br | $-NH_2$ | H | 1 | $CH_3$ | |
| 6.469 | F | Br | 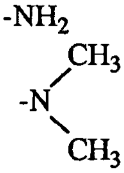 | H | 1 | $CH_3$ | |
| 6.470 | F | Br | 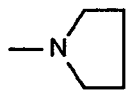 | H | 1 | $CH_3$ | |
| 6.471 | F | Br | 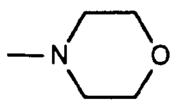 | H | 1 | $CH_3$ | |
| 6.472 | F | Br | 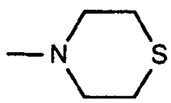 | H | 1 | $CH_3$ | |
| 6.473 | F | Br | 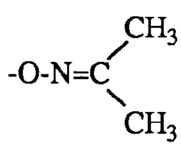 | H | 1 | $CH_3$ | |
| 6.474 | F | Br | $-O-CH_2-CH_2-Cl$ | H | 1 | $CH_3$ | |
| 6.475 | F | Br | $-O-CH_2-CN$ | H | 1 | $CH_3$ | |
| 6.476 | F | Br | $-O-CH_2-CH=CH_2$ | H | 1 | $CH_3$ | |
| 6.477 | F | Br | $-O-CH_2-C\equiv CH$ | H | 1 | $CH_3$ | |
| 6.478 | F | Br | 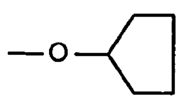 | H | 1 | $CH_3$ | |
| 6.479 | F | Br | 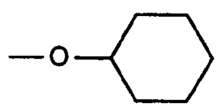 | H | 1 | $CH_3$ | |
| 6.480 | F | Br | 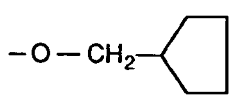 | H | 1 | $CH_3$ | |
| 6.481 | F | Br | 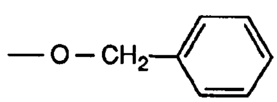 | H | 1 | $CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.482 | F | Br | $-SCH_3$ | H | 1 | $CH_3$ | |
| 6.483 | F | Br | $-S-CH_2-COOCH_3$ | H | 1 | $CH_3$ | |
| 6.484 | F | Br | $-S-CH(CH_3)-COOCH_3$ | H | 1 | $CH_3$ | |
| 6.485 | F | Br | $-O-CH_2-COOCH_3$ | H | 1 | $CH_3$ | |
| 6.486 | F | Br | $-O-CH(CH_3)COOCH_3$ | H | 1 | $CH_3$ | |
| 6.487 | F | CN | -Cl | H | 1 | $CH_3$ | |
| 6.488 | F | CN | -OH | H | 1 | $CH_3$ | |
| 6.489 | F | CN | $-OCH_3$ | H | 1 | $CH_3$ | |
| 6.490 | H | Cl | -Cl | H | 1 | $CH_3$ | |
| 6.491 | H | Cl | -OH | H | 1 | $CH_3$ | |
| 6.492 | H | Cl | $-OCH_3$ | H | 1 | $CH_3$ | |
| 6.493 | H | Cl | $-OC_2H_5$ | H | 1 | $CH_3$ | |
| 6.494 | H | Cl | 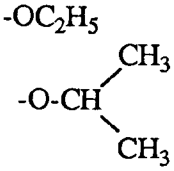 | H | 1 | $CH_3$ | |
| 6.495 | H | Cl | $-O-CH_2-COOCH_3$ | H | 1 | $CH_3$ | |
| 6.496 | H | Cl | $-O-CH(CH_3)COOCH_3$ | H | 1 | $CH_3$ | |
| 6.497 | H | Cl | $-S-CH_2-COOCH_3$ | H | 1 | $CH_3$ | |
| 6.498 | H | Cl | $-S-CH(CH_3)COOCH_3$ | H | 1 | $CH_3$ | |
| 6.499 | H | Cl | 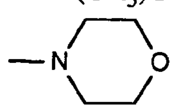 | H | 1 | $CH_3$ | |
| 6.500 | F | Cl | Cl | H | 2 | $CH_3$ | |
| 6.501 | F | Cl | OH | H | 2 | $CH_3$ | |
| 6.502 | F | Cl | $OCH_3$ | H | 2 | $CH_3$ | |
| 6.503 | F | Cl | $-OC_2H_5$ | H | 2 | $CH_3$ | |
| 6.504 | F | Cl | 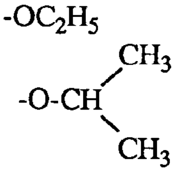 | H | 2 | $CH_3$ | |
| 6.505 | F | Cl | $-O-CH_2-CH_2-O-CH_3$ | H | 2 | $CH_3$ | |
| 6.506 | F | Cl | $-O-CH(CH_3)-CH_2-S-CH_3$ | H | 2 | $CH_3$ | |
| 6.507 | F | Cl | $-O-CH_2-COOCH_3$ | H | 2 | $CH_3$ | |
| 6.508 | F | Cl | $-S-CH_2-COOCH_3$ | H | 2 | $CH_3$ | |
| 6.509 | F | Cl | $-O-CH_2-CH=CH_2$ | H | 2 | $CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.510 | F | Cl | $-O-CH_2-C{\equiv}CH$ | H | 2 | $CH_3$ | |
| 6.511 | F | Cl | Cl | H | 3 | $CH_3$ | |
| 6.512 | F | Cl | OH | H | 3 | $CH_3$ | |
| 6.513 | F | Cl | $OCH_3$ | H | 3 | $CH_3$ | |
| 6.514 | F | Cl | $OC_2H_5$ | H | 3 | $CH_3$ | |
| 6.515 | F | Cl | 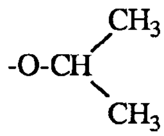 | H | 3 | $CH_3$ | |
| 6.516 | F | Cl | $-O-CH_2-CH_2-O-CH$ | H | 3 | $CH_3$ | |
| 6.517 | F | Cl | $-O-CH(CH_3)-CH_2-S-CH_3$ | H | 3 | $CH_3$ | |
| 6.518 | F | Cl | $-O-CH_2-COOCH_3$ | H | 3 | $CH_3$ | |
| 6.519 | F | Cl | $-S-CH_2-COOCH_3$ | H | 3 | $CH_3$ | |
| 6.520 | F | Cl | $-O-CH_2-C{\equiv}CH$ | H | 3 | $CH_3$ | |
| 6.521 | F | Cl | -Cl | Cl | 0 | $CH_3$ | |
| 6.522 | F | Cl | -OH | Cl | 0 | $CH_3$ | |
| 6.523 | F | Cl | $-OCH_3$ | Cl | 0 | $CH_3$ | |
| 6.524 | F | Cl | $-OC_2H_5$ | Cl | 0 | $CH_3$ | |
| 6.525 | F | Cl | 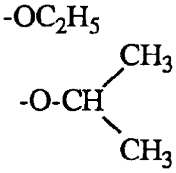 | Cl | 0 | $CH_3$ | |
| 6.526 | F | Cl | $-O-CH_2-COOCH_3$ | Cl | 0 | $CH_3$ | |
| 6.527 | F | Cl | $-S-CH_2-COOCH_3$ | Cl | 0 | $CH_3$ | |
| 6.528 | F | Cl | $-OCH_3$ | Br | 0 | $CH_3$ | |
| 6.529 | F | Cl | 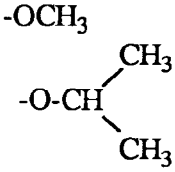 | Br | 0 | $CH_3$ | |
| 6.530 | F | Cl | $-OCH_3$ | F | 0 | $CH_3$ | |
| 6.531 | F | Cl | $-OCH_3$ | $CH_3$ | 0 | $CH_3$ | |
| 6.532 | F | Cl | $-OC_2H_5$ | $CH_3$ | 0 | $CH_3$ | |
| 6.533 | F | Cl | 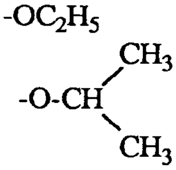 | $CH_3$ | 0 | $CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.534 | F | Cl | $-O-CH_2-CH_2-O-CH_3$ | $CH_3$ | 0 | $CH_3$ | |
| 6.535 | F | Cl | $-O-CH_2-COOCH_3$ | $CH_3$ | 0 | $CH_3$ | |
| 6.536 | F | Cl | $-O-CH(CH_3)COOCH_3$ | $CH_3$ | 0 | $CH_3$ | |
| 6.537 | F | Cl | $-S-CH_2-COOCH_3$ | $CH_3$ | 0 | $CH_3$ | |
| 6.538 | F | Cl | $-OCH_3$ | $CF_3$ | 0 | $CH_3$ | |
| 6.539 | F | Cl | $-OC_2H_5$ | $CF_3$ | 0 | $CH_3$ | |
| 6.540 | F | Cl | 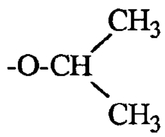 | $CF_3$ | 0 | $CH_3$ | |
| 6.541 | F | Cl | $-O-CH_2-COOCH_3$ | $CF_3$ | 0 | $CH_3$ | |
| 6.542 | F | Cl | $-S-CH_2-COOCH_3$ | $CF_3$ | 0 | $CH_3$ | |
| 6.543 | F | Cl | -Cl | H | 0 | $-CH(CH_3)-CH_3$ | |
| 6.544 | F | Cl | -OH | H | 0 | $-CH(CH_3)-CH_3$ | |
| 6.545 | F | Cl | $-OCH_3$ | H | 0 | $-CH(CH_3)-CH_3$ | |
| 6.546 | F | Cl | $-OC_2H_5$ | H | 0 | $-CH(CH_3)-CH_3$ | |
| 6.547 | F | Cl | $-OC_3H_7$ | H | 0 | $-CH(CH_3)-CH_3$ | |
| 6.548 | F | Cl | 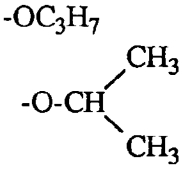 | H | 0 | $-CH(CH_3)-CH_3$ | |
| 6.549 | F | Cl | $-OC_4H_9$ | H | 0 | $-CH(CH_3)-CH_3$ | |
| 6.550 | F | Cl | $-O-CH(CH_3)-C_2H_5$ | H | 0 | $-CH(CH_3)-CH_3$ | |
| 6.551 | F | Cl | 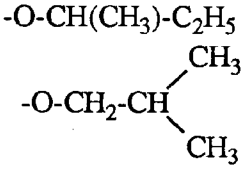 | H | 0 | $-CH(CH_3)-CH_3$ | |
| 6.552 | F | Cl | $-OC_5H_{11}$ | H | 0 | $-CH(CH_3)-CH_3$ | |
| 6.553 | F | Cl | $-O-CH_2-CH_2-O-CH_3$ | H | 0 | $-CH(CH_3)-CH_3$ | |
| 6.554 | F | Cl | $-O-CH_2-CH_2-O-C_2H_5$ | H | 0 | $-CH(CH_3)-CH_3$ | |
| 6.555 | F | Cl | $-O-CH-(CH_3)-CH_2-O-CH_3$ | H | 0 | $-CH(CH_3)-CH_3$ | |
| 6.556 | F | Cl | $-O-CH_2-CH_2-S-CH_3$ | H | 0 | $-CH(CH_3)-CH_3$ | |
| 6.557 | F | Cl | $-O-CH(CH_3)-CH_2-S-CH_3$ | H | 0 | $-CH(CH_3)-CH_3$ | |
| 6.558 | F | Cl | $-O-CH(CH_3)-CH_2-S-C_2H_5$ | H | 0 | $-CH(CH_3)-CH_3$ | |
| 6.559 | F | Cl | $-O-CH(CH_3)-CH_2-S-C_3H_7$ | H | 0 | $-CH(CH_3)-CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.560 | F | Cl | 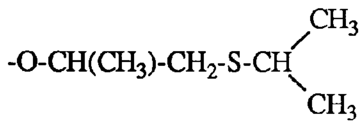 | H | 0 | $-CH(CH_3)-CH_3$ | |
| 6.561 | F | Cl | $-O-CH(CH_3)-CH_2-S-C_4H_9$ | H | 0 | $-CH(CH_3)-CH_3$ | |
| 6.562 | F | Cl | $-O-CH(CH_3)-CH_2-S-C_5H_{11}$ | H | 0 | $-CH(CH_3)-CH_3$ | |
| 6.563 | F | Cl | 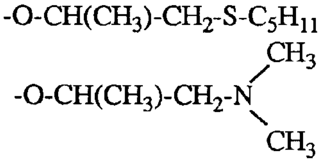 | H | 0 | $-CH(CH_3)-CH_3$ | |
| 6.564 | F | Cl | 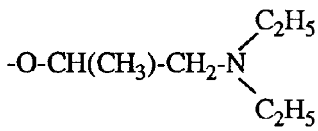 | H | 0 | $-CH(CH_3)-CH_3$ | |
| 6.565 | F | Cl | $-NH_2$ | H | 0 | $-CH(CH_3)-CH_3$ | |
| 6.566 | F | Cl | 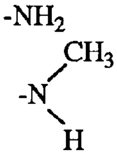 | H | 0 | $-CH(CH_3)-CH_3$ | |
| 6.567 | F | Cl | 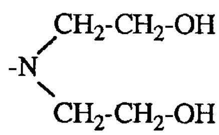 | H | 0 | $-CH(CH_3)-CH_3$ | |
| 6.568 | F | Cl | $-NH-CH_2-CH=CH_2$ | H | 0 | $-CH(CH_3)-CH_3$ | |
| 6.569 | F | Cl | $-N-(CH_2-CH=CH_2)_2$ | H | 0 | $-CH(CH_3)-CH_3$ | |
| 6.570 | F | Cl | 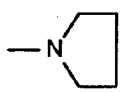 | H | 0 | $-CH(CH_3)-CH_3$ | |
| 6.571 | F | Cl | 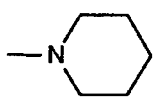 | H | 0 | $-CH(CH_3)-CH_3$ | |
| 6.572 | F | Cl | 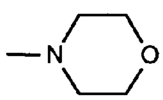 | H | 0 | $-CH(CH_3)-CH_3$ | |
| 6.573 | F | Cl | 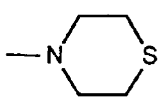 | H | 0 | $-CH(CH_3)-CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.574 | F | Cl | 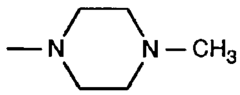 | H | 0 | $-CH(CH_3)-CH_3$ | |
| 6.575 | F | Cl | 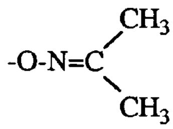 | H | 0 | $-CH(CH_3)-CH_3$ | |
| 6.576 | F | Cl | $-O-CH_2-CH_2-Cl$ | H | 0 | $-CH(CH_3)-CH_3$ | |
| 6.577 | F | Cl | $-O-CH_2-CN$ | H | 0 | $-CH(CH_3)-CH_3$ | |
| 6.578 | F | Cl | 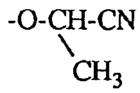 | H | 0 | $-CH(CH_3)-CH_3$ | |
| 6.579 | F | Cl | $-O-CH_2-CH=CH_2$ | H | 0 | $-CH(CH_3)-CH_3$ | |
| 6.580 | F | Cl | $-O-CH_2-CH=CHCl$ | H | 0 | $-CH(CH_3)-CH_3$ | |
| 6.581 | F | Cl | 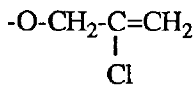 | H | 0 | $-CH(CH_3)-CH_3$ | |
| 6.582 | F | Cl | $-O-CH_2=C\equiv CH$ | H | 0 | $-CH(CH_3)-CH_3$ | |
| 6.583 | F | Cl | 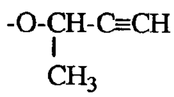 | H | 0 | $-CH(CH_3)-CH_3$ | |
| 6.584 | F | Cl | 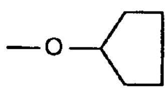 | H | 0 | $-CH(CH_3)-CH_3$ | |
| 6.585 | F | Cl | 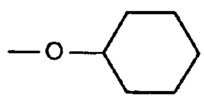 | H | 0 | $-CH(CH_3)-CH_3$ | |
| 6.586 | F | Cl | 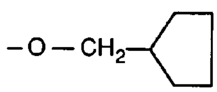 | H | 0 | $-CH(CH_3)-CH_3$ | |
| 6.587 | F | Cl | 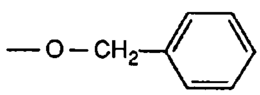 | H | 0 | $-CH(CH_3)-CH_3$ | |
| 6.588 | F | Cl | 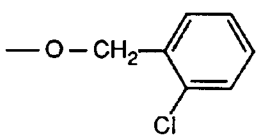 | H | 0 | $-CH(CH_3)-CH_3$ | |
| 6.589 | F | Cl | 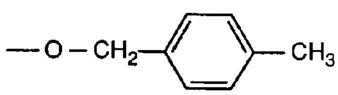 | H | 0 | $-CH(CH_3)-CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.590 | F | Cl | $-S-CH_3$ | H | 0 | $-CH(CH_3)-CH_3$ | |
| 6.591 | F | Cl | $-S-C_2H_5$ | H | 0 | $-CH(CH_3)-CH_3$ | |
| 6.592 | F | Cl | $-S-C_3H_7$ | H | 0 | $-CH(CH_3)-CH_3$ | |
| 6.593 | F | Cl | $-S-CH_2-CH=CH_2$ | H | 0 | $-CH(CH_3)-CH_3$ | |
| 6.594 | F | Cl | $-S-CH_2-COOCH_3$ | H | 0 | $-CH(CH_3)-CH_3$ | |
| 6.595 | F | Cl | $-S-CH_2-COOC_2H_5$ | H | 0 | $-CH(CH_3)-CH_3$ | |
| 6.596 | F | Cl | $-S-CH_2-COOC_5H_{11}$ | H | 0 | $-CH(CH_3)-CH_3$ | |
| 6.597 | F | Cl | $-S-CH(CH_3)-COOCH_3$ | H | 0 | $-CH(CH_3)-CH_3$ | |
| 6.598 | F | Cl | $-S-(CH_3)-COOC_2H_5$ | H | 0 | $-CH(CH_3)-CH_3$ | |
| 6.599 | F | Cl | $-S-CH(CH_3)-COOC_3H_7$ | H | 0 | $-CH(CH_3)-CH_3$ | |
| 6.600 | F | Cl | $-S-CH_2-CH_2-COOCH_3$ | H | 0 | $-CH(CH_3)-CH_3$ | |
| 6.601 | F | Cl | $-S-CH_2-COOCH_2-CH_2-O-CH_3$ | H | 0 | $-CH(CH_3)-CH_3$ | |
| 6.602 | F | Cl | $-O-CH_2-COOCH_3$ | H | 0 | $-CH(CH_3)-CH_3$ | |
| 6.603 | F | Cl | $-O-CH(CH_3)-COOCH_3$ | H | 0 | $-CH(CH_3)-CH_3$ | |
| 6.604 | F | Cl | $-O-CH_2-COOC_5H_{11}$ | H | 0 | $-CH(CH_3)-CH_3$ | |
| 6.605 | F | Cl | $-O-CH_2-CH_3-Si(CH_3)_3$ | H | 0 | $-CH(CH_3)-CH_3$ | |
| 6.606 | F | Cl | -ONa | H | 0 | $-CH(CH_3)-CH_3$ | |
| 6.607 | F | Br | -Cl | H | 0 | $-CH(CH_3)-CH_3$ | |
| 6.608 | F | Br | -OH | H | 0 | $-CH(CH_3)-CH_3$ | |
| 6.609 | F | Br | $-OCH_3$ | H | 0 | $-CH(CH_3)-CH_3$ | |
| 6.610 | F | Br | $-OC_2H_5$ | H | 0 | $-CH(CH_3)-CH_3$ | |
| 6.611 | F | Br | $-OC_3H_7$ | H | 0 | $-CH(CH_3)-CH_3$ | |
| 6.612 | F | Br | 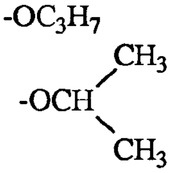 | H | 0 | $-CH(CH_3)-CH_3$ | |
| 6.613 | F | Br | $-OC_4H_9$ | H | 0 | $-CH(CH_3)-CH_3$ | |
| 6.614 | F | Br | $-OCH(CH_3)-CH_2-CH_3$ | H | 0 | $-CH(CH_3)-CH_3$ | |
| 6.615 | F | Br | 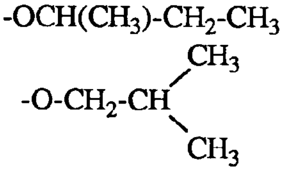 | H | 0 | $-CH(CH_3)-CH_3$ | |
| 6.616 | F | Br | $-O-C_5H_{11}$ | H | 0 | $-CH(CH_3)-CH_3$ | |
| 6.617 | F | Br | $-O-CH_2-CH_2-O-CH_3$ | H | 0 | $-CH(CH_3)-CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.618 | F | Br | $-O-CH_2-CH_2-O-C_2H_5$ | H | 0 | $-CH(CH_3)-CH_3$ | |
| 6.619 | F | Br | $-O-CH(CH_3)-CH_2-O-CH_3$ | H | 0 | $-CH(CH_3)-CH_3$ | |
| 6.620 | F | Br | $-O-CH_2-CH_2-S-CH_3$ | H | 0 | $-CH(CH_3)-CH_3$ | |
| 6.621 | F | Br | $-O-CH_2(CH_3)-S-CH_3$ | H | 0 | $-CH(CH_3)-CH_3$ | |
| 6.622 | F | Br | $-O-CH-(CH_3)-S-C_2H_5$ | H | 0 | $-CH(CH_3)-CH_3$ | |
| 6.623 | F | Br | $-O-CH(CH_3)-S-C_3H_7$ | H | 0 | $-CH(CH_3)-CH_3$ | |
| 6.624 | F | Br | 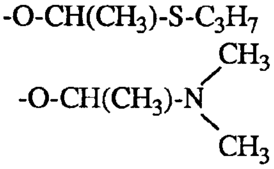 | H | 0 | $-CH(CH_3)-CH_3$ | |
| 6.625 | F | Br | $-NH_2$ | H | 0 | $-CH(CH_3)-CH_3$ | |
| 6.626 | F | Br | 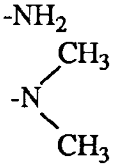 | H | 0 | $-CH(CH_3)-CH_3$ | |
| 6.627 | F | Br | 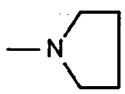 | H | 0 | $-CH(CH_3)-CH_3$ | |
| 6.628 | F | Br | 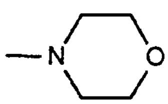 | H | 0 | $-CH(CH_3)-CH_3$ | |
| 6.629 | F | Br | 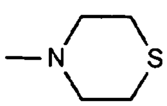 | H | 0 | $-CH(CH_3)-CH_3$ | |
| 6.630 | F | Br | 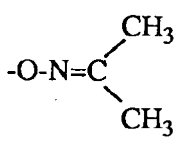 | H | 0 | $-CH(CH_3)-CH_3$ | |
| 6.631 | F | Br | $-O-CH_2-CH_2-Cl$ | H | 0 | $-CH(CH_3)-CH_3$ | |
| 6.632 | F | Br | $-O-CH_2-CN$ | H | 0 | $-CH(CH_3)-CH_3$ | |
| 6.633 | F | Br | $-O-CH_2-CH=CH_2$ | H | 0 | $-CH(CH_3)-CH_3$ | |
| 6.634 | F | Br | $-O-CH_2-C\equiv CH$ | H | 0 | $-CH(CH_3)-CH_3$ | |
| 6.635 | F | Br | 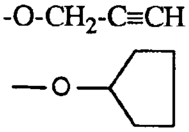 | H | 0 | $-CH(CH_3)-CH_3$ | |
| 6.636 | F | Br | 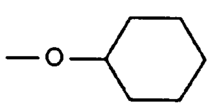 | H | 0 | $-CH(CH_3)-CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.637 | F | Br | 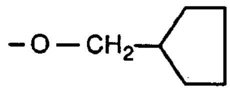 | H | 0 | $-CH(CH_3)-CH_3$ | |
| 6.638 | F | Br | 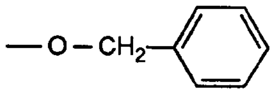 | H | 0 | $-CH(CH_3)-CH_3$ | |
| 6.639 | F | Br | $-SCH_3$ | H | 0 | $-CH(CH_3)-CH_3$ | |
| 6.640 | F | Br | $-S-CH_2-COOCH_3$ | H | 0 | $-CH(CH_3)-CH_3$ | |
| 6.641 | F | Br | $-S-CH(CH_3)-COOCH_3$ | H | 0 | $-CH(CH_3)-CH_3$ | |
| 6.642 | F | Br | $-O-CH_2-COOCH_3$ | H | 0 | $-CH(CH_3)-CH_3$ | |
| 6.643 | F | Br | $-O-CH(CH_3)COOCH_3$ | H | 0 | $-CH(CH_3)-CH_3$ | |
| 6.644 | F | CN | -Cl | H | 0 | $-CH(CH_3)-CH_3$ | |
| 6.645 | F | CN | -OH | H | 0 | $-CH(CH_3)-CH_3$ | |
| 6.646 | F | CN | $-OCH_3$ | H | 0 | $-CH(CH_3)-CH_3$ | |
| 6.647 | H | Cl | -Cl | H | 0 | $-CH(CH_3)-CH_3$ | |
| 6.648 | H | Cl | -OH | H | 0 | $-CH(CH_3)-CH_3$ | |
| 6.649 | H | Cl | $-OCH_3$ | H | 0 | $-CH(CH_3)-CH_3$ | |
| 6.650 | H | Cl | $-OC_2H_5$ | H | 0 | $-CH(CH_3)-CH_3$ | |
| 6.651 | H | Cl | 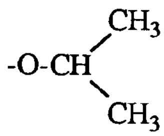 | H | 0 | $-CH(CH_3)-CH_3$ | |
| 6.652 | H | Cl | $-O-CH_2-COOCH_3$ | H | 0 | $-CH(CH_3)-CH_3$ | |
| 6.653 | H | Cl | $-O-CH(CH_3)COOCH_3$ | H | 0 | $-CH(CH_3)-CH_3$ | |
| 6.654 | H | Cl | $-S-CH_2-COOCH_3$ | H | 0 | $-CH(CH_3)-CH_3$ | |
| 6.655 | H | Cl | $-S-CH(CH_3)COOCH_3$ | H | 0 | $-CH(CH_3)-CH_3$ | |
| 6.656 | H | Cl | 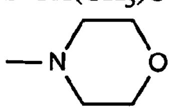 | H | 0 | $-CH(CH_3)-CH_3$ | |
| 6.657 | F | Cl | -Cl | H | 1 | $-CH(CH_3)-CH_3$ | |
| 6.658 | F | Cl | -OH | H | 1 | $-CH(CH_3)-CH_3$ | |
| 6.659 | F | Cl | $-OCH_3$ | H | 1 | $-CH(CH_3)-CH_3$ | |
| 6.660 | F | Cl | $-OC_2H_5$ | H | 1 | $-CH(CH_3)-CH_3$ | |
| 6.661 | F | Cl | $-OC_3H_7$ | H | 1 | $-CH(CH_3)-CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.662 | F | Cl | 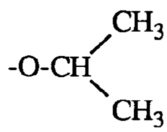 | H | 1 | $-CH(CH_3)-CH_3$ | |
| 6.663 | F | Cl | $-OC_4H_9$ | H | 1 | $-CH(CH_3)-CH_3$ | |
| 6.664 | F | Cl | $-O-CH(CH_3)-C_2H_5$ | H | 1 | $-CH(CH_3)-CH_3$ | |
| 6.665 | F | Cl | 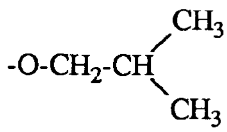 | H | 1 | $-CH(CH_3)-CH_3$ | |
| 6.666 | F | Cl | $-OC_5H_{11}$ | H | 1 | $-CH(CH_3)-CH_3$ | |
| 6.667 | F | Cl | $-O-CH_2-CH_2-O-CH_3$ | H | 1 | $-CH(CH_3)-CH_3$ | |
| 6.668 | F | Cl | $-O-CH_2-CH_2-O-C_2H_5$ | H | 1 | $-CH(CH_3)-CH_3$ | |
| 6.669 | F | Cl | $-O-CH-(CH_3)-CH_2-O-CH_3$ | H | 1 | $-CH(CH_3)-CH_3$ | |
| 6.670 | F | Cl | $-O-CH_2-CH_2-S-CH_3$ | H | 1 | $-CH(CH_3)-CH_3$ | |
| 6.671 | F | Cl | $-O-CH(CH_3)-CH_2-S-CH_3$ | H | 1 | $-CH(CH_3)-CH_3$ | |
| 6.672 | F | Cl | $-O-CH(CH_3)-CH_2-S-C_2H_5$ | H | 1 | $-CH(CH_3)-CH_3$ | |
| 6.673 | F | Cl | $-O-CH(CH_3)-CH_2-S-C_3H_7$ | H | 1 | $-CH(CH_3)-CH_3$ | |
| 6.674 | F | Cl | 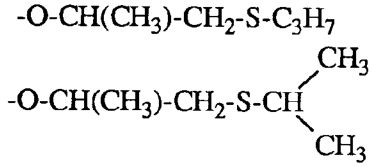 | H | 1 | $-CH(CH_3)-CH_3$ | |
| 6.675 | F | Cl | $-O-CH(CH_3)-CH_2-S-C_4H_9$ | H | 1 | $-CH(CH_3)-CH_3$ | |
| 6.676 | F | Cl | $-O-CH(CH_3)-CH_2-S-C_5H_{11}$ | H | 1 | $-CH(CH_3)-CH_3$ | |
| 6.677 | F | Cl | 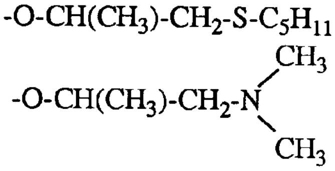 | H | 1 | $-CH(CH_3)-CH_3$ | |
| 6.678 | F | Cl | 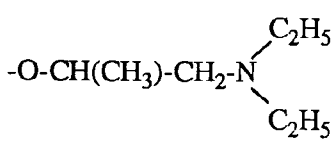 | H | 1 | $-CH(CH_3)-CH_3$ | |
| 6.679 | F | Cl | $-NH_2$ | H | 1 | $-CH(CH_3)-CH_3$ | |
| 6.680 | F | Cl | 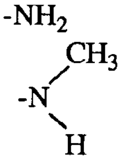 | H | 1 | $-CH(CH_3)-CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.681 | F | Cl | 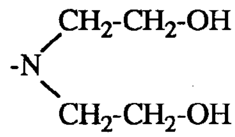 | H | 1 | $-CH(CH_3)-CH_3$ | |
| 6.682 | F | Cl | $-NH-CH_2-CH=CH_2$ | H | 1 | $-CH(CH_3)-CH_3$ | |
| 6.683 | F | Cl | $-N-(CH_2-CH=CH_2)_2$ | H | 1 | $-CH(CH_3)-CH_3$ | |
| 6.684 | F | Cl | 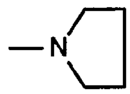 | H | 1 | $-CH(CH_3)-CH_3$ | |
| 6.685 | F | Cl | 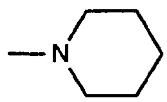 | H | 1 | $-CH(CH_3)-CH_3$ | |
| 6.686 | F | Cl | 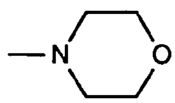 | H | 1 | $-CH(CH_3)-CH_3$ | |
| 6.687 | F | Cl | 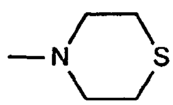 | H | 1 | $-CH(CH_3)-CH_3$ | |
| 6.688 | F | Cl | 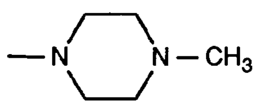 | H | 1 | $-CH(CH_3)-CH_3$ | |
| 6.689 | F | Cl | 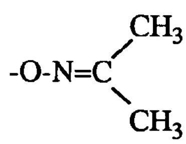 | H | 1 | $-CH(CH_3)-CH_3$ | |
| 6.690 | F | Cl | $-O-CH_2-CH_2-Cl$ | H | 1 | $-CH(CH_3)-CH_3$ | |
| 6.691 | F | Cl | $-O-CH_2-CN$ | H | 1 | $-CH(CH_3)-CH_3$ | |
| 6.692 | F | Cl | 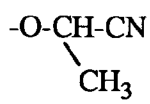 | H | 1 | $-CH(CH_3)-CH_3$ | |
| 6.693 | F | Cl | $-O-CH_2-CH=CH_2$ | H | 1 | $-CH(CH_3)-CH_3$ | |
| 6.694 | F | Cl | $-O-CH_2-CH=CHCl$ | H | 1 | $-CH(CH_3)-CH_3$ | |
| 6.695 | F | Cl | 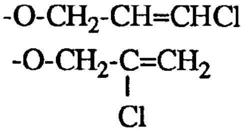 | H | 1 | $-CH(CH_3)-CH_3$ | |
| 6.696 | F | Cl | $-O-CH_2=C\equiv CH$ | H | 1 | $-CH(CH_3)-CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.697 | F | Cl | -O-CH-C≡CH<br>\|<br>$CH_3$ | H | 1 | -CH($CH_3$)-$CH_3$ | |
| 6.698 | F | Cl | 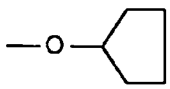 | H | 1 | -CH($CH_3$)-$CH_3$ | |
| 6.699 | F | Cl | 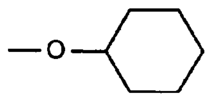 | H | 1 | -CH($CH_3$)-$CH_3$ | |
| 6.700 | F | Cl | 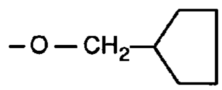 | H | 1 | -CH($CH_3$)-$CH_3$ | |
| 6.701 | F | Cl | 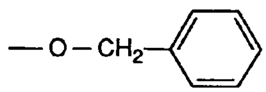 | H | 1 | -CH($CH_3$)-$CH_3$ | |
| 6.702 | F | Cl | 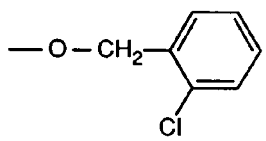 | H | 1 | -CH($CH_3$)-$CH_3$ | |
| 6.703 | F | Cl | 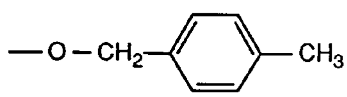 | H | 1 | -CH($CH_3$)-$CH_3$ | |
| 6.704 | F | Cl | -S-$CH_3$ | H | 1 | -CH($CH_3$)-$CH_3$ | |
| 6.705 | F | Cl | -S-$C_2H_5$ | H | 1 | -CH($CH_3$)-$CH_3$ | |
| 6.706 | F | Cl | -S-$C_3H_7$ | H | 1 | -CH($CH_3$)-$CH_3$ | |
| 6.707 | F | Cl | -S-$CH_2$-CH=$CH_2$ | H | 1 | -CH($CH_3$)-$CH_3$ | |
| 6.708 | F | Cl | -S-$CH_2$-$COOCH_3$ | H | 1 | -CH($CH_3$)-$CH_3$ | |
| 6.709 | F | Cl | -S-$CH_2$-$COOC_2H_5$ | H | 1 | -CH($CH_3$)-$CH_3$ | |
| 6.710 | F | Cl | -S-$CH_2$-$COOC_5H_{11}$ | H | 1 | -CH($CH_3$)-$CH_3$ | |
| 6.711 | F | Cl | -S-CH($CH_3$)-$COOCH_3$ | H | 1 | -CH($CH_3$)-$CH_3$ | |
| 6.712 | F | Cl | -S-($CH_3$)-$COOC_2H_5$ | H | 1 | -CH($CH_3$)-$CH_3$ | |
| 6.713 | F | Cl | -S-CH($CH_3$)-$COOC_3H_7$ | H | 1 | -CH($CH_3$)-$CH_3$ | |
| 6.714 | F | Cl | -S-$CH_2$-$CH_2$-$COOCH_3$ | H | 1 | -CH($CH_3$)-$CH_3$ | |
| 6.715 | F | Cl | -S-$CH_2$-$COOCH_2$-$CH_2$-O-$CH_3$ | H | 1 | -CH($CH_3$)-$CH_3$ | |
| 6.716 | F | Cl | -O-$CH_2$-$COOCH_3$ | H | 1 | -CH($CH_3$)-$CH_3$ | |
| 6.717 | F | Cl | -O-CH($CH_3$)-$COOCH_3$ | H | 1 | -CH($CH_3$)-$CH_3$ | |
| 6.718 | F | Cl | -O-$CH_2$-$COOC_5H_{11}$ | H | 1 | -CH($CH_3$)-$CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.719 | F | Cl | $-O-CH_2-CH_3-Si(CH_3)_3$ | H | 1 | $-CH(CH_3)-CH_3$ | |
| 6.720 | F | Cl | -ONa | H | 1 | $-CH(CH_3)-CH_3$ | |
| 6.721 | F | Br | -Cl | H | 1 | $-CH(CH_3)-CH_3$ | |
| 6.722 | F | Br | -OH | H | 1 | $-CH(CH_3)-CH_3$ | |
| 6.723 | F | Br | $-OCH_3$ | H | 1 | $-CH(CH_3)-CH_3$ | |
| 6.724 | F | Br | $-OC_2H_5$ | H | 1 | $-CH(CH_3)-CH_3$ | |
| 6.725 | F | Br | $-OC_3H_7$ | H | 1 | $-CH(CH_3)-CH_3$ | |
| 6.726 | F | Br | 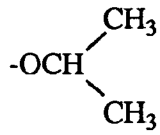 | H | 1 | $-CH(CH_3)-CH_3$ | |
| 6.727 | F | Br | $-OC_4H_9$ | H | 1 | $-CH(CH_3)-CH_3$ | |
| 6.728 | F | Br | $-OCH(CH_3)-CH_2-CH_3$ | H | 1 | $-CH(CH_3)-CH_3$ | |
| 6.729 | F | Br | 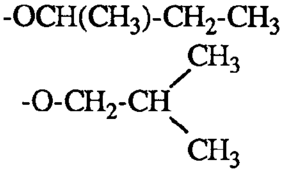 | H | 1 | $-CH(CH_3)-CH_3$ | |
| 6.730 | F | Br | $-O-C_5H_{11}$ | H | 1 | $-CH(CH_3)-CH_3$ | |
| 6.731 | F | Br | $-O-CH_2-CH_2-O-CH_3$ | H | 1 | $-CH(CH_3)-CH_3$ | |
| 6.732 | F | Br | $-O-CH_2-CH_2-O-C_2H_5$ | H | 1 | $-CH(CH_3)-CH_3$ | |
| 6.733 | F | Br | $-O-CH(CH_3)-CH_2-O-CH_3$ | H | 1 | $-CH(CH_3)-CH_3$ | |
| 6.734 | F | Br | $-O-CH_2-CH_2-S-CH_3$ | H | 1 | $-CH(CH_3)-CH_3$ | |
| 6.735 | F | Br | $-O-CH_2(CH_3)-S-CH_3$ | H | 1 | $-CH(CH_3)-CH_3$ | |
| 6.736 | F | Br | $-O-CH-(CH_3)-S-C_2H_5$ | H | 1 | $-CH(CH_3)-CH_3$ | |
| 6.737 | F | Br | $-O-CH(CH_3)-S-C_3H_7$ | H | 1 | $-CH(CH_3)-CH_3$ | |
| 6.738 | F | Br | 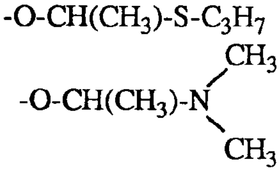 | H | 1 | $-CH(CH_3)-CH_3$ | |
| 6.739 | F | Br | $-NH_2$ | H | 1 | $-CH(CH_3)-CH_3$ | |
| 6.740 | F | Br | 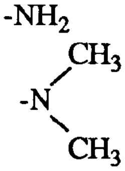 | H | 1 | $-CH(CH_3)-CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.741 | F | Br | 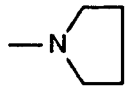 | H | 1 | $-CH(CH_3)-CH_3$ | |
| 6.742 | F | Br | 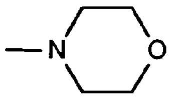 | H | 1 | $-CH(CH_3)-CH_3$ | |
| 6.743 | F | Br | 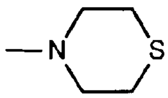 | H | 1 | $-CH(CH_3)-CH_3$ | |
| 6.744 | F | Br | 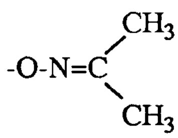 | H | 1 | $-CH(CH_3)-CH_3$ | |
| 6.745 | F | Br | $-O-CH_2-CH_2-Cl$ | H | 1 | $-CH(CH_3)-CH_3$ | |
| 6.746 | F | Br | $-O-CH_2-CN$ | H | 1 | $-CH(CH_3)-CH_3$ | |
| 6.747 | F | Br | $-O-CH_2-CH=CH_2$ | H | 1 | $-CH(CH_3)-CH_3$ | |
| 6.748 | F | Br | $-O-CH_2-C\equiv CH$ | H | 1 | $-CH(CH_3)-CH_3$ | |
| 6.749 | F | Br | 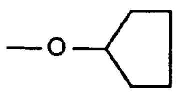 | H | 1 | $-CH(CH_3)-CH_3$ | |
| 6.750 | F | Br | 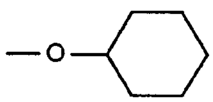 | H | 1 | $-CH(CH_3)-CH_3$ | |
| 6.751 | F | Br | 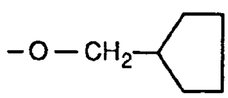 | H | 1 | $-CH(CH_3)-CH_3$ | |
| 6.752 | F | Br | 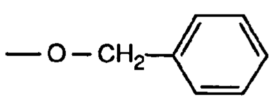 | H | 1 | $-CH(CH_3)-CH_3$ | |
| 6.753 | F | Br | $-SCH_3$ | H | 1 | $-CH(CH_3)-CH_3$ | |
| 6.754 | F | Br | $-S-CH_2-COOCH_3$ | H | 1 | $-CH(CH_3)-CH_3$ | |
| 6.755 | F | Br | $-S-CH(CH_3)-COOCH_3$ | H | 1 | $-CH(CH_3)-CH_3$ | |
| 6.756 | F | Br | $-O-CH_2-COOCH_3$ | H | 1 | $-CH(CH_3)-CH_3$ | |
| 6.757 | F | Br | $-O-CH(CH_3)COOCH_3$ | H | 1 | $-CH(CH_3)-CH_3$ | |
| 6.758 | F | CN | -Cl | H | 1 | $-CH(CH_3)-CH_3$ | |
| 6.759 | F | CN | -OH | H | 1 | $-CH(CH_3)-CH_3$ | |
| 6.760 | F | CN | $-OCH_3$ | H | 1 | $-CH(CH_3)-CH_3$ | |
| 6.761 | H | Cl | -Cl | H | 1 | $-CH(CH_3)-CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.762 | H | Cl | -OH | H | 1 | $-CH(CH_3)-CH_3$ | |
| 6.763 | H | Cl | $-OCH_3$ | H | 1 | $-CH(CH_3)-CH_3$ | |
| 6.764 | H | Cl | $-OC_2H_5$ | H | 1 | $-CH(CH_3)-CH_3$ | |
| 6.765 | H | Cl | 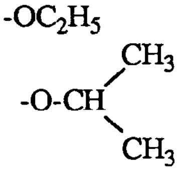 | H | 1 | $-CH(CH_3)-CH_3$ | |
| 6.766 | H | Cl | $-O-CH_2-COOCH_3$ | H | 1 | $-CH(CH_3)-CH_3$ | |
| 6.767 | H | Cl | $-O-CH(CH_3)COOCH_3$ | H | 1 | $-CH(CH_3)-CH_3$ | |
| 6.768 | H | Cl | $-S-CH_2-COOCH_3$ | H | 1 | $-CH(CH_3)-CH_3$ | |
| 6.769 | H | Cl | $-S-CH(CH_3)COOCH_3$ | H | 1 | $-CH(CH_3)-CH_3$ | |
| 6.770 | H | Cl | 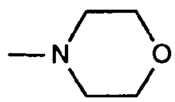 | H | 1 | $-CH(CH_3)-CH_3$ | |
| 6.771 | F | Cl | Cl | H | 2 | $-CH(CH_3)-CH_3$ | |
| 6.772 | F | Cl | OH | H | 2 | $-CH(CH_3)-CH_3$ | |
| 6.773 | F | Cl | $OCH_3$ | H | 2 | $-CH(CH_3)-CH_3$ | |
| 6.774 | F | Cl | $-OC_2H_5$ | H | 2 | $-CH(CH_3)-CH_3$ | |
| 6.775 | F | Cl | 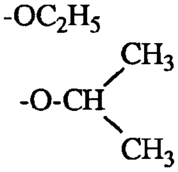 | H | 2 | $-CH(CH_3)-CH_3$ | |
| 6.776 | F | Cl | $-O-CH_2-CH_2-O-CH_3$ | H | 2 | $-CH(CH_3)-CH_3$ | |
| 6.777 | F | Cl | $-O-CH(CH_3)-CH_2-S-CH_3$ | H | 2 | $-CH(CH_3)-CH_3$ | |
| 6.778 | F | Cl | $-O-CH_2-COOCH_3$ | H | 2 | $-CH(CH_3)-CH_3$ | |
| 6.779 | F | Cl | $-S-CH_2-COOCH_3$ | H | 2 | $-CH(CH_3)-CH_3$ | |
| 6.780 | F | Cl | $-CH_2-CH=CH_2$ | H | 2 | $-CH(CH_3)-CH_3$ | |
| 6.781 | F | Cl | $-CH_2-C\equiv CH$ | H | 2 | $-CH(CH_3)-CH_3$ | |
| 6.782 | F | Cl | Cl | H | 3 | $-CH(CH_3)-CH_3$ | |
| 6.783 | F | Cl | OH | H | 3 | $-CH(CH_3)-CH_3$ | |
| 6.784 | F | Cl | $OCH_3$ | H | 3 | $-CH(CH_3)-CH_3$ | |
| 6.785 | F | Cl | $OC_2H_5$ | H | 3 | $-CH(CH_3)-CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.786 | F | Cl | 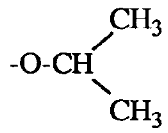 | H | 3 | $-CH(CH_3)-CH_3$ | |
| 6.787 | F | Cl | $-O-CH_2-CH_2-O-CH$ | H | 3 | $-CH(CH_3)-CH_3$ | |
| 6.788 | F | Cl | $-O-CH(CH_3)-CH_2-S-CH_3$ | H | 3 | $-CH(CH_3)-CH_3$ | |
| 6.789 | F | Cl | $-O-CH_2-COOCH_3$ | H | 3 | $-CH(CH_3)-CH_3$ | |
| 6.790 | F | Cl | $-S-CH_2-COOCH_3$ | H | 3 | $-CH(CH_3)-CH_3$ | |
| 6.791 | F | Cl | $-O-CH_2-C\equiv CH$ | H | 3 | $-CH(CH_3)-CH_3$ | |
| 6.792 | F | Cl | -Cl | Cl | 0 | $-CH(CH_3)-CH_3$ | |
| 6.793 | F | Cl | -OH | Cl | 0 | $-CH(CH_3)-CH_3$ | |
| 6.794 | F | Cl | $-OCH_3$ | Cl | 0 | $-CH(CH_3)-CH_3$ | |
| 6.795 | F | Cl | $-OC_2H_5$ | Cl | 0 | $-CH(CH_3)-CH_3$ | |
| 6.796 | F | Cl | 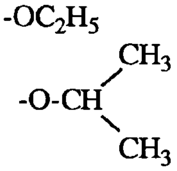 | Cl | 0 | $-CH(CH_3)-CH_3$ | |
| 6.797 | F | Cl | $-O-CH_2-COOCH_3$ | Cl | 0 | $-CH(CH_3)-CH_3$ | |
| 6.798 | F | Cl | $-S-CH_2-COOCH_3$ | Cl | 0 | $-CH(CH_3)-CH_3$ | |
| 6.799 | F | Cl | $-OCH_3$ | Br | 0 | $-CH(CH_3)-CH_3$ | |
| 6.800 | F | Cl | 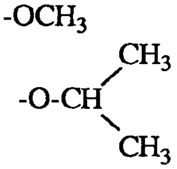 | Br | 0 | $-CH(CH_3)-CH_3$ | |
| 6.801 | F | Cl | $-OCH_3$ | F | 0 | $-CH(CH_3)-CH_3$ | |
| 6.802 | F | Cl | $-OCH_3$ | $CH_3$ | 0 | $-CH(CH_3)-CH_3$ | |
| 6.803 | F | Cl | $-OC_2H_5$ | $CH_3$ | 0 | $-CH(CH_3)-CH_3$ | |
| 6.804 | F | Cl | 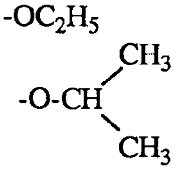 | $CH_3$ | 0 | $-CH(CH_3)-CH_3$ | |
| 6.805 | F | Cl | $-O-CH_2-CH_2-O-CH_3$ | $CH_3$ | 0 | $-CH(CH_3)-CH_3$ | |
| 6.806 | F | Cl | $-O-CH_2-COOCH_3$ | $CH_3$ | 0 | $-CH(CH_3)-CH_3$ | |
| 6.807 | F | Cl | $-O-CH(CH_3)COOCH_3$ | $CH_3$ | 0 | $-CH(CH_3)-CH_3$ | |
| 6.808 | F | Cl | $-S-CH_2-COOCH_3$ | $CH_3$ | 0 | $-CH(CH_3)-CH_3$ | |
| 6.809 | F | Cl | $-OCH_3$ | $CF_3$ | 0 | $-CH(CH_3)-CH_3$ | |
| 6.810 | F | Cl | $-OC_2H_5$ | $CF_3$ | 0 | $-CH(CH_3)-CH_3$ | |

Tabelle 6a (Fortsetzung)

| Verb. Nr. | $R_1$ | $R_2$ | $R_3$ | $R_4$ | n | $R_{13}$ | Smp. [°C] |
|---|---|---|---|---|---|---|---|
| 6.811 | F | Cl | 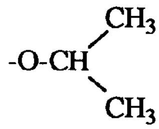 | $CF_3$ | 0 | $-CH(CH_3)-CH_3$ | |
| 6.812 | F | Cl | $-O-CH_2-COOCH_3$ | $CF_3$ | 0 | $-CH(CH_3)-CH_3$ | |
| 6.813 | F | Cl | $-S-CH_2-COOCH_3$ | $CF_3$ | 0 | $-CH(CH_3)-CH_3$ | |

Tabelle 6b:

Verbindungen der Formel I mit $W = W_6$, A = -CN und $R_{14}$ = H:

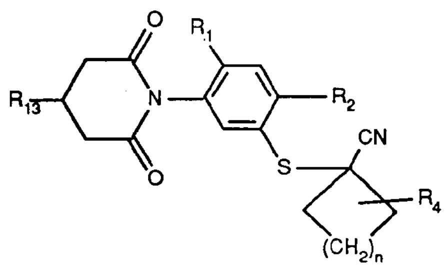 (I)

Tabelle 6b

| Verb. Nr. | $R_1$ | $R_2$ | $R_3$ | $R_4$ | n | $R_{13}$ | Smp. [°C] |
|---|---|---|---|---|---|---|---|
| 6.814 | F | Cl | - | H | 0 | $CF_3$ | |
| 6.815 | F | Cl | - | H | 1 | $CF_3$ | |
| 6.816 | F | Cl | - | H | 2 | $CF_3$ | |
| 6.817 | F | Cl | - | H | 3 | $CF_3$ | |
| 6.818 | F | Cl | - | H | 4 | $CF_3$ | |
| 6.819 | F | Cl | - | H | 0 | $CH_3$ | |
| 6.820 | F | Cl | - | H | 1 | $CH_3$ | |
| 6.821 | F | Cl | - | H | 2 | $CH_3$ | |
| 6.822 | F | Cl | - | H | 3 | $CH_3$ | |
| 6.823 | F | Cl | - | H | 4 | $CH_3$ | |
| 6.824 | F | Cl | - | H | 0 | $-CH(CH_3)-CH_3$ | |
| 6.825 | F | Cl | - | H | 1 | $-CH(CH_3)-CH_3$ | |
| 6.826 | F | Cl | - | H | 2 | $-CH(CH_3)-CH_3$ | |
| 6.827 | F | Cl | - | H | 3 | $-CH(CH_3)-CH_3$ | |
| 6.828 | F | Cl | - | H | 4 | $-CH(CH_3)-CH_3$ | |

Tabelle 7:

Verbindungen der Formel I, worin W für $W_7$, $Y_4$ für Sauerstoff und $R_{14}$ für Wasserstoff steht:

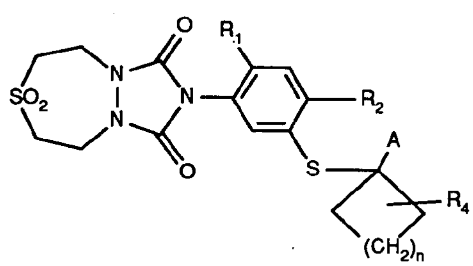

(I)

Tabelle 7

| Verb. Nr. | $R_1$ | $R_2$ | $R_3$ | $R_4$ | n | A | Smp. [°C] |
|---|---|---|---|---|---|---|---|
| 7.001 | F | Cl | -Cl | H | 0 | $-CO-R_3$ | |
| 7.002 | F | Cl | -OH | H | 0 | $-CO-R_3$ | |
| 7.003 | F | Cl | $-OCH_3$ | H | 0 | $-CO-R_3$ | |
| 7.004 | F | Cl | $-OC_2H_5$ | H | 0 | $-CO-R_3$ | |
| 7.005 | F | Cl | $-OC_3H_7$ | H | 0 | $-CO-R_3$ | |
| 7.006 | F | Cl | 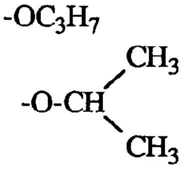 | H | 0 | $-CO-R_3$ | |
| 7.007 | F | Cl | $-OC_4H_9$ | H | 0 | $-CO-R_3$ | |
| 7.008 | F | Cl | $-O-CH(CH_3)-C_2H_5$ | H | 0 | $-CO-R_3$ | |
| 7.009 | F | Cl | 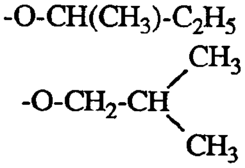 | H | 0 | $-CO-R_3$ | |
| 7.010 | F | Cl | $-OC_5H_{11}$ | H | 0 | $-CO-R_3$ | |
| 7.011 | F | Cl | $-O-CH_2-CH_2-O-CH_3$ | H | 0 | $-CO-R_3$ | |
| 7.012 | F | Cl | $-O-CH_2-CH_2-O-C_2H_5$ | H | 0 | $-CO-R_3$ | |
| 7.013 | F | Cl | $-O-CH-(CH_3)-CH_2-O-CH_3$ | H | 0 | $-CO-R_3$ | |
| 7.014 | F | Cl | $-O-CH_2-CH_2-S-CH_3$ | H | 0 | $-CO-R_3$ | |
| 7.015 | F | Cl | $-O-CH(CH_3)-CH_2-S-CH_3$ | H | 0 | $-CO-R_3$ | |
| 7.016 | F | Cl | $-O-CH(CH_3)-CH_2-S-C_2H_5$ | H | 0 | $-CO-R_3$ | |
| 7.017 | F | Cl | $-O-CH(CH_3)-CH_2-S-C_3H_7$ | H | 0 | $-CO-R_3$ | |
| 7.018 | F | Cl | 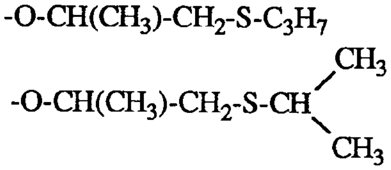 | H | 0 | $-CO-R_3$ | |
| 7.019 | F | Cl | $-O-CH(CH_3)-CH_2-S-C_4H_9$ | H | 0 | $-CO-R_3$ | |
| 7.020 | F | Cl | $-O-CH(CH_3)-CH_2-S-C_5H_{11}$ | H | 0 | $-CO-R_3$ | |
| 7.021 | F | Cl | 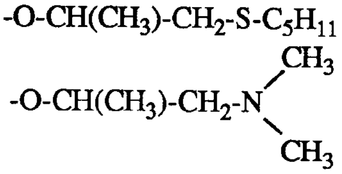 | H | 0 | $-CO-R_3$ | |

Tabelle 7 (Fortsetzung)

| Verb. Nr. | $R_1$ | $R_2$ | $R_3$ | $R_4$ | n | A | Smp. [°C] |
|---|---|---|---|---|---|---|---|
| 7.022 | F | Cl | 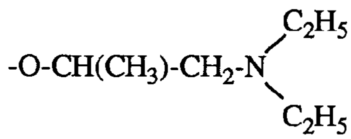 | H | 0 | $-CO-R_3$ | |
| 7.023 | F | Cl | $-NH_2$ | H | 0 | $-CO-R_3$ | |
| 7.024 | F | Cl | 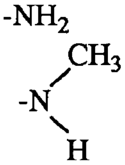 | H | 0 | $-CO-R_3$ | |
| 7.025 | F | Cl | 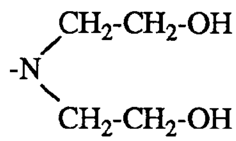 | H | 0 | $-CO-R_3$ | |
| 7.026 | F | Cl | $-NH-CH_2-CH=CH_2$ | H | 0 | $-CO-R_3$ | |
| 7.027 | F | Cl | $-N-(CH_2-CH=CH_2)_2$ | H | 0 | $-CO-R_3$ | |
| 7.028 | F | Cl | 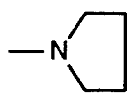 | H | 0 | $-CO-R_3$ | |
| 7.029 | F | Cl | 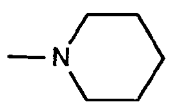 | H | 0 | $-CO-R_3$ | |
| 7.030 | F | Cl | 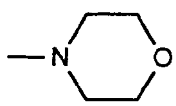 | H | 0 | $-CO-R_3$ | |
| 7.031 | F | Cl | 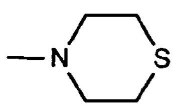 | H | 0 | $-CO-R_3$ | |
| 7.032 | F | Cl | 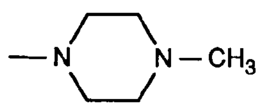 | H | 0 | $-CO-R_3$ | |
| 7.033 | F | Cl | 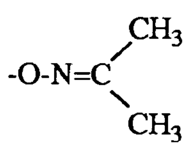 | H | 0 | $-CO-R_3$ | |
| 7.034 | F | Cl | $-O-CH_2-CH_2-Cl$ | H | 0 | $-CO-R_3$ | |
| 7.035 | F | Cl | $-O-CH_2-CN$ | H | 0 | $-CO-R_3$ | |
| 7.036 | F | Cl | 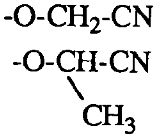 | H | 0 | $-CO-R_3$ | |
| 7.037 | F | Cl | $-O-CH_2-CH=CH_2$ | H | 0 | $-CO-R_3$ | |

Tabelle 7 (Fortsetzung)

| Verb. Nr. | $R_1$ | $R_2$ | $R_3$ | $R_4$ | n | A | Smp. [°C] |
|---|---|---|---|---|---|---|---|
| 7.038 | F | Cl | $-O-CH_2-CH=CHCl$ | H | 0 | $-CO-R_3$ | |
| 7.039 | F | Cl | $-O-CH_2-C=CH_2$<br>Cl | H | 0 | $-CO-R_3$ | |
| 7.040 | F | Cl | $-O-CH_2=C\equiv CH$ | H | 0 | $-CO-R_3$ | |
| 7.041 | F | Cl | $-O-CH-C\equiv CH$<br>$CH_3$ | H | 0 | $-CO-R_3$ | |
| 7.042 | F | Cl | 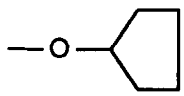 | H | 0 | $-CO-R_3$ | |
| 7.043 | F | Cl | 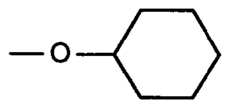 | H | 0 | $-CO-R_3$ | |
| 7.044 | F | Cl | 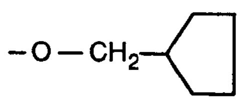 | H | 0 | $-CO-R_3$ | |
| 7.045 | F | Cl | 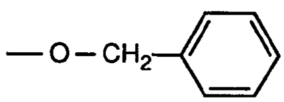 | H | 0 | $-CO-R_3$ | |
| 7.046 | F | Cl | 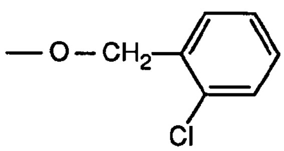 | H | 0 | $-CO-R_3$ | |
| 7.047 | F | Cl | 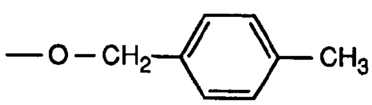 | H | 0 | $-CO-R_3$ | |
| 7.048 | F | Cl | $-S-CH_3$ | H | 0 | $-CO-R_3$ | |
| 7.049 | F | Cl | $-S-C_2H_5$ | H | 0 | $-CO-R_3$ | |
| 7.050 | F | Cl | $-S-C_3H_7$ | H | 0 | $-CO-R_3$ | |
| 7.051 | F | Cl | $-S-CH_2-CH=CH_2$ | H | 0 | $-CO-R_3$ | |
| 7.052 | F | Cl | $-S-CH_2-COOCH_3$ | H | 0 | $-CO-R_3$ | |
| 7.053 | F | Cl | $-S-CH_2-COOC_2H_5$ | H | 0 | $-CO-R_3$ | |
| 7.054 | F | Cl | $-S-CH_2-COOC_5H_{11}$ | H | 0 | $-CO-R_3$ | |
| 7.055 | F | Cl | $-S-CH(CH_3)-COOCH_3$ | H | 0 | $-CO-R_3$ | |
| 7.056 | F | Cl | $-S-(CH_3)-COOC_2H_5$ | H | 0 | $-CO-R_3$ | |

Tabelle 7 (Fortsetzung)

| Verb. Nr. | $R_1$ | $R_2$ | $R_3$ | $R_4$ | n | A | Smp. [°C] |
|---|---|---|---|---|---|---|---|
| 7.057 | F | Cl | $-S-CH(CH_3)-COOC_3H_7$ | H | 0 | $-CO-R_3$ | |
| 7.058 | F | Cl | $-S-CH_2-CH_2-COOCH_3$ | H | 0 | $-CO-R_3$ | |
| 7.059 | F | Cl | $-S-CH_2-COOCH_2-CH_2-O-CH_3$ | H | 0 | $-CO-R_3$ | |
| 7.060 | F | Cl | $-O-CH_2-COOCH_3$ | H | 0 | $-CO-R_3$ | |
| 7.061 | F | Cl | $-O-CH(CH_3)-COOCH_3$ | H | 0 | $-CO-R_3$ | |
| 7.062 | F | Cl | $-O-CH_2-COOC_5H_{11}$ | H | 0 | $-CO-R_3$ | |
| 7.063 | F | Cl | $-O-CH_2-CH_3-Si(CH_3)_3$ | H | 0 | $-CO-R_3$ | |
| 7.064 | F | Cl | -ONa | H | 0 | $-CO-R_3$ | |
| 7.065 | F | Br | -Cl | H | 0 | $-CO-R_3$ | |
| 7.066 | F | Br | -OH | H | 0 | $-CO-R_3$ | |
| 7.067 | F | Br | $-OCH_3$ | H | 0 | $-CO-R_3$ | |
| 7.068 | F | Br | $-OC_2H_5$ | H | 0 | $-CO-R_3$ | |
| 7.069 | F | Br | $-OC_3H_7$ | H | 0 | $-CO-R_3$ | |
| 7.070 | F | Br | 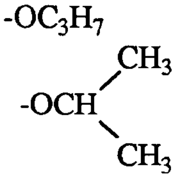 | H | 0 | $-CO-R_3$ | |
| 7.071 | F | Br | $-OC_4H_9$ | H | 0 | $-CO-R_3$ | |
| 7.072 | F | Br | $-OCH(CH_3)-CH_2-CH_3$ | H | 0 | $-CO-R_3$ | |
| 7.073 | F | Br | 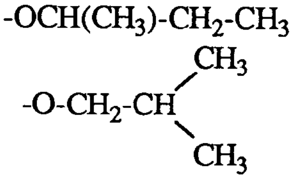 | H | 0 | $-CO-R_3$ | |
| 7.074 | F | Br | $-O-C_5H_{11}$ | H | 0 | $-CO-R_3$ | |
| 7.075 | F | Br | $-O-CH_2-CH_2-O-CH_3$ | H | 0 | $-CO-R_3$ | |
| 7.076 | F | Br | $-O-CH_2-CH_2-O-C_2H_5$ | H | 0 | $-CO-R_3$ | |
| 7.077 | F | Br | $-O-CH(CH_3)-CH_2-O-CH_3$ | H | 0 | $-CO-R_3$ | |
| 7.078 | F | Br | $-O-CH_2-CH_2-S-CH_3$ | H | 0 | $-CO-R_3$ | |
| 7.079 | F | Br | $-O-CH_2(CH_3)-S-CH_3$ | H | 0 | $-CO-R_3$ | |
| 7.080 | F | Br | $-O-CH-(CH_3)-S-C_2H_5$ | H | 0 | $-CO-R_3$ | |
| 7.081 | F | Br | $-O-CH(CH_3)-S-C_3H_7$ | H | 0 | $-CO-R_3$ | |
| 7.082 | F | Br | 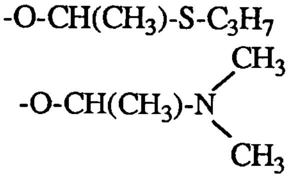 | H | 0 | $-CO-R_3$ | |
| 7.083 | F | Br | $-NH_2$ | H | 0 | $-CO-R_3$ | |

Tabelle 7 (Fortsetzung)

| Verb. Nr. | $R_1$ | $R_2$ | $R_3$ | $R_4$ | n | A | Smp. [°C] |
|---|---|---|---|---|---|---|---|
| 7.084 | F | Br | 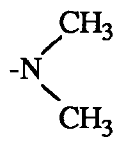 | H | 0 | $-CO-R_3$ | |
| 7.085 | F | Br | 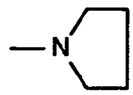 | H | 0 | $-CO-R_3$ | |
| 7.086 | F | Br | 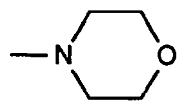 | H | 0 | $-CO-R_3$ | |
| 7.087 | F | Br | 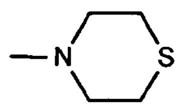 | H | 0 | $-CO-R_3$ | |
| 7.088 | F | Br | 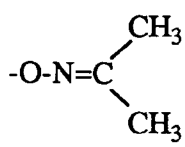 | H | 0 | $-CO-R_3$ | |
| 7.089 | F | Br | $-O-CH_2-CH_2-Cl$ | H | 0 | $-CO-R_3$ | |
| 7.090 | F | Br | $-O-CH_2-CN$ | H | 0 | $-CO-R_3$ | |
| 7.091 | F | Br | $-O-CH_2-CH=CH_2$ | H | 0 | $-CO-R_3$ | |
| 7.092 | F | Br | $-O-CH_2-C\equiv CH$ | H | 0 | $-CO-R_3$ | |
| 7.093 | F | Br | 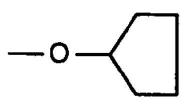 | H | 0 | $-CO-R_3$ | |
| 7.094 | F | Br | 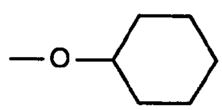 | H | 0 | $-CO-R_3$ | |
| 7.095 | F | Br | 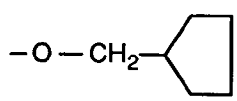 | H | 0 | $-CO-R_3$ | |
| 7.096 | F | Br | 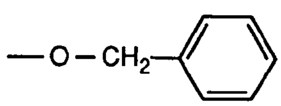 | H | 0 | $-CO-R_3$ | |
| 7.097 | F | Br | $-SCH_3$ | H | 0 | $-CO-R_3$ | |
| 7.098 | F | Br | $-S-CH_2-COOCH_3$ | H | 0 | $-CO-R_3$ | |
| 7.099 | F | Br | $-S-CH(CH_3)-COOCH_3$ | H | 0 | $-CO-R_3$ | |
| 7.100 | F | Br | $-O-CH_2-COOCH_3$ | H | 0 | $-CO-R_3$ | |

Tabelle 7 (Fortsetzung)

| Verb. Nr. | $R_1$ | $R_2$ | $R_3$ | $R_4$ | n | A | Smp. [°C] |
|---|---|---|---|---|---|---|---|
| 7.101 | F | Br | $-O-CH(CH_3)COOCH_3$ | H | 0 | $-CO-R_3$ | |
| 7.102 | F | CN | -Cl | H | 0 | $-CO-R_3$ | |
| 7.103 | F | CN | -OH | H | 0 | $-CO-R_3$ | |
| 7.104 | F | CN | $-OCH_3$ | H | 0 | $-CO-R_3$ | |
| 7.105 | H | Cl | -Cl | H | 0 | $-CO-R_3$ | |
| 7.106 | H | Cl | -OH | H | 0 | $-CO-R_3$ | |
| 7.107 | H | Cl | $-OCH_3$ | H | 0 | $-CO-R_3$ | |
| 7.108 | H | Cl | $-OC_2H_5$ | H | 0 | $-CO-R_3$ | |
| 7.109 | H | Cl | 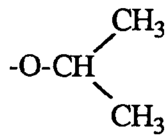 | H | 0 | $-CO-R_3$ | |
| 7.110 | H | Cl | $-O-CH_2-COOCH_3$ | H | 0 | $-CO-R_3$ | |
| 7.111 | H | Cl | $-O-CH(CH_3)COOCH_3$ | H | 0 | $-CO-R_3$ | |
| 7.112 | H | Cl | $-S-CH_2-COOCH_3$ | H | 0 | $-CO-R_3$ | |
| 7.113 | H | Cl | $-S-CH(CH_3)COOCH_3$ | H | 0 | $-CO-R_3$ | |
| 7.114 | H | Cl | 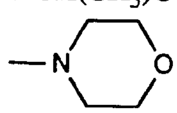 | H | 0 | $-CO-R_3$ | |
| 7.115 | F | Cl | -Cl | H | 1 | $-CO-R_3$ | |
| 7.116 | F | Cl | -OH | H | 1 | $-CO-R_3$ | |
| 7.117 | F | Cl | $-OCH_3$ | H | 1 | $-CO-R_3$ | |
| 7.118 | F | Cl | $-OC_2H_5$ | H | 1 | $-CO-R_3$ | |
| 7.119 | F | Cl | $-OC_3H_7$ | H | 1 | $-CO-R_3$ | |
| 7.120 | F | Cl | 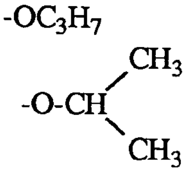 | H | 1 | $-CO-R_3$ | |
| 7.121 | F | Cl | $-OC_4H_9$ | H | 1 | $-CO-R_3$ | |
| 7.122 | F | Cl | $-O-CH(CH_3)-C_2H_5$ | H | 1 | $-CO-R_3$ | |
| 7.123 | F | Cl | 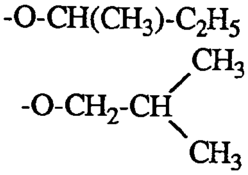 | H | 1 | $-CO-R_3$ | |
| 7.124 | F | Cl | $-OC_5H_{11}$ | H | 1 | $-CO-R_3$ | |
| 7.125 | F | Cl | $-O-CH_2-CH_2-O-CH_3$ | H | 1 | $-CO-R_3$ | |
| 7.126 | F | Cl | $-O-CH_2-CH_2-O-C_2H_5$ | H | 1 | $-CO-R_3$ | |

Tabelle 7 (Fortsetzung)

| Verb. Nr. | $R_1$ | $R_2$ | $R_3$ | $R_4$ | n | A | Smp. [°C] |
|---|---|---|---|---|---|---|---|
| 7.127 | F | Cl | $-O-CH-(CH_3)-CH_2-O-CH_3$ | H | 1 | $-CO-R_3$ | |
| 7.128 | F | Cl | $-O-CH_2-CH_2-S-CH_3$ | H | 1 | $-CO-R_3$ | |
| 7.129 | F | Cl | $-O-CH(CH_3)-CH_2-S-CH_3$ | H | 1 | $-CO-R_3$ | |
| 7.130 | F | Cl | $-O-CH(CH_3)-CH_2-S-C_2H_5$ | H | 1 | $-CO-R_3$ | |
| 7.131 | F | Cl | $-O-CH(CH_3)-CH_2-S-C_3H_7$ | H | 1 | $-CO-R_3$ | |
| 7.132 | F | Cl | 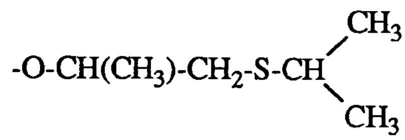 | H | 1 | $-CO-R_3$ | |
| 7.133 | F | Cl | $-O-CH(CH_3)-CH_2-S-C_4H_9$ | H | 1 | $-CO-R_3$ | |
| 7.134 | F | Cl | $-O-CH(CH_3)-CH_2-S-C_5H_{11}$ | H | 1 | $-CO-R_3$ | |
| 7.135 | F | Cl | 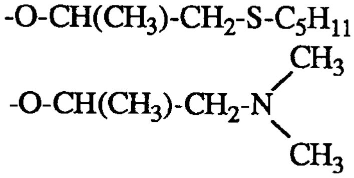 | H | 1 | $-CO-R_3$ | |
| 7.136 | F | Cl | 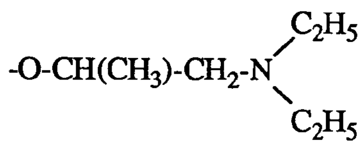 | H | 1 | $-CO-R_3$ | |
| 7.137 | F | Cl | $-NH_2$ | H | 1 | $-CO-R_3$ | |
| 7.138 | F | Cl | 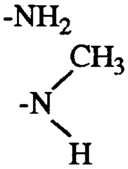 | H | 1 | $-CO-R_3$ | |
| 7.139 | F | Cl | 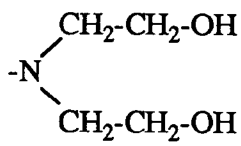 | H | 1 | $-CO-R_3$ | |
| 7.140 | F | Cl | $-NH-CH_2-CH=CH_2$ | H | 1 | $-CO-R_3$ | |
| 7.141 | F | Cl | $-N-(CH_2-CH=CH_2)_2$ | H | 1 | $-CO-R_3$ | |
| 7.142 | F | Cl | 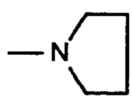 | H | 1 | $-CO-R_3$ | |
| 7.143 | F | Cl | 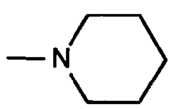 | H | 1 | $-CO-R_3$ | |

Tabelle 7 (Fortsetzung)

| Verb. Nr. | $R_1$ | $R_2$ | $R_3$ | $R_4$ | n | A | Smp. [°C] |
|---|---|---|---|---|---|---|---|
| 7.144 | F | Cl | 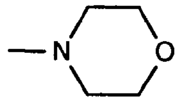 | H | 1 | -CO-$R_3$ | |
| 7.145 | F | Cl | 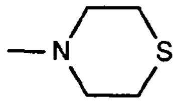 | H | 1 | -CO-$R_3$ | |
| 7.146 | F | Cl | 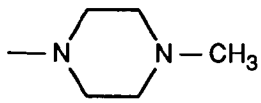 | H | 1 | -CO-$R_3$ | |
| 7.147 | F | Cl | $CH_3$<br>-O-N=C<br>$CH_3$ | H | 1 | -CO-$R_3$ | |
| 7.148 | F | Cl | -O-$CH_2$-$CH_2$-Cl | H | 1 | -CO-$R_3$ | |
| 7.149 | F | Cl | -O-$CH_2$-CN | H | 1 | -CO-$R_3$ | |
| 7.150 | F | Cl | -O-CH-CN<br>$CH_3$ | H | 1 | -CO-$R_3$ | |
| 7.151 | F | Cl | -O-$CH_2$-CH=$CH_2$ | H | 1 | -CO-$R_3$ | |
| 7.152 | F | Cl | -O-$CH_2$-CH=CHCl | H | 1 | -CO-$R_3$ | |
| 7.153 | F | Cl | -O-$CH_2$-C=$CH_2$<br>Cl | H | 1 | -CO-$R_3$ | |
| 7.154 | F | Cl | -O-$CH_2$=C≡CH | H | 1 | -CO-$R_3$ | |
| 7.155 | F | Cl | -O-CH-C≡CH<br>$CH_3$ | H | 1 | -CO-$R_3$ | |
| 7.156 | F | Cl | 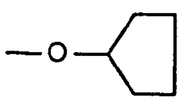 | H | 1 | -CO-$R_3$ | |
| 7.157 | F | Cl | 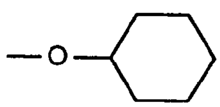 | H | 1 | -CO-$R_3$ | |
| 7.158 | F | Cl | 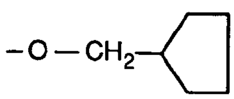 | H | 1 | -CO-$R_3$ | |
| 7.159 | F | Cl | 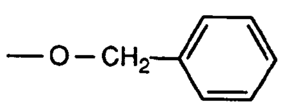 | H | 1 | -CO-$R_3$ | |

Tabelle 7 (Fortsetzung)

| Verb. Nr. | $R_1$ | $R_2$ | $R_3$ | $R_4$ | n | A | Smp. [°C] |
|---|---|---|---|---|---|---|---|
| 7.160 | F | Cl | 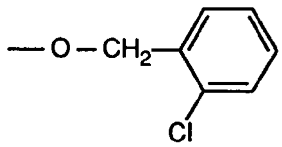 | H | 1 | $-CO-R_3$ | |
| 7.161 | F | Cl | 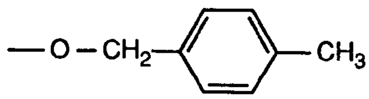 | H | 1 | $-CO-R_3$ | |
| 7.162 | F | Cl | $-S-CH_3$ | H | 1 | $-CO-R_3$ | |
| 7.163 | F | Cl | $-S-C_2H_5$ | H | 1 | $-CO-R_3$ | |
| 7.164 | F | Cl | $-S-C_3H_7$ | H | 1 | $-CO-R_3$ | |
| 7.165 | F | Cl | $-S-CH_2-CH=CH_2$ | H | 1 | $-CO-R_3$ | |
| 7.166 | F | Cl | $-S-CH_2-COOCH_3$ | H | 1 | $-CO-R_3$ | |
| 7.167 | F | Cl | $-S-CH_2-COOC_2H_5$ | H | 1 | $-CO-R_3$ | |
| 7.168 | F | Cl | $-S-CH_2-COOC_5H_{11}$ | H | 1 | $-CO-R_3$ | |
| 7.169 | F | Cl | $-S-CH(CH_3)-COOCH_3$ | H | 1 | $-CO-R_3$ | |
| 7.170 | F | Cl | $-S-(CH_3)-COOC_2H_5$ | H | 1 | $-CO-R_3$ | |
| 7.171 | F | Cl | $-S-CH(CH_3)-COOC_3H_7$ | H | 1 | $-CO-R_3$ | |
| 7.172 | F | Cl | $-S-CH_2-CH_2-COOCH_3$ | H | 1 | $-CO-R_3$ | |
| 7.173 | F | Cl | $-S-CH_2-COOCH_2-CH_2-O-CH_3$ | H | 1 | $-CO-R_3$ | |
| 7.174 | F | Cl | $-O-CH_2-COOCH_3$ | H | 1 | $-CO-R_3$ | |
| 7.175 | F | Cl | $-O-CH(CH_3)-COOCH_3$ | H | 1 | $-CO-R_3$ | |
| 7.176 | F | Cl | $-O-CH_2-COOC_5H_{11}$ | H | 1 | $-CO-R_3$ | |
| 7.177 | F | Cl | $-O-CH_2-CH_3-Si(CH_3)_3$ | H | 1 | $-CO-R_3$ | |
| 7.178 | F | Cl | -ONa | H | 1 | $-CO-R_3$ | |
| 7.179 | F | Br | -Cl | H | 1 | $-CO-R_3$ | |
| 7.180 | F | Br | -OH | H | 1 | $-CO-R_3$ | |
| 7.181 | F | Br | $-OCH_3$ | H | 1 | $-CO-R_3$ | |
| 7.182 | F | Br | $-OC_2H_5$ | H | 1 | $-CO-R_3$ | |
| 7.183 | F | Br | $-OC_3H_7$ | H | 1 | $-CO-R_3$ | |
| 7.184 | F | Br | 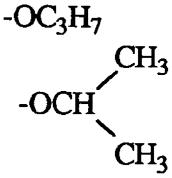 | H | 1 | $-CO-R_3$ | |
| 7.185 | F | Br | $-OC_4H_9$ | H | 1 | $-CO-R_3$ | |

Tabelle 7 (Fortsetzung)

| Verb. Nr. | $R_1$ | $R_2$ | $R_3$ | $R_4$ | n | A | Smp. [°C] |
|---|---|---|---|---|---|---|---|
| 7.186 | F | Br | $-OCH(CH_3)-CH_2-CH_3$ | H | 1 | $-CO-R_3$ | |
| 7.187 | F | Br | 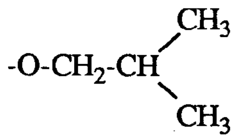 | H | 1 | $-CO-R_3$ | |
| 7.188 | F | Br | $-O-C_5H_{11}$ | H | 1 | $-CO-R_3$ | |
| 7.189 | F | Br | $-O-CH_2-CH_2-O-CH_3$ | H | 1 | $-CO-R_3$ | |
| 7.190 | F | Br | $-O-CH_2-CH_2-O-C_2H_5$ | H | 1 | $-CO-R_3$ | |
| 7.191 | F | Br | $-O-CH(CH_3)-CH_2-O-CH_3$ | H | 1 | $-CO-R_3$ | |
| 7.192 | F | Br | $-O-CH_2-CH_2-S-CH_3$ | H | 1 | $-CO-R_3$ | |
| 7.193 | F | Br | $-O-CH_2(CH_3)-S-CH_3$ | H | 1 | $-CO-R_3$ | |
| 7.194 | F | Br | $-O-CH-(CH_3)-S-C_2H_5$ | H | 1 | $-CO-R_3$ | |
| 7.195 | F | Br | $-O-CH(CH_3)-S-C_3H_7$ | H | 1 | $-CO-R_3$ | |
| 7.196 | F | Br | 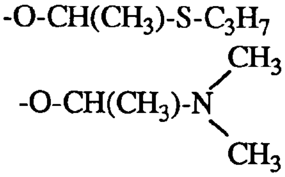 | H | 1 | $-CO-R_3$ | |
| 7.197 | F | Br | $-NH_2$ | H | 1 | $-CO-R_3$ | |
| 7.198 | F | Br | 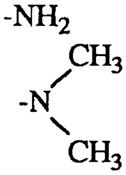 | H | 1 | $-CO-R_3$ | |
| 7.199 | F | Br | 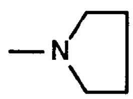 | H | 1 | $-CO-R_3$ | |
| 7.200 | F | Br | 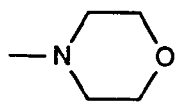 | H | 1 | $-CO-R_3$ | |
| 7.201 | F | Br | 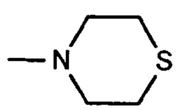 | H | 1 | $-CO-R_3$ | |
| 7.202 | F | Br | 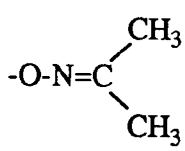 | H | 1 | $-CO-R_3$ | |
| 7.203 | F | Br | $-O-CH_2-CH_2-Cl$ | H | 1 | $-CO-R_3$ | |
| 7.204 | F | Br | $-O-CH_2-CN$ | H | 1 | $-CO-R_3$ | |
| 7.205 | F | Br | $-O-CH_2-CH=CH_2$ | H | 1 | $-CO-R_3$ | |
| 7.206 | F | Br | $-O-CH_2-C\equiv CH$ | H | 1 | $-CO-R_3$ | |

Tabelle 7 (Fortsetzung)

| Verb. Nr. | $R_1$ | $R_2$ | $R_3$ | $R_4$ | n | A | Smp. [°C] |
|---|---|---|---|---|---|---|---|
| 7.207 | F | Br | 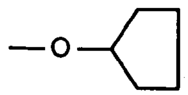 | H | 1 | $-CO-R_3$ | |
| 7.208 | F | Br | 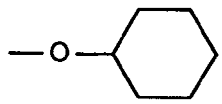 | H | 1 | $-CO-R_3$ | |
| 7.209 | F | Br | 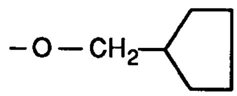 | H | 1 | $-CO-R_3$ | |
| 7.210 | F | Br | 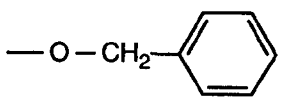 | H | 1 | $-CO-R_3$ | |
| 7.211 | F | Br | $-SCH_3$ | H | 1 | $-CO-R_3$ | |
| 7.212 | F | Br | $-S-CH_2-COOCH_3$ | H | 1 | $-CO-R_3$ | |
| 7.213 | F | Br | $-S-CH(CH_3)-COOCH_3$ | H | 1 | $-CO-R_3$ | |
| 7.214 | F | Br | $-O-CH_2-COOCH_3$ | H | 1 | $-CO-R_3$ | |
| 7.215 | F | Br | $-O-CH(CH_3)COOCH_3$ | H | 1 | $-CO-R_3$ | |
| 7.216 | F | CN | -Cl | H | 1 | $-CO-R_3$ | |
| 7.217 | F | CN | -OH | H | 1 | $-CO-R_3$ | |
| 7.218 | F | CN | $-OCH_3$ | H | 1 | $-CO-R_3$ | |
| 7.219 | H | Cl | -Cl | H | 1 | $-CO-R_3$ | |
| 7.220 | H | Cl | -OH | H | 1 | $-CO-R_3$ | |
| 7.221 | H | Cl | $-OCH_3$ | H | 1 | $-CO-R_3$ | |
| 7.222 | H | Cl | $-OC_2H_5$ | H | 1 | $-CO-R_3$ | |
| 7.223 | H | Cl | 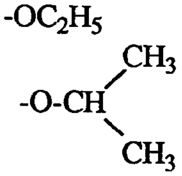 | H | 1 | $-CO-R_3$ | |
| 7.224 | H | Cl | $-O-CH_2-COOCH_3$ | H | 1 | $-CO-R_3$ | |
| 7.225 | H | Cl | $-O-CH(CH_3)COOCH_3$ | H | 1 | $-CO-R_3$ | |
| 7.226 | H | Cl | $-S-CH_2-COOCH_3$ | H | 1 | $-CO-R_3$ | |
| 7.227 | H | Cl | $-S-CH(CH_3)COOCH_3$ | H | 1 | $-CO-R_3$ | |
| 7.228 | H | Cl | 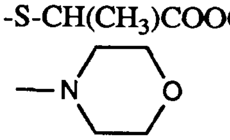 | H | 1 | $-CO-R_3$ | |
| 7.229 | F | Cl | Cl | H | 2 | $-CO-R_3$ | |

Tabelle 7 (Fortsetzung)

| Verb. Nr. | $R_1$ | $R_2$ | $R_3$ | $R_4$ | n | A | Smp. [°C] |
|---|---|---|---|---|---|---|---|
| 7.230 | F | Cl | OH | H | 2 | $-CO-R_3$ | |
| 7.231 | F | Cl | $OCH_3$ | H | 2 | $-CO-R_3$ | |
| 7.232 | F | Cl | $-OC_2H_5$ | H | 2 | $-CO-R_3$ | |
| 7.233 | F | Cl | 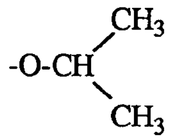 | H | 2 | $-CO-R_3$ | |
| 7.234 | F | Cl | $-O-CH_2-CH_2-O-CH_3$ | H | 2 | $-CO-R_3$ | |
| 7.235 | F | Cl | $-O-CH(CH_3)-CH_2-S-CH_3$ | H | 2 | $-CO-R_3$ | |
| 7.236 | F | Cl | $-O-CH_2-COOCH_3$ | H | 2 | $-CO-R_3$ | |
| 7.237 | F | Cl | $-S-CH_2-COOCH_3$ | H | 2 | $-CO-R_3$ | |
| 7.238 | F | Cl | $-CH_2-CH=CH_2$ | H | 2 | $-CO-R_3$ | |
| 7.239 | F | Cl | $-CH_2-C\equiv CH$ | H | 2 | $-CO-R_3$ | |
| 7.240 | F | Cl | Cl | H | 3 | $-CO-R_3$ | |
| 7.241 | F | Cl | OH | H | 3 | $-CO-R_3$ | |
| 7.242 | F | Cl | $OCH_3$ | H | 3 | $-CO-R_3$ | |
| 7.243 | F | Cl | $OC_2H_5$ | H | 3 | $-CO-R_3$ | |
| 7.244 | F | Cl | 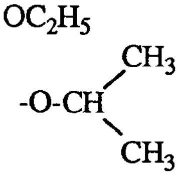 | H | 3 | $-CO-R_3$ | |
| 7.245 | F | Cl | $-O-CH_2-CH_2-O-CH$ | H | 3 | $-CO-R_3$ | |
| 7.246 | F | Cl | $-O-CH(CH_3)-CH_2-S-CH_3$ | H | 3 | $-CO-R_3$ | |
| 7.247 | F | Cl | $-O-CH_2-COOCH_3$ | H | 3 | $-CO-R_3$ | |
| 7.248 | F | Cl | $-S-CH_2-COOCH_3$ | H | 3 | $-CO-R_3$ | |
| 7.249 | F | Cl | $-O-CH_2-C\equiv CH$ | H | 3 | $-CO-R_3$ | |
| 7.250 | F | Cl | -Cl | Cl | 0 | $-CO-R_3$ | |
| 7.251 | F | Cl | -OH | Cl | 0 | $-CO-R_3$ | |
| 7.252 | F | Cl | $-OCH_3$ | Cl | 0 | $-CO-R_3$ | |
| 7.253 | F | Cl | $-OC_2H_5$ | Cl | 0 | $-CO-R_3$ | |
| 7.254 | F | Cl | 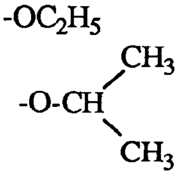 | Cl | 0 | $-CO-R_3$ | |
| 7.255 | F | Cl | $-O-CH_2-COOCH_3$ | Cl | 0 | $-CO-R_3$ | |
| 7.256 | F | Cl | $-S-CH_2-COOCH_3$ | Cl | 0 | $-CO-R_3$ | |
| 7.257 | F | Cl | $-OCH_3$ | Br | 0 | $-CO-R_3$ | |

Tabelle 7 (Fortsetzung)

| Verb. Nr. | $R_1$ | $R_2$ | $R_3$ | $R_4$ | n | A | Smp. [°C] |
|---|---|---|---|---|---|---|---|
| 7.258 | F | Cl | 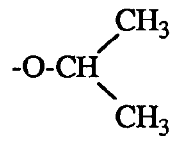 | Br | 0 | $-CO-R_3$ | |
| 7.259 | F | Cl | $-OCH_3$ | F | 0 | $-CO-R_3$ | |
| 7.260 | F | Cl | $-OCH_3$ | $CH_3$ | 0 | $-CO-R_3$ | |
| 7.261 | F | Cl | $-OC_2H_5$ | $CH_3$ | 0 | $-CO-R_3$ | |
| 7.262 | F | Cl | 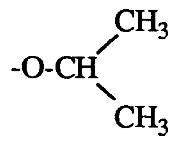 | $CH_3$ | 0 | $-CO-R_3$ | |
| 7.263 | F | Cl | $-O-CH_2-CH_2-O-CH_3$ | $CH_3$ | 0 | $-CO-R_3$ | |
| 7.264 | F | Cl | $-O-CH_2-COOCH_3$ | $CH_3$ | 0 | $-CO-R_3$ | |
| 7.265 | F | Cl | $-O-CH(CH_3)COOCH_3$ | $CH_3$ | 0 | $-CO-R_3$ | |
| 7.266 | F | Cl | $-S-CH_2-COOCH_3$ | $CH_3$ | 0 | $-CO-R_3$ | |
| 7.267 | F | Cl | $-OCH_3$ | $CF_3$ | 0 | $-CO-R_3$ | |
| 7.268 | F | Cl | $-OC_2H_5$ | $CF_3$ | 0 | $-CO-R_3$ | |
| 7.269 | F | Cl | 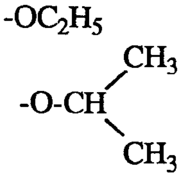 | $CF_3$ | 0 | $-CO-R_3$ | |
| 7.270 | F | Cl | $-O-CH_2-COOCH_3$ | $CF_3$ | 0 | $-CO-R_3$ | |
| 7.271 | F | Cl | $-S-CH_2-COOCH_3$ | $CF_3$ | 0 | $-CO-R_3$ | |
| 7.272 | F | Cl | - | H | 0 | -CN | |
| 7.273 | F | Cl | - | H | 1 | -CN | |
| 7.274 | F | Cl | - | H | 2 | -CN | |
| 7.275 | F | Cl | - | H | 3 | -CN | |
| 7.276 | F | Cl | - | H | 4 | -CN | |

Tabelle 8:

Verbindungen der Formel I, worin W für $W_8$ und $Z_1$ für Sauerstoff steht:

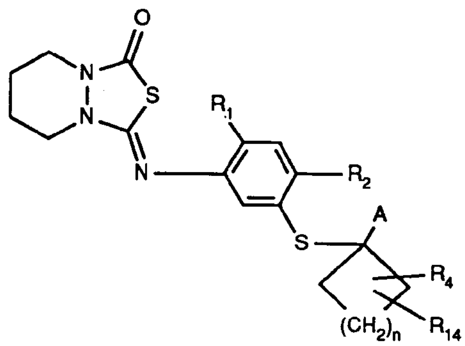

(I)

Tabelle 8 (Fortsetzung)

| Verb. Nr. | $R_1$ | $R_2$ | $R_3$ | $R_{14}$ | $R_4$ | n | A | Smp. [°C] |
|---|---|---|---|---|---|---|---|---|
| 8.001 | F | Cl | -Cl | H | H | 0 | $-CO-R_3$ | |
| 8.002 | F | Cl | -OH | H | H | 0 | $-CO-R_3$ | 162-164 |
| 8.003 | F | Cl | $-OCH_3$ | H | H | 0 | $-CO-R_3$ | 149 |
| 8.004 | F | Cl | $-OC_2H_5$ | H | H | 0 | $-CO-R_3$ | 123-124 |
| 8.005 | F | Cl | $-OC_3H_7$ | H | H | 0 | $-CO-R_3$ | |
| 8.006 | F | Cl | 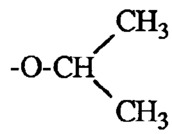 | H | H | 0 | $-CO-R_3$ | |
| 8.007 | F | Cl | $-OC_4H_9$ | H | H | 0 | $-CO-R_3$ | 75-78 |
| 8.008 | F | Cl | $-O-CH(CH_3)-C_2H_5$ | H | H | 0 | $-CO-R_3$ | |
| 8.009 | F | Cl | 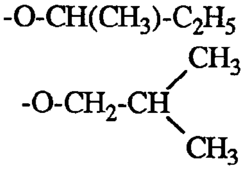 | H | H | 0 | $-CO-R_3$ | 109-111 |
| 8.010 | F | Cl | $-OC_5H_{11}$ | H | H | 0 | $-CO-R_3$ | |
| 8.011 | F | Cl | $-O-CH_2-CH_2-O-CH_3$ | H | H | 0 | $-CO-R_3$ | 89-90 |
| 8.012 | F | Cl | $-O-CH_2-CH_2-O-C_2H_5$ | H | H | 0 | $-CO-R_3$ | |
| 8.013 | F | Cl | $-O-CH-(CH_3)-CH_2-O-CH_3$ | H | H | 0 | $-CO-R_3$ | |
| 8.014 | F | Cl | $-O-CH_2-CH_2-S-CH_3$ | H | H | 0 | $-CO-R_3$ | |
| 8.015 | F | Cl | $-O-CH(CH_3)-CH_2-S-CH_3$ | H | H | 0 | $-CO-R_3$ | |
| 8.016 | F | Cl | $-O-CH(CH_3)-CH_2-S-C_2H_5$ | H | H | 0 | $-CO-R_3$ | |
| 8.017 | F | Cl | $-O-CH(CH_3)-CH_2-S-C_3H_7$ | H | H | 0 | $-CO-R_3$ | |
| 8.018 | F | Cl | 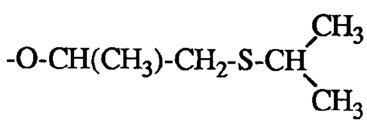 | H | H | 0 | $-CO-R_3$ | |
| 8.019 | F | Cl | $-O-CH(CH_3)-CH_2-S-C_4H_9$ | H | H | 0 | $-CO-R_3$ | |
| 8.020 | F | Cl | $-O-CH(CH_3)-CH_2-S-C_5H_{11}$ | H | H | 0 | $-CO-R_3$ | |
| 8.021 | F | Cl | 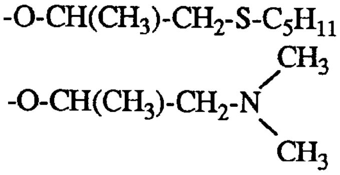 | H | H | 0 | $-CO-R_3$ | 124-126 |

Tabelle 8 (Fortsetzung)

| Verb. Nr. | $R_1$ | $R_2$ | $R_3$ | $R_{14}$ | $R_4$ | n | A | Smp. [°C] |
|---|---|---|---|---|---|---|---|---|
| 8.022 | F | Cl | 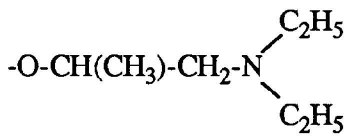 | H | H | 0 | $-CO-R_3$ | |
| 8.023 | F | Cl | $-NH_2$ | H | H | 0 | $-CO-R_3$ | |
| 8.024 | F | Cl | 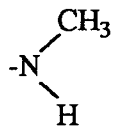 | H | H | 0 | $-CO-R_3$ | |
| 8.025 | F | Cl | 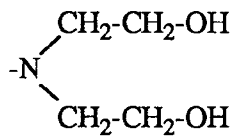 | H | H | 0 | $-CO-R_3$ | |
| 8.026 | F | Cl | $-NH-CH_2-CH=CH_2$ | H | H | 0 | $-CO-R_3$ | |
| 8.027 | F | Cl | $-N-(CH_2-CH=CH_2)_2$ | H | H | 0 | $-CO-R_3$ | |
| 8.028 | F | Cl | 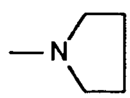 | H | H | 0 | $-CO-R_3$ | |
| 8.029 | F | Cl | 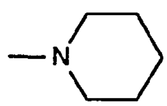 | H | H | 0 | $-CO-R_3$ | |
| 8.030 | F | Cl | 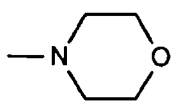 | H | H | 0 | $-CO-R_3$ | |
| 8.031 | F | Cl | 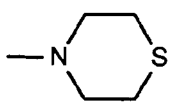 | H | H | 0 | $-CO-R_3$ | |
| 8.032 | F | Cl | 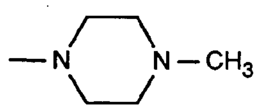 | H | H | 0 | $-CO-R_3$ | |
| 8.033 | F | Cl | 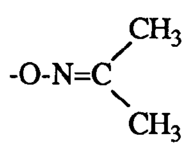 | H | H | 0 | $-CO-R_3$ | 149-151 |
| 8.034 | F | Cl | $-O-CH_2-CH_2-Cl$ | H | H | 0 | $-CO-R_3$ | |
| 8.035 | F | Cl | $-O-CH_2-CN$ | H | H | 0 | $-CO-R_3$ | |
| 8.036 | F | Cl | 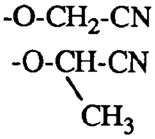 | H | H | 0 | $-CO-R_3$ | |
| 8.037 | F | Cl | $-O-CH_2-CH=CH_2$ | H | H | 0 | $-CO-R_3$ | 94-96 |

Tabelle 8 (Fortsetzung)

| Verb. Nr. | $R_1$ | $R_2$ | $R_3$ | $R_{14}$ | $R_4$ | n | A | Smp. [°C] |
|---|---|---|---|---|---|---|---|---|
| 8.038 | F | Cl | $-O-CH_2-CH=CHCl$ | H | H | 0 | $-CO-R_3$ | 121-123 |
| 8.039 | F | Cl | $-O-CH_2-C=CH_2$ <br> $\mid$ <br> Cl | H | H | 0 | $-CO-R_3$ | 86-88 |
| 8.040 | F | Cl | $-O-CH_2=C\equiv CH$ | H | H | 0 | $-CO-R_3$ | 119-120 |
| 8.041 | F | Cl | $-O-CH-C\equiv CH$ <br> $\mid$ <br> $CH_3$ | H | H | 0 | $-CO-R_3$ | |
| 8.042 | F | Cl | 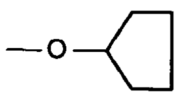 | H | H | 0 | $-CO-R_3$ | |
| 8.043 | F | Cl | 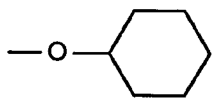 | H | H | 0 | $-CO-R_3$ | |
| 8.044 | F | Cl | 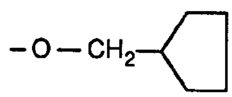 | H | H | 0 | $-CO-R_3$ | |
| 8.045 | F | Cl | 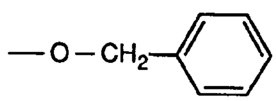 | H | H | 0 | $-CO-R_3$ | |
| 8.046 | F | Cl | 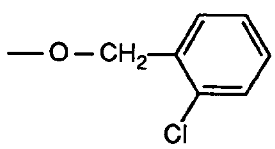 | H | 0 | | $-CO-R_3$ | |
| 8.047 | F | Cl | 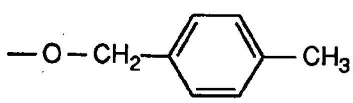 | H | H | 0 | $-CO-R_3$ | |
| 8.048 | F | Cl | $-S-CH_3$ | H | H | 0 | $-CO-R_3$ | |
| 8.049 | F | Cl | $-S-C_2H_5$ | H | H | 0 | $-CO-R_3$ | |
| 8.050 | F | Cl | $-S-C_3H_7$ | H | H | 0 | $-CO-R_3$ | |
| 8.051 | F | Cl | $-S-CH_2-CH=CH_2$ | H | H | 0 | $-CO-R_3$ | |
| 8.052 | F | Cl | $-S-CH_2-COOCH_3$ | H | H | 0 | $-CO-R_3$ | |
| 8.053 | F | Cl | $-S-CH_2-COOC_2H_5$ | H | H | 0 | $-CO-R_3$ | |
| 8.054 | F | Cl | $-S-CH_2-COOC_5H_{11}$ | H | H | 0 | $-CO-R_3$ | |
| 8.055 | F | Cl | $-S-CH(CH_3)-COOCH_3$ | H | H | 0 | $-CO-R_3$ | |
| 8.056 | F | Cl | $-S-(CH_3)-COOC_2H_5$ | H | H | 0 | $-CO-R_3$ | |

Tabelle 8 (Fortsetzung)

| Verb. Nr. | $R_1$ | $R_2$ | $R_3$ | $R_{14}$ | $R_4$ | n | A | Smp. [°C] |
|---|---|---|---|---|---|---|---|---|
| 8.057 | F | Cl | $-S-CH(CH_3)-COOC_3H_7$ | H | H | 0 | $-CO-R_3$ | |
| 8.058 | F | Cl | $-S-CH_2-CH_2-COOCH_3$ | H | H | 0 | $-CO-R_3$ | |
| 8.059 | F | Cl | $-S-CH_2-COOCH_2-CH_2-O-CH_3$ | H | H | 0 | $-CO-R_3$ | |
| 8.060 | F | Cl | $-O-CH_2-COOCH_3$ | H | H | 0 | $-CO-R_3$ | 140-141 |
| 8.061 | F | Cl | $-O-CH(CH_3)-COOCH_3$ | H | H | 0 | $-CO-R_3$ | 122-123 |
| 8.062 | F | Cl | $-O-CH_2-COOC_5H_{11}$ | H | H | 0 | $-CO-R_3$ | |
| 8.063 | F | Cl | $-O-CH_2-CH_3-Si(CH_3)_3$ | H | H | 0 | $-CO-R_3$ | |
| 8.064 | F | Cl | -ONa | H | H | 0 | $-CO-R_3$ | |
| 8.065 | F | Br | -Cl | H | H | 0 | $-CO-R_3$ | |
| 8.066 | F | Br | -OH | H | H | 0 | $-CO-R_3$ | |
| 8.067 | F | Br | $-OCH_3$ | H | H | 0 | $-CO-R_3$ | |
| 8.068 | F | Br | $-OC_2H_5$ | H | H | 0 | $-CO-R_3$ | |
| 8.069 | F | Br | $-OC_3H_7$ | H | H | 0 | $-CO-R_3$ | |
| 8.070 | F | Br | 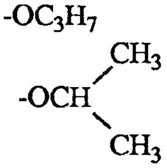 | H | H | 0 | $-CO-R_3$ | |
| 8.071 | F | Br | $-OC_4H_9$ | H | H | 0 | $-CO-R_3$ | |
| 8.072 | F | Br | $-OCH(CH_3)-CH_2-CH_3$ | H | H | 0 | $-CO-R_3$ | |
| 8.073 | F | Br | 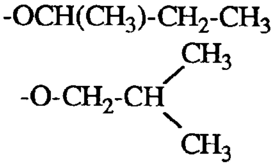 | H | H | 0 | $-CO-R_3$ | |
| 8.074 | F | Br | $-O-C_5H_{11}$ | H | H | 0 | $-CO-R_3$ | |
| 8.075 | F | Br | $-O-CH_2-CH_2-O-CH_3$ | H | H | 0 | $-CO-R_3$ | |
| 8.076 | F | Br | $-O-CH_2-CH_2-O-C_2H_5$ | H | H | 0 | $-CO-R_3$ | |
| 8.077 | F | Br | $-O-CH(CH_3)-CH_2-O-CH_3$ | H | H | 0 | $-CO-R_3$ | |
| 8.078 | F | Br | $-O-CH_2-CH_2-S-CH_3$ | H | H | 0 | $-CO-R_3$ | |
| 8.079 | F | Br | $-O-CH_2(CH_3)-S-CH_3$ | H | H | 0 | $-CO-R_3$ | |
| 8.080 | F | Br | $-O-CH-(CH_3)-S-C_2H_5$ | H | H | 0 | $-CO-R_3$ | |
| 8.081 | F | Br | $-O-CH(CH_3)-S-C_3H_7$ | H | H | 0 | $-CO-R_3$ | |
| 8.082 | F | Br | 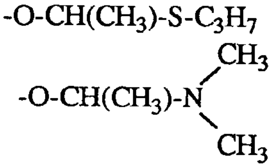 | H | H | 0 | $-CO-R_3$ | |
| 8.083 | F | Br | $-NH_2$ | H | H | 0 | $-CO-R_3$ | |

Tabelle 8 (Fortsetzung)

| Verb. Nr. | $R_1$ | $R_2$ | $R_3$ | $R_{14}$ | $R_4$ | n | A | Smp. [°C] |
|---|---|---|---|---|---|---|---|---|
| 8.084 | F | Br | 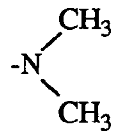 | H | H | 0 | $-CO-R_3$ | |
| 8.085 | F | Br | 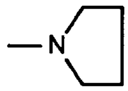 | H | H | 0 | $-CO-R_3$ | |
| 8.086 | F | Br | 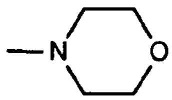 | H | H | 0 | $-CO-R_3$ | |
| 8.087 | F | Br | 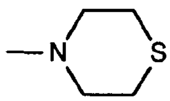 | H | H | 0 | $-CO-R_3$ | |
| 8.088 | F | Br | 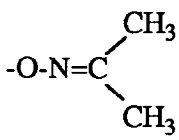 | H | H | 0 | $-CO-R_3$ | |
| 8.089 | F | Br | $-O-CH_2-CH_2-Cl$ | H | H | 0 | $-CO-R_3$ | |
| 8.090 | F | Br | $-O-CH_2-CN$ | H | H | 0 | $-CO-R_3$ | |
| 8.091 | F | Br | $-O-CH_2-CH=CH_2$ | H | H | 0 | $-CO-R_3$ | |
| 8.092 | F | Br | $-O-CH_2-C\equiv CH$ | H | H | 0 | $-CO-R_3$ | |
| 8.093 | F | Br | 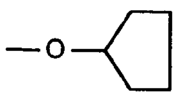 | H | H | 0 | $-CO-R_3$ | |
| 8.094 | F | Br | 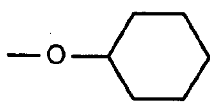 | H | H | 0 | $-CO-R_3$ | |
| 8.095 | F | Br | 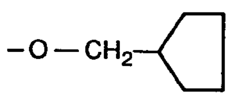 | H | H | 0 | $-CO-R_3$ | |
| 8.096 | F | Br | 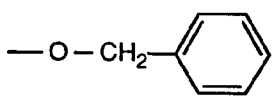 | H | H | 0 | $-CO-R_3$ | |
| 8.097 | F | Br | $-SCH_3$ | H | H | 0 | $-CO-R_3$ | |
| 8.098 | F | Br | $-S-CH_2-COOCH_3$ | H | H | 0 | $-CO-R_3$ | |
| 8.099 | F | Br | $-S-CH(CH_3)-COOCH_3$ | H | H | 0 | $-CO-R_3$ | |
| 8.100 | F | Br | $-O-CH_2-COOCH_3$ | H | H | 0 | $-CO-R_3$ | |

Tabelle 8 (Fortsetzung)

| Verb. Nr. | $R_1$ | $R_2$ | $R_3$ | $R_{14}$ | $R_4$ | n | A | Smp. [°C] |
|---|---|---|---|---|---|---|---|---|
| 8.101 | F | Br | $-O-CH(CH_3)COOCH_3$ | H | H | 0 | $-CO-R_3$ | |
| 8.102 | F | CN | -Cl | H | H | 0 | $-CO-R_3$ | |
| 8.103 | F | CN | -OH | H | H | 0 | $-CO-R_3$ | |
| 8.104 | F | CN | $-OCH_3$ | H | H | 0 | $-CO-R_3$ | |
| 8.105 | H | Cl | -Cl | H | H | 0 | $-CO-R_3$ | |
| 8.106 | H | Cl | -OH | H | H | 0 | $-CO-R_3$ | |
| 8.107 | H | Cl | $-OCH_3$ | H | 0 | $-CO-R_3$ | | |
| 8.108 | H | Cl | $-OC_2H_5$ | H | H | 0 | $-CO-R_3$ | |
| 8.109 | H | Cl | 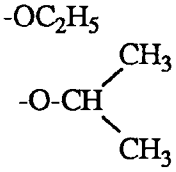 | H | H | 0 | $-CO-R_3$ | |
| 8.110 | H | Cl | $-O-CH_2-COOCH_3$ | H | H | 0 | $-CO-R_3$ | |
| 8.111 | H | Cl | $-O-CH(CH_3)COOCH_3$ | H | H | 0 | $-CO-R_3$ | |
| 8.112 | H | Cl | $-S-CH_2-COOCH_3$ | H | H | 0 | $-CO-R_3$ | |
| 8.113 | H | Cl | $-S-CH(CH_3)COOCH_3$ | H | H | 0 | $-CO-R_3$ | |
| 8.114 | H | Cl | 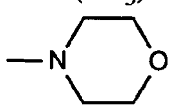 | H | H | 0 | $-CO-R_3$ | |
| 8.115 | F | Cl | -Cl | H | H | 1 | $-CO-R_3$ | |
| 8.116 | F | Cl | -OH | H | H | 1 | $-CO-R_3$ | |
| 8.117 | F | Cl | $-OCH_3$ | H | H | 1 | $-CO-R_3$ | $\eta_D^{22}$ 1.6010 |
| 8.118 | F | Cl | $-OC_2H_5$ | H | H | 1 | $-CO-R_3$ | |
| 8.119 | F | Cl | $-OC_3H_7$ | H | H | 1 | $-CO-R_3$ | |
| 8.120 | F | Cl | 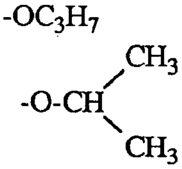 | H | H | 1 | $-CO-R_3$ | |
| 8.121 | F | Cl | $-OC_4H_9$ | H | H | 1 | $-CO-R_3$ | |
| 8.122 | F | Cl | $-O-CH(CH_3)-C_2H_5$ | H | H | 1 | $-CO-R_3$ | |
| 8.123 | F | Cl | 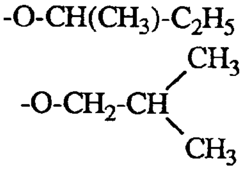 | H | H | 1 | $-CO-R_3$ | |
| 8.124 | F | Cl | $-OC_5H_{11}$ | H | H | 1 | $-CO-R_3$ | |
| 8.125 | F | Cl | $-O-CH_2-CH_2-O-CH_3$ | H | H | 1 | $-CO-R_3$ | |
| 8.126 | F | Cl | $-O-CH_2-CH_2-O-C_2H_5$ | H | H | 1 | $-CO-R_3$ | |

Tabelle 8 (Fortsetzung)

| Verb. Nr. | $R_1$ | $R_2$ | $R_3$ | $R_{14}$ | $R_4$ | n | A | Smp. [°C] |
|---|---|---|---|---|---|---|---|---|
| 8.127 | F | Cl | $-O-CH-(CH_3)-CH_2-O-CH_3$ | H | H | 1 | $-CO-R_3$ | |
| 8.128 | F | Cl | $-O-CH_2-CH_2-S-CH_3$ | H | H | 1 | $-CO-R_3$ | |
| 8.129 | F | Cl | $-O-CH(CH_3)-CH_2-S-CH_3$ | H | H | 1 | $-CO-R_3$ | |
| 8.130 | F | Cl | $-O-CH(CH_3)-CH_2-S-C_2H_5$ | H | H | 1 | $-CO-R_3$ | |
| 8.131 | F | Cl | $-O-CH(CH_3)-CH_2-S-C_3H_7$ | H | H | 1 | $-CO-R_3$ | |
| 8.132 | F | Cl | 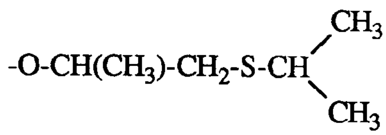 | H | H | 1 | $-CO-R_3$ | |
| 8.133 | F | Cl | $-O-CH(CH_3)-CH_2-S-C_4H_9$ | H | H | 1 | $-CO-R_3$ | |
| 8.134 | F | Cl | $-O-CH(CH_3)-CH_2-S-C_5H_{11}$ | H | H | 1 | $-CO-R_3$ | |
| 8.135 | F | Cl | 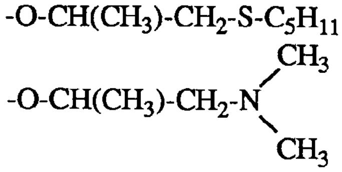 | H | H | 1 | $-CO-R_3$ | |
| 8.136 | F | Cl | 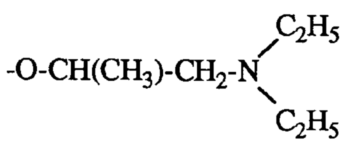 | H | H | 1 | $-CO-R_3$ | |
| 8.137 | F | Cl | $-NH_2$ | H | H | 1 | $-CO-R_3$ | |
| 8.138 | F | Cl | 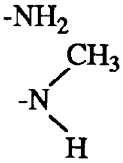 | H | H | 1 | $-CO-R_3$ | |
| 8.139 | F | Cl | 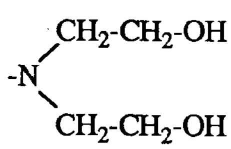 | H | H | 1 | $-CO-R_3$ | |
| 8.140 | F | Cl | $-NH-CH_2-CH=CH_2$ | H | H | 1 | $-CO-R_3$ | |
| 8.141 | F | Cl | $-N-(CH_2-CH=CH_2)_2$ | H | H | 1 | $-CO-R_3$ | |
| 8.142 | F | Cl | 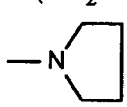 | H | H | 1 | $-CO-R_3$ | |
| 8.143 | F | Cl | 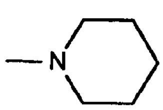 | H | H | 1 | $-CO-R_3$ | |

Tabelle 8 (Fortsetzung)

| Verb. Nr. | $R_1$ | $R_2$ | $R_3$ | $R_{14}$ | $R_4$ | n | A | Smp. [°C] |
|---|---|---|---|---|---|---|---|---|
| 8.144 | F | Cl | 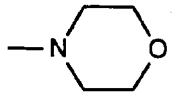 | H | H | 1 | $-CO-R_3$ | |
| 8.145 | F | Cl | 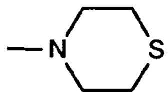 | H | H | 1 | $-CO-R_3$ | |
| 8.146 | F | Cl | 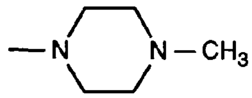 | H | H | 1 | $-CO-R_3$ | |
| 8.147 | F | Cl | 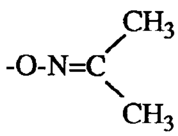 | H | H | 1 | $-CO-R_3$ | |
| 8.148 | F | Cl | $-O-CH_2-CH_2-Cl$ | H | H | 1 | $-CO-R_3$ | |
| 8.149 | F | Cl | $-O-CH_2-CN$ | H | H | 1 | $-CO-R_3$ | |
| 8.150 | F | Cl | 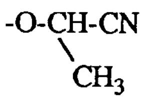 | H | H | 1 | $-CO-R_3$ | |
| 8.151 | F | Cl | $-O-CH_2-CH=CH_2$ | H | H | 1 | $-CO-R_3$ | |
| 8.152 | F | Cl | $-O-CH_2-CH=CHCl$ | H | H | 1 | $-CO-R_3$ | |
| 8.153 | F | Cl | 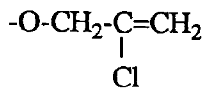 | H | H | 1 | $-CO-R_3$ | |
| 8.154 | F | Cl | $-O-CH_2=C\equiv CH$ | H | H | 1 | $-CO-R_3$ | |
| 8.155 | F | Cl | 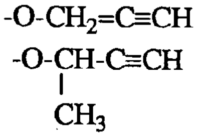 | H | H | 1 | $-CO-R_3$ | |
| 8.156 | F | Cl | 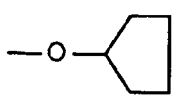 | H | H | 1 | $-CO-R_3$ | |
| 8.157 | F | Cl | 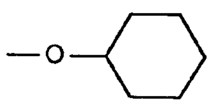 | H | H | 1 | $-CO-R_3$ | |
| 8.158 | F | Cl | 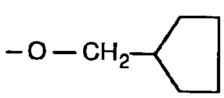 | H | H | 1 | $-CO-R_3$ | |
| 8.159 | F | Cl | 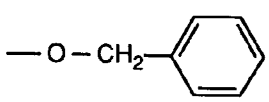 | H | H | 1 | $-CO-R_3$ | |

Tabelle 8 (Fortsetzung)

| Verb. Nr. | $R_1$ | $R_2$ | $R_3$ | $R_{14}$ | $R_4$ | n | A | Smp. [°C] |
|---|---|---|---|---|---|---|---|---|
| 8.160 | F | Cl | 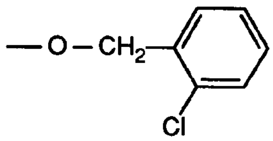 | H | H | 1 | $-CO-R_3$ | |
| 8.161 | F | Cl | 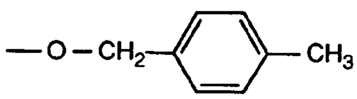 | H | H | 1 | $-CO-R_3$ | |
| 8.162 | F | Cl | $-S-CH_3$ | H | H | 1 | $-CO-R_3$ | |
| 8.163 | F | Cl | $-S-C_2H_5$ | H | H | 1 | $-CO-R_3$ | |
| 8.164 | F | Cl | $-S-C_3H_7$ | H | H | 1 | $-CO-R_3$ | |
| 8.165 | F | Cl | $-S-CH_2-CH=CH_2$ | H | H | 1 | $-CO-R_3$ | |
| 8.166 | F | Cl | $-S-CH_2-COOCH_3$ | H | H | 1 | $-CO-R_3$ | |
| 8.167 | F | Cl | $-S-CH_2-COOC_2H_5$ | H | H | 1 | $-CO-R_3$ | |
| 8.168 | F | Cl | $-S-CH_2-COOC_5H_{11}$ | H | H | 1 | $-CO-R_3$ | |
| 8.169 | F | Cl | $-S-CH(CH_3)-COOCH_3$ | H | H | 1 | $-CO-R_3$ | |
| 8.170 | F | Cl | $-S-(CH_3)-COOC_2H_5$ | H | H | 1 | $-CO-R_3$ | |
| 8.171 | F | Cl | $-S-CH(CH_3)-COOC_3H_7$ | H | H | 1 | $-CO-R_3$ | |
| 8.172 | F | Cl | $-S-CH_2-CH_2-COOCH_3$ | H | H | 1 | $-CO-R_3$ | |
| 8.173 | F | Cl | $-S-CH_2-COOCH_2-CH_2-O-CH_3$ | H | H | 1 | $-CO-R_3$ | |
| 8.174 | F | Cl | $-O-CH_2-COOCH_3$ | H | H | 1 | $-CO-R_3$ | |
| 8.175 | F | Cl | $-O-CH(CH_3)-COOCH_3$ | H | H | 1 | $-CO-R_3$ | |
| 8.176 | F | Cl | $-O-CH_2-COOC_5H_{11}$ | H | H | 1 | $-CO-R_3$ | |
| 8.177 | F | Cl | $-O-CH_2-CH_3-Si(CH_3)_3$ | H | H | 1 | $-CO-R_3$ | |
| 8.178 | F | Cl | -ONa | H | H | 1 | $-CO-R_3$ | |
| 8.179 | F | Br | -Cl | H | H | 1 | $-CO-R_3$ | |
| 8.180 | F | Br | -OH | H | H | 1 | $-CO-R_3$ | |
| 8.181 | F | Br | $-OCH_3$ | H | H | 1 | $-CO-R_3$ | |
| 8.182 | F | Br | $-OC_2H_5$ | H | H | 1 | $-CO-R_3$ | |
| 8.183 | F | Br | $-OC_3H_7$ | H | H | 1 | $-CO-R_3$ | |
| 8.184 | F | Br | 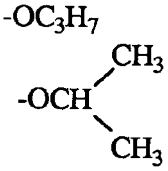 | H | H | 1 | $-CO-R_3$ | |
| 8.185 | F | Br | $-OC_4H_9$ | H | H | 1 | $-CO-R_3$ | |

Tabelle 8 (Fortsetzung)

| Verb. Nr. | $R_1$ | $R_2$ | $R_3$ | $R_{14}$ | $R_4$ | n | A | Smp. [°C] |
|---|---|---|---|---|---|---|---|---|
| 8.186 | F | Br | $-OCH(CH_3)-CH_2-CH_3$ | H | H | 1 | $-CO-R_3$ | |
| 8.187 | F | Br | 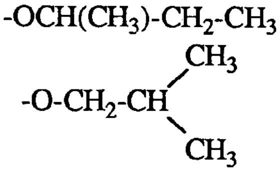 | H | H | 1 | $-CO-R_3$ | |
| 8.188 | F | Br | $-O-C_5H_{11}$ | H | H | 1 | $-CO-R_3$ | |
| 8.189 | F | Br | $-O-CH_2-CH_2-O-CH_3$ | H | H | 1 | $-CO-R_3$ | |
| 8.190 | F | Br | $-O-CH_2-CH_2-O-C_2H_5$ | H | H | 1 | $-CO-R_3$ | |
| 8.191 | F | Br | $-O-CH(CH_3)-CH_2-O-CH_3$ | H | H | 1 | $-CO-R_3$ | |
| 8.192 | F | Br | $-O-CH_2-CH_2-S-CH_3$ | H | H | 1 | $-CO-R_3$ | |
| 8.193 | F | Br | $-O-CH_2(CH_3)-S-CH_3$ | H | H | 1 | $-CO-R_3$ | |
| 8.194 | F | Br | $-O-CH-(CH_3)-S-C_2H_5$ | H | H | 1 | $-CO-R_3$ | |
| 8.195 | F | Br | $-O-CH(CH_3)-S-C_3H_7$ | H | H | 1 | $-CO-R_3$ | |
| 8,196 | F | Br | 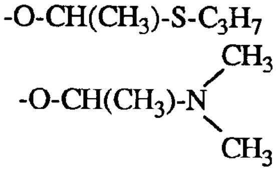 | H | H | 1 | $-CO-R_3$ | |
| 8.197 | F | Br | $-NH_2$ | H | H | 1 | $-CO-R_3$ | |
| 8.198 | F | Br | 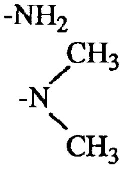 | H | H | 1 | $-CO-R_3$ | |
| 8.199 | F | Br | 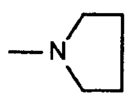 | H | H | 1 | $-CO-R_3$ | |
| 8.200 | F | Br | 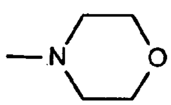 | H | H | 1 | $-CO-R_3$ | |
| 8.201 | F | Br | 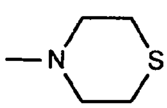 | H | H | 1 | $-CO-R_3$ | |
| 8.202 | F | Br | 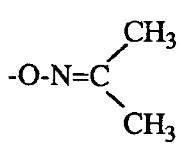 | H | H | 1 | $-CO-R_3$ | |
| 8.203 | F | Br | $-O-CH_2-CH_2-Cl$ | H | H | 1 | $-CO-R_3$ | |
| 8.204 | F | Br | $-O-CH_2-CN$ | H | H | 1 | $-CO-R_3$ | |
| 8.205 | F | Br | $-O-CH_2-CH=CH_2$ | H | H | 1 | $-CO-R_3$ | |
| 8.206 | F | Br | $-O-CH_2-C\equiv CH$ | H | H | 1 | $-CO-R_3$ | |

Tabelle 8 (Fortsetzung)

| Verb. Nr. | $R_1$ | $R_2$ | $R_3$ | $R_{14}$ | $R_4$ | n | A | Smp. [°C] |
|---|---|---|---|---|---|---|---|---|
| 8.207 | F | Br | 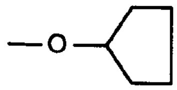 | H | H | 1 | $-CO-R_3$ | |
| 8.208 | F | Br | 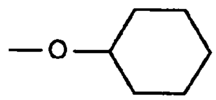 | H | H | 1 | $-CO-R_3$ | |
| 8.209 | F | Br | 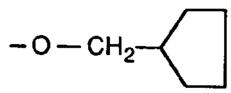 | H | H | 1 | $-CO-R_3$ | |
| 8.210 | F | Br | 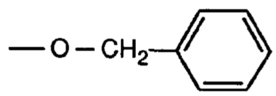 | H | H | 1 | $-CO-R_3$ | |
| 8.211 | F | Br | $-SCH_3$ | H | H | 1 | $-CO-R_3$ | |
| 8.212 | F | Br | $-S-CH_2-COOCH_3$ | H | H | 1 | $-CO-R_3$ | |
| 8.213 | F | Br | $-S-CH(CH_3)-COOCH_3$ | H | H | 1 | $-CO-R_3$ | |
| 8.214 | F | Br | $-O-CH_2-COOCH_3$ | H | H | 1 | $-CO-R_3$ | |
| 8.215 | F | Br | $-O-CH(CH_3)COOCH_3$ | H | H | 1 | $-CO-R_3$ | |
| 8.216 | F | CN | -Cl | H | H | 1 | $-CO-R_3$ | |
| 8.217 | F | CN | -OH | H | H | 1 | $-CO-R_3$ | |
| 8.218 | F | CN | $-OCH_3$ | H | H | 1 | $-CO-R_3$ | |
| 8.219 | H | Cl | -Cl | H | H | 1 | $-CO-R_3$ | |
| 8.220 | H | Cl | -OH | H | | 1 | $-CO-R_3$ | |
| 8.221 | H | Cl | $-OCH_3$ | H | H | 1 | $-CO-R_3$ | |
| 8.222 | H | Cl | $-OC_2H_5$ | H | H | 1 | $-CO-R_3$ | |
| 8.223 | H | Cl | 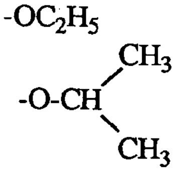 | H | H | 1 | $-CO-R_3$ | |
| 8.224 | H | Cl | $-O-CH_2-COOCH_3$ | H | H | 1 | $-CO-R_3$ | |
| 8.225 | H | Cl | $-O-CH(CH_3)COOCH_3$ | H | H | 1 | $-CO-R_3$ | |
| 8.226 | H | Cl | $-S-CH_2-COOCH_3$ | H | H | 1 | $-CO-R_3$ | |
| 8.227 | H | Cl | $-S-CH(CH_3)COOCH_3$ | H | H | 1 | $-CO-R_3$ | |
| 8.228 | H | Cl | 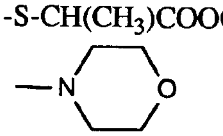 | H | H | 1 | $-CO-R_3$ | |
| 8.229 | F | Cl | Cl | H | H | 2 | $-CO-R_3$ | |

Tabelle 8 (Fortsetzung)

| Verb. Nr. | $R_1$ | $R_2$ | $R_3$ | $R_{14}$ | $R_4$ | n | A | Smp. [°C] |
|---|---|---|---|---|---|---|---|---|
| 8.230 | F | Cl | OH | H | H | 2 | $-CO-R_3$ | |
| 8.231 | F | Cl | $OCH_3$ | H | H | 2 | $-CO-R_3$ | |
| 8.232 | F | Cl | $-OC_2H_5$ | H | H | 2 | $-CO-R_3$ | |
| 8.233 | F | Cl | 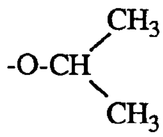 | H | H | 2 | $-CO-R_3$ | |
| 8.234 | F | Cl | $-O-CH_2-CH_2-O-CH_3$ | H | H | 2 | $-CO-R_3$ | |
| 8.235 | F | Cl | $-O-CH(CH_3)-CH_2-S-CH_3$ | H | H | 2 | $-CO-R_3$ | |
| 8.236 | F | Cl | $-O-CH_2-COOCH_3$ | H | H | 2 | $-CO-R_3$ | |
| 8.237 | F | Cl | $-S-CH_2-COOCH_3$ | H | H | 2 | $-CO-R_3$ | |
| 8.238 | F | Cl | $-CH_2-CH=CH_2$ | H | H | 2 | $-CO-R_3$ | |
| 8.239 | F | Cl | $-CH_2-C\equiv CH$ | H | H | 2 | $-CO-R_3$ | |
| 8.240 | F | Cl | Cl | H | H | 3 | $-CO-R_3$ | |
| 8.241 | F | Cl | OH | H | H | 3 | $-CO-R_3$ | |
| 8.242 | F | Cl | $OCH_3$ | H | H | 3 | $-CO-R_3$ | |
| 8.243 | F | Cl | $OC_2H_5$ | H | H | 3 | $-CO-R_3$ | |
| 8.244 | F | Cl | 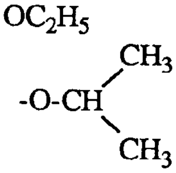 | H | H | 3 | $-CO-R_3$ | |
| 8.245 | F | Cl | $-O-CH_2-CH_2-O-CH$ | H | H | 3 | $-CO-R_3$ | |
| 8.246 | F | Cl | $-O-CH(CH_3)-CH_2-S-CH_3$ | H | H | 3 | $-CO-R_3$ | |
| 8.247 | F | Cl | $-O-CH_2-COOCH_3$ | H | H | 3 | $-CO-R_3$ | |
| 8.248 | F | Cl | $-S-CH_2-COOCH_3$ | H | H | 3 | $-CO-R_3$ | |
| 8.249 | F | Cl | $-O-CH_2-C\equiv CH$ | H | H | 3 | $-CO-R_3$ | |
| 8.250 | F | Cl | -Cl | H | Cl | 0 | $-CO-R_3$ | |
| 8.251 | F | Cl | -OH | H | Cl | 0 | $-CO-R_3$ | |
| 8.252 | F | Cl | $-OCH_3$ | H | Cl | 0 | $-CO-R_3$ | |
| 8.253 | F | Cl | $-OC_2H_5$ | H | Cl | 0 | $-CO-R_3$ | |
| 8.254 | F | Cl | 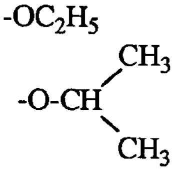 | H | Cl | 0 | $-CO-R_3$ | |
| 8.255 | F | Cl | $-O-CH_2-COOCH_3$ | H | Cl | 0 | $-CO-R_3$ | |
| 8.256 | F | Cl | $-S-CH_2-COOCH_3$ | H | Cl | 0 | $-CO-R_3$ | |
| 8.257 | F | Cl | $-OCH_3$ | H | Br | 0 | $-CO-R_3$ | |

Tabelle 8 (Fortsetzung)

| Verb. Nr. | $R_1$ | $R_2$ | $R_3$ | $R_{14}$ | $R_4$ | n | A | Smp. [°C] |
|---|---|---|---|---|---|---|---|---|
| 8.258 | F | Cl | 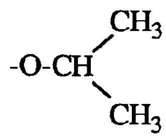 | H | Br | 0 | $-CO-R_3$ | |
| 8.259 | F | Cl | $-OCH_3$ | H | F | 0 | $-CO-R_3$ | |
| 8.260 | F | Cl | $-OCH_3$ | H | $CH_3$ | 0 | $-CO-R_3$ | |
| 8.261 | F | Cl | $-OC_2H_5$ | H | $CH_3$ | 0 | $-CO-R_3$ | |
| 8.262 | F | Cl | 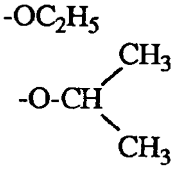 | H | $CH_3$ | 0 | | $-CO-R_3$ |
| 8.263 | F | Cl | $-O-CH_2-CH_2-O-CH_3$ | H | $CH_3$ | 0 | $-CO-R_3$ | |
| 8.264 | F | Cl | $-O-CH_2-COOCH_3$ | H | $CH_3$ | 0 | $-CO-R_3$ | |
| 8.265 | F | Cl | $-O-CH(CH_3)COOCH_3$ | H | $CH_3$ | 0 | $-CO-R_3$ | |
| 8.266 | F | Cl | $-S-CH_2-COOCH_3$ | H | $CH_3$ | 0 | $-CO-R_3$ | |
| 8.267 | F | Cl | $-OCH_3$ | H | $CF_3$ | 0 | $-CO-R_3$ | |
| 8.268 | F | Cl | $-OC_2H_5$ | H | $CF_3$ | 0 | $-CO-R_3$ | |
| 8.269 | F | Cl | 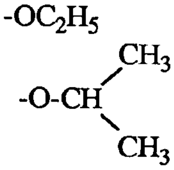 | H | $CF_3$ | 0 | $-CO-R_3$ | |
| 8.270 | F | Cl | $-O-CH_2-COOCH_3$ | H | $CF_3$ | 0 | $-CO-R_3$ | |
| 8.271 | F | Cl | $-S-CH_2-COOCH_3$ | H | $CF_3$ | 0 | $-CO-R_3$ | |
| 8.272 | F | Cl | - | H | H | 0 | -CN | |
| 8.273 | F | Cl | - | H | H | 1 | -CN | |
| 8.274 | F | Cl | - | H | H | 2 | -CN | |
| 8.275 | F | Cl | - | H | H | 3 | -CN | |
| 8.276 | F | Cl | - | H | H | 4 | -CN | |
| 8.277 | F | Cl | - | 2-Cl | 2-Cl | 0 | -CN | 152-153 |
| 8.278 | F | Cl | 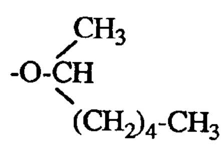 | H | H | 0 | $-CO-R_3$ | 120-121 |
| 8.279 | F | Cl | $-O-C_2H_5$ | H | H | 0 | $-CO-R_3$ | 94-96 |
| 8.280 | F | Cl | $-O-C_2H_5$ | H | H | 0 | $-CO-R_3$ | $\eta_D^{20}$ 1,6011 |

| | | | | | | | | |
|---|---|---|---|---|---|---|---|---|
| 8.281 | F | Cl | $-O-CH_2-CF_3$ | H | H | 0 | $-CO-R_3$ | 134-136 |
| 8.282 | F | Cl | 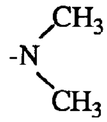 | H | H | 0 | $-CO-R_3$ | 98-100 |

Tabelle 9:

Verbindungen der Formel I, worin W für $W_9$, $Z_2$ für Sauerstoff und $R_{14}$ für Wasserstoff steht:

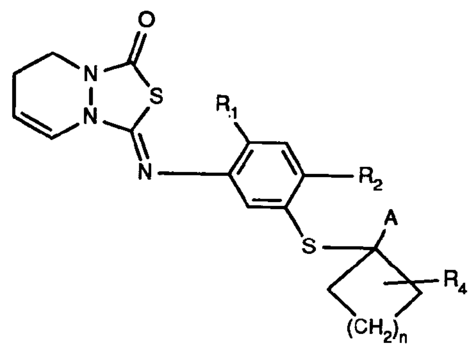

(I)

Tabelle 9

| Verb. Nr. | $R_1$ | $R_2$ | $R_3$ | $R_4$ | n | A | Smp. [°C] |
|---|---|---|---|---|---|---|---|
| 9.001 | F | Cl | -Cl | H | 0 | $-CO-R_3$ | |
| 9.002 | F | Cl | -OH | H | 0 | $-CO-R_3$ | |
| 9.003 | F | Cl | $-OCH_3$ | H | 0 | $-CO-R_3$ | |
| 9.004 | F | Cl | $-OC_2H_5$ | H | 0 | $-CO-R_3$ | |
| 9.005 | F | Cl | $-OC_3H_7$ | H | 0 | $-CO-R_3$ | |
| 9.006 | F | Cl | 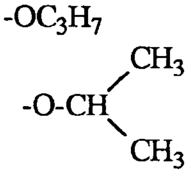 | H | 0 | $-CO-R_3$ | |
| 9.007 | F | Cl | $-OC_4H_9$ | H | 0 | $-CO-R_3$ | |
| 9.008 | F | Cl | $-O-CH(CH_3)-C_2H_5$ | H | 0 | $-CO-R_3$ | |
| 9.009 | F | Cl | 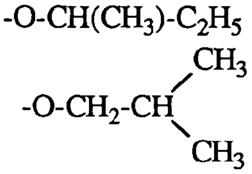 | H | 0 | $-CO-R_3$ | |
| 9.010 | F | Cl | $-OC_5H_{11}$ | H | 0 | $-CO-R_3$ | |
| 9.011 | F | Cl | $-O-CH_2-CH_2-O-CH_3$ | H | 0 | $-CO-R_3$ | |
| 9.012 | F | Cl | $-O-CH_2-CH_2-O-C_2H_5$ | H | 0 | $-CO-R_3$ | |
| 9.013 | F | Cl | $-O-CH-(CH_3)-CH_2-O-CH_3$ | H | 0 | $-CO-R_3$ | |
| 9.014 | F | Cl | $-O-CH_2-CH_2-S-CH_3$ | H | 0 | $-CO-R_3$ | |
| 9.015 | F | Cl | $-O-CH(CH_3)-CH_2-S-CH_3$ | H | 0 | $-CO-R_3$ | |
| 9.016 | F | Cl | $-O-CH(CH_3)-CH_2-S-C_2H_5$ | H | 0 | $-CO-R_3$ | |
| 9.017 | F | Cl | $-O-CH(CH_3)-CH_2-S-C_3H_7$ | H | 0 | $-CO-R_3$ | |
| 9.018 | F | Cl | 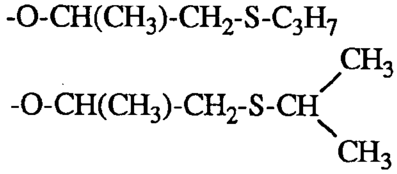 | H | 0 | $-CO-R_3$ | |
| 9.019 | F | Cl | $-O-CH(CH_3)-CH_2-S-C_4H_9$ | H | 0 | $-CO-R_3$ | |
| 9.020 | F | Cl | $-O-CH(CH_3)-CH_2-S-C_5H_{11}$ | H | 0 | $-CO-R_3$ | |
| 9.021 | F | Cl | 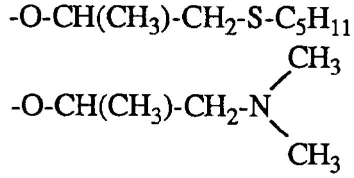 | H | 0 | $-CO-R_3$ | |
| 9.022 | F | Cl | 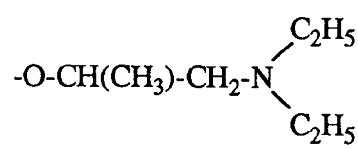 | H | 0 | $-CO-R_3$ | |

Tabelle 9 (Fortsetzung)

| Verb. Nr. | $R_1$ | $R_2$ | $R_3$ | $R_4$ | n | A | Smp. [°C] |
|---|---|---|---|---|---|---|---|
| 9.023 | F | Cl | $-NH_2$ | H | 0 | $-CO-R_3$ | |
| 9.024 | F | Cl | 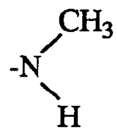 | H | 0 | $-CO-R_3$ | |
| 9.025 | F | Cl | 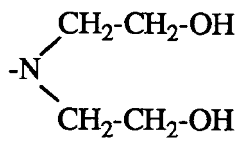 | H | 0 | $-CO-R_3$ | |
| 9.026 | F | Cl | $-NH-CH_2-CH=CH_2$ | H | 0 | $-CO-R_3$ | |
| 9.027 | F | Cl | $-N-(CH_2-CH=CH_2)_2$ | H | 0 | $-CO-R_3$ | |
| 9.028 | F | Cl | 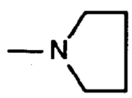 | H | 0 | $-CO-R_3$ | |
| 9.029 | F | Cl | 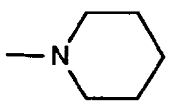 | H | 0 | $-CO-R_3$ | |
| 9.030 | F | Cl | 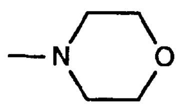 | H | 0 | $-CO-R_3$ | |
| 9.031 | F | Cl | 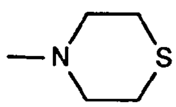 | H | 0 | $-CO-R_3$ | |
| 9.032 | F | Cl | 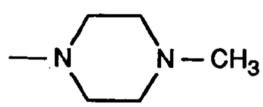 | H | 0 | $-CO-R_3$ | |
| 9.033 | F | Cl | 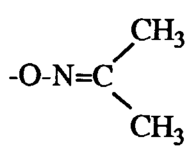 | H | 0 | $-CO-R_3$ | |
| 9.034 | F | Cl | $-O-CH_2-CH_2-Cl$ | H | 0 | $-CO-R_3$ | |
| 9.035 | F | Cl | $-O-CH_2-CN$ | H | 0 | $-CO-R_3$ | |
| 9.036 | F | Cl | 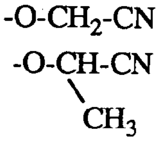 | H | 0 | $-CO-R_3$ | |
| 9.037 | F | Cl | $-O-CH_2-CH=CH_2$ | H | 0 | $-CO-R_3$ | |
| 9.038 | F | Cl | $-O-CH_2-CH=CHCl$ | H | 0 | $-CO-R_3$ | |

Tabelle 9 (Fortsetzung)

| Verb. Nr. | $R_1$ | $R_2$ | $R_3$ | $R_4$ | n | A | Smp. [°C] |
|---|---|---|---|---|---|---|---|
| 9.039 | F | Cl | $-O-CH_2-C=CH_2$<br>\|<br>Cl | H | 0 | $-CO-R_3$ | |
| 9.040 | F | Cl | $-O-CH_2=C\equiv CH$ | H | 0 | $-CO-R_3$ | |
| 9.041 | F | Cl | $-O-CH-C\equiv CH$<br>\|<br>$CH_3$ | H | 0 | $-CO-R_3$ | |
| 9.042 | F | Cl | 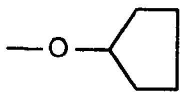 | H | 0 | $-CO-R_3$ | |
| 9.043 | F | Cl | 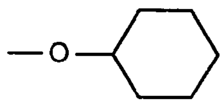 | H | 0 | $-CO-R_3$ | |
| 9.044 | F | Cl | 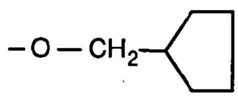 | H | 0 | $-CO-R_3$ | |
| 9.045 | F | Cl | 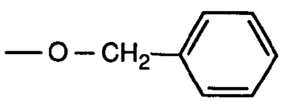 | H | 0 | $-CO-R_3$ | |
| 9.046 | F | Cl | 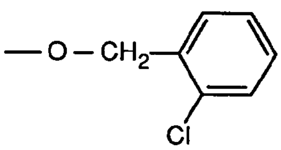 | H | 0 | $-CO-R_3$ | |
| 9.047 | F | Cl | 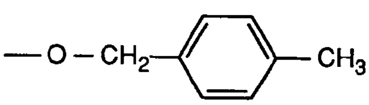 | H | 0 | $-CO-R_3$ | |
| 9.048 | F | Cl | $-S-CH_3$ | H | 0 | $-CO-R_3$ | |
| 9.049 | F | Cl | $-S-C_2H_5$ | H | 0 | $-CO-R_3$ | |
| 9.050 | F | Cl | $-S-C_3H_7$ | H | 0 | $-CO-R_3$ | |
| 9.051 | F | Cl | $-S-CH_2-CH=CH_2$ | H | 0 | $-CO-R_3$ | |
| 9.052 | F | Cl | $-S-CH_2-COOCH_3$ | H | 0 | $-CO-R_3$ | |
| 9.053 | F | Cl | $-S-CH_2-COOC_2H_5$ | H | 0 | $-CO-R_3$ | |
| 9.054 | F | Cl | $-S-CH_2-COOC_5H_{11}$ | H | 0 | $-CO-R_3$ | |
| 9.055 | F | Cl | $-S-CH(CH_3)-COOCH_3$ | H | 0 | $-CO-R_3$ | |
| 9.056 | F | Cl | $-S-(CH_3)-COOC_2H_5$ | H | 0 | $-CO-R_3$ | |
| 9.057 | F | Cl | $-S-CH(CH_3)-COOC_3H_7$ | H | 0 | $-CO-R_3$ | |
| 9.058 | F | Cl | $-S-CH_2-CH_2-COOCH_3$ | H | 0 | $-CO-R_3$ | |
| 9.059 | F | Cl | $-S-CH_2-COOCH_2-CH_2-O-CH_3$ | H | 0 | $-CO-R_3$ | |

Tabelle 9 (Fortsetzung)

| Verb. Nr. | $R_1$ | $R_2$ | $R_3$ | $R_4$ | n | A | Smp. [°C] |
|---|---|---|---|---|---|---|---|
| 9.060 | F | Cl | $-O-CH_2-COOCH_3$ | H | 0 | $-CO-R_3$ | |
| 9.061 | F | Cl | $-O-CH(CH_3)-COOCH_3$ | H | 0 | $-CO-R_3$ | |
| 9.062 | F | Cl | $-O-CH_2-COOC_5H_{11}$ | H | 0 | $-CO-R_3$ | |
| 9.063 | F | Cl | $-O-CH_2-CH_3-Si(CH_3)_3$ | H | 0 | $-CO-R_3$ | |
| 9.064 | F | Cl | -ONa | H | 0 | $-CO-R_3$ | |
| 9.065 | F | Br | -Cl | H | 0 | $-CO-R_3$ | |
| 9.066 | F | Br | -OH | H | 0 | $-CO-R_3$ | |
| 9.067 | F | Br | $-OCH_3$ | H | 0 | $-CO-R_3$ | |
| 9.068 | F | Br | $-OC_2H_5$ | H | 0 | $-CO-R_3$ | |
| 9.069 | F | Br | $-OC_3H_7$ | H | 0 | $-CO-R_3$ | |
| 9.070 | F | Br | 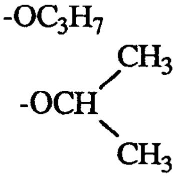 | H | 0 | $-CO-R_3$ | |
| 9.071 | F | Br | $-OC_4H_9$ | H | 0 | $-CO-R_3$ | |
| 9.072 | F | Br | $-OCH(CH_3)-CH_2-CH_3$ | H | 0 | $-CO-R_3$ | |
| 9.073 | F | Br | 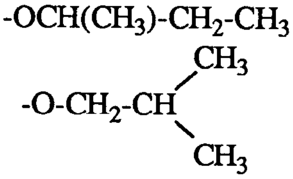 | H | 0 | $-CO-R_3$ | |
| 9.074 | F | Br | $-O-C_5H_{11}$ | H | 0 | $-CO-R_3$ | |
| 9.075 | F | Br | $-O-CH_2-CH_2-O-CH_3$ | H | 0 | $-CO-R_3$ | |
| 9.076 | F | Br | $-O-CH_2-CH_2-O-C_2H_5$ | H | 0 | $-CO-R_3$ | |
| 9.077 | F | Br | $-O-CH(CH_3)-CH_2-O-CH_3$ | H | 0 | $-CO-R_3$ | |
| 9.078 | F | Br | $-O-CH_2-CH_2-S-CH_3$ | H | 0 | $-CO-R_3$ | |
| 9.079 | F | Br | $-O-CH_2(CH_3)-S-CH_3$ | H | 0 | $-CO-R_3$ | |
| 9.080 | F | Br | $-O-CH-(CH_3)-S-C_2H_5$ | H | 0 | $-CO-R_3$ | |
| 9.081 | F | Br | $-O-CH(CH_3)-S-C_3H_7$ | H | 0 | $-CO-R_3$ | |
| 9.082 | F | Br | 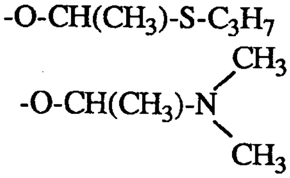 | H | 0 | $-CO-R_3$ | |
| 9.083 | F | Br | $-NH_2$ | H | 0 | $-CO-R_3$ | |

Tabelle 9 (Fortsetzung)

| Verb. Nr. | $R_1$ | $R_2$ | $R_3$ | $R_4$ | n | A | Smp. [°C] |
|---|---|---|---|---|---|---|---|
| 9.084 | F | Br | 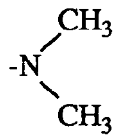 | H | 0 | $-CO-R_3$ | |
| 9.085 | F | Br | 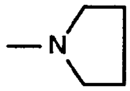 | H | 0 | $-CO-R_3$ | |
| 9.086 | F | Br | 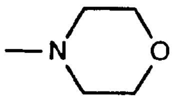 | H | 0 | $-CO-R_3$ | |
| 9.087 | F | Br | 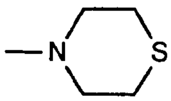 | H | 0 | $-CO-R_3$ | |
| 9.088 | F | Br | 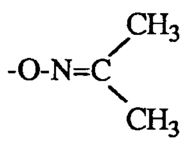 | H | 0 | $-CO-R_3$ | |
| 9.089 | F | Br | $-O-CH_2-CH_2-Cl$ | H | 0 | $-CO-R_3$ | |
| 9.090 | F | Br | $-O-CH_2-CN$ | H | 0 | $-CO-R_3$ | |
| 9.091 | F | Br | $-O-CH_2-CH=CH_2$ | H | 0 | $-CO-R_3$ | |
| 9.092 | F | Br | $-O-CH_2-C\equiv CH$ | H | 0 | $-CO-R_3$ | |
| 9.093 | F | Br | 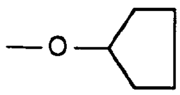 | H | 0 | $-CO-R_3$ | |
| 9.094 | F | Br | 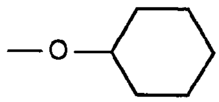 | H | 0 | $-CO-R_3$ | |
| 9.095 | F | Br | 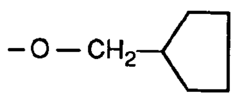 | H | 0 | $-CO-R_3$ | |
| 9.096 | F | Br | 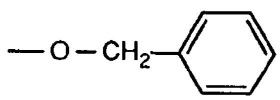 | H | 0 | $-CO-R_3$ | |
| 9.097 | F | Br | $-SCH_3$ | H | 0 | $-CO-R_3$ | |
| 9.098 | F | Br | $-S-CH_2-COOCH_3$ | H | 0 | $-CO-R_3$ | |
| 9.099 | F | Br | $-S-CH(CH_3)-COOCH_3$ | H | 0 | $-CO-R_3$ | |
| 9.100 | F | Br | $-O-CH_2-COOCH_3$ | H | 0 | $-CO-R_3$ | |
| 9.101 | F | Br | $-O-CH(CH_3)COOCH_3$ | H | 0 | $-CO-R_3$ | |
| 9.102 | F | CN | -Cl | H | 0 | $-CO-R_3$ | |

Tabelle 9 (Fortsetzung)

| Verb. Nr. | $R_1$ | $R_2$ | $R_3$ | $R_4$ | n | A | Smp. [°C] |
|---|---|---|---|---|---|---|---|
| 9.103 | F | CN | $-OH$ | H | 0 | $-CO-R_3$ | |
| 9.104 | F | CN | $-OCH_3$ | H | 0 | $-CO-R_3$ | |
| 9.105 | H | Cl | -Cl | H | 0 | $-CO-R_3$ | |
| 9.106 | H | Cl | $-OH$ | H | 0 | $-CO-R_3$ | |
| 9.107 | H | Cl | $-OCH_3$ | H | 0 | $-CO-R_3$ | |
| 9.108 | H | Cl | $-OC_2H_5$ | H | 0 | $-CO-R_3$ | |
| 9.109 | H | Cl | 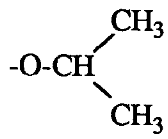 | H | 0 | $-CO-R_3$ | |
| 9.110 | H | Cl | $-O-CH_2-COOCH_3$ | H | 0 | $-CO-R_3$ | |
| 9.111 | H | Cl | $-O-CH(CH_3)COOCH_3$ | H | 0 | $-CO-R_3$ | |
| 9.112 | H | Cl | $-S-CH_2-COOCH_3$ | H | 0 | $-CO-R_3$ | |
| 9.113 | H | Cl | $-S-CH(CH_3)COOCH_3$ | H | 0 | $-CO-R_3$ | |
| 9.114 | H | Cl | 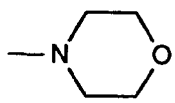 | H | 0 | $-CO-R_3$ | |
| 9.115 | F | Cl | -Cl | H | 1 | $-CO-R_3$ | |
| 9.116 | F | Cl | $-OH$ | H | 1 | $-CO-R_3$ | |
| 9.117 | F | Cl | $-OCH_3$ | H | 1 | $-CO-R_3$ | |
| 9.118 | F | Cl | $-OC_2H_5$ | H | 1 | $-CO-R_3$ | |
| 9.119 | F | Cl | $-OC_3H_7$ | H | 1 | $-CO-R_3$ | |
| 9.120 | F | Cl | 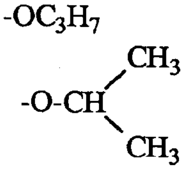 | H | 1 | $-CO-R_3$ | |
| 9.121 | F | Cl | $-OC_4H_9$ | H | 1 | $-CO-R_3$ | |
| 9.122 | F | Cl | $-O-CH(CH_3)-C_2H_5$ | H | 1 | $-CO-R_3$ | |
| 9.123 | F | Cl | 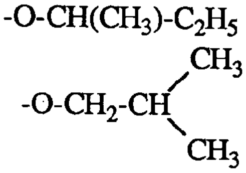 | H | 1 | $-CO-R_3$ | |
| 9.124 | F | Cl | $-OC_5H_{11}$ | H | 1 | $-CO-R_3$ | |
| 9.125 | F | Cl | $-O-CH_2-CH_2-O-CH_3$ | H | 1 | $-CO-R_3$ | |
| 9.126 | F | Cl | $-O-CH_2-CH_2-O-C_2H_5$ | H | 1 | $-CO-R_3$ | |

Tabelle 9 (Fortsetzung)

| Verb. Nr. | $R_1$ | $R_2$ | $R_3$ | $R_4$ | n | A | Smp. [°C] |
|---|---|---|---|---|---|---|---|
| 9.127 | F | Cl | $-O-CH-(CH_3)-CH_2-O-CH_3$ | H | 1 | $-CO-R_3$ | |
| 9.128 | F | Cl | $-O-CH_2-CH_2-S-CH_3$ | H | 1 | $-CO-R_3$ | |
| 9.129 | F | Cl | $-O-CH(CH_3)-CH_2-S-CH_3$ | H | 1 | $-CO-R_3$ | |
| 9.130 | F | Cl | $-O-CH(CH_3)-CH_2-S-C_2H_5$ | H | 1 | $-CO-R_3$ | |
| 9.131 | F | Cl | $-O-CH(CH_3)-CH_2-S-C_3H_7$ | H | 1 | $-CO-R_3$ | |
| 9.132 | F | Cl | 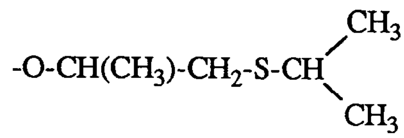 | H | 1 | $-CO-R_3$ | |
| 9.133 | F | Cl | $-O-CH(CH_3)-CH_2-S-C_4H_9$ | H | 1 | $-CO-R_3$ | |
| 9.134 | F | Cl | $-O-CH(CH_3)-CH_2-S-C_5H_{11}$ | H | 1 | $-CO-R_3$ | |
| 9.135 | F | Cl | 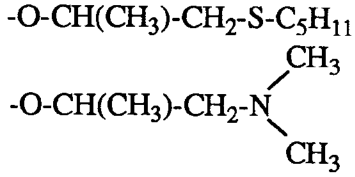 | H | 1 | $-CO-R_3$ | |
| 9.136 | F | Cl | 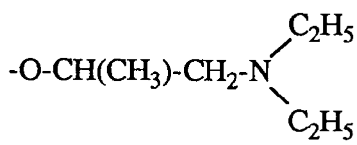 | H | 1 | $-CO-R_3$ | |
| 9.137 | F | Cl | $-NH_2$ | H | 1 | $-CO-R_3$ | |
| 9.138 | F | Cl | 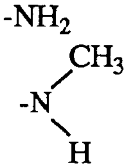 | H | 1 | $-CO-R_3$ | |
| 9.139 | F | Cl | 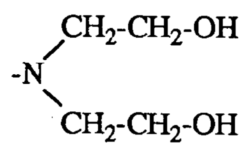 | H | 1 | $-CO-R_3$ | |
| 9.140 | F | Cl | $-NH-CH_2-CH=CH_2$ | H | 1 | $-CO-R_3$ | |
| 9.141 | F | Cl | $-N-(CH_2-CH=CH_2)_2$ | H | 1 | $-CO-R_3$ | |
| 9.142 | F | Cl | 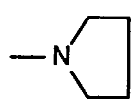 | H | 1 | $-CO-R_3$ | |
| 9.143 | F | Cl | 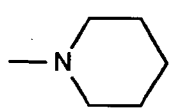 | H | 1 | $-CO-R_3$ | |
| 9.144 | F | Cl | 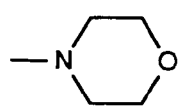 | H | 1 | $-CO-R_3$ | |

Tabelle 9 (Fortsetzung)

| Verb. Nr. | $R_1$ | $R_2$ | $R_3$ | $R_4$ | n | A | Smp. [°C] |
|---|---|---|---|---|---|---|---|
| 9.145 | F | Cl | 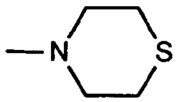 | H | 1 | $-CO-R_3$ | |
| 9.146 | F | Cl | 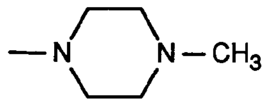 | H | 1 | $-CO-R_3$ | |
| 9.147 | F | Cl | 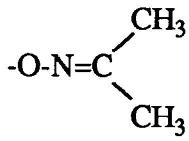 | H | 1 | $-CO-R_3$ | |
| 9.148 | F | Cl | $-O-CH_2-CH_2-Cl$ | H | 1 | $-CO-R_3$ | |
| 9.149 | F | Cl | $-O-CH_2-CN$ | H | 1 | $-CO-R_3$ | |
| 9.150 | F | Cl | 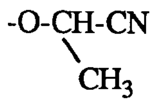 | H | 1 | $-CO-R_3$ | |
| 9.151 | F | Cl | $-O-CH_2-CH=CH_2$ | H | 1 | $-CO-R_3$ | |
| 9.152 | F | Cl | $-O-CH_2-CH=CHCl$ | H | 1 | $-CO-R_3$ | |
| 9.153 | F | Cl | 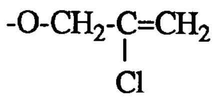 | H | 1 | $-CO-R_3$ | |
| 9.154 | F | Cl | $-O-CH_2=C\equiv CH$ | H | 1 | $-CO-R_3$ | |
| 9.155 | F | Cl | 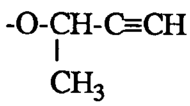 | H | 1 | $-CO-R_3$ | |
| 9.156 | F | Cl | 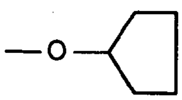 | H | 1 | $-CO-R_3$ | |
| 9.157 | F | Cl | 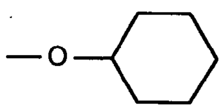 | H | 1 | $-CO-R_3$ | |
| 9.158 | F | Cl | 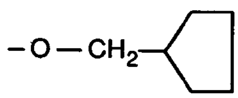 | H | 1 | $-CO-R_3$ | |
| 9.159 | F | Cl | 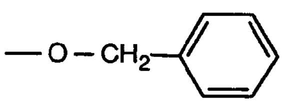 | H | 1 | $-CO-R_3$ | |

Tabelle 9 (Fortsetzung)

| Verb. Nr. | $R_1$ | $R_2$ | $R_3$ | $R_4$ | n | A | Smp. [°C] |
|---|---|---|---|---|---|---|---|
| 9.160 | F | Cl | 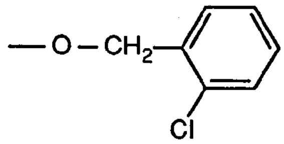 | H | 1 | $-CO-R_3$ | |
| 9.161 | F | Cl | 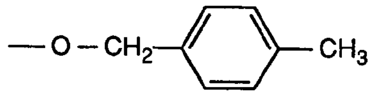 | H | 1 | $-CO-R_3$ | |
| 9.162 | F | Cl | $-S-CH_3$ | H | 1 | $-CO-R_3$ | |
| 9.163 | F | Cl | $-S-C_2H_5$ | H | 1 | $-CO-R_3$ | |
| 9.164 | F | Cl | $-S-C_3H_7$ | H | 1 | $-CO-R_3$ | |
| 9.165 | F | Cl | $-S-CH_2-CH=CH_2$ | H | 1 | $-CO-R_3$ | |
| 9.166 | F | Cl | $-S-CH_2-COOCH_3$ | H | 1 | $-CO-R_3$ | |
| 9.167 | F | Cl | $-S-CH_2-COOC_2H_5$ | H | 1 | $-CO-R_3$ | |
| 9.168 | F | Cl | $-S-CH_2-COOC_5H_{11}$ | H | 1 | $-CO-R_3$ | |
| 9.169 | F | Cl | $-S-CH(CH_3)-COOCH_3$ | H | 1 | $-CO-R_3$ | |
| 9.170 | F | Cl | $-S-(CH_3)-COOC_2H_5$ | H | 1 | $-CO-R_3$ | |
| 9.171 | F | Cl | $-S-CH(CH_3)-COOC_3H_7$ | H | 1 | $-CO-R_3$ | |
| 9.172 | F | Cl | $-S-CH_2-CH_2-COOCH_3$ | H | 1 | $-CO-R_3$ | |
| 9.173 | F | Cl | $-S-CH_2-COOCH_2-CH_2-O-CH_3$ | H | 1 | $-CO-R_3$ | |
| 9.174 | F | Cl | $-O-CH_2-COOCH_3$ | H | 1 | $-CO-R_3$ | |
| 9.175 | F | Cl | $-O-CH(CH_3)-COOCH_3$ | H | 1 | $-CO-R_3$ | |
| 9.176 | F | Cl | $-O-CH_2-COOC_5H_{11}$ | H | 1 | $-CO-R_3$ | |
| 9.177 | F | Cl | $-O-CH_2-CH_3-Si(CH_3)_3$ | H | 1 | $-CO-R_3$ | |
| 9.178 | F | Cl | -ONa | H | 1 | $-CO-R_3$ | |
| 9.179 | F | Br | -Cl | H | 1 | $-CO-R_3$ | |
| 9.180 | F | Br | -OH | H | 1 | $-CO-R_3$ | |
| 9.181 | F | Br | $-OCH_3$ | H | 1 | $-CO-R_3$ | |
| 9.182 | F | Br | $-OC_2H_5$ | H | 1 | $-CO-R_3$ | |
| 9.183 | F | Br | $-OC_3H_7$ | H | 1 | $-CO-R_3$ | |
| 9.184 | F | Br | 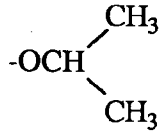 | H | 1 | $-CO-R_3$ | |
| 9.185 | F | Br | $-OC_4H_9$ | H | 1 | $-CO-R_3$ | |
| 9.186 | F | Br | $-OCH(CH_3)-CH_2-CH_3$ | H | 1 | $-CO-R_3$ | |

<u>Tabelle 9</u> (Fortsetzung)

| Verb. Nr. | $R_1$ | $R_2$ | $R_3$ | $R_4$ | n | A | Smp. [°C] |
|---|---|---|---|---|---|---|---|
| 9.187 | F | Br | 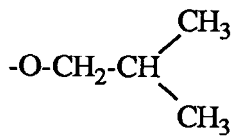 | H | 1 | $-CO-R_3$ | |
| 9.188 | F | Br | $-O-C_5H_{11}$ | H | 1 | $-CO-R_3$ | |
| 9.189 | F | Br | $-O-CH_2-CH_2-O-CH_3$ | H | 1 | $-CO-R_3$ | |
| 9.190 | F | Br | $-O-CH_2-CH_2-O-C_2H_5$ | H | 1 | $-CO-R_3$ | |
| 9.191 | F | Br | $-O-CH(CH_3)-CH_2-O-CH_3$ | H | 1 | $-CO-R_3$ | |
| 9.192 | F | Br | $-O-CH_2-CH_2-S-CH_3$ | H | 1 | $-CO-R_3$ | |
| 9.193 | F | Br | $-O-CH_2(CH_3)-S-CH_3$ | H | 1 | $-CO-R_3$ | |
| 9.194 | F | Br | $-O-CH-(CH_3)-S-C_2H_5$ | H | 1 | $-CO-R_3$ | |
| 9.195 | F | Br | $-O-CH(CH_3)-S-C_3H_7$ | H | 1 | $-CO-R_3$ | |
| 9.196 | F | Br | 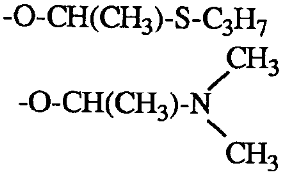 | H | 1 | $-CO-R_3$ | |
| 9.197 | F | Br | $-NH_2$ | H | 1 | $-CO-R_3$ | |
| 9.198 | F | Br | 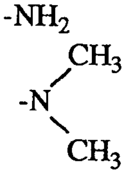 | H | 1 | $-CO-R_3$ | |
| 9.199 | F | Br | 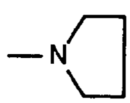 | H | 1 | $-CO-R_3$ | |
| 9.200 | F | Br | 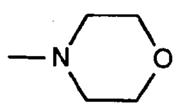 | H | 1 | $-CO-R_3$ | |
| 9.201 | F | Br | 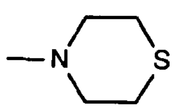 | H | 1 | $-CO-R_3$ | |
| 9.202 | F | Br | 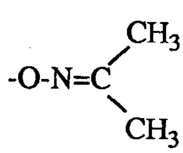 | H | 1 | $-CO-R_3$ | |
| 9.203 | F | Br | $-O-CH_2-CH_2-Cl$ | H | 1 | $-CO-R_3$ | |
| 9.204 | F | Br | $-O-CH_2-CN$ | H | 1 | $-CO-R_3$ | |
| 9.205 | F | Br | $-O-CH_2-CH=CH_2$ | H | 1 | $-CO-R_3$ | |

Tabelle 9 (Fortsetzung)

| Verb. Nr. | $R_1$ | $R_2$ | $R_3$ | $R_4$ | n | A | Smp. [°C] |
|---|---|---|---|---|---|---|---|
| 9.206 | F | Br | $-O-CH_2-C{\equiv}CH$ | H | 1 | $-CO-R_3$ | |
| 9.207 | F | Br | 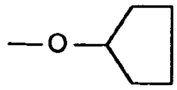 | H | 1 | $-CO-R_3$ | |
| 9.208 | F | Br | 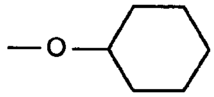 | H | 1 | $-CO-R_3$ | |
| 9.209 | F | Br | 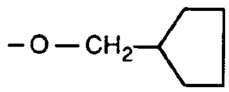 | H | 1 | $-CO-R_3$ | |
| 9.210 | F | Br | 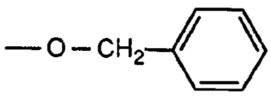 | H | 1 | $-CO-R_3$ | |
| 9.211 | F | Br | $-SCH_3$ | H | 1 | $-CO-R_3$ | |
| 9.212 | F | Br | $-S-CH_2-COOCH_3$ | H | 1 | $-CO-R_3$ | |
| 9.213 | F | Br | $-S-CH(CH_3)-COOCH_3$ | H | 1 | $-CO-R_3$ | |
| 9.214 | F | Br | $-O-CH_2-COOCH_3$ | H | 1 | $-CO-R_3$ | |
| 9.215 | F | Br | $-O-CH(CH_3)COOCH_3$ | H | 1 | $-CO-R_3$ | |
| 9.216 | F | CN | -Cl | H | 1 | $-CO-R_3$ | |
| 9.217 | F | CN | -OH | H | 1 | $-CO-R_3$ | |
| 9.218 | F | CN | $-OCH_3$ | H | 1 | $-CO-R_3$ | |
| 9.219 | H | Cl | -Cl | H | 1 | $-CO-R_3$ | |
| 9.220 | H | Cl | -OH | H | 1 | $-CO-R_3$ | |
| 9.221 | H | Cl | $-OCH_3$ | H | 1 | $-CO-R_3$ | |
| 9.222 | H | Cl | $-OC_2H_5$ | H | 1 | $-CO-R_3$ | |
| 9.223 | H | Cl | 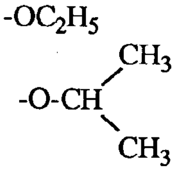 | H | 1 | $-CO-R_3$ | |
| 9.224 | H | Cl | $-O-CH_2-COOCH_3$ | H | 1 | $-CO-R_3$ | |
| 9.225 | H | Cl | $-O-CH(CH_3)COOCH_3$ | H | 1 | $-CO-R_3$ | |
| 9.226 | H | Cl | $-S-CH_2-COOCH_3$ | H | 1 | $-CO-R_3$ | |
| 9.227 | H | Cl | $-S-CH(CH_3)COOCH_3$ | H | 1 | $-CO-R_3$ | |
| 9.228 | H | Cl | 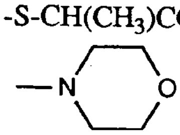 | H | 1 | $-CO-R_3$ | |
| 9.229 | F | Cl | Cl | H | 2 | $-CO-R_3$ | |
| 9.230 | F | Cl | OH | H | 2 | $-CO-R_3$ | |

Tabelle 9 (Fortsetzung)

| Verb. Nr. | $R_1$ | $R_2$ | $R_3$ | $R_4$ | n | A | Smp. [°C] |
|---|---|---|---|---|---|---|---|
| 9.231 | F | Cl | $OCH_3$ | H | 2 | $-CO-R_3$ | |
| 9.232 | F | Cl | $-OC_2H_5$ | H | 2 | $-CO-R_3$ | |
| 9.233 | F | Cl | 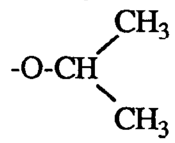 | H | 2 | $-CO-R_3$ | |
| 9.234 | F | Cl | $-O-CH_2-CH_2-O-CH_3$ | H | 2 | $-CO-R_3$ | |
| 9.235 | F | Cl | $-O-CH(CH_3)-CH_2-S-CH_3$ | H | 2 | $-CO-R_3$ | |
| 9.236 | F | Cl | $-O-CH_2-COOCH_3$ | H | 2 | $-CO-R_3$ | |
| 9.237 | F | Cl | $-S-CH_2-COOCH_3$ | H | 2 | $-CO-R_3$ | |
| 9.238 | F | Cl | $-CH_2-CH=CH_2$ | H | 2 | $-CO-R_3$ | |
| 9.239 | F | Cl | $-CH_2-C\equiv CH$ | H | 2 | $-CO-R_3$ | |
| 9.240 | F | Cl | Cl | H | 3 | $-CO-R_3$ | |
| 9.241 | F | Cl | OH | H | 3 | $-CO-R_3$ | |
| 9.242 | F | Cl | $OCH_3$ | H | 3 | $-CO-R_3$ | |
| 9.243 | F | Cl | $OC_2H_5$ | H | 3 | $-CO-R_3$ | |
| 9.244 | F | Cl | 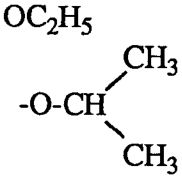 | H | 3 | $-CO-R_3$ | |
| 9.245 | F | Cl | $-O-CH_2-CH_2-O-CH$ | H | 3 | $-CO-R_3$ | |
| 9.246 | F | Cl | $-O-CH(CH_3)-CH_2-S-CH_3$ | H | 3 | $-CO-R_3$ | |
| 9.247 | F | Cl | $-O-CH_2-COOCH_3$ | H | 3 | $-CO-R_3$ | |
| 9.248 | F | Cl | $-S-CH_2-COOCH_3$ | H | 3 | $-CO-R_3$ | |
| 9.249 | F | Cl | $-O-CH_2-C\equiv CH$ | H | 3 | $-CO-R_3$ | |
| 9.250 | F | Cl | -Cl | Cl | 0 | $-CO-R_3$ | |
| 9.251 | F | Cl | -OH | Cl | 0 | $-CO-R_3$ | |
| 9.252 | F | Cl | $-OCH_3$ | Cl | 0 | $-CO-R_3$ | |
| 9.253 | F | Cl | $-OC_2H_5$ | Cl | 0 | $-CO-R_3$ | |
| 9.254 | F | Cl | 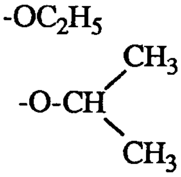 | Cl | 0 | $-CO-R_3$ | |
| 9.255 | F | Cl | $-O-CH_2-COOCH_3$ | Cl | 0 | $-CO-R_3$ | |
| 9.256 | F | Cl | $-S-CH_2-COOCH_3$ | Cl | 0 | $-CO-R_3$ | |

Tabelle 9 (Fortsetzung)

| Verb. Nr. | $R_1$ | $R_2$ | $R_3$ | $R_4$ | n | A | Smp. [°C] |
|---|---|---|---|---|---|---|---|
| 9.257 | F | Cl | $-OCH_3$ | Br | 0 | $-CO-R_3$ | |
| 9.258 | F | Cl | 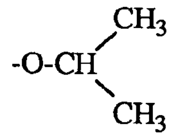 | Br | 0 | $-CO-R_3$ | |
| 9.259 | F | Cl | $-OCH_3$ | F | 0 | $-CO-R_3$ | |
| 9.260 | F | Cl | $-OCH_3$ | $CH_3$ | 0 | $-CO-R_3$ | |
| 9.261 | F | Cl | $-OC_2H_5$ | $CH_3$ | 0 | $-CO-R_3$ | |
| 9.262 | F | Cl | 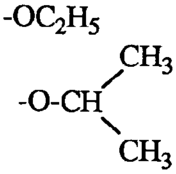 | $CH_3$ | 0 | $-CO-R_3$ | |
| 9.263 | F | Cl | $-O-CH_2-CH_2-O-CH_3$ | $CH_3$ | 0 | $-CO-R_3$ | |
| 9.264 | F | Cl | $-O-CH_2-COOCH_3$ | $CH_3$ | 0 | $-CO-R_3$ | |
| 9.265 | F | Cl | $-O-CH(CH_3)COOCH_3$ | $CH_3$ | 0 | $-CO-R_3$ | |
| 9.266 | F | Cl | $-S-CH_2-COOCH_3$ | $CH_3$ | 0 | $-CO-R_3$ | |
| 9.267 | F | Cl | $-OCH_3$ | $CF_3$ | 0 | $-CO-R_3$ | |
| 9.268 | F | Cl | $-OC_2H_5$ | $CF_3$ | 0 | $-CO-R_3$ | |
| 9.269 | F | Cl | 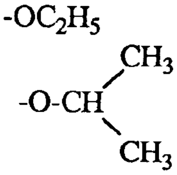 | $CF_3$ | 0 | $-CO-R_3$ | |
| 9.270 | F | Cl | $-O-CH_2-COOCH_3$ | $CF_3$ | 0 | $-CO-R_3$ | |
| 9.271 | F | Cl | $-S-CH_2-COOCH_3$ | $CF_3$ | 0 | $-CO-R_3$ | |
| 9.272 | F | Cl | - | H | 0 | -CN | |
| 9.273 | F | Cl | - | H | 1 | -CN | |
| 9.274 | F | Cl | - | H | 2 | -CN | |
| 9.275 | F | Cl | - | H | 3 | -CN | |
| 9.276 | F | Cl | - | H | 4 | -CN | |

Tabelle 10a:

Verbindungen der Formel I, worin W für $W_{10}$, $Y_1$ für Sauerstoff, $Y_2$ für Sauerstoff, $R_{14}$ für Wasserstoff und A für $-CO-R_3$ steht:

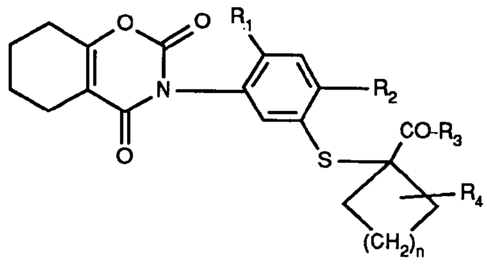

(I)

Tabelle 10a

| Verb. Nr. | $R_1$ | $R_2$ | $R_3$ | $R_4$ | n | Smp. [°C] |
|---|---|---|---|---|---|---|
| 10.001 | F | Cl | -Cl | H | 0 | |
| 10.002 | F | Cl | -OH | H | 0 | |
| 10.003 | F | Cl | $-OCH_3$ | H | 0 | |
| 10.004 | F | Cl | $-OC_2H_5$ | H | 0 | |
| 10.005 | F | Cl | $-OC_3H_7$ | H | 0 | |
| 10.006 | F | Cl | 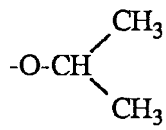 | H | 0 | |
| 10.007 | F | Cl | $-OC_4H_9$ | H | 0 | |
| 10.008 | F | Cl | $-O-CH(CH_3)-C_2H_5$ | H | 0 | |
| 10.009 | F | Cl | 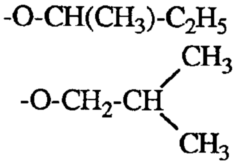 | H | 0 | |
| 10.010 | F | Cl | $-OC_5H_{11}$ | H | 0 | |
| 10.011 | F | Cl | $-O-CH_2-CH_2-O-CH_3$ | H | 0 | |
| 10.012 | F | Cl | $-O-CH_2-CH_2-O-C_2H_5$ | H | 0 | |
| 10.013 | F | Cl | $-O-CH-(CH_3)-CH_2-O-CH_3$ | H | 0 | |
| 10.014 | F | Cl | $-O-CH_2-CH_2-S-CH_3$ | H | 0 | |
| 10.015 | F | Cl | $-O-CH(CH_3)-CH_2-S-CH_3$ | H | 0 | |
| 10.016 | F | Cl | $-O-CH(CH_3)-CH_2-S-C_2H_5$ | H | 0 | |
| 10.017 | F | Cl | $-O-CH(CH_3)-CH_2-S-C_3H_7$ | H | 0 | |
| 10.018 | F | Cl | 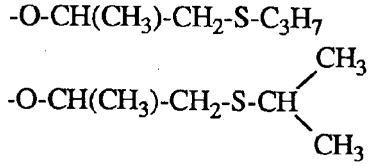 | H | 0 | |
| 10.019 | F | Cl | $-O-CH(CH_3)-CH_2-S-C_4H_9$ | H | 0 | |
| 10.020 | F | Cl | $-O-CH(CH_3)-CH_2-S-C_5H_{11}$ | H | 0 | |
| 10.021 | F | Cl | 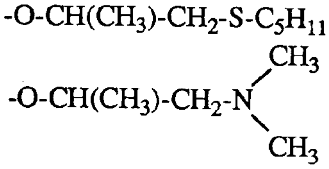 | H | 0 | |
| 10.022 | F | Cl | 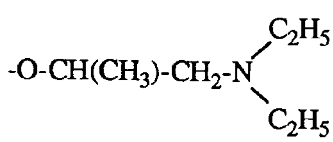 | H | 0 | |

Tabelle 10a (Fortsetzung)

| Verb. Nr. | $R_1$ | $R_2$ | $R_3$ | $R_4$ | n | Smp. [°C] |
|---|---|---|---|---|---|---|
| 10.023 | F | Cl | $-NH_2$ | H | 0 | |
| 10.024 | F | Cl | 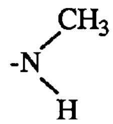 | H | 0 | |
| 10.025 | F | Cl | 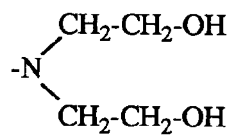 | H | 0 | |
| 10.026 | F | Cl | $-NH-CH_2-CH=CH_2$ | H | 0 | |
| 10.027 | F | Cl | $-N-(CH_2-CH=CH_2)_2$ | H | 0 | |
| 10.028 | F | Cl | 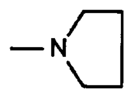 | H | 0 | |
| 10.029 | F | Cl | 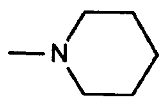 | H | 0 | |
| 10.030 | F | Cl | 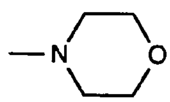 | H | 0 | |
| 10.031 | F | Cl | 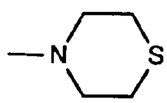 | H | 0 | |
| 10.032 | F | Cl | 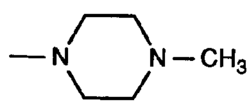 | H | 0 | |
| 10.033 | F | Cl | 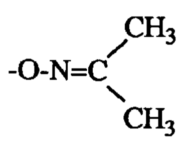 | H | 0 | |
| 10.034 | F | Cl | $-O-CH_2-CH_2-Cl$ | H | 0 | |
| 10.035 | F | Cl | $-O-CH_2-CN$ | H | 0 | |
| 10.036 | F | Cl | 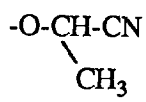 | H | 0 | |
| 10.037 | F | Cl | $-O-CH_2-CH=CH_2$ | H | 0 | |
| 10.038 | F | Cl | $-O-CH_2-CH=CHCl$ | H | 0 | |

<u>Tabelle 10a</u> (Fortsetzung)

| Verb. Nr. | $R_1$ | $R_2$ | $R_3$ | $R_4$ | n | Smp. [°C] |
|---|---|---|---|---|---|---|
| 10.039 | F | Cl | $-O-CH_2-C=CH_2$<br>\|<br>Cl | H | 0 | |
| 10.040 | F | Cl | $-O-CH_2=C\equiv CH$ | H | 0 | |
| 10.041 | F | Cl | $-O-CH-C\equiv CH$<br>\|<br>$CH_3$ | H | 0 | |
| 10.042 | F | Cl | 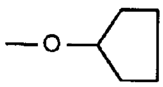 | H | 0 | |
| 10.043 | F | Cl | 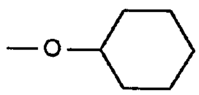 | H | 0 | |
| 10.044 | F | Cl | 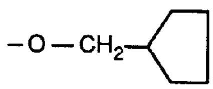 | H | 0 | |
| 10.045 | F | Cl | 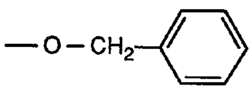 | H | 0 | |
| 10.046 | F | Cl | 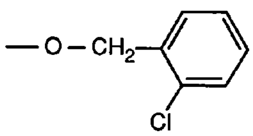 | H | 0 | |
| 10.047 | F | Cl | 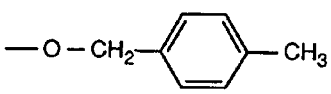 | H | 0 | |
| 10.048 | F | Cl | $-S-CH_3$ | H | 0 | |
| 10.049 | F | Cl | $-S-C_2H_5$ | H | 0 | |
| 10.050 | F | Cl | $-S-C_3H_7$ | H | 0 | |
| 10.051 | F | Cl | $-S-CH_2-CH=CH_2$ | H | 0 | |
| 10.052 | F | Cl | $-S-CH_2-COOCH_3$ | H | 0 | |
| 10.053 | F | Cl | $-S-CH_2-COOC_2H_5$ | H | 0 | |
| 10.054 | F | Cl | $-S-CH_2-COOC_5H_{11}$ | H | 0 | |
| 10.055 | F | Cl | $-S-CH(CH_3)-COOCH_3$ | H | 0 | |
| 10.056 | F | Cl | $-S-(CH_3)-COOC_2H_5$ | H | 0 | |
| 10.057 | F | Cl | $-S-CH(CH_3)-COOC_3H_7$ | H | 0 | |
| 10.058 | F | Cl | $-S-CH_2-CH_2-COOCH_3$ | H | 0 | |
| 10.059 | F | Cl | $-S-CH_2-COOCH_2-CH_2-O-CH_3$ | H | 0 | |

Tabelle 10a (Fortsetzung)

| Verb. Nr. | $R_1$ | $R_2$ | $R_3$ | $R_4$ | n | Smp. [°C] |
|---|---|---|---|---|---|---|
| 10.060 | F | Cl | $-O-CH_2-COOCH_3$ | H | 0 | |
| 10.061 | F | Cl | $-O-CH(CH_3)-COOCH_3$ | H | 0 | |
| 10.062 | F | Cl | $-O-CH_2-COOC_5H_{11}$ | H | 0 | |
| 10.063 | F | Cl | $-O-CH_2-CH_3-Si(CH_3)_3$ | H | 0 | |
| 10.064 | F | Cl | -ONa | H | 0 | |
| 10.065 | F | Br | -Cl | H | 0 | |
| 10.066 | F | Br | -OH | H | 0 | |
| 10.067 | F | Br | $-OCH_3$ | H | 0 | |
| 10.068 | F | Br | $-OC_2H_5$ | H | 0 | |
| 10.069 | F | Br | $-OC_3H_7$ | H | 0 | |
| 10.070 | F | Br | 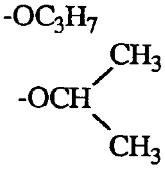 | H | 0 | |
| 10.071 | F | Br | $-OC_4H_9$ | H | 0 | |
| 10.072 | F | Br | $-OCH(CH_3)-CH_2-CH_3$ | H | 0 | |
| 10.073 | F | Br | 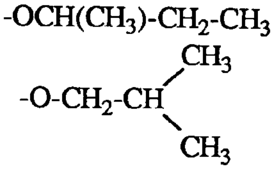 | H | 0 | |
| 10.074 | F | Br | $-O-C_5H_{11}$ | H | 0 | |
| 10.075 | F | Br | $-O-CH_2-CH_2-O-CH_3$ | H | 0 | |
| 10.076 | F | Br | $-O-CH_2-CH_2-O-C_2H_5$ | H | 0 | |
| 10.077 | F | Br | $-O-CH(CH_3)-CH_2-O-CH_3$ | H | 0 | |
| 10.078 | F | Br | $-O-CH_2-CH_2-S-CH_3$ | H | 0 | |
| 10.079 | F | Br | $-O-CH_2(CH_3)-S-CH_3$ | H | 0 | |
| 10.080 | F | Br | $-O-CH-(CH_3)-S-C_2H_5$ | H | 0 | |
| 10.081 | F | Br | $-O-CH(CH_3)-S-C_3H_7$ | H | 0 | |
| 10.082 | F | Br | 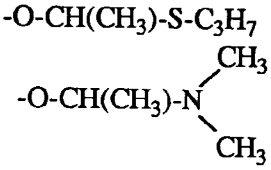 | H | 0 | |
| 10.083 | F | Br | $-NH_2$ | H | 0 | |

Tabelle 10a (Fortsetzung)

| Verb. Nr. | $R_1$ | $R_2$ | $R_3$ | $R_4$ | n | Smp. [°C] |
|---|---|---|---|---|---|---|
| 10.084 | F | Br | 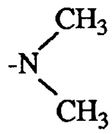 | H | 0 | |
| 10.085 | F | Br | 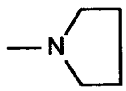 | H | 0 | |
| 10.086 | F | Br | 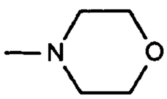 | H | 0 | |
| 10.087 | F | Br | 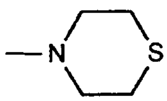 | H | 0 | |
| 10.088 | F | Br | 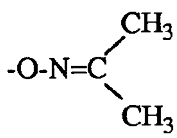 | H | 0 | |
| 10.089 | F | Br | $-O-CH_2-CH_2-Cl$ | H | 0 | |
| 10.090 | F | Br | $-O-CH_2-CN$ | H | 0 | |
| 10.091 | F | Br | $-O-CH_2-CH=CH_2$ | H | 0 | |
| 10.092 | F | Br | $-O-CH_2-C\equiv CH$ | H | 0 | |
| 10.093 | F | Br | 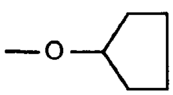 | H | 0 | |
| 10.094 | F | Br | 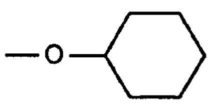 | H | 0 | |
| 10.095 | F | Br | 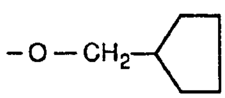 | H | 0 | |
| 10.096 | F | Br | 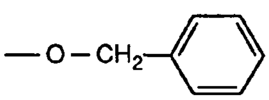 | H | 0 | |
| 10.097 | F | Br | $-SCH_3$ | H | 0 | |
| 10.098 | F | Br | $-S-CH_2-COOCH_3$ | H | 0 | |
| 10.099 | F | Br | $-S-CH(CH_3)-COOCH_3$ | H | 0 | |
| 10.100 | F | Br | $-O-CH_2-COOCH_3$ | H | 0 | |
| 10.101 | F | Br | $-O-CH(CH_3)COOCH_3$ | H | 0 | |
| 10.102 | F | CN | -Cl | H | 0 | |

Tabelle 10a (Fortsetzung)

| Verb. Nr. | $R_1$ | $R_2$ | $R_3$ | $R_4$ | n | Smp. [°C] |
|---|---|---|---|---|---|---|
| 10.103 | F | CN | $-OH$ | H | 0 | |
| 10.104 | F | CN | $-OCH_3$ | H | 0 | |
| 10.105 | H | Cl | -Cl | H | 0 | |
| 10.106 | H | Cl | $-OH$ | H | 0 | |
| 10.107 | H | Cl | $-OCH_3$ | H | 0 | |
| 10.108 | H | Cl | $-OC_2H_5$ | H | 0 | |
| 10.109 | H | Cl | 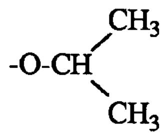 | H | 0 | |
| 10.110 | H | Cl | $-O-CH_2-COOCH_3$ | H | 0 | |
| 10.111 | H | Cl | $-O-CH(CH_3)COOCH_3$ | H | 0 | |
| 10.112 | H | Cl | $-S-CH_2-COOCH_3$ | H | 0 | |
| 10.113 | H | Cl | $-S-CH(CH_3)COOCH_3$ | H | 0 | |
| 10.114 | H | Cl | 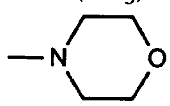 | H | 0 | |
| 10.115 | F | Cl | -Cl | H | 1 | |
| 10.116 | F | Cl | $-OH$ | H | 1 | |
| 10.117 | F | Cl | $-OCH_3$ | H | 1 | 105-107 |
| 10.118 | F | Cl | $-OC_2H_5$ | H | 1 | |
| 10.119 | F | Cl | $-OC_3H_7$ | H | 1 | |
| 10.120 | F | Cl | 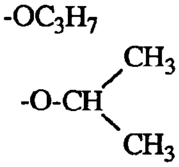 | H | 1 | |
| 10.121 | F | Cl | $-OC_4H_9$ | H | 1 | |
| 10.122 | F | Cl | $-O-CH(CH_3)-C_2H_5$ | H | 1 | |
| 10.123 | F | Cl | 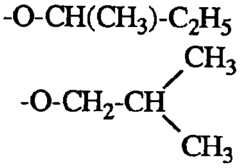 | H | 1 | |
| 10.124 | F | Cl | $-OC_5H_{11}$ | H | 1 | |
| 10.125 | F | Cl | $-O-CH_2-CH_2-O-CH_3$ | H | 1 | |
| 10.126 | F | Cl | $-O-CH_2-CH_2-O-C_2H_5$ | H | 1 | |

Tabelle 10a (Fortsetzung)

| Verb. Nr. | $R_1$ | $R_2$ | $R_3$ | $R_4$ | n | Smp. [°C] |
|---|---|---|---|---|---|---|
| 10.127 | F | Cl | $-O-CH-(CH_3)-CH_2-O-CH_3$ | H | 1 | |
| 10.128 | F | Cl | $-O-CH_2-CH_2-S-CH_3$ | H | 1 | |
| 10.129 | F | Cl | $-O-CH(CH_3)-CH_2-S-CH_3$ | H | 1 | |
| 10.130 | F | Cl | $-O-CH(CH_3)-CH_2-S-C_2H_5$ | H | 1 | |
| 10.131 | F | Cl | $-O-CH(CH_3)-CH_2-S-C_3H_7$ | H | 1 | |
| 10.132 | F | Cl | 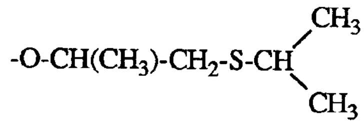 | H | 1 | |
| 10.133 | F | Cl | $-O-CH(CH_3)-CH_2-S-C_4H_9$ | H | 1 | |
| 10.134 | F | Cl | $-O-CH(CH_3)-CH_2-S-C_5H_{11}$ | H | 1 | |
| 10.135 | F | Cl | 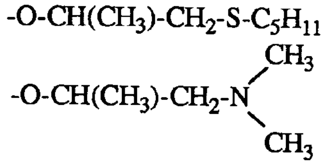 | H | 1 | |
| 10.136 | F | Cl | 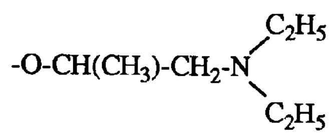 | H | 1 | |
| 10.137 | F | Cl | $-NH_2$ | H | 1 | |
| 10.138 | F | Cl | 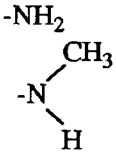 | H | 1 | |
| 10.139 | F | Cl | 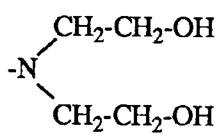 | H | 1 | |
| 10.140 | F | Cl | $-NH-CH_2-CH=CH_2$ | H | 1 | |
| 10.141 | F | Cl | $-N-(CH_2-CH=CH_2)_2$ | H | 1 | |
| 10.142 | F | Cl | 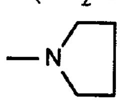 | H | 1 | |
| 10.143 | F | Cl | 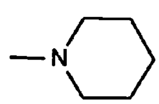 | H | 1 | |
| 10.144 | F | Cl | 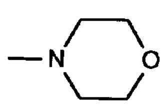 | H | 1 | |

<u>Tabelle 10a</u> (Fortsetzung)

| Verb. Nr. | $R_1$ | $R_2$ | $R_3$ | $R_4$ | n | Smp. [°C] |
|---|---|---|---|---|---|---|
| 10.145 | F | Cl | 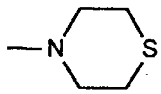 | H | 1 | |
| 10.146 | F | Cl | 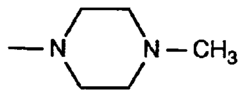 | H | 1 | |
| 10.147 | F | Cl | 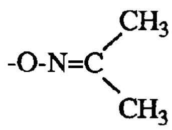 | H | 1 | |
| 10.148 | F | Cl | $-O-CH_2-CH_2-Cl$ | H | 1 | |
| 10.149 | F | Cl | $-O-CH_2-CN$ | H | 1 | |
| 10.150 | F | Cl | 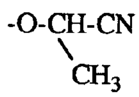 | H | 1 | |
| 10.151 | F | Cl | $-O-CH_2-CH=CH_2$ | H | 1 | |
| 10.152 | F | Cl | $-O-CH_2-CH=CHCl$ | H | 1 | |
| 10.153 | F | Cl | 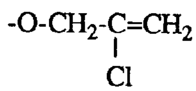 | H | 1 | |
| 10.154 | F | Cl | $-O-CH_2=C\equiv CH$ | H | 1 | |
| 10.155 | F | Cl | 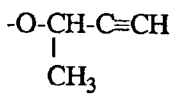 | H | 1 | |
| 10.156 | F | Cl | 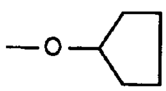 | H | 1 | |
| 10.157 | F | Cl | 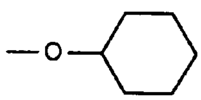 | H | 1 | |
| 10.158 | F | Cl | 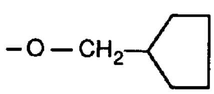 | H | 1 | |
| 10.159 | F | Cl | 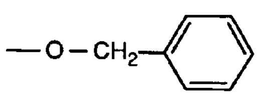 | H | 1 | |

Tabelle 10a (Fortsetzung)

| Verb. Nr. | $R_1$ | $R_2$ | $R_3$ | $R_4$ | n | Smp. [°C] |
|---|---|---|---|---|---|---|
| 10.160 | F | Cl | 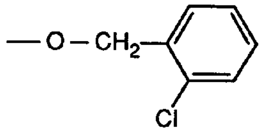 | H | 1 | |
| 10.161 | F | Cl | 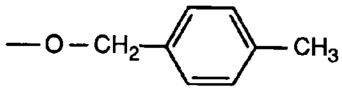 | H | 1 | |
| 10.162 | F | Cl | $-S-CH_3$ | H | 1 | |
| 10.163 | F | Cl | $-S-C_2H_5$ | H | 1 | |
| 10.164 | F | Cl | $-S-C_3H_7$ | H | 1 | |
| 10.165 | F | Cl | $-S-CH_2-CH=CH_2$ | H | 1 | |
| 10.166 | F | Cl | $-S-CH_2-COOCH_3$ | H | 1 | |
| 10.167 | F | Cl | $-S-CH_2-COOC_2H_5$ | H | 1 | |
| 10.168 | F | Cl | $-S-CH_2-COOC_5H_{11}$ | H | 1 | |
| 10.169 | F | Cl | $-S-CH(CH_3)-COOCH_3$ | H | 1 | |
| 10.170 | F | Cl | $-S-(CH_3)-COOC_2H_5$ | H | 1 | |
| 10.171 | F | Cl | $-S-CH(CH_3)-COOC_3H_7$ | H | 1 | |
| 10.172 | F | Cl | $-S-CH_2-CH_2-COOCH_3$ | H | 1 | |
| 10.173 | F | Cl | $-S-CH_2-COOCH_2-CH_2-O-CH_3$ | H | 1 | |
| 10.174 | F | Cl | $-O-CH_2-COOCH_3$ | H | 1 | |
| 10.175 | F | Cl | $-O-CH(CH_3)-COOCH_3$ | H | 1 | |
| 10.176 | F | Cl | $-O-CH_2-COOC_5H_{11}$ | H | 1 | |
| 10.177 | F | Cl | $-O-CH_2-CH_3-Si(CH_3)_3$ | H | 1 | |
| 10.178 | F | Cl | -ONa | H | 1 | |
| 10.179 | F | Br | -Cl | H | 1 | |
| 10.180 | F | Br | -OH | H | 1 | |
| 10.181 | F | Br | $-OCH_3$ | H | 1 | |
| 10.182 | F | Br | $-OC_2H_5$ | H | 1 | |
| 10.183 | F | Br | $-OC_3H_7$ | H | 1 | |
| 10.184 | F | Br | 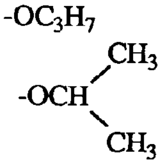 | H | 1 | |
| 10.185 | F | Br | $-OC_4H_9$ | H | 1 | |
| 10.186 | F | Br | $-OCH(CH_3)-CH_2-CH_3$ | H | 1 | |

Tabelle 10a (Fortsetzung)

| Verb. Nr. | $R_1$ | $R_2$ | $R_3$ | $R_4$ | n | Smp. [°C] |
|---|---|---|---|---|---|---|
| 10.187 | F | Br | 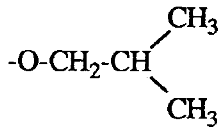 | H | 1 | |
| 10.188 | F | Br | $-O-C_5H_{11}$ | H | 1 | |
| 10.189 | F | Br | $-O-CH_2-CH_2-O-CH_3$ | H | 1 | |
| 10.190 | F | Br | $-O-CH_2-CH_2-O-C_2H_5$ | H | 1 | |
| 10.191 | F | Br | $-O-CH(CH_3)-CH_2-O-CH_3$ | H | 1 | |
| 10.192 | F | Br | $-O-CH_2-CH_2-S-CH_3$ | H | 1 | |
| 10.193 | F | Br | $-O-CH_2(CH_3)-S-CH_3$ | H | 1 | |
| 10.194 | F | Br | $-O-CH-(CH_3)-S-C_2H_5$ | H | 1 | |
| 10.195 | F | Br | $-O-CH(CH_3)-S-C_3H_7$ | H | 1 | |
| 10.196 | F | Br | 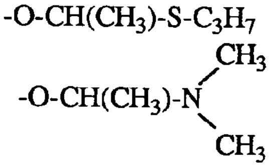 | H | 1 | |
| 10.197 | F | Br | $-NH_2$ | H | 1 | |
| 10.198 | F | Br | 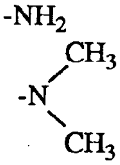 | H | 1 | |
| 10.199 | F | Br | 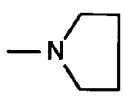 | H | 1 | |
| 10.200 | F | Br | 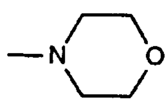 | H | 1 | |
| 10.201 | F | Br | 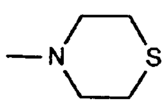 | H | 1 | |
| 10.202 | F | Br | 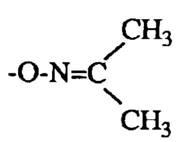 | H | 1 | |
| 10.203 | F | Br | $-O-CH_2-CH_2-Cl$ | H | 1 | |
| 10.204 | F | Br | $-O-CH_2-CN$ | H | 1 | |
| 10.205 | F | Br | $-O-CH_2-CH=CH_2$ | H | 1 | |

Tabelle 10a (Fortsetzung)

| Verb. Nr. | $R_1$ | $R_2$ | $R_3$ | $R_4$ | n | Smp. [°C] |
|---|---|---|---|---|---|---|
| 10.206 | F | Br | $-O-CH_2-C{\equiv}CH$ | H | 1 | |
| 10.207 | F | Br | 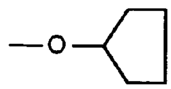 | H | 1 | |
| 10.208 | F | Br | 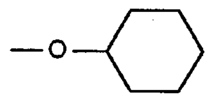 | H | 1 | |
| 10.209 | F | Br | 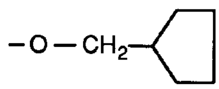 | H | 1 | |
| 10.210 | F | Br | 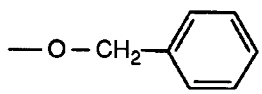 | H | 1 | |
| 10.211 | F | Br | $-SCH_3$ | H | 1 | |
| 10.212 | F | Br | $-S-CH_2-COOCH_3$ | H | 1 | |
| 10.213 | F | Br | $-S-CH(CH_3)-COOCH_3$ | H | 1 | |
| 10.214 | F | Br | $-O-CH_2-COOCH_3$ | H | 1 | |
| 10.215 | F | Br | $-O-CH(CH_3)COOCH_3$ | H | 1 | |
| 10.216 | F | CN | -Cl | H | 1 | |
| 10.217 | F | CN | -OH | H | 1 | |
| 10.218 | F | CN | $-OCH_3$ | H | 1 | |
| 10.219 | H | Cl | -Cl | H | 1 | |
| 10.220 | H | Cl | -OH | H | 1 | |
| 10.221 | H | Cl | $-OCH_3$ | H | 1 | |
| 10.222 | H | Cl | $-OC_2H_5$ | H | 1 | |
| 10.223 | H | Cl | 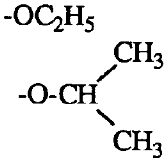 | H | 1 | |
| 10.224 | H | Cl | $-O-CH_2-COOCH_3$ | H | 1 | |
| 10.225 | H | Cl | $-O-CH(CH_3)COOCH_3$ | H | 1 | |
| 10.226 | H | Cl | $-S-CH_2-COOCH_3$ | H | 1 | |
| 10.227 | H | Cl | $-S-CH(CH_3)COOCH_3$ | H | 1 | |
| 10.228 | H | Cl | 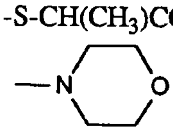 | H | 1 | |
| 10.229 | F | Cl | Cl | H | 2 | |
| 10.230 | F | Cl | OH | H | 2 | |

Tabelle 10a (Fortsetzung)

| Verb. Nr. | $R_1$ | $R_2$ | $R_3$ | $R_4$ | n | Smp. [°C] |
|---|---|---|---|---|---|---|
| 10.231 | F | Cl | $OCH_3$ | H | 2 | |
| 10.232 | F | Cl | $-OC_2H_5$ | H | 2 | |
| 10.233 | F | Cl | 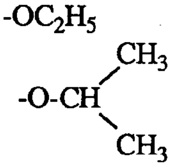 | H | 2 | |
| 10.234 | F | Cl | $-O-CH_2-CH_2-O-CH_3$ | H | 2 | |
| 10.235 | F | Cl | $-O-CH(CH_3)-CH_2-S-CH_3$ | H | 2 | |
| 10.236 | F | Cl | $-O-CH_2-COOCH_3$ | H | 2 | |
| 10.237 | F | Cl | $-S-CH_2-COOCH_3$ | H | 2 | |
| 10.238 | F | Cl | $-CH_2-CH=CH_2$ | H | 2 | |
| 10.239 | F | Cl | $-CH_2-C\equiv CH$ | H | 2 | |
| 10.240 | F | Cl | Cl | H | 3 | |
| 10.241 | F | Cl | OH | H | 3 | |
| 10.242 | F | Cl | $OCH_3$ | H | 3 | |
| 10.243 | F | Cl | $OC_2H_5$ | H | 3 | |
| 10.244 | F | Cl | 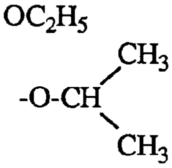 | H | 3 | |
| 10.245 | F | Cl | $-O-CH_2-CH_2-O-CH$ | H | 3 | |
| 10.246 | F | Cl | $-O-CH(CH_3)-CH_2-S-CH_3$ | H | 3 | |
| 10.247 | F | Cl | $-O-CH_2-COOCH_3$ | H | 3 | |
| 10.248 | F | Cl | $-S-CH_2-COOCH_3$ | H | 3 | |
| 10.249 | F | Cl | $-O-CH_2-C\equiv CH$ | H | 3 | |
| 10.250 | F | Cl | -Cl | Cl | 0 | |
| 10.251 | F | Cl | -OH | Cl | 0 | |
| 10.252 | F | Cl | $-OCH_3$ | Cl | 0 | |
| 10.253 | F | Cl | $-OC_2H_5$ | Cl | 0 | |
| 10.254 | F | Cl | 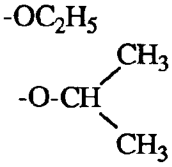 | Cl | 0 | |
| 10.255 | F | Cl | $-O-CH_2-COOCH_3$ | Cl | 0 | |
| 10.256 | F | Cl | $-S-CH_2-COOCH_3$ | Cl | 0 | |

Tabelle 10a (Fortsetzung)

| Verb. Nr. | $R_1$ | $R_2$ | $R_3$ | $R_4$ | n | Smp. [°C] |
|---|---|---|---|---|---|---|
| 10.257 | F | Cl | $-OCH_3$ | Br | 0 | |
| 10.258 | F | Cl | 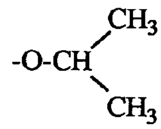 | Br | 0 | |
| 10.259 | F | Cl | $-OCH_3$ | F | 0 | |
| 10.260 | F | Cl | $-OCH_3$ | $CH_3$ | 0 | |
| 10.261 | F | Cl | $-OC_2H_5$ | $CH_3$ | 0 | |
| 10.262 | F | Cl | 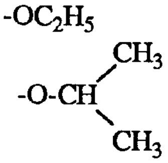 | $CH_3$ | 0 | |
| 10.263 | F | Cl | $-O-CH_2-CH_2-O-CH_3$ | $CH_3$ | 0 | |
| 10.264 | F | Cl | $-O-CH_2-COOCH_3$ | $CH_3$ | 0 | |
| 10.265 | F | Cl | $-O-CH(CH_3)COOCH_3$ | $CH_3$ | 0 | |
| 10.266 | F | Cl | $-S-CH_2-COOCH_3$ | $CH_3$ | 0 | |
| 10.267 | F | Cl | $-OCH_3$ | $CF_3$ | 0 | |
| 10.268 | F | Cl | $-OC_2H_5$ | $CF_3$ | 0 | |
| 10.269 | F | Cl | 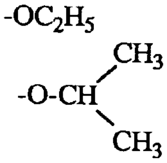 | $CF_3$ | 0 | |
| 10.270 | F | Cl | $-O-CH_2-COOCH_3$ | $CF_3$ | 0 | |
| 10.271 | F | Cl | $-S-CH_2-COOCH_3$ | $CF_3$ | 0 | |

Tabelle 10b:

Verbindungen der Formel I, worin W für $W_{10}$, $Y_1$ für Sauerstoff, $Y_2$ für Sauerstoff, $R_{14}$ für Wasserstoff und A für -CN steht:

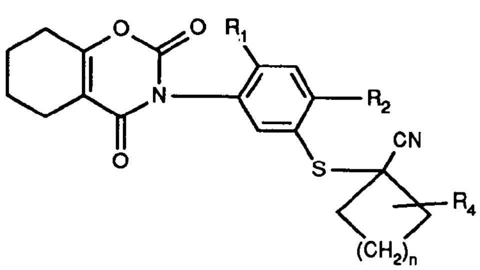

(I)

Tabelle 10b

| Verb. Nr. | $R_1$ | $R_2$ | $R_3$ | $R_4$ | n | Smp. [°C] |
|---|---|---|---|---|---|---|
| 10.272 | F | Cl | - | H | 0 | |
| 10.273 | F | Cl | - | H | 1 | |
| 10.274 | F | Cl | - | H | 2 | |
| 10.275 | F | Cl | - | H | 3 | |
| 10.276 | F | Cl | - | H | 4 | |

Tabelle 11:

Zwischenprodukte der Formel III worin $R_{14}$ für Wasserstoff steht:

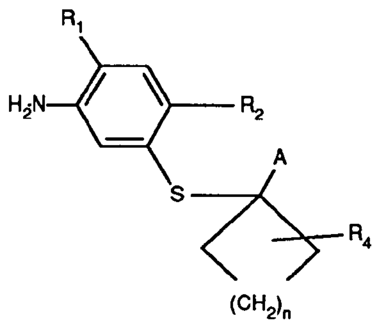

(III)

<u>Tabelle 11</u>

| Verb. Nr. | $R_1$ | $R_2$ | $R_3$ | $R_4$ | n | A | Smp. [°C] |
|---|---|---|---|---|---|---|---|
| 11.001 | F | Cl | OH | H | 0 | $-CO-R_3$ | 227-229 |
| 11.002 | F | Cl | $OCH_3$ | H | 0 | $-CO-R_3$ | 223-224 |
| 11.003 | F | Cl | $OC_2H_5$ | H | 0 | $-CO-R_3$ | 96-99 |
| 11.004 | F | Cl | 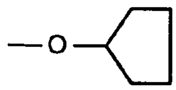 | H | 0 | $-CO-R_3$ | |
| 11.005 | F | Cl | $-O-CH_2-CH_2-O-CH_3$ | H | 0 | $-CO-R_3$ | |
| 11.006 | F | Cl | -OH | H | 1 | $-CO-R_3$ | |
| 11.007 | F | Cl | $OCH_3$ | H | 1 | $-CO-R_3$ | 89-90 |
| 11.008 | F | Cl | $OC_2H_5$ | H | 1 | $-CO-R_3$ | $\eta_D^{22}$ 1.5711 |
| 11.009 | F | Cl | 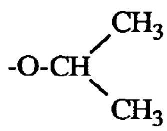 | H | 1 | $-CO-R_3$ | |
| 11.010 | F | Cl | -OH | H | 2 | $-CO-R_3$ | |
| 11.011 | F | Cl | $-OCH_3$ | H | 2 | $-CO-R_3$ | |
| 11.012 | F | Cl | $-OCH_3$ | H | 3 | $-CO-R_3$ | |
| 11.013 | H | Cl | OH | H | 0 | $-CO-R_3$ | |
| 11.014 | H | Cl | $OCH_3$ | H | 0 | $-CO-R_3$ | |
| 11.015 | H | Cl | 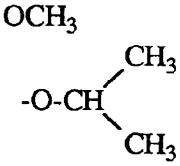 | H | 0 | $-CO-R_3$ | |
| 11.016 | H | Cl | -OH | H | 1 | $-CO-R_3$ | |
| 11.017 | H | Cl | $-OCH_3$ | H | 1 | $-CO-R_3$ | |
| 11.018 | F | Br | -OH | H | 0 | $-CO-R_3$ | |
| 11.019 | F | Br | $-OCH_3$ | H | 0 | $-CO-R_3$ | |
| 11.020 | F | Br | $-OC_3H_5$ | H | 0 | $-CO-R_3$ | |
| 11.021 | F | Cl | OH | Cl | 0 | $-CO-R_3$ | 173-174 |
| 11.022 | F | Cl | $OCH_3$ | Cl | 0 | $-CO-R_3$ | |
| 11.023 | F | Cl | $O_2H_5$ | Cl | 0 | $-CO-R_3$ | |
| 11.024 | F | Cl | -OH | Cl | 1 | $-CO-R_3$ | |
| 11.025 | F | Cl | $-OCH_3$ | Cl | 1 | $-CO-R_3$ | |
| 11.026 | F | Cl | $-OC_2H_5$ | Cl | 1 | $-CO-R_3$ | |
| 11.027 | F | Cl | -OH | F | 0 | $-CO-R_3$ | |

Tabelle 11 (Fortsetzung)

| Verb. Nr. | $R_1$ | $R_2$ | $R_3$ | $R_4$ | n | A | Smp. [°C] |
|---|---|---|---|---|---|---|---|
| 11.028 | F | Cl | $-OCH_3$ | F | 0 | $-CO-R_3$ | |
| 11.029 | F | Cl | $-OCH_3$ | $CH_3$ | 0 | $-CO-R_3$ | |
| 11.030 | F | Cl | $-OCH_3$ | $CF_3$ | 0 | $-CO-R_3$ | |
| 11.031 | F | Cl | - | H | 0 | -CN | |
| 11.032 | F | Cl | - | H | 1 | -CN | |
| 11.033 | F | Cl | - | H | 2 | -CN | |
| 11.034 | H | Cl | - | H | 0 | -CN | |

Tabelle 12:

Zwischenprodukte der Formel VII worin $R_{14}$ für Wasserstoff steht:

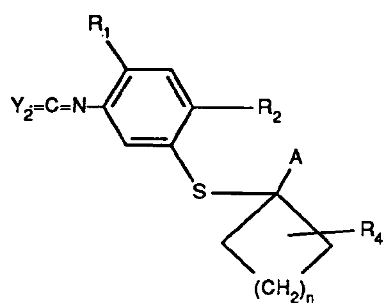

(VII)

Tabelle 12

| Verb. Nr. | $R_1$ | $R_2$ | $R_3$ | $R_4$ | n | A | $Y_2$ | Smp. [°C] |
|---|---|---|---|---|---|---|---|---|
| 12.001 | F | Cl | OH | H | 0 | $-CO-R_3$ | S | 181-183 |
| 12.002 | F | Cl | $OCH_3$ | H | 0 | $-CO-R_3$ | S | 100-101 |
| 12.003 | F | Cl | $OC_2H_5$ | H | 0 | $-CO-R_3$ | S | 68-71 |
| 12.004 | F | Cl | 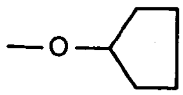 | H | 0 | $-CO-R_3$ | S | |
| 12.005 | F | Cl | $-O-CH_2-CH_2-O-CH_3$ | H | 0 | $-CO-R_3$ | S | |
| 12.006 | F | Cl | -OH | H | 1 | $-CO-R_3$ | S | |
| 12.007 | F | Cl | $OCH_3$ | H | 1 | $-CO-R_3$ | S | |
| 12.008 | F | Cl | $OC_2H_5$ | H | 1 | $-CO-R_3$ | S | |
| 12.009 | F | Cl | 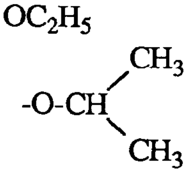 | H | 1 | $-CO-R_3$ | S | |
| 12.010 | F | Cl | -OH | H | 2 | $-CO-R_3$ | S | |
| 12.011 | F | Cl | $-OCH_3$ | H | 2 | $-CO-R_3$ | S | |
| 12.012 | F | Cl | $-OCH_3$ | H | 3 | $-CO-R_3$ | S | |
| 12.013 | H | Cl | OH | H | 0 | $-CO-R_3$ | S | |
| 12.014 | H | Cl | $OCH_3$ | H | 0 | $-CO-R_3$ | S | |
| 12.015 | H | Cl | 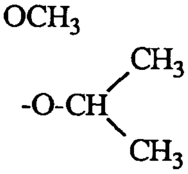 | H | 0 | $-CO-R_3$ | S | |
| 12.016 | H | Cl | -OH | H | 1 | $-CO-R_3$ | S | |
| 12.017 | H | Cl | $-OCH_3$ | H | 1 | $-CO-R_3$ | S | |
| 12.018 | F | Br | -OH | H | 0 | $-CO-R_3$ | S | |
| 12.019 | F | Br | $-OCH_3$ | H | 0 | $-CO-R_3$ | S | |
| 12.020 | F | Br | $-OC_3H_5$ | H | 0 | $-CO-R_3$ | S | |
| 12.021 | F | Cl | OH | Cl | 0 | $-CO-R_3$ | S | |
| 12.022 | F | Cl | $OCH_3$ | Cl | 0 | $-CO-R_3$ | S | |
| 12.023 | F | Cl | $O_2H_5$ | Cl | 0 | $-CO-R_3$ | S | |
| 12.024 | F | Cl | -OH | Cl | 1 | $-CO-R_3$ | S | |
| 12.025 | F | Cl | $-OCH_3$ | Cl | 1 | $-CO-R_3$ | S | |
| 12.026 | F | Cl | $-OC_2H_5$ | Cl | 1 | $-CO-R_3$ | S | |

Tabelle 12 (Fortsetzung)

| Verb. Nr. | $R_1$ | $R_2$ | $R_3$ | $R_4$ | n | A | $Y_2$ | Smp. [°C] |
|---|---|---|---|---|---|---|---|---|
| 12.027 | F | Cl | -OH | F | 0 | $-CO-R_3$ | S | |
| 12.028 | F | Cl | $-OCH_3$ | F | 0 | $-CO-R_3$ | S | |
| 12.029 | F | Cl | $-OCH_3$ | $CH_3$ | 0 | $-CO-R_3$ | S | |
| 12.030 | F | Cl | $-OCH_3$ | $CF_3$ | 0 | $-CO-R_3$ | S | |
| 12.031 | F | Cl | - | H | 0 | -CN | S | |
| 12.032 | F | Cl | - | H | 1 | -CN | S | |
| 12.033 | F | Cl | - | H | 2 | -CN | S | |
| 12.034 | H | Cl | - | H | 0 | -CN | S | |
| 12.035 | F | Cl | OH | H | 0 | $-CO-R_3$ | O | |
| 12.036 | F | Cl | $OCH_3$ | H | 0 | $-CO-R_3$ | O | |
| 12.037 | F | Cl | $OC_2H_5$ | H | 0 | $-CO-R_3$ | O | |
| 12.038 | F | Cl | 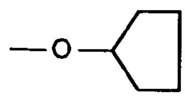 | H | 0 | $-CO-R_3$ | O | |
| 12.039 | F | Cl | $-O-CH_2-CH_2-O-CH_3$ | H | 0 | $-CO-R_3$ | O | |
| 12.040 | F | Cl | -OH | H | 1 | $-CO-R_3$ | O | |
| 12.041 | F | Cl | $OCH_3$ | H | 1 | $-CO-R_3$ | O | |
| 12.042 | F | Cl | $OC_2H_5$ | H | 1 | $-CO-R_3$ | O | |
| 12.043 | F | Cl | 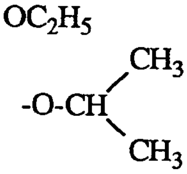 | H | 1 | $-CO-R_3$ | O | |
| 12.044 | F | Cl | -OH | H | 2 | $-CO-R_3$ | O | |
| 12.045 | F | Cl | $-OCH_3$ | H | 2 | $-CO-R_3$ | O | |
| 12.046 | F | Cl | $-OCH_3$ | H | 3 | $-CO-R_3$ | O | |
| 12.047 | H | Cl | OH | H | 0 | $-CO-R_3$ | O | |
| 12.048 | H | Cl | $OCH_3$ | H | 0 | $-CO-R_3$ | O | |
| 12.049 | H | Cl | 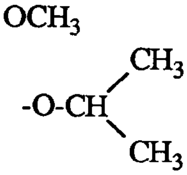 | H | 0 | $-CO-R_3$ | O | |
| 12.050 | H | Cl | -OH | H | 1 | $-CO-R_3$ | O | |
| 12.051 | H | Cl | $-OCH_3$ | H | 1 | $-CO-R_3$ | O | |
| 12.052 | F | Br | -OH | H | 0 | $-CO-R_3$ | O | |
| 12.053 | F | Br | $-OCH_3$ | H | 0 | $-CO-R_3$ | O | |
| 12.054 | F | Br | $-OC_3H_5$ | H | 0 | $-CO-R_3$ | O | |

Tabelle 12 (Fortsetzung)

| Verb. Nr. | $R_1$ | $R_2$ | $R_3$ | $R_4$ | n | A | $Y_2$ | Smp. [°C] |
|---|---|---|---|---|---|---|---|---|
| 12.055 | F | Cl | OH | Cl | 0 | $-CO-R_3$ | O | |
| 12.056 | F | Cl | $OCH_3$ | Cl | 0 | $-CO-R_3$ | O | |
| 12.057 | F | Cl | $O_2H_5$ | Cl | 0 | $-CO-R_3$ | O | |
| 12.058 | F | Cl | -OH | Cl | 1 | $-CO-R_3$ | O | |
| 12.059 | F | Cl | $-OCH_3$ | Cl | 1 | $-CO-R_3$ | O | |
| 12.060 | F | Cl | $-OC_2H_5$ | Cl | 1 | $-CO-R_3$ | O | |
| 12.061 | F | Cl | -OH | F | 0 | $-CO-R_3$ | O | |
| 12.062 | F | Cl | $-OCH_3$ | F | 0 | $-CO-R_3$ | O | |
| 12.063 | F | Cl | $-OCH_3$ | $CH_3$ | 0 | $-CO-R_3$ | O | |
| 12.064 | F | Cl | $-OCH_3$ | $CF_3$ | 0 | $-CO-R_3$ | O | |
| 12.065 | F | Cl | - | H | 0 | -CN | O | |
| 12.066 | F | Cl | - | H | 1 | -CN | O | |
| 12.067 | F | Cl | - | H | 2 | -CN | O | |
| 12.068 | H | Cl | - | H | 0 | -CN | O | |

Die Wirkstoffe der Formel I werden in der Regel mit Aufwandmengen von 0,001 bis 4 kg/ha insbesondere 0,005 bis 1 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und herbizide Eigenschaften aus, die sie ausgezeichnet zum Einsatz in Kulturen von Nutzpflanzen, insbesondere in Getreide, Baumwolle, Soja, Raps, Mais und Reis befähigen, wobei der Einsatz in Maiskultur ganz besonders bevorzugt ist.

Die Erfindung betrifft auch herbizide und pflanzenwachstumsregulierende Mittel, welche einen neuen Wirkstoff der Formel I enthalten, sowie Verfahren zur Hemmung des Pflanzenwuchses.

Pflanzenwachstumsregulatoren sind Substanzen, die in/an der Pflanze agronomisch erwünschte biochemische und/oder physiologische und/oder morphologische Veränderungen bewirken.

Die in den erfindungsgemäßen Mitteln enthaltenen Wirkstoffe beeinflussen das Pflanzenwachstum je nach Applikationszeitpunkt, Dosierung, Applikationsart und Umweltbedingungen in verschiedener Weise. Pflanzenwuchsregulatoren der Formel I können zum Beispiel das vegetative Wachstum von Pflanzen hemmen. Diese Art der Wirkung ist von Interesse auf Rasenflächen, im Zierpflanzenbau, in Obstplantagen, an Straßenböschungen, auf Sport- und Industrieanlagen, aber auch bei der gezielten Hemmung von Nebentrieben wie bei Tabak. Im Ackerbau führt die Hemmung des vegetativen Wachstums bei Getreide über eine Halmverstärkung zu reduziertem Lager, ähnliche agronomische Wirkungen erreicht man in Raps, Sonnenblumen, Mais und anderen Kulturpflanzen. Des weiteren kann durch Hemmung des vegetativen Wachstums die Anzahl Pflanzen pro Fläche erhöht werden. Ein weiteres Einsatzgebiet von Wuchshemmern ist die selektive Kontrolle von bodendeckenden Pflanzen in Plantagen oder weitreihigen Kulturen durch starke Wuchshemmung, ohne diese Bodendecker abzutöten, sodaß die Konkurrenz gegenüber der Hauptkultur ausgeschaltet ist, die agronomisch positiven Effekte wie Erosionsverhinderung, Stickstoffbindung und Bodenlockerung aber erhalten bleiben.

Unter einem Verfahren zur Hemmung des Pflanzenwachstums ist eine Steuerung der natürlichen Pflanzenentwicklung zu verstehen, ohne den von genetischen Eigenschaften determinierten Lebenszyklus der Pflanze im Sinne einer Mutation zu verändern. Das Verfahren der Wuchsregulierung wird zu einem im Einzelfall

zu bestimmenden Entwicklungszeitpunkt der Pflanze angewendet. Die Applikation der Wirkstoffe der Formel I kann vor oder nach dem Auflaufen der Pflanzen erfolgen, beispielsweise bereits auf die Samen oder die Sämlinge, auf Wurzeln, Knollen, Stengel, Blätter, Blüten oder andere Pflanzenteile. Dies kann z.B. durch Aufbringen des Wirkstoffes selbst oder in Form eines Mittels auf die Pflanzen und/oder durch Behandlung des Nährmediums der Pflanze (Erdboden) geschehen.

Für die Verwendung der Verbindung der Formel I oder sie enthaltender Mittel zur Regulierung des Pflanzenwachstums kommen verschiedene Methoden und Techniken in Betracht, wie beispielsweise die folgenden:

i) Samenbeizung

a) Beizung der Samen mit einem als Spritzpulver formulierten Wirkstoff durch Schütteln in einem Gefäß bis zur gleichmäßigen Verteilung auf der Samenoberfläche (Trockenbeizung). Man verwendet dabei bis zu 4 g Wirkstoff der Formel I (bei einer 50 %igen Formulierung: bis zu 8,0 g Spritzpulver) pro 1 kg Saatgut.
b) Beizung der Samen mit einem Emulsionskonzentrat des Wirkstoffs oder mit einer wäßrigen Lösung des als Spritzpulver formulierten Wirkstoffs der Formel I nach Methode a) (Naßbeizung).
c) Beizung durch Tauchen des Saatguts in einer Brühe mit bis zu 1000 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäß die bevorzugten Methoden der Applikation, weil die Wirkstoffbehandlung vollständig auf die Zielkultur gerichtet ist. Man verwendet in der Regel 0,001 g bis 4,0 g Aktivsubstanz pro 1 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Überzug aufgebracht werden (Umhüllungsgranulate), der es erlaubt, den Wirkstoff über einen bestimmten Zeitraum dosiert abzugeben.

Die Verbindungen der Formel I werden in unveränderter Form, wie aus der Synthese erhältlich, oder vorzugsweise mit den in der Formulierungstechnik üblichen Hilfsmitteln eingesetzt und werden daher z.B. zu emulgierbaren Konzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Benetzen, Verstreuen oder Gießen werden gleich wie die Art der Mittel den angestrebten Zielen und den gegebenen Verhältnissen entsprechend gewählt.

Die Formulierungen, d.h. die den Wirkstoff der Formel I und gegebenenfalls einen oder mehrere feste oder flüssige Zusatzstoffe enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, insbesondere die Fraktionen $C_8$ bis $C_{12}$, wie Mischungen von Alkylbenzolen, z.B. Xylolgemische oder alkylierte Naphthaline; aliphatische und cycloaliphatische Kohlenwasserstoffe wie Paraffine, Cyclohexan oder Tetrahydronaphthalin; Alkohole, wie Ethanol, Propanol oder Butanol; Glykole sowie deren Ether und Ester, wie Propylenglykol oder Dipropylenglykolether, Ketone wie Cyclohexanon, Isophoron oder Diacetonalkohol, starke polare Lösungsmittel wie N-Methyl-2-pyrrolidon, Dimethylsulfoxid oder Wasser Pflanzenöle sowie deren Ester, wie Raps-, Ricinus- oder Sojaöl; gegebenenfalls auch Silikonöle.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali oder gegebenenfalls substituierten Ammoniumsalze von höheren Fettsäuren ($C_{10}$-$C_{22}$), wie z.B. die Na- oder K-Salze der Öl- oder Stearinsäure, oder von natürlichen Fettsäuregemischen genannt, die z.B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylarylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, die Dibutylnaphthalinsulfonsäure, oder eines Naphthalinsulfonsäure-Formaldehydkondensationsproduktes. Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen, enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht ionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside sind u.a. in folgenden Publikationen beschrieben:

- "Mc Cutcheon's Detergents and Emulsifiers Annual", Mc Publishing Corp., Glen Rock, New Jersey, 1988.
- M. and J. Ash, "Encyclopedia of Surfactants", Vol. I-III, Chemical Publishing Co., New York, 1980-1981.
- Dr. Helmut Stache "Tensid-Taschenbuch", Carl Hanser Verlag, München/Wien 1981.

Die pestiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Insbesondere setzen sich bevorzugte Formulierungen folgendermaßen zusammen:
(% = Gewichtsprozent)

Emulgierbare Konzentrate:

| | |
|---|---|
| Aktiver Wirkstoff: | 1 bis 90 %, vorzugsweise 5 bis 20 % |
| oberflächenaktives Mittel: | 1 bis 30 %, vorzugsweise 10 bis 20 % |
| flüssiges Trägermittel: | 50 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspensions-Konzentrate:

| | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

Benetzbare Pulver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

Granulate:

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Formulierungsbeispiele für flüssige Wirkstoffe der Formel I (% = Gewichtsprozent)

| 1. Emulsions-Konzentrate | a) | b) | c) |
|---|---|---|---|
| Wirkstoff gemäß Tabelle 1-10 | 25 % | 40 % | 50 % |
| Ca-Dodecylbenzolsulfonat | 5 % | 8 % | 6 % |
| Ricinusöl-polyethylenglykolether (36 Mol AeO) | 5 % | - | - |
| Tributylphenol-polyethylenglykol-ether (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff gemäß Tabelle 1-10 | 80 % | 10 % | 5 % | 95 % |
| Propylenglykol-monomethylether | 20 % | - | - | - |
| Polyethylenglykol MG400 | - | 70 % | - | - |
| N-Methyl-2-pyrrolidon | - | 20 % | - | - |
| Epoxidiertes Kokosnußöl | - | - | 1 % | 5 % |
| Benzin (Siedegrenzen 160-190°C) | - | - | 94 % | - |

Die Lösungen sind zur Anwendung in Form kleinster Tropfen geeignet.

| 3. Granulate | a) | b) | c) | d) |
|---|---|---|---|---|
| Wirkstoff gemäß Tabelle 1-10 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Methylenchlorid gelöst, auf den Träger aufgesprüht und das Lösungsmittel anschließend im Vakuum abgedampft.

| 4. Stäubemittel | a) | b) |
|---|---|---|
| Wirkstoff gemäß Tabelle 1-10 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| 5. Spritzpulver | a) | b) | c) |
|---|---|---|---|
| Wirkstoff gemäß Tabelle 1-10 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | |
|---|---|
| Wirkstoff gemäß Tabelle 1-10 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden

7. Stäubemittel

| | a) | b) |
|---|---|---|
| Wirkstoff gemäß Tabelle 1-10 | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

8. Extruder-Granulat

| | |
|---|---|
| Wirkstoff gemäß Tabelle 1-10 | 10 % |
| Na-ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschließend im Luftstrom getrocknet.

9. Umhüllungs-Granulat

| | |
|---|---|
| Wirkstoff gemäß Tabelle 1-10 | 3 % |
| Polyäthylenglykol (MG200) | 3 % |
| Kaolin | 94 % |

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

10. Suspensions-Konzentrat

| | |
|---|---|
| Wirkstoff gemäß Tabelle 1-10 | 40 % |
| Propylenglykol | 10 % |
| Nonylphenolpolyethylenglykolether (15 Mol AeO) | 6 % |
| Na-Ligninsulfonat | 10 % |
| Carboxymethylcellulose | 1 % |
| Silikonöl in Form einer 75%-igen wäßrigen Emulsion | 1 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.Die Verbidungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

Biologische Beispiele

Beispiel B1: Pre-emergente Herbizid-Wirkung

Im Gewächshaus wird unmittelbar nach der Einsaat der Versuchspflanzen in Saatschalen die Erdoberfläche mit einer wäßrigen Spritzbrühe entsprechend einer Aufwandmenge von 4 kg Wirksubstanz/Hektar behandelt. Die Saatschalen werden im Gewächshaus bei 22 - 25°C und 50 - 70 % relativer Luftfeuchtigkeit gehalten.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Tabelle 1 starke Herbizidwirkung.

Beispiel B2: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 250-1000 g Wirksubstanz pro Hektar auf die Pflanzen gespritzt und diese bei 24°-26°C und 45-60 % relativer Luftfeuchtigkeit gehalten. 15 Tage nach der Behandlung wird die Herbizidwirkung mit einem neunstufigen (1 = vollständige Schädigung, 9 = keine Wirkung) Boniturschema im Vergleich mit einer unbehandelten Kontrollgruppe bewertet. Einzelergebnisse sind in den Tabellen B1 und B2 zusammengestellt:

Tabelle B1: Herbizid-Wirkung der Verbindung Nr. 1.117

| Pflanze | Aufwandmenge [g/ha] | | |
|---|---|---|---|
| | 1000 | 500 | 250 |
| Abutilon | 1 | 1 | 1 |
| Sida spinosa | 1 | 1 | 1 |
| Xanthium Sp. | 1 | 1 | 1 |
| Amaranthus ret. | 1 | 1 | 1 |
| Chenopodium Sp. | 1 | 1 | 1 |
| Ipomoea | 1 | 1 | 1 |
| Sinapis | 1 | 1 | 1 |
| Stellaria | 2 | 2 | 2 |
| Chrysanthe. leuc. | 1 | 1 | 1 |
| Galium aparine | 1 | 1 | 1 |
| Viola tricolor | 1 | 1 | 1 |
| Veronica Sp. | 1 | 1 | 3 |
| Solanum nigrum | 1 | 1 | 1 |

Tabelle B2: Herbizid-Wirkung der Verbindung Nr. 1.118

| Pflanze | Aufwandmenge [g/ha] | | |
|---|---|---|---|
| | 1000 | 500 | 250 |
| Abutilon | 1 | 1 | 1 |
| Sida spinosa | 1 | 1 | 1 |
| Xanthium Sp. | 1 | 1 | 1 |
| Amaranthus ret. | 1 | 1 | 1 |
| Chenopodium Sp. | 1 | 1 | 1 |
| Ipomoea | 1 | 1 | 1 |
| Sinapis | 1 | 1 | 1 |
| Stellaria | 3 | 4 | 4 |
| Chrysanthe. leuc. | 1 | 1 | 2 |
| Galium aparine | 1 | 1 | 2 |
| Viola tricolor | 1 | 1 | 1 |
| Veronica Sp. | 1 | 1 | 3 |
| Solanum nigrum | 1 | 1 | 1 |

Beispiel B3: Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat durch eine

Spritzung der Prüfsubstanzen auf die Gefäße. Die verwendete Dosis entspricht einer Wirkstoffmenge von 2 kg AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit.Die Auswertung der Versuche findet 3 Wochen nach Applikation statt. Die Verbindungen der Tabelle I schädigen dabei die Unkräuter, nicht aber den Reis.

Beispiel B4: Wuchshemmung bei tropischen Bodenbedecker-Leguminosen (cover crops)

Die Versuchspflanzen Centrosema pubescens und Psophocarpus palustris werden in 4 cm - Torftöpfen mit Landerde (45 %), Torf (45 %) und Zonolit (10 %) durch Stecklinge vermehrt. Die Anzucht erfolgt im Gewächshaus bei einer Tagestemperatur von 27°C und einer Nachttemperatur von 23°C. Die Beleuchtungsdauer ist mindestens 14 Stunden/Tag bei einer Intensität von mindestens 7000 Lux.

Ca. 50 Tage nach Ansatz der Stecklinge erfolgt das Vertopfen in 13 cm-Töpfe, 4 - 5 Pflanzen/Topf. Nach weiteren 60 Tagen werden die Pflanzen auf ca. 15 cm Höhe zurückgeschnitten und appliziert. Dabei werden sie mit 0,1 bis 300 g Wirkstoff/ha (in der Regel 25 %ig formuliert) in wäßriger Spritzbrühe besprüht. Die Wasseraufwandmenge beträgt ca. 200 l/ha.

4 Wochen nach der Applikation wird der Neuzuwachs im Gewicht ermittelt und in % zum Durchschnitt der unbehandelten Kontrollen dargestellt. Die nekrotischen Schäden sind in % zur gesamten Blattfläche aufgeführt.

Der Neuzuwachs der behandelten Pflanzen erweist sich als deutlich geringer als der der unbehandelten

Kontrollen.

Beispiel B5: Wuchsregulierung an Sojabohnen

Versuchspflanzen der Sorte Williams werden in 11 cm - Tontöpfen mit Landerde (45 %), Torf (45 %) und Zonolit (10 %) angesät und in der Klimakammer bei einer Tagestemperatur von 24°C und einer Nachttemperatur von 19°C angezogen. Die Beleuchtungsdauer ist 16 Stunden pro Tage bei einer Intensität von ca. 350 Mikro-Einstein.

Ca. 24 Tage nach der Saat erfolgt das Umtopfen in 18 cm - Töpfe, 2 Pflanzen/Topf. Nach weiteren 12 Tagen und im Stadium von 5 - 6 Trifolia-Blätter findet die Applikation mit 0,1 bis 300 g Wirkstoff/ha statt, in der Regel 25 %ig formuliert und in wäßriger Spritzbrühe. Die Waßeraufwandmenge beträgt ca. 200 l/ha.

Ca. 4 Wochen nach der Applikation findet die Auswertung statt. Hierbei wird die Höhe des Neuzuwachses gemessen und in % zum Durchschnitt der unbehandelten Kontrollen dargestellt. Die nekrotischen Schäden sind in % zur gesamten Blattfläche aufgeführt.

Die behandelten Pflanzen zeigen einen deutlich geringeren Neuzuwachs als die unbehandelten Kontrollen.

Beispiel B6: Wuchshemmung bei Getreide

Versuchspflanzen (Sommergerste der Sorte Iban) werden in 15 cm-Kunststofftöpfen mit steriler Landerde angesät und in der Klimakammer bei einer Tagestemperatur von 10 - 15°C und einer Nachttemperatur von 5 - 10°C angezogen. Die Beleuchtungsdauer ist 13.5 Stunden pro Tag bei einer Intensität von ca. 25000 Lux.

Ca. 34 Tage nach der Saat und dem Ausdünnen auf 4 Pflanzen/Topf erfolgt die Applikation mit 0,1 bis 300 g Wirkstoff/ha, in der Regel 25 %ig formuliert und in wäßriger Spritzbrühe. Die Wasseraufwandmenge ist ca. 500 l/ha. Nach der Applikation werden die Pflanzen im Gewächshaus bei einer Tagestemperatur von mindestens 10°C aufgestellt. Die Beleuchtungsdauer ist mind. 13.5 Stunden/Tag.

Ca. 28 Tage nach der Behandlung findet die Auswertung statt. Hierbei wird die Höhe des Neuzuwachses in % zum Durchschnitt der unbehandelten Kontrollen dargestellt. Die nekrotischen Schäden sind in % zur gesamten Blattfläche aufgeführt.

Die behandelten Pflanzen weisen im Vergleich zu unbehandelten Kontrollen eine Verringerung des Neuzuwachsese auf.

Beispiel B7: Wuchshemmung bei Gräsern

Eine Mischung von Gräsern (z.B. Poa, Festuca, Lolium, Bromus, Cynosurus) und Klee (Trifolium pratense/repens) wird in 15 cm-Kunststofftöpfen mit steriler Landerde angesät und im Gewächshaus bei einer Tagestemperatur von 21°C und einer Nachttemperatur von 17°C angezogen. Die Beleuchtungsdauer ist 13.5 Stunden/Tag bei einer Lichtintensität von mind. 7000 Lux. Nach dem Auflauf werden die Pflanzen wöchentlich auf ca. 6 cm Höhe zurückgeschnitten. Ca. 42 Tage nach der Saat und 1 Tag nach dem letzten Schnitt erfolgt die Applikation mit 0,1 bis 300 g Wirkstoff/ha, in der Regel 25 %ig formuliert und in wäßriger Spritzbrühe. Die Wasseraufwandmenge ist ca. 500 l/ha.

Ca. 3 Wochen nach der Behandlung findet die Auswertung statt. Hierbei wird die Höhe des Neuzuwachses gemessen und in % zum Durchschnitt der unbehandelten Kontrollen dargestellt. Die nekrotischen Schäden sind in % zur gesamten Blattfläche aufgeführt.

Die geprüften Verbindungen der Tabelle 1 bewirken eine Reduzierung des Neuzuwachses im Vergleich zur unbehandelten Kontrolle.

**Patentansprüche**

1. Cycloalkancarbonsäurederivate der Formel I

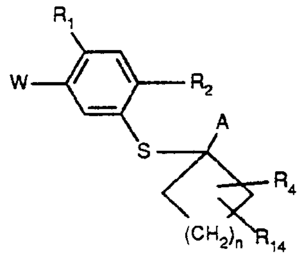 (I),

worin W für

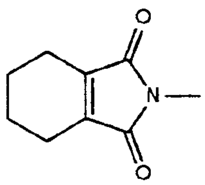 ($W_1$), 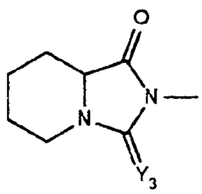 ($W_2$), 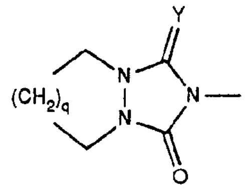 ($W_3$),

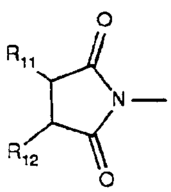 ($W_4$), 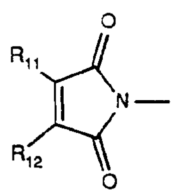 ($W_5$), 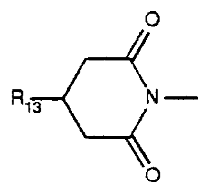 ($W_6$),

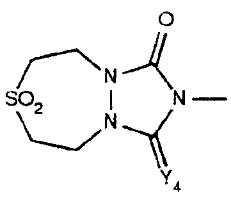 ($W_7$), 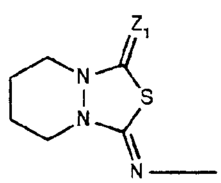 ($W_8$) 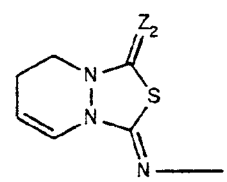 ($W_9$) oder

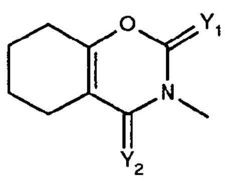 ($W_{10}$)

steht; und
A $CO-R_3$ oder CN;
$R_1$ Wasserstoff oder Fluor,
$R_2$ Halogen oder Cyano;
$R_3$ Chlor, $X-R_5$, Amino, $C_1$-$C_4$-Alkylamino, Di-$C_1$-$C_4$-alkylamino, $C_2$-$C_4$-Halogenalkylamino, Di-$C_2$-$C_4$-halogenalkylamino, $C_1$-$C_4$-Hydroxyalkylamino, Di-$C_1$-$C_4$-hydroxyalkylamino, $C_3$-$C_4$-Alkenylamino, Diallylamino, -N-Pyrrolidino, -N-Piperidino, -N-Morpholino, -N-Thiomorpholino, -N-Piperidazino, die Gruppe -O- N= C-$(R_9)R_{10}$ oder die Gruppe -N-$R_5(OR_5)$;

$R_4$ und $R_{14}$ unabhängig voneinander Wasserstoff, Fluor, Chlor, Brom, $C_1$-$C_4$-Alkyl oder Trifluormethyl;
$R_5$ Wasserstoff, $C_1$-$C_{10}$-Alkyl, $C_1$-$C_4$-Alkoxy-$C_1$-$C_4$-alkyl, Halogen-$C_1$-$C_8$-alkyl, $C_1$-$C_{10}$-Alkylthio-$C_1$-$C_4$-alkyl, Di-$C_1$-$C_4$-alkylamino-$C_1$-$C_4$-alkyl, Cyano-$C_1$-$C_8$-alkyl, $C_3$-$C_8$-Alkenyl, Halogen-$C_3$-$C_8$-alkenyl, $C_3$-$C_8$-Alkinyl, $C_3$-$C_7$-Cycloalkyl, $C_3$-$C_7$-Cycloalkyl-$C_1$-$C_4$-alkyl, Halogen-$C_3$-$C_7$-cycloalkyl oder Benzyl, das unsubstituiert oder am Phenylring gleich oder verschieden bis zu dreifach durch Halogen, $C_1$-$C_4$-Alkyl, Halogen-$C_1$-$C_4$-alkyl, Halogen-$C_1$-$C_4$-alkoxy oder $C_1$-$C_4$-alkoxy substituiert ist oder Alkali-, Erdalkali-Ionen und Ammoniumionen, die Gruppe - $[CHR_6$-$(CH_2)_m]$-$COOR_7$, oder die Gruppe $[CHR_6$-$(CH_2)_t$-$Si(R_6)_3]$;
$R_6$ Wasserstoff oder $C_1$-$C_4$-Alkyl;
$R_7$ Wasserstoff, $C_1$-$C_6$-Alkyl, $C_3$-$C_8$-Alkenyl, $C_3$-$C_8$-Alkinyl, $C_1$-$C_8$-Alkoxy-$C_2$-$C_8$- alkyl, $C_1$-$C_8$-Alkylthio-$C_2$-$C_8$- alkyl oder $C_3$-$C_7$-Cycloalkyl;
$R_8$ $C_1$-$C_4$-Alkyl;
$R_9$ $C_1$-$C_4$-Alkyl;
$R_{10}$ $C_1$-$C_4$-Alkyl oder Phenyl;
oder $R_9$ und $R_{10}$ zusammen mit dem Kohlenstoffatom, an welches sie gebunden sind, eien Cyclohexanring;
$R_{11}$ $C_1$ -$C_8$-Alkyl;
$R_{12}$ Wasserstoff oder $C_1$-$C_8$-Alkyl;
$R_{13}$ Wasserstoff, $C_1$-$C_6$-Alkyl, $C_3$-$C_7$-Cycloalkyl; $C_1$-$C_4$-Alkoxy-$C_1$-$C_4$-Alkyl, $C_1$-$C_4$-Alkylthio-$C_1$-$C_4$-alkyl; Halogen-$C_1$-$C_7$-alkyl, $C_3$-$C_7$-Alkenyl oder $C_3$-$C_7$-Alkinyl;
X Sauerstoff oder Schwefel;
Y Sauerstoff oder Schwefel;
$Y_1$ Sauerstoff oder Schwefel;
$Y_2$ Sauerstoff oder Schwefel;
$Y_3$ Sauerstoff oder Schwefel;
$Y_4$ Sauerstoff oder Schwefel;
$Z_1$ Sauerstoff oder Schwefel;
$Z_2$ Sauerstoff oder Schwefel;
n 0, 1, 2, 3 oder 4;
m 0, 1, 2, 3 oder 4;
q 1 oder 2; und
t 0, 1, 2, 3 oder 4
bedeutet, unter Einschluss der Salze und Komplexe mit Säuren, Basen oder Komplexbildnern, sowie der möglichen Stereoisomeren, die in Form von Enantiomeren, Diastereomeren oder deren Gemische vorliegen.

**2.** Cycloalkancarbonsäurederivate der Formel I gemäss Anspruch 1, dadurch gekennzeichnet, dass $R_{14}$ Wasserstoff bedeutet.

**3.** Cycloalkancarbonsäurederivate der Formel I gemäß Anspruch 1, dadurch gekennzeichnet, daß W für $W_8$ oder $W_9$ steht.

**4.** Cycloalkancarbonsäurederivate der Formel I gemäß Anspruch 1, dadurch gekennzeichnet, daß W für $W_1$ steht.

**5.** Cycloalkancarbonsäurederivate der Formel I gemäß Anspruch 1, dadurch gekennzeichnet, daß W für $W_2$ oder $W_3$ steht.

**6.** Cycloalkancarbonsäurederivate der Formel I gemäß Anspruch 1, dadurch gekennzeichnet, daß W für $W_6$ steht.

**7.** Cycloalkancarbonsäurederivate der Formel I gemäß Anspruch 1, dadurch gekennzeichnet, daß $R_1$ für Fluor steht.

**8.** Cycloalkancarbonsäurederivate der Formel I gemäß Anspruch 1, dadurch gekennzeichnet, daß $R_1$ für Fluor und $R_2$ für Chlor steht.

**9.** Cycloalkancarbonsäurederivate der Formel I gemäß Anspruch 1, dadurch gekennzeichnet, daß A für CO-$R_3$ und $R_3$ für X-$R_6$ steht.

**10.** Cycloalkancarbonsäurederivate der Formel I gemäß Anspruch 1, dadurch gekennzeichnet, daß A für CO-$R_3$ und $R_3$ für X-$R_5$ steht und $R_5$ $C_1$-$C_{10}$-Alkyl bedeutet.

**11.** Cycloalkancarbonsäurederivate der Formel I gemäß Anspruch 3, dadurch gekennzeichnet, daß A für CO-$R_3$ und $R_1$ für Fluor steht.

**12.** Cycloalkancarbonsäurederivate der Formel I gemäß Anspruch 3, dadurch gekennzeichnet, daß A für CO-$R_3$ und $R_1$ für Fluor und $R_2$ für Chlor steht.

**13.** Cycloalkancarbonsäurederivate der Formel I gemäß Anspruch 3, dadurch gekennzeichnet, daß A für CO-$R_3$ und $R_3$ für X-$R_5$ steht.

**14.** Cycloalkancarbonsäurederivate der Formel I gemäß Anspruch 3, dadurch gekennzeichnet, daß A CO-$R_3$, $R_3$ X-$R_5$ und $R_5$ $C_1$-$C_{10}$-Alkyl bedeutet.

**15.** Cycloalkancarbonsäurederivate der Formel I gemäß Anspruch 3, dadurch gekennzeichnet, daß A für CO-$R_3$ und $R_1$ Fluor, $R_2$ Chlor, $R_3$ X-$R_5$, und $R_5$ $C_1$-$C_{10}$-Alkyl bedeutet.

**16.** Cycloalkancarbonsäurederivate der Formel I gemäß Anspruch 3, dadurch gekennzeichnet, daß A für CO-$R_3$ und $R_4$ Wasserstoff bedeutet.

**17.** Cycloalkancarbonsäurederivate der Formel I gemäß Anspruch 15, dadurch gekennzeichnet, daß X Sauerstoff und $R_5$ $C_1$-$C_4$-Alkyl bedeutet.

**18.** Cycloalkancarbonsäurederivate der Formel I gemäß Anspruch 15, dadurch gekennzeichnet, daß X Sauerstoff und $R_5$ $C_1$-$C_4$-Alkyl bedeutet, und n für die Zahl 1 steht.

**19.** 1-[2-Chlor-4-fluor-5-(1,2,3,4,5,6-hexahydro-1,3-dioxo-isoindolyl)-phenylthio]-cyclopropancarbonsäuremethylester.

**20.** 9-[4-chlor-2-fluor-5-(1-methoxycarbonyl-cyclobutylthio)-phenylimino]-8-thia-1,6-diazabicyclo[4.3.0]-nonan-7-on.

**21.** 1-[4-Chlor-2-fluor-5-(1-methoxycarbonyl-cyclobutyl)-phenylthio]-4-trifluormethyl-piperidin-2,6-dion.

**22.** 9-[4-chlor-2-fluor-5-(1-methoxycarbonyl-cyclopropylthio)-phenylimino]-8-thia-1,6-diazabicyclo[4.3.0]-nonan-7-on.

**23.** Verfahren zur Herstellung der Verbindungen der Formel I, in welcher W für $W_8$ steht, dadurch gekennzeichnet, daß man ein Isothiocyanat der Formel VIIa

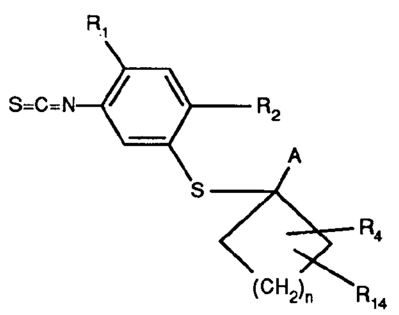

VIIa),

worin die Substituenten A, $R_1$, $R_2$, $R_4$, $R_{14}$ sowie n die unter Formel I im Anspruch 1 angegebene Bedeutung haben, mit einem Hexahydropyridazin der Formel VIII

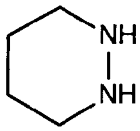

(VIII)

in die Verbindung der Formel IX

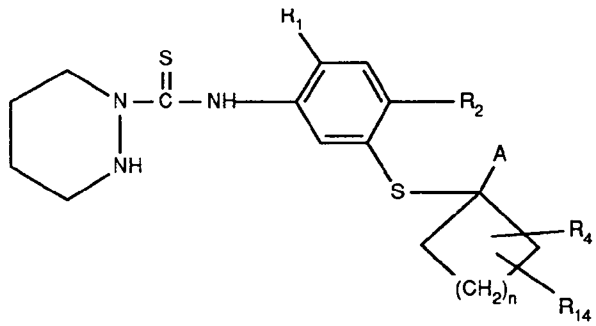

(IX)

überführt und diese anschliessend mit einer Verbindung der Formel X

$CZ_1Cl_2$ (X),

worin $Z_1$ für Sauerstoff oder Schwefel steht, in Gegenwart einer Base umsetzt.

**24.** Verfahren zur Herstellung der Verbindungen der Formel I, in welcher W für $W_9$ steht, dadurch gekennzeichnet, daß man ein Isothiocyanat der Formel VIIa

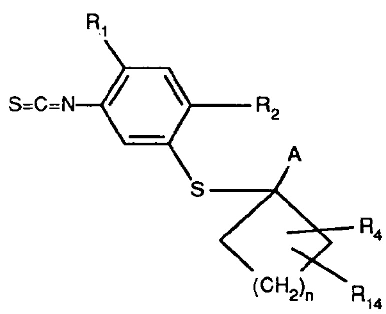

(VIIa),

worin die Substituenten A, $R_1$, $R_2$, $R_4$, $R_{14}$ sowie n die unter Formel I in Anspruch I angegebene Bedeutung haben, mit einem 1,4,5,6-Tetrahydropyridazin der Formel XI

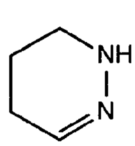

(XI)

in die Verbindung der Formel XII

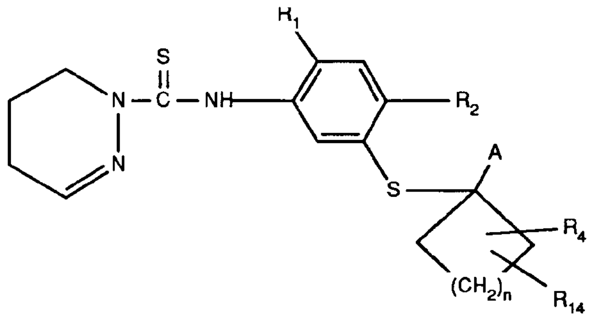

(XII)

überführt und diese anschliessend mit einer Verbindung der Formel Xa

$CZ_2Cl_2$ (Xa),

worin $Z_2$ für Sauerstoff oder Schwefel steht, in Gegenwart einer Base umsetzt.

**25.** Amine der Formel III

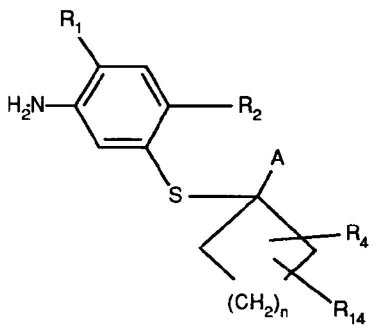

(III),

worin A, $R_1$, $R_2$, $R_3$, $R_4$, $R_{14}$ und n wie unter Formel I in Anspruch 1 definiert sind.

**26.** Verbindungen der Formel VII

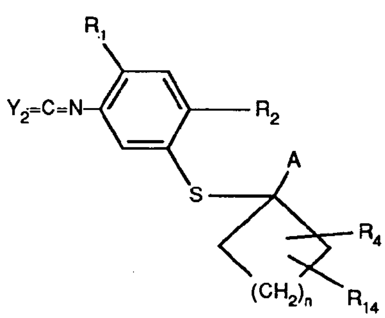

(VII)

worin $Y_2$, $R_1$, $R_2$, $R_4$, $R_{14}$ und A die unter Formel I im Anspruch 1 angegebene Bedeutung haben.

**27.** 2-Hydroxy-cyclohex-1-en-carbonsäure- oder -thiocarbonsäureanilide der Formel XIX

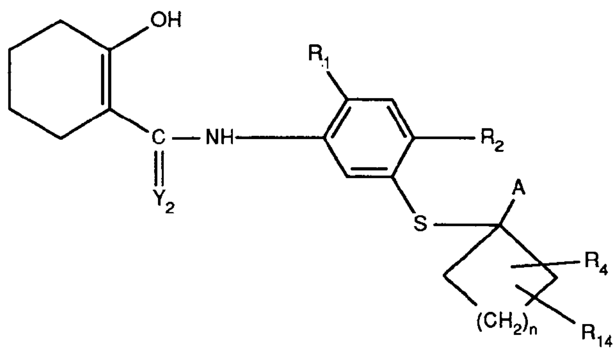

(XIX)

worin A, $R_1$, $R_2$, $R_4$, $R_{14}$, $Y_2$ und n die unter der Formel I im Anspruch 1 gegebene Bedeutung haben.

**28.** Ein herbizides und den Pflanzenwuchs hemmendes Mittel, gekennzeichnet durch ein Gehalt an einem oder mehreren Cycloalkancarbonsäurederivaten der Formel I, gemäß Anspruch 1.

**29.** Mittel gemäß Anspruch 28, dadurch gekennzeichnet, daß es zwischen 0,1 % und 95 % Wirkstoff der Formel I gemäß Anspruch 1 enthält.

**30.** Verfahren zur Bekämpfung unerwünschten Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

**31.** Verfahren gemäß Anspruch 30, dadurch gekennzeichnet, daß man eine Wirkstoffmenge zwischen 0,001 und 2 kg pro Hektar appliziert.

**32.** Verfahren zur Hemmung des Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

**33.** Verfahren gemäß Anspruch 30, zur selektiven pre- oder post-emergenten Bekämpfung von Unkräutern in Nutzpflanzenkulturen.

**Patentansprüche für folgenden Verstragsstaat: ES**

**1.** Verfahren zur Herstellung von Cycloalkancarbonsäurederivaten der Formel I

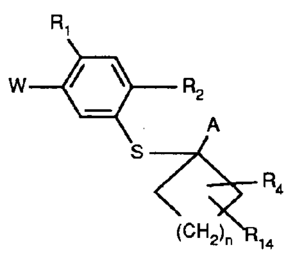

(I),

worin W für

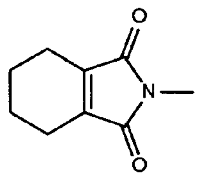 ($W_1$), 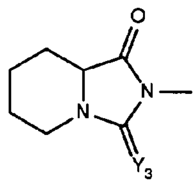 ($W_2$), 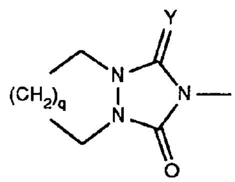 ($W_3$),

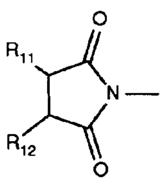 ($W_4$), 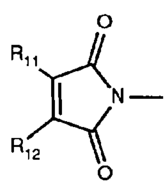 ($W_5$), 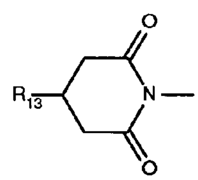 ($W_6$),

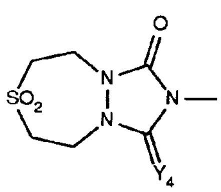 ($W_7$), 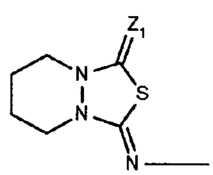 ($W_8$) 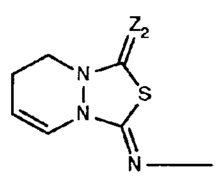 ($W_9$) oder

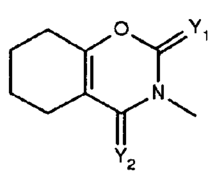 ($W_{10}$)

steht; und

A $CO-R_3$ oder CN;

$R_1$ Wasserstoff oder Fluor,

$R_2$ Halogen oder Cyano;

$R_3$ Chlor, $X-R_5$, Amino, $C_1$-$C_4$-Alkylamino, Di-$C_1$-$C_4$-alkylamino, $C_2$-$C_4$-Halogenalkylamino, Di-$C_2$-$C_4$-halogenalkylamino, $C_1$-$C_4$-Hydroxyalkylamino, Di-$C_1$-$C_4$-hydroxyalkylamino, $C_3$-$C_4$-Alkenylamino, Diallylamino, -N-Pyrrolidino, -N-Piperidino, -N-Morpholino, -N-Thiomorpholino, -N-Piperidazino, die Gruppe $-O-N=C-(R_9)R_{10}$ oder die Gruppe $-N-R_5(OR_5)$;

$R_4$ und $R_{14}$ unabhängig voneinander Wasserstoff, Fluor, Chlor, Brom, $C_1$-$C_4$-Alkyl oder Trifluormethyl;

$R_5$ Wasserstoff, $C_1$-$C_{10}$-Alkyl, $C_1$-$C_4$-Alkoxy-$C_1$-$C_4$-alkyl, Halogen-$C_1$-$C_8$-alkyl, $C_1$-$C_{10}$-Alkylthio-$C_1$-$C_4$-alkyl, Di-$C_1$-$C_4$-alkylamino-$C_1$-$C_4$-alkyl, Cyano-$C_1$-$C_8$-alkyl, $C_3$-$C_8$-Alkenyl, Halogen-$C_3$-$C_8$-alkenyl, $C_3$-$C_8$-Alkinyl, $C_3$-$C_7$-Cycloalkyl, $C_3$-$C_7$-Cycloalkyl-$C_1$-$C_4$-alkyl, Halogen-$C_3$-$C_7$-cycloalkyl oder Benzyl, das unsubstituiert oder am Phenylring gleich oder verschieden bis zu dreifach durch Halogen, $C_1$-$C_4$-Alkyl, Halogen-$C_1$-$C_4$-alkyl, Halogen-$C_1$-$C_4$-alkoxy oder $C_1$-$C_4$-alkoxy substituiert ist oder Alkali-, Erdalkali-Ionen und Ammoniumionen, die Gruppe $-[CHR_6-(CH_2)_m]-COOR_7$, oder die Gruppe $[CHR_5-(CH_2)_t-Si(R_8)_3]$;

$R_6$ Wasserstoff oder $C_1$-$C_4$-Alkyl;

$R_7$ Wasserstoff, $C_1$-$C_6$-Alkyl, $C_3$-$C_8$-Alkenyl, $C_3$-$C_8$-Alkinyl, $C_1$-$C_8$-Alkoxy-$C_2$-$C_8$-alkyl, $C_1$-$C_8$-Alkylthio-$C_2$-

$C_8$-alkyl oder $C_3$-$C_7$-Cycloalkyl;
$R_8$ $C_1$-$C_4$-Alkyl;
$R_9$ $C_1$-$C_4$-Alkyl;
$R_{10}$ $C_1$-$C_4$-Alkyl oder Phenyl;
oder $R_9$ und $R_{10}$ zusammen mit dem Kohlenstoffatom, an welches sie gebunden sind, eien Cyclohexanring;
$R_{11}$ $C_1$-$C_8$-Alkyl;
$R_{12}$ Wasserstoff oder $C_1$-$C_8$-Alkyl;
$R_{13}$ Wasserstoff, $C_1$-$C_6$-Alkyl, $C_3$-$C_7$-Cycloalkyl; $C_1$-$C_4$-Alkoxy-$C_1$-$C_4$-Alkyl, $C_1$-$C_4$-Alkylthio-$C_1$-$C_4$-alkyl; Halogen-$C_1$-$C_7$-alkyl, $C_3$-$C_7$-Alkenyl oder $C_3$-$C_7$-Alkinyl;
X Sauerstoff oder Schwefel;
Y Sauerstoff oder Schwefel;
$Y_1$ Sauerstoff oder Schwefel;
$Y_2$ Sauerstoff oder Schwefel;
$Y_3$ Sauerstoff oder Schwefel;
$Y_4$ Sauerstoff oder Schwefel;
$Z_1$ Sauerstoff oder Schwefel;
$Z_2$ Sauerstoff oder Schwefel;
n 0, 1, 2, 3 oder 4;
m 0, 1, 2, 3 oder 4;
q 1 oder 2; und
t 0, 1, 2, 3 oder 4

bedeutet, unter Einschluss der Salze und Komplexe mit Säuren, Basen oder Komplexbildnern, sowie der möglichen Stereoisomeren, die in Form von Enantiomeren, Diastereomeren oder deren Gemische vorliegen, dadurch gekennzeichnet, dass man

a) bei Verbindungen der Formel I, in welcher W für $W_1$, $W_4$, $W_5$ oder $W_6$ steht, die Anhydride der Reste $W_1$,$W_4$, $W_5$ und $W_6$ mit Aminen der Formel III

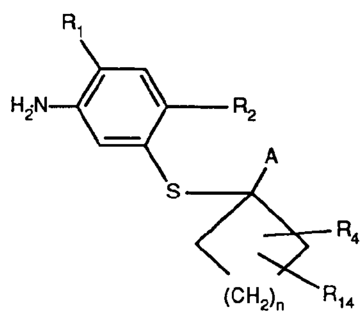

(III),

worin A, $R_1$, $R_2$, $R_4$, $R_{14}$ und n wie unter Formel I definiert sind, umsetzt, oder

b) bei den Verbindungen der Formel I, worin W für $W_2$ steht und $Y_3$ Sauerstoff bedeutet, ein Isocyanat der Formel VIIb

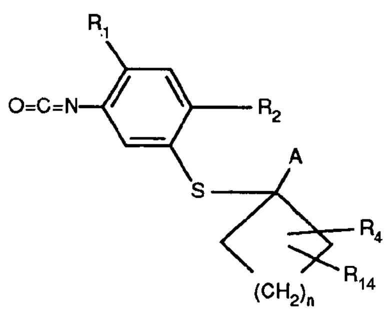

(VIIb)

worin $R_1$, $R_2$, $R_4$, $R_{14}$, A und n die unter Formel I angegebene Bedeutung haben, in einem organischem Lösungsmittel in Gegenwart einer Base bei einer Temperatur von 0 bis +150°C mit einem 2-Carboxylpiperidin der Formel XXIV

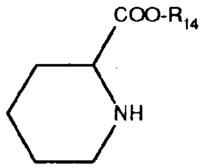

(XXIV)

worin $R_{14}$ für Wasserstoff, $C_1$-$C_6$-Alkyl oder Benzyl steht, zur Verbindung der Formel XXV

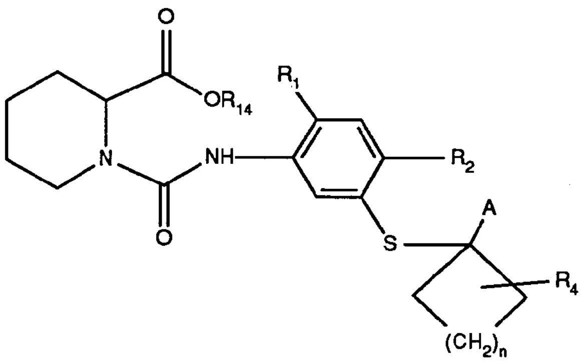

(XXV)

kondensiert, welche man anschließend unter sauren Bedingungen bei einer Temperatur von +50 bis +150°C zur Verbindung der Formel I cyclisiert, oder

c) bei Verbindungen der Formel I, worin W für $W_2$ steht und $Y_3$ Schwefel bedeutet, ein Isothiocyanatcyanat der Formel VIIa

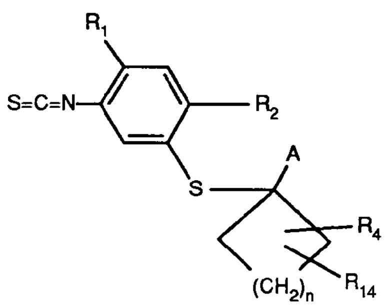

(VIIa)

worin $R_1$, $R_2$, $R_4$, $R_{14}$, A und n die unter Formel I angegebene Bedeutung haben, in einem organischem Lösungsmittel in Gegenwart einer Base bei einer Temperatur von 0 bis +150°C mit einem 2-Carboxylpiperidin der Formel XXIV

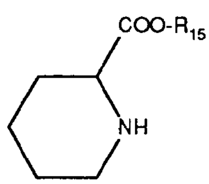

(XXIV)

worin $R_{15}$ für Wasserstoff, $C_1$-$C_6$-Alkyl oder Benzyl steht, zur Verbindung der Formel XXVa

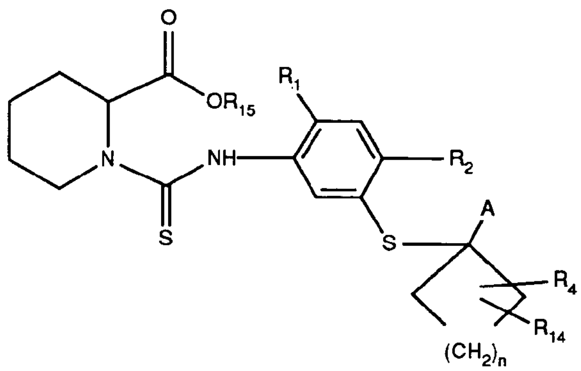

(XXVa)

kondensiert, welche man anschließend unter sauren Bedingungen bei einer Temperatur von +50 bis +150°C zur Verbindung der Formel I cyclisiert, oder

d) bei Verbindungen der Formel I, worin W für $W_3$ steht und Y Sauerstoff bedeutet, ein Isocyanat der Formel VIIb

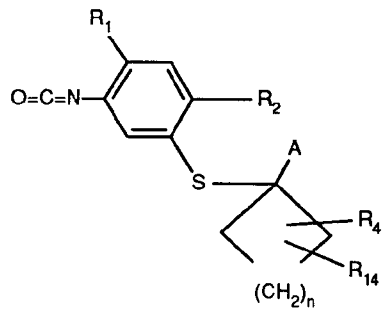

(VIIb)

worin $R_1$, $R_2$, $R_4$, $R_{14}$, A und n die unter Formel I angegebene Bedeutung haben, in einem organischem Lösungsmittel in Gegenwart einer Base bei einer Temperatur von 0 bis +150°C mit einer Verbindung der Formel XXVI

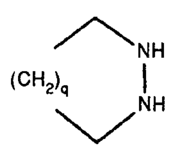

(XXVI)

worin q die unter Formel I angegebene Bedeutung hat, kondensiert, die entstandene Verbindung der Formel XXVII

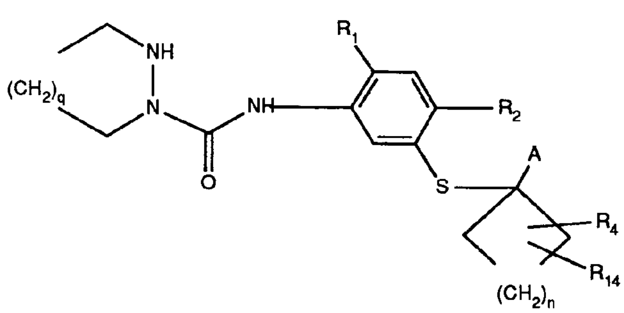

(XXVII)

anschließend in Gegenwart einer Base bei einer Temperatur von 0 bis +150°C mit einem Chlorkohlensäureester der Formel XXVIII

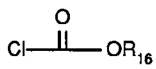 XXVIII

worin $R_{16}$ für $C_1$-$C_6$-Alkyl oder Benzyl steht, zu einer Verbindung der Formel XXIX

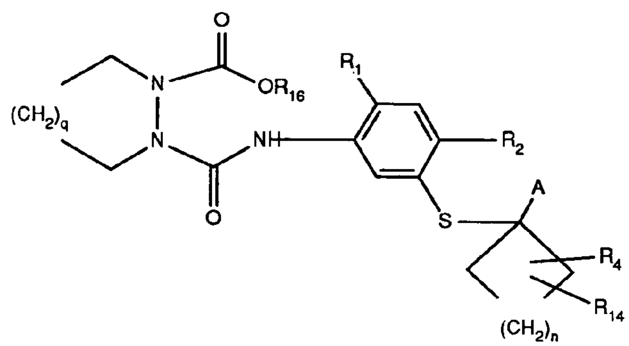 (XXIX)

umsetzt, welche man in einem inerten Lösungsmittel in Gegenwart einer Base bei einer Temperatur von +50 bis + 180°C zur Verbindung der Formel I cyclisiert, oder

e) bei Verbindungen der Formel I, in welcher W für $W_7$ steht und $Y_4$ Sauerstoff bedeutet, ein Isocyanat der Formel VIIb

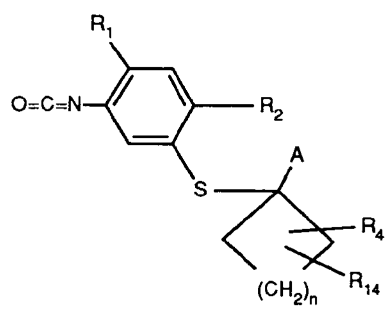 (VIIb)

worin die Substituenten A, $R_1$, $R_2$, $R_4$, $R_{14}$ sowie n die unter Formel I angegebene Bedeutung haben, mit einer Verbindung der Formel XXX

$$H_2N\text{-}NH\text{-}COOR_{16} \qquad (XXX)$$

worin $R_{16}$ die oben angegebene Bedeutung hat, zur Verbindung der Formel XXXI

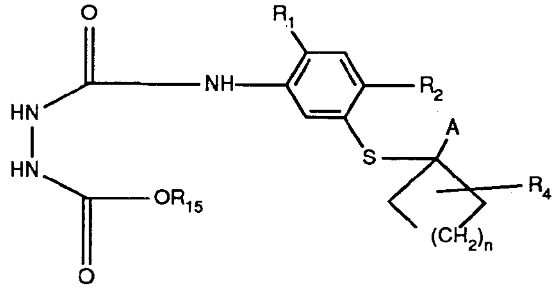 (XXXI)

umsetzt, welche man in Gegenwart einer Base bei einer Temperatur von +50 bis +180°C zur Verbindung der Formel XXXII

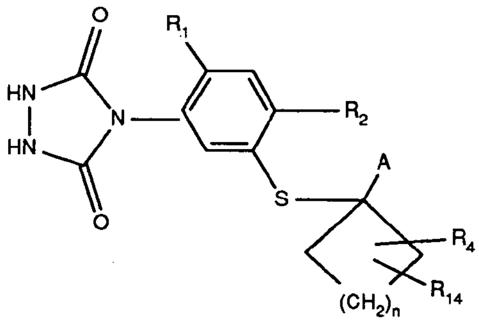

(XXXII)

cyclisiert, welche man bei einer Temperatur von 0 bis +150°C in Gegenwart einer katalytischen Menge an Base mit Divinylsulfon $(CH_2{=}CH)_2SO_2$ umsetzt, oder

f) bei Verbindungen der Formel I, in welcher W für $W_7$ steht und $Y_4$ Schwefel bedeutet, ein Isothiocyanat der Formel VIIa

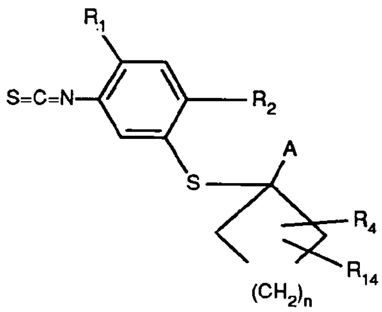

(VIIa)

worin die Substituenten A, $R_1$, $R_2$, $R_4$, $R_{14}$ sowie n die unter Formel I angegebene Bedeutung haben, mit einer Verbindung der Formel XXX

$H_2N\text{-}NH\text{-}COOR_{16}$ (XXX)

worin $R_{16}$ die oben angegebene Bedeutung hat, zur Verbindung der Formel XXXIa

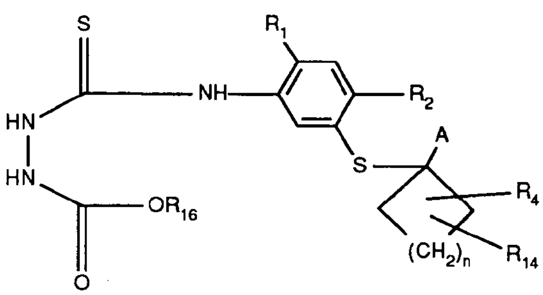

(XXXIa)

umsetzt, welche man in Gegenwart einer Base bei einer Temperatur von +50 bis + 180°C zur Verbindung der Formel XXXIIa

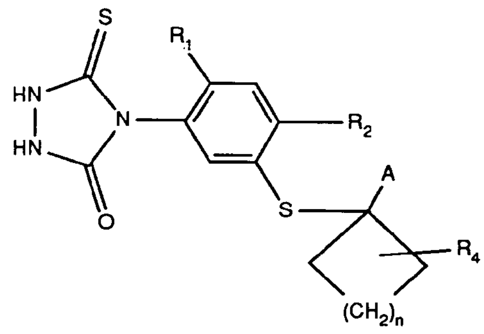

(XXXIIa)

cyclisiert, welche man bei einer Temperatur von 0 bis +150°C in Gegenwart einer katalytischen Menge an Base mit Divinylsulfon $(CH_2{=}CH)_2SO_2$ umsetzt, oder

f) bei Verbindungen der Formel I, in welcher W für $W_8$ steht, ein Isothiocyanat der Formel VIIa

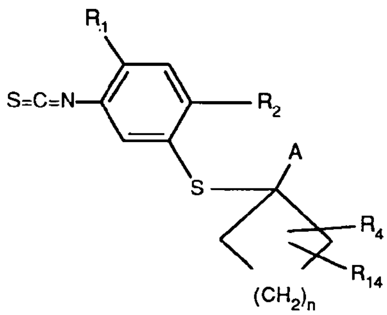

(VIIa),

worin die Substituenten A, $R_1$, $R_2$, $R_4$, $R_{14}$ sowie n die unter Formel I angegebene Bedeutung haben, mit einem Hexahydropyridazin der Formel VIII

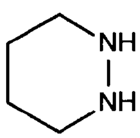

(VIII)

in die Verbindung der Formel IX

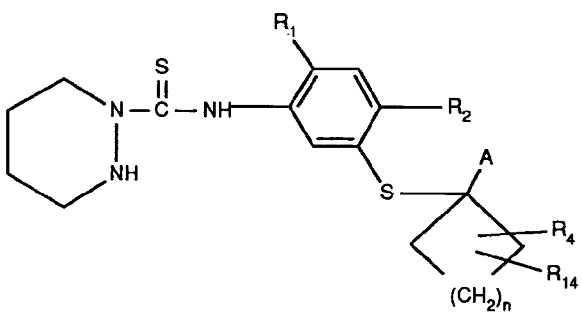

(IX)

überführt und diese anschliessend mit einer Verbindung der Formel X

$CZ_1Cl_2$ (X),

worin $Z_1$ für Sauerstoff oder Schwefel steht, in Gegenwart einer Base umsetzt, oder

g) bei Verbindungen der Formel I, in welcher W für $W_9$ steht, ein Isothiocyanat der Formel VIIa

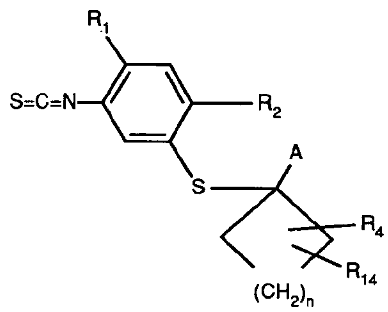

(VIIa),

worin die Substituenten A, $R_1$, $R_2$, $R_4$, $R_{14}$ sowie n die unter Formel I angegebene Bedeutung haben, mit einem 1,4,5,6-Tetrahydropyridazin der Formel XI

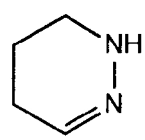

(XI)

in die Verbindung der Formel XII

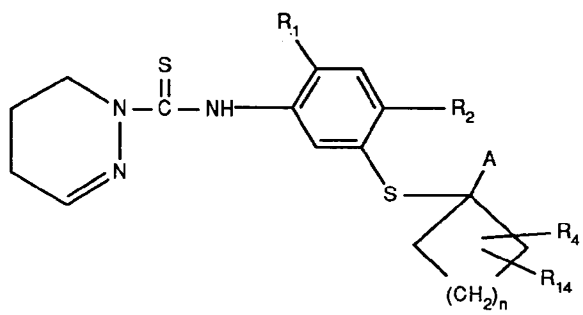

(XII)

überführt und diese anschliessend mit einer Verbindung der Formel Xa

$CZ_2Cl_2$ (Xa),

worin $Z_2$ für Sauerstoff oder Schwefel steht, in Gegenwart einer Base umsetzt oder

h) ein Tetrahydropyridazin-3-on der Formel XIII

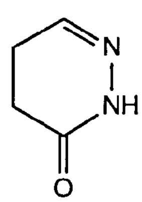

(XIII)

zum Hexahydropyridazin-3-on der Formel XIV

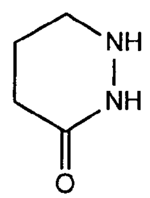

(XIV)

hydriert, dieses mit einem Isothiocyanat der Formel VIIa

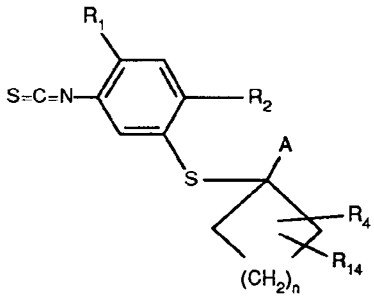

(VIIa)

worin die Substituenten A, $R_1$, $R_2$, $R_4$, $R_{14}$ sowie n die unter Formel I angegebene Bedeutung haben, zur Verbindung der Formel XVI

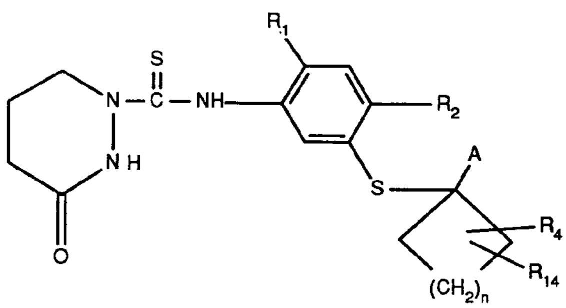

(XVI)

umsetzt, diese mit einer Verbindung der Formel Xa

$CZ_2Cl_2$ (Xa)

worin $Z_2$ für Sauerstoff oder Schwefel steht, oder mit $Cl_3(CO)_3Cl_3$ in die Verbindung der Formel XVII

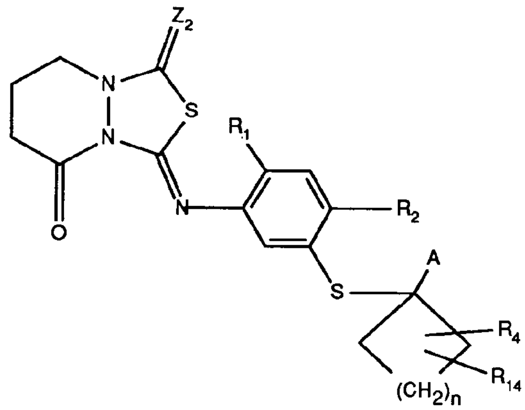

(XVII)

überführt, diese mit Natriumborhydrid zur Verbindung der Formel XVIII

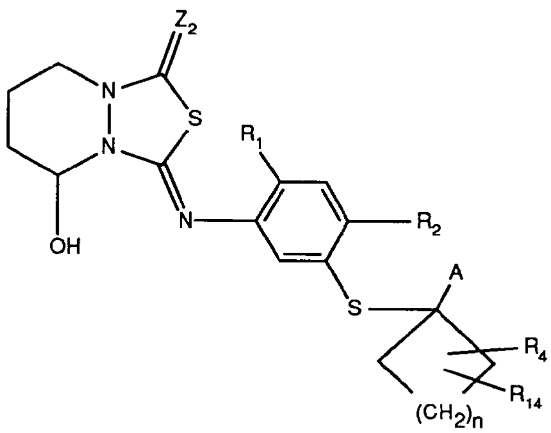

(XVIII)

umsetzt und diese anschliessend in Gegenwart von Säure dehydratisiert.

2. Verfahren gemäss Anspruch 1 zur Herstellung von Verbindungen der Formel I, in denen $R_{14}$ Wasserstoff bedeutet.

3. Verfahren gemäß Anspruch 1 zur Herstellung von Verbindungen der Formel I, in denen W für $W_8$ oder $W_9$ steht.

4. Verfahren gemäß Anspruch 1 zur Herstellung von Verbindungen der Formel I, in denen W für $W_1$ steht.

5. Verfahren gemäß Anspruch 1 zur Herstellung von Verbindungen der Formel I, in denen W für $W_2$ oder $W_3$ steht.

6. Verfahren gemäß Anspruch 1 zur Herstellung von Verbindungen der Formel I, in denen W für $W_6$ steht.

7. Verfahren gemäß Anspruch 1 zur Herstellung von Verbindungen der Formel I, in denen $R_1$ für Fluor steht.

8. Verfahren gemäß Anspruch 1 zur Herstellung von Verbindungen der Formel I, in denen $R_1$ für Fluor und $R_2$ für Chlor steht.

9. Verfahren gemäß Anspruch 1 zur Herstellung von Verbindungen der Formel I, in denen A für $CO-R_3$ und $R_3$ für $X-R_5$ steht.

10. Verfahren gemäß Anspruch 1 zur Herstellung von Verbindungen der Formel I, in denen A für $CO-R_3$ und $R_3$ für $X-R_5$ steht und $R_5$ $C_1$-$C_{10}$-Alkyl bedeutet.

**11.** Verfahren gemäß Anspruch 3, dadurch gekennzeichnet, daß A für CO-$R_3$ und $R_1$ für Fluor steht.

**12.** Verfahren gemäß Anspruch 3, dadurch gekennzeichnet, daß A für CO-$R_3$ und $R_1$ für Fluor und $R_2$ für Chlor steht.

**13.** Verfahren gemäß Anspruch 3, dadurch gekennzeichnet, daß A für CO-$R_3$ und $R_3$ für X-$R_5$ steht.

**14.** Verfahren gemäß Anspruch 3, dadurch gekennzeichnet, daß A CO-$R_3$, $R_3$ X-$R_5$ und $R_5$ $C_1$-$C_{10}$-Alkyl bedeutet.

**15.** Verfahren gemäß Anspruch 3, dadurch gekennzeichnet, daß A für CO-$R_3$ und $R_1$ Fluor, $R_2$ Chlor, $R_3$ X-$R_5$, und $R_5$ $C_1$-$C_{10}$-Alkyl bedeutet.

**16.** Verfahren gemäß Anspruch 3, dadurch gekennzeichnet, daß A für CO-$R_3$ und $R_4$ Wasserstoff bedeutet.

**17.** Verfahren gemäß Anspruch 15, dadurch gekennzeichnet, daß X Sauerstoff und $R_5$ $C_1$-$C_4$-Alkyl bedeutet.

**18.** Verfahren gemäß Anspruch 15, dadurch gekennzeichnet, daß X Sauerstoff und $R_5$ $C_1$-$C_4$-Alkyl bedeutet, und n für die Zahl 1 steht.

**19.** Verfahren gemäß Anspruch 1 zur Herstellung von 1-[2-Chlor-4-fluor-5-(1,2,3,4,5,6-hexahydro- 1,3-dioxo-isoindolyl)-phenylthio]-cyclopropancarbonsäuremethylester.

**20.** Verfahren gemäß Anspruch 1 zur Herstellung von 9-[4-chlor-2-fluor-5-(1-methoxycarbonyl-cyclobutylthio)-phenylimino]-8-thia-1,6-diazabicyclo[4.3.0]-nonan-7-on.

**21.** Verfahren gemäß Anspruch 1 zur Herstellung von 1-[4-Chlor-2-fluor-5-(1-methoxycarbonyl-cyclobutyl)-phenylthio]-4-trifluormethylpiperidin-2,6-dion.

**22.** Verfahren gemäß Anspruch 1 zur Herstellung von 9-[4-chlor-2-fluor-5-(1-methoxycarbonyl-cyclopropylthio)-phenylimino]-8-thia-1,6-diazabicyclo[4.3.0]-nonan-7-on.

**23.** Verfahren zur Bekämpfung unerwünschten Pflanzenwachstums, dadurch gekennzeichnet, daß man einen Wirkstoff der Formel I, gemäß Anspruch 1 in einer wirksamen Menge auf die Pflanzen oder deren Lebensraum appliziert.

**24.** Verfahren gemäß Anspruch 23, dadurch gekennzeichnet, daß man eine Wirkstoffmenge zwischen 0,001 und 2 kg pro Hektar appliziert.

**25.** Verfahren zur Hemmung des Pflanzenwachstums, dadurch gekennzeichnet, daß man einen Wirkstoff der Formel I, gemäß Anspruch 1 in einer wirksamen Menge auf die Pflanzen oder deren Lebensraum appliziert.

**26.** Verfahren gemäß Anspruch 23, zur selektiven pre- oder post-emergenten Bekämpfung von Unkräutern in Nutzpflanzenkulturen.